(19) Europäisches Patentamt
European Patent Office
Office européen des brevets

(11) EP 2 103 628 A1

(12) EUROPEAN PATENT APPLICATION
published in accordance with Art. 153(4) EPC

(43) Date of publication:
**23.09.2009 Bulletin 2009/39**

(21) Application number: **07850593.0**

(22) Date of filing: **14.12.2007**

(51) Int Cl.:
***C07K 16/28** (2006.01)* ***A61K 39/395** (2006.01)*
***A61P 35/00** (2006.01)* ***A61P 35/04** (2006.01)*
***A61P 43/00** (2006.01)* ***C07K 16/46** (2006.01)*
***G01N 33/574** (2006.01)* ***C12N 15/13** (2006.01)*
***C12P 21/08** (2006.01)*

(86) International application number:
**PCT/JP2007/074081**

(87) International publication number:
**WO 2008/072723 (19.06.2008 Gazette 2008/25)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **14.12.2006 JP 2006336555**

(71) Applicant: **Forerunner Pharma Research Co., Ltd.**
**Meguro-ku**
**Tokyo 153-0041 (JP)**

(72) Inventor: **YOSHIDA, Kenji**
**Tokyo 153-0041 (JP)**

(74) Representative: **Vossius & Partner**
**Siebertstraße 4**
**81675 München (DE)**

# (54) ANTI-CLAUDIN-3 MONOCLONAL ANTIBODY, AND TREATMENT AND DIAGNOSIS OF CANCER USING THE SAME

(57) Monoclonal antibodies that bind specifically to Claudin 3 expressed on cell surface are provided. The antibodies of the present invention are useful for diagnosis of cancers that have enhanced expression of Claudin 3, such as ovarian cancer, prostate cancer, breast cancer, uterine cancer, liver cancer, lung cancer, pancreatic cancer, stomach cancer, bladder cancer, and colon cancer. The present invention provides monoclonal antibodies showing cytotoxic effects against cells of these cancers. Methods for inducing cell injury in Claudin 3-expressing cells and methods for suppressing proliferation of Claudin 3-expressing cells by contacting Claudin 3-expressing cells with a Claudin 3-binding antibody are disclosed. The present application also discloses methods for diagnosis or treatment of cancers.



Printed by Jouve, 75001 PARIS (FR)

## Description

### Technical Field

The present invention relates to methods for diagnosis and treatment of cancer, as well as cell proliferation-suppressing and anticancer agents.

### Background Art

In recent years, cancer treatment that uses monoclonal antibodies as therapeutic agents, by utilizing the characteristics of monoclonal antibodies, *i.e.*, high target specificity and low incidence of side-effects, is receiving attention. The main antitumor mechanisms of antibodies used as therapeutic agents include, for example, the following:

discrimination of tumor cells from normal cells by antibodies;
binding of effector cells having cytotoxic activity to antibodies specifically bound to antigens expressed on tumor cells, or formation of complement complexes that bind to the antibodies; and
effector cell- or complement-mediated cytotoxic activity against tumor cells.

Examples of antibodies used for cancer treatment include, trastuzumab which is an antibody for breast cancer treatment targeting HER-2, and rituximab which is an antibody for non-Hodgkin lymphoma treatment targeting CD20. However, the number of antibodies actually showing clinical efficacy is very few at present. Therefore, the types of cancers that could be applied to antibody therapy are very limited at this time. It is highly desirable to develop antibody therapeutic agents with few side effects and high anti-tumor efficiency, and to establish new therapeutic methods against cancers for which there are few therapeutic options and the currently available therapeutic agents are ineffective.

Claudin 3 is a protein that belongs to the Claudin family. It is localized at tight junctions, and has a characteristic role in eliminating the intercellular space at tight junctions. "Tight junction" refers to a rigid structure that links adjacent cell membranes in tissues of organisms such as animals. Claudin 3 is a structural protein that regulates the intercellular permeability of small solutes such as ions. It has been reported that the expression of the Claudin 3 molecule is elevated in many cancer tissues such as ovarian cancer, prostate cancer, breast cancer, uterine cancer, liver cancer, lung cancer, pancreatic cancer, stomach cancer, bladder cancer, and colon cancer tissues (Non-patent Documents 1-6). The Swedish Human Protein Atlas (HPA) Web site (http://www.proteinatlas.org/) is available as a reference for the Claudin 3 expression profile. It has been shown that the expression of Claudin 3 and Claudin 4 is particularly elevated in chemotherapy-resistant and/or recurrent uterine cancer, which is considered to have the highest fatality among gynecological cancers in the United States. As with Claudin 3, Claudin 4 is a protein of the Claudin family. To date, the 24 genes that belong to the Claudin family, including Claudin 3 and Claudin 4, have been reported to be present on the human chromosome.

Both Claudin 3 and Claudin 4 are known to function as toxin receptors for the *Clostridium perfringens* enterotoxin (CPE). CPE binds to Claudin 3 and Claudin 4, and then produces a pore in the cell membrane by forming a large complex to cause cellular necrosis. Published Data show that administration of a sublethal amount of CPE to gallbladder cancer model mice produced by transplanting Claudin 3- and Claudin 4-expressing human tumor cells decreased tumor volume and increased survival rate (Non-Patent Documents 6 and 7). Although the possibility of clinically applying CPE to human has been suggested, such application has yet to be performed in practice due to its narrow therapeutic window between the dosage that shows drug efficacy and the dosage that causes lethal toxicity, and the concern regarding antigenicity of CPE in human.

Claudin 3 is a protein with four transmembrane regions, and has a structure that exposes two peptide loops to the outside of the cell. As shown below, the polypeptide portions which constitute the peptide sequences predicted to be the extracellular loops consist of only 51 amino acid residues (loop 1) and 23 amino acid residues (loop 2).

```
                  Extracellular loop 1              Extracellular loop 2
                       [30-80]                           [137-159]
Cell membrane=[9-29]=========[81-101]======[116-136]======[160-180]===========
[NH2-1-8]                          [102-115]                     [181-219-COOH]
Cytoplasmic                        Cytoplasmic                   Cytoplasmic
```

Since the sequence identity of the Claudin family is high among animal species, it was extremely difficult to obtain antibodies that recognize the extracellular domains by general immunization methods. Furthermore, since molecules that belong to the Claudin family have similar structures to each other, methods for obtaining an antibody that specifically recognizes a member of the Claudin family have not been established (Non-patent Document 8).

For antibodies that recognize Claudin 3 expressed on cells, only Non-patent Document 3 is available, which reports the isolation of polyclonal antibodies obtained by immunizing chickens with a partial peptide of Claudin 3 and then performing affinity purification using the peptide. To date, there is no report on examples of isolation of monoclonal antibodies that bind to the native structure of Claudin-3 expressed on cell surface or determination of their antitumor activity.

[Non-patent Document 1] Soini (2005) Histopathology 46, 551.
[Non-patent Document 2] Santin et al. (2005) Br. J. Cancer 92, 1561.
[Non-patent Document 3] Offner et al. (2005) Cancer Immunol. Immunother. 54, 431.
[Non-patent Document 4] Long et al. (2001) Cancer Res. 61, 7878.
[Non-patent Document 5] Rangel et al. (2003) Clin. Cancer Res. 9, 2567.
[Non-patent Document 6] Kominsky et al. (2004) Am. J. Path. 164, 1627.
[Non-patent Document 7] Santin et al. (2005) Cancer Res. 65, 4334.
[Non-patent Document 8] Hoevel et al. (2002) J. Cell. Physiol. 191, 60.

Disclosure of the Invention

[Problems to be Solved by the Invention]

An objective of the present invention is to provide anti-Claudin 3 antibodies and uses thereof. More specifically, an objective is to provide novel anti-Claudin 3 antibodies, novel methods for treating cancer using anti-Claudin 3 antibodies, and novel cell proliferation inhibitors or anti-cancer agents containing an anti-Claudin 3 antibody.

[Means for Solving the Problems]

The present inventors successfully obtained anti-Claudin 3 antibodies by immunizing mice with a Claudin 3 polypeptide-encoding DNA. Furthermore, the present inventors measured the activity of the thus-obtained antibodies to bind to the following Claudin family molecules, which are expressed on the cell surface:

the human Claudin 3 protein,
mouse Claudin 3 protein,
human Claudin 1 protein,
human Claudin 4 protein, and
human Claudin 6 protein.

As a result, the anti-Claudin 3 antibodies of the present invention were confirmed to have any or all of the following binding activities:

strong binding activity to the human Claudin 3 protein;
strong binding activity to the human and mouse Claudin 3 proteins; and
binding activity to the human Claudin 3 protein and human Claudin 4 protein.

Furthermore, the present inventors discovered that the obtained anti-Claudin 3 antibodies include antibodies that do not substantially show binding activity to synthetic peptides having the peptide sequences predicted to be the extracellular loops, but bind specifically to the human Claudin 3 protein expressed on the cell surface.

The present inventors also discovered that the obtained anti-Claudin 3 antibodies show binding activity to the MCF7 human breast cancer cell line endogenously expressing Claudin 3, and demonstrated that the antibodies are useful for diagnosis of various types of primary or metastatic cancer cells. Furthermore, the present inventors discovered that any or all of the various types of cancer tissues described below can be diagnosed using the anti-Claudin 3 antibodies:

various types of cancer tissues expressing a Claudin 3 protein;
various types of cancer tissues expressing a Claudin 4 protein; and
cancer tissues expressing both the Claudin 3 and Claudin 4 proteins.

Furthermore, the present inventors measured the complement-dependent cytotoxicity (CDC) activity of the anti-Claudin 3 antibodies against DG44 cells stably expressing the human Claudin 3 protein and the aforementioned MCF7 cells. The present inventors discovered that the anti-Claudin 3 antibodies of the present invention have CDC activity against both of these cells. The present inventors also measured the antibody-dependent cell-mediated cytotoxicity (ADCC) activity of the anti-Claudin 3 antibodies against MCF7 cells, and discovered that the anti-Claudin 3 antibodies of the present invention have ADCC activity against MCF7 cells.

From the above-mentioned findings, the present inventors discovered that anti-Claudin 3 antibodies are effective for diagnosing, preventing, or treating various types of primary or metastatic cancers, and completed the present invention.

The present invention provides monoclonal antibodies that bind to a Claudin 3 protein. The present invention also provides pharmaceutical compositions comprising an antibody that binds to a Claudin 3 protein as an active ingredient. The present invention also provides anticancer agents comprising an antibody that binds to a Claudin 3 protein as an active ingredient. Preferably, the antibodies that bind to a Claudin 3 protein have cytotoxic activity. In a preferred embodiment of the present invention, cancers that can be targeted for treatment are ovarian cancer, prostate cancer, breast cancer, uterine cancer, liver cancer, lung cancer, pancreatic cancer, stomach cancer, bladder cancer, and colon cancer. Breast cancer is particularly preferred. Anticancer agents containing an anti-Claudin 3 antibody of the present invention are useful for treating these cancers which are primary or metastatic cancers and have elevated expression of Claudin 3.

Furthermore, the present invention provides pharmaceutical compositions comprising an antibody that binds to a Claudin 3 protein and a pharmaceutically acceptable carrier. Pharmaceutical compositions of the present invention are useful for treating and/or preventing cancers that have elevated expression of Claudin 3. That is, the present invention relates to the use of an antibody that binds to a Claudin 3 protein for the production of pharmaceutical compositions for treating and/or preventing cancer.

In another embodiment, the present invention provides methods for inducing cell injury in cells that express a Claudin 3 protein by contacting Claudin 3-expressing cells with an antibody that binds to a Claudin 3 protein. The present invention also provides methods for suppressing proliferation of cells that express a Claudin 3 protein by contacting Claudin 3 protein-expressing cells with an antibody that binds to a Claudin 3 protein. The antibody that binds to a Claudin 3 protein preferably has cytotoxic activity. Cells that express a Claudin 3 protein are preferably cancer cells.

Furthermore, in another embodiment, the present invention provides antibodies that bind to a Claudin 3 protein and have cytotoxic activity in cells that express the Claudin 3 protein. Preferably, the cytotoxic activity is ADCC activity. Preferably, the cytotoxic activity is CDC activity. The present invention also provides antibodies to which a cytotoxic substance is bound. In the present invention, the cytotoxic substances that may be bound to the antibody include chemotherapeutic agents, radioisotopes, and toxic peptides. Preferably, in the present invention, an antibody itself has cytotoxic activity.

Furthermore, in another embodiment, the present invention provides methods for diagnosing cancer, which comprises detecting a Claudin 3 protein using an antibody that binds to the Claudin 3 protein. In the methods of the present invention, preferably, the extracellular region of a Claudin 3 protein is detected. Preferably, the methods of the present invention are carried out using an antibody that recognizes a Claudin 3 protein.

In another embodiment, the present invention provides methods for diagnosis of cancer which comprise the following steps of:

(a) collecting a sample from a subject; and
(b) using an antibody that binds to a Claudin 3 protein to detect the Claudin 3 protein contained in the collected sample.

In the present invention, any sample can be used as the above-mentioned sample as long as it can be collected from the subject. In one embodiment, blood sample collected from a subject is used. In another embodiment, samples collected surgically or by biopsy from a subject may be used. The methods of diagnosis can be used for any cancer as long as it is a cancer in which the target cancer cells express a Claudin 3 protein. Cancers that are preferred in the present invention are ovarian cancer, prostate cancer, breast cancer, uterine cancer, liver cancer, lung cancer, pancreatic cancer, stomach cancer, bladder cancer, and colon cancer. Breast cancer is particularly preferable. Based on the present invention, both primary and metastatic foci of these cancers can be diagnosed. In the present invention, the step of collecting a sample from a subject can also be expressed as the step of providing a sample collected from a subject.

Furthermore, in another embodiment, the present invention provides methods for diagnosis of cancer, which comprise the steps of: (1) administering to a subject a radioisotope-labeled antibody that binds to a Claudin 3 protein; and (2) detecting accumulation of the radioisotope. In a certain embodiment, the radioisotope is a positron-emitting nuclide.

A preferred positron-emitting nuclide of the present invention can be selected, for example, from the group consisting of $^{11}C$, $^{13}N$, $^{15}O$, $^{18}F$, $^{45}Ti$ $^{55}Co$, $^{64}Cu$, $^{66}Ga$, $^{68}Ga$, $^{76}Br$, $^{89}Zr$, and $^{124}I$.

Furthermore, in another embodiment, the present invention provides methods for diagnosis of cancer, which comprise detecting the expression of a gene encoding the Claudin 3 protein. Furthermore, in another embodiment, the present invention provides diagnostic agents and kits to be used in the diagnostic methods of the present invention.

More specifically, the present invention provides the following:

[1] a monoclonal antibody that binds to a Claudin 3 protein;
[2] the monoclonal antibody of [1], wherein the antibody binds to a protein expressed on the cell membrane and comprising the amino acid sequence of SEQ ID NO: 2;
[3] the monoclonal antibody of [2], wherein the antibody does not substantially cross-react with a peptide comprising the amino acid sequence of positions 30 to 80 or positions 137 to 159 in the amino acid sequence of SEQ ID NO: 2;
[4] the monoclonal antibody of any one of [1] to [3], wherein the antibody binds to a protein expressed on the cell membrane and comprising the amino acid sequence of SEQ ID NO: 4;
[5] the monoclonal antibody of any one of [1] to [3], wherein the antibody binds to a protein expressed on the cell membrane and comprising the amino acid sequence of SEQ ID NO: 8;
[6] an antibody that binds to a protein expressed on the cell membrane and comprising the amino acid sequence of any one of SEQ ID NOs: 2, 4, and 8, by recognizing a conformation formed by two extracellular loops of the protein;
[7] the antibody of [6], wherein the antibody does not substantially cross-react with a peptide comprising the amino acid sequence of positions 30 to 80 or positions 137 to 159 in the amino acid sequence of SEQ ID NO: 2;
[8] the antibody of [6] or [7], wherein the antibody is a monoclonal antibody;
[9] the antibody of any one of [1] to [8], wherein the antibody has cytotoxic activity;
[10] the antibody of [9], wherein the cytotoxic activity is ADCC activity;
[11] the antibody of [9], wherein the cytotoxic activity is CDC activity;
[12] the antibody of any one of [1] to [11], wherein a chemotherapeutic agent or a toxic peptide is bound to the antibody;
[13] an antibody that binds to a Claudin 3 protein, wherein a cytotoxic substance selected from the group consisting of a chemotherapeutic agent, toxic peptide, and radioisotope is bound to the antibody;
[14] the antibody of any one of [1] to [13], which is an antibody described in any of (1) to (61) below:

(1) an antibody comprising an H chain having the amino acid sequence of SEQ ID NO: 12 as CDR1, the amino acid sequence of SEQ ID NO: 14 as CDR2, and the amino acid sequence of SEQ ID NO: 16 as CDR3;
(2) an antibody comprising the H chain of (1), wherein the H chain has the amino acid sequence of positions 139 to 462 in the amino acid sequence of SEQ ID NO: 20 as CH;
(3) an antibody comprising the H chain of (1), wherein the H chain has the amino acid sequence of SEQ ID NO: 22 as CH;
(4) an antibody comprising an L chain having the amino acid sequence of SEQ ID NO: 24 as CDR1, the amino acid sequence of SEQ ID NO: 26 as CDR2, and the amino acid sequence of SEQ ID NO: 28 as CDR3;
(5) an antibody comprising the L chain of (4), wherein the L chain has the amino acid sequence of positions 135 to 240 in the amino acid sequence of SEQ ID NO: 32 as CL;
(6) an antibody comprising the L chain of (4), wherein the L chain has the amino acid sequence of SEQ ID NO: 34 as CL;
(7) an antibody comprising the H chain of (1) and the L chain of (4);
(8) an antibody comprising the H chain of (2) and the L chain of (5);
(9) an antibody comprising the H chain of (3) and the L chain of (6);
(10) an antibody having one or more amino acid substitutions, deletions, additions, and/or insertions in the antibody of any of (1) to (9), which has equivalent activity as the antibody of any of (1) to (9);
(11) an antibody comprising an H chain having the amino acid sequence of SEQ ID NO: 36 as CDR1, the amino acid sequence of SEQ ID NO: 38 as CDR2, and the amino acid sequence of SEQ ID NO: 40 as CDR3;
(12) an antibody comprising the H chain of (11), wherein the H chain has the amino acid sequence of positions 140 to 476 in the amino acid sequence of SEQ ID NO: 44 as CH;
(13) an antibody comprising the H chain of (11), wherein the H chain has the amino acid sequence of SEQ ID NO: 22 as CH;
(14) an antibody comprising an L chain having the amino acid sequence of SEQ ID NO: 46 as CDR1, the amino acid sequence of SEQ ID NO: 48 as CDR2, and the amino acid sequence of SEQ ID NO: 50 as CDR3;
(15) an antibody comprising the L chain of (14), wherein the L chain has the amino acid sequence of positions 133 to 238 in the amino acid sequence of SEQ ID NO: 54 as CL;
(16) an antibody comprising the L chain of (14), wherein the L chain has the amino acid sequence of SEQ ID NO: 34 as CL;
(17) an antibody comprising the H chain of (11) and the L chain of (14);
(18) an antibody comprising the H chain of (12) and the L chain of (15);
(19) an antibody comprising the H chain of (13) and the L chain of (16);
(20) an antibody having one or more amino acid substitutions, deletions, additions, and/or insertions in the antibody of any of (11) to (19), which has equivalent activity as the antibody of any of (11) to (19);

(21) an antibody comprising an H chain having the amino acid sequence of SEQ ID NO: 56 as CDR1, the amino acid sequence of SEQ ID NO: 58 as CDR2, and the amino acid sequence of SEQ ID NO: 60 as CDR3;
(22) an antibody comprising the H chain of (21), wherein the H chain has the amino acid sequence of positions 137 to 471 in the amino acid sequence of SEQ ID NO: 64 as CH;
(23) an antibody comprising the H chain of (21), wherein the H chain has the amino acid sequence of SEQ ID NO: 22 as CH;
(24) an antibody comprising an L chain having the amino acid sequence of SEQ ID NO: 66 as CDR1, the amino acid sequence of SEQ ID NO: 68 as CDR2, and the amino acid sequence of SEQ ID NO: 70 as CDR3;
(25) an antibody comprising the L chain of (24), wherein the L chain has the amino acid sequence of positions 133 to 238 in the amino acid sequence of SEQ ID NO: 74 as CL;
(26) an antibody comprising the L chain of (24), wherein the L chain has the amino acid sequence of SEQ ID NO: 34 as CL;
(27) an antibody comprising the H chain of (21) and the L chain of (24);
(28) an antibody comprising the H chain of (22) and the L chain of (25);
(29) an antibody comprising the H chain of (23) and the L chain of (26);
(30) an antibody having one or more amino acid substitutions, deletions, additions, and/or insertions in the antibody of any of (21) to (29), which has equivalent activity as the antibody of any of (21) to (29);
(31) an antibody comprising an H chain having the amino acid sequence of SEQ ID NO: 76 as CDR1, the amino acid sequence of SEQ ID NO: 78 as CDR2, and the amino acid sequence of SEQ ID NO: 80 as CDR3;
(32) an antibody comprising the H chain of (31), wherein the H chain has the amino acid sequence of positions 140 to 463 in the amino acid sequence of SEQ ID NO: 84 as CH;
(33) an antibody comprising the H chain of (31), wherein the H chain has the amino acid sequence of SEQ ID NO: 22 as CH;
(34) an antibody comprising an L chain having the amino acid sequence of SEQ ID NO: 86 as CDR1, the amino acid sequence of SEQ ID NO: 88 as CDR2, and the amino acid sequence of SEQ ID NO: 90 as CDR3;
(35) an antibody comprising the L chain of (34), wherein the L chain has the amino acid sequence of positions 133 to 238 in the amino acid sequence of SEQ ID NO: 94 as CL;
(36) an antibody comprising the L chain of (34), wherein the L chain has the amino acid sequence of SEQ ID NO: 34 as CL;
(37) an antibody comprising the H chain of (31) and the L chain of (34);
(38) an antibody comprising the H chain of (32) and the L chain of (35);
(39) an antibody comprising the H chain of (33) and the L chain of (36);
(40) an antibody having one or more amino acid substitutions, deletions, additions, and/or insertions in the antibody of any of (31) to (39), which has equivalent activity as the antibody of any of (31) to (39);
(41) an antibody comprising an H chain having the amino acid sequence of SEQ ID NO: 96 as CDR1, the amino acid sequence of SEQ ID NO: 98 as CDR2, and the amino acid sequence of SEQ ID NO: 100 as CDR3;
(42) an antibody comprising the H chain of (41), wherein the H chain has the amino acid sequence of positions 140 to 474 in the amino acid sequence of SEQ ID NO: 104 as CH;
(43) an antibody comprising the H chain of (41), wherein the H chain has the amino acid sequence of SEQ ID NO: 22 as CH;
(44) an antibody comprising an L chain having the amino acid sequence of SEQ ID NO: 106 as CDR1, the amino acid sequence of SEQ ID NO: 108 as CDR2, and the amino acid sequence of SEQ ID NO: 110 as CDR3;
(45) an antibody comprising the L chain of (44), wherein the L chain has the amino acid sequence of positions 133 to 238 in the amino acid sequence of SEQ ID NO: 114 as CL;
(46) an antibody comprising the L chain of (44), wherein the L chain has the amino acid sequence of SEQ ID NO: 34 as CL;
(47) an antibody comprising the H chain of (41) and the L chain of (44);
(48) an antibody comprising the H chain of (42) and the L chain of (45);
(49) an antibody comprising the H chain of (43) and the L chain of (46);
(50) an antibody having one or more amino acid substitutions, deletions, additions, and/or insertions in the antibody of any of (41) to (49), which has equivalent activity as the antibody of any of (41) to (49);
(51) an antibody comprising an H chain having the amino acid sequence of SEQ ID NO: 167 as CDR1, the amino acid sequence of SEQ ID NO: 169 as CDR2, and the amino acid sequence of SEQ ID NO: 171 as CDR3;
(52) an antibody comprising the H chain of (51), wherein the H chain has the amino acid sequence of positions 118 to 447 in the amino acid sequence of SEQ ID NO: 173 as CH;
(53) an antibody comprising the H chain of (51), wherein the H chain has the amino acid sequence of SEQ ID NO: 22 as CH;
(54) an antibody comprising an L chain having the amino acid sequence of SEQ ID NO: 179 as CDR1, the amino acid sequence of SEQ ID NO: 181 as CDR2, and the amino acid sequence of SEQ ID NO: 183 as CDR3;
(55) an antibody comprising the L chain of (54), wherein the L chain has the amino acid sequence of positions 113 to 218 in the amino acid sequence of SEQ ID NO: 185 as CL;
(56) an antibody comprising the L chain of (54), wherein the L chain has the amino acid sequence of SEQ ID NO: 34 as CL;
(57) an antibody comprising the H chain of (51) and the L chain of (54);
(58) an antibody comprising the H chain of (52) and the L chain of (55);
(59) an antibody comprising the H chain of (53) and the L chain of (56);
(60) an antibody having one or more amino acid substitutions, deletions, additions, and/or insertions in the antibody of any of (51) to (59), which has equivalent activity as the antibody of any of (51) to (59);
(61) an antibody that binds to the same epitope as the Claudin 3 protein epitope bound by the antibody of any of (1) to (60);

[15] a method for diagnosis of cancer, comprising the step of binding the antibody of any one of [1] to [14] to a Claudin 3 protein;
[16] a method for diagnosis of cancer, comprising the steps of:

(a) collecting a sample from a subject; and
(b) detecting a Claudin 3 protein contained in the collected sample using an antibody that binds to the Claudin 3 protein;

[17] a method for diagnosis of cancer, comprising the steps of: (1) administering to a subject a radioisotope-labeled antibody that binds to a Claudin 3 protein; and (2) detecting accumulation of said radioisotope;
[18] the diagnostic method of [17], wherein the radioisotope is a positron-emitting nuclide;
[19] the diagnostic method of [18], wherein the positron-emitting nuclide is any nuclide selected from the group consisting of $^{11}C$, $^{13}N$, $^{15}O$, $^{18}F$, $^{45}Ti$, $^{55}Co$, $^{64}Cu$, $^{66}Ga$, $^{68}Ga$, $^{76}Br$, $^{89}Zr$, and $^{124}I$;
[20] the diagnostic method of any one of [15] to [19], wherein the cancer is any cancer selected from the group consisting of ovarian cancer, prostate cancer, breast cancer, uterine cancer, liver cancer, lung cancer, pancreatic cancer, stomach cancer, bladder cancer, and colon cancer;
[21] the diagnostic method of [20], wherein the cancer is primary cancer;
[22] the diagnostic method of [20], wherein the cancer is metastatic cancer;
[23] a diagnostic agent for use in a method of cancer diagnosis, which comprises an antibody that binds to a Claudin 3 protein;
[24] a kit for use in a method of cancer diagnosis, which comprises an antibody that binds to a Claudin 3 protein, and a biological sample comprising a Claudin 3 protein;
[25] a pharmaceutical composition comprising an antibody that binds to a Claudin 3 protein as an active ingredient;
[26] a cell proliferation inhibitor comprising an antibody that binds to a Claudin 3 protein as an active ingredient;
[27] an anticancer agent comprising an antibody that binds to a Claudin 3 protein as an active ingredient;
[28] the anticancer agent of [27], wherein the cancer is any cancer selected from the group consisting of ovarian cancer, prostate cancer, breast cancer, uterine cancer, liver cancer, lung cancer, pancreatic cancer, stomach cancer, bladder cancer, and colon cancer;
[29] the anticancer agent of [28], wherein the cancer is primary cancer;
[30] the anticancer agent of [28], wherein the cancer is metastatic cancer;
[31] the anticancer agent of [28], wherein said antibody is the antibody of any one of [1] to [14];
[32] use of an antibody that binds to a Claudin 3 protein in the production of a cell proliferation inhibitor;
[33] use of an antibody that binds to a Claudin 3 protein in the production of an anticancer agent;
[34] the use of [33], wherein the cancer is any cancer selected from the group consisting of ovarian cancer, prostate cancer, breast cancer, uterine cancer, liver cancer, lung cancer, pancreatic cancer, stomach cancer, bladder cancer, and colon cancer;
[35] the use of [33], wherein the cancer is primary cancer;
[36] the use of [33], wherein the cancer is metastatic cancer;
[37] the use of [32] or [33], wherein the antibody is the antibody of any one of [1] to [14];
[38] a method of suppressing cell proliferation, which comprises the step of administering to a subject an antibody that binds to a Claudin 3 protein;
[39] a method of preventing or treating cancer, which comprises the step of administering to a subject an antibody that binds to a Claudin 3 protein;
[40] the method of [39], wherein the cancer is any cancer selected from the group consisting of ovarian cancer, prostate cancer, breast cancer, uterine cancer, liver cancer, lung cancer, pancreatic cancer, stomach cancer, bladder cancer, and colon cancer;
[41] the method of [39], wherein the cancer is primary cancer;
[42] the method of [39], wherein the cancer is metastatic cancer;
[43] the method of [38] or [39], wherein the antibody is the antibody of any one of [1] to [14];
[44] a method of inducing cell injury in a Claudin 3 protein-expressing cell, which comprises the step of contacting the Claudin 3 protein-expressing cell with an antibody that binds to a Claudin 3 protein;
[45] a method of suppressing proliferation of a Claudin 3 protein-expressing cell, which comprises the step of contacting the Claudin 3 protein-expressing cell with an antibody that binds to a Claudin 3 protein;
[46] the method of [44] or [45], wherein the Claudin 3 protein-expressing cell is a cancer cell;
[47] the method of any one of [44] to [46], wherein the antibody is an antibody having cytotoxic activity;
[48] the method of any one of [44] to [46], wherein the antibody is the antibody of any one of [1] to [14];
[49] an antibody that binds to a Claudin 3 protein for use in a method of cancer treatment;
[50] the antibody of [49], wherein the cancer is any cancer selected from the group consisting of ovarian cancer, prostate cancer, breast cancer, uterine cancer, liver cancer, lung cancer, pancreatic cancer, stomach cancer, bladder cancer, and colon cancer;
[51] the antibody of [50], wherein the cancer is primary cancer;
[52] the antibody of [50], wherein the cancer is metastatic cancer; and
[53] the antibody of [50], which is the antibody of any one of [1] to [14].

Brief Description of the Drawings

Fig. 1 shows a dendrogram and alignment of the extracellular loops of Claudin 3 and other members of human Claudin family.
Fig. 2 shows an amino acid sequence alignment of human and mouse Claudin 3. The underlines indicate the putative transmembrane regions, and the boxes indicate the putative extracellular regions.
Fig. 3 shows differences in the binding reactivity of anti-Claudin 3 antibodies for Claudin 3-expressing DG44 cells and Ba/F3 cells or the MCF7 breast cancer cell line: (A) Ba/F3 cells, (B) the MCF7 breast cancer cell line. The X geometric mean values (relative fluorescence intensity values) are shown on the vertical and horizontal axes.
Fig. 4 shows the affinity of anti-Claudin 3 monoclonal antibodies for the Claudin 3 extracellular loop peptide regions. The vertical axis indicates the absorbance at 405 nm (reference wavelength of 655 nm), and the horizontal axis indicates the anti-Claudin 3 antibody concentration (ng/mL).
Fig. 5 shows the antigen -dependent induction of cytotoxic activity by anti-Claudin 3 antibodies. The vertical axis indicates the percentage increase in dead cells by complement addition.
Fig. 6 shows the anti-Claudin 3 antibody-mediated induction of complement-dependent cytotoxic activity against MCF7 cells. The vertical axis indicates the specific chromium release rate (%).
Fig. 7 shows the anti-Claudin 3 antibody-mediated induction of antibody-dependent cell-mediated cytotoxic activity against MCF7 cells. The vertical axis indicates the specific chromium release rate (%).
Fig. 8 shows, by flow cytometry, the specific binding of recombinant chimeric antibodies to cells forced to express Claudin 3. The vertical axis indicates cell count (fluorescence coefficient), and the horizontal axis indicates fluorescence intensity.
Fig. 9-1 presents graphs showing the results of FACS analysis of the binding activity of various anti-Claudin 3 antibodies to the recombinant cells below (results for the CDC1, CDN2, and CDN3 monoclonal antibodies). "CLD3/3": wild-type Claudin 3-expressing cells; "1/3": CLD1/3 chimeric protein-expressing cells; "3/1": CLD3/1 chimeric protein-expressing cells; and "Ba/F3": Ba/F3 cells as a parental cell. The X axis indicates fluorescence intensity, and the Y axis indicates the relative cell count at each fluorescence intensity.
Fig. 9-2 is the continuation of Fig. 9-1 (results for the CDN4, CDN5, and CDN7 anti-Claudin 3 monoclonal antibodies).
Fig. 9-3 is the continuation of Fig. 9-2 (results for the CDN8, CDN16, and CDN17 anti-Claudin 3 monoclonal antibodies).
Fig. 9-4 is the continuation of Fig. 9-3 (results for the CDN24, CDN27, and CDN28 anti-Claudin 3 monoclonal antibodies).
Fig. 9-5 is the continuation of Fig. 9-4 (results for the CDN29, CDN30, and CDN31 anti-Claudin 3 monoclonal antibodies).
Fig. 9-6 is the continuation of Fig. 9-5 (results for the CDN32, CDN33, and CDN35 anti-Claudin 3 monoclonal antibodies).
Fig. 9-7 is the continuation of Fig. 9-6 (results for the CDN36, CDN37, and CDN38 anti-Claudin 3 monoclonal antibodies).

Fig. 9-8 is the continuation of Fig. 9-7 (results for anti-Claudin 3 antiserum diluted 200-fold, anti-Claudin 3 antiserum diluted 1000-fold, and the negative control without antibody addition).

Fig. 10 is a graph showing the proportion of cells having a relative fluorescence intensity of 100 or more with respect to total cells. The fluorescence intensity is shown on the horizontal axis of the histogram plots of Figs. 9-1 to 9-7.

Fig. 11 shows, by flow cytometry, the binding of recombinant chimeric antibodies to cells forced to express Claudin 3. The vertical axis indicates cell number (fluorescence coefficient), and the horizontal axis indicates fluorescence intensity. The black solid line and the grey dotted line represent the fluorescence intensity distribution of cells with and without addition of a chimeric antibody, respectively.

Fig. 12 is a graph showing suppression of the proliferation of MCF7 cells by anti-Claudin 3 antibodies in a soft agar colony formation/MTT hybrid assay.

Fig. 13 presents photographs showing suppression of the cell motility by addition of an anti-Claudin 3 antibody (CDN04) in a wound-healing assay. Cells in the region between the dashed lines are the cells that migrated to the scratched site.

Mode for Carrying Out the Invention

Claudin 3 is a protein that belongs to the Claudin family, and is a structural protein that regulates intercellular permeability. The amino acid sequence of human Claudin 3 and a nucleotide sequence encoding the protein are shown in SEQ ID NOs: 2 and 1, respectively (GenBank Accession No. NM_001306). In the present invention, the Claudin 3 proteins recognized by the monoclonal antibodies are proteins that maintain the native conformation of a Claudin 3 protein. Furthermore, the monoclonal antibodies of the present invention bind to Claudin 3 preferably by recognizing its extracellular regions. Positions 30 to 80 in the amino acid sequence of SEQ ID NO: 2 (loop 1) and positions 137 to 159 in the amino acid sequence of SEQ ID NO: 2 (loop 2) correspond to the extracellular regions of the Claudin 3 protein.

As long as polypeptides containing the amino acid sequences of these extracellular regions maintain the cell-surface conformation of Claudin 3, monoclonal antibodies of the present invention can recognize the polypeptides. The monoclonal antibodies of the present invention preferably bind to polypeptides that maintain the native conformation of Claudin 3 expressed on the cell surface. Whether polypeptides maintain the native conformation of Claudin 3 can be checked, for example, as follows. When the immunological binding between the monoclonal antibodies of the present invention and cells expressing Claudin 3 on the cell surface is inhibited by certain polypeptides, it can be confirmed that these polypeptides maintain the conformation of the extracellular regions of naturally occurring Claudin 3.

Particularly, in a preferred embodiment of the present invention, monoclonal antibodies of the present invention recognize epitopes comprising the conformation formed by the two extracellular loop regions of Claudin 3. In the present invention, the epitopes comprising the conformation includes a structure formed by interactions within one polypeptide chain or interactions among multiple peptide chains. Such epitopes are also called "conformational epitopes". For example, an epitope formed by the interaction between the two loops constituting the extracellular domains of Claudin 3 is included in the epitopes comprising the conformation of the present invention. That is, monoclonal antibodies of the present invention preferably recognize a conformational epitope formed by the two extracellular loops of Claudin 3.

Recognition of a conformational epitope by a monoclonal antibody can be confirmed, for example, as follows. For example, a linear peptide comprising the amino acid sequences constituting the extracellular loops of Claudin 3 is synthesized. Such a peptide can be synthesized chemically. Alternatively, the peptide can be obtained by a genetic engineering method using the regions in a Claudin 3 cDNA that encode the amino acid sequences corresponding to the loop portions. Then, the binding between a test antibody and the linear peptide comprising the amino acid sequences constituting the loop portions is evaluated. For example, the activity of a test antibody to bind to the linear peptide can be evaluated by ELISA using an immobilized form of the peptide as antigen. Alternatively, the activity to bind to the linear peptide can be assessed based on the level of inhibition of binding between a test antibody and Claudin 3-expressing cells by the linear peptide. These experiments can evaluate the activity of a test antibody to bind to the linear peptide.

In the present invention, when an antibody that binds to Claudin 3-expressing cells also has activity to bind to the linear peptide, "the antibody has cross-reactivity to the linear peptide". In the present invention, preferred monoclonal antibodies do not substantially have cross-reactivity to the linear peptide comprising the amino acid sequences constituting the extracellular loops of Claudin 3. "Not substantially having cross-reactivity to the linear peptide" means that the activity of an antibody to bind to the linear peptide is, for example, 50% or less, generally 30% or less, or preferably 20% or less, as compared to the activity of the antibody to bind to Claudin 3-expressing cells.

Alternatively, recognition of a conformational epitope by a monoclonal antibody of the present invention can be confirmed as follows. Cells that express a chimeric molecule produced by linking one of the two extracellular loops of Claudin 3 with the other extracellular loop of a Claudin 3-like molecule are prepared. For example, human Claudin 1 can be used as a Claudin 3-like molecule. More specifically, cells forced to express the following chimeric molecules are produced.

| | 3/1 chimera | 1/3 chimera | Native |
|---|---|---|---|
| Loop 1 | Claudin 3 | Claudin 1 | Claudin 3 |
| Loop 2 | Claudin 1 | Claudin 3 | Claudin 3 |

A test antibody is contacted with these forced expression cells. If a monoclonal antibody has a lower activity of binding to both 3/1 chimera-expressing cells and 1/3 chimera-expressing cells, as compared to binding to native-type Claudin 3-expressing cells, the monoclonal body is an antibody that recognizes a conformational epitope of Claudin 3. In an embodiment, for example, a monoclonal antibody that recognizes an epitope formed by interactions between the two loops constituting the extracellular domains of Claudin 3 is one of the preferable antibodies that recognize a conformational epitope of Claudin 3 of the present invention. More specifically, a preferred monoclonal antibody of the present invention binds strongly to cells forced to express human Claudin 3, but does not substantially bind to either 3/1 chimera-expressing cells or 1/3 chimera-expressing cells. Herein, "not substantially binding" refers to a binding activity that is 80% or less, normally 50% or less, preferably 30% or less, or particularly preferably 15% or less compared to the activity of binding to human Claudin 3-expressing cells.

Examples of methods for evaluating the binding activity of an antibody to a cell include the method described on pages 359-420 of "Antibodies A Laboratory Manual" (Ed Harlow, David Lane, Cold Spring Harbor Laboratory, 1988). That is, the evaluation can be performed by the ELISA or fluorescence activated cell sorting (FACS) method using the cell as antigen.

In the ELISA format, the binding activity of an antibody to a cell is evaluated quantitatively by comparing the signal levels produced by enzymatic reaction. More specifically, a test antibody is added to ELISA plates on which the respective forced expression cells are immobilized, and the antibody bound to the cells is detected using an enzyme-labeled antibody that recognizes the test antibody. In FACS, the activity of binding to cells can be compared by producing a dilution series of a test antibody, and determining the binding titer of the antibody towards the respective forced expression cells.

The binding of an antibody to antigens expressed on the surface of cells suspended in a buffer solution or such can be detected by a flow cytometer. Known flow cytometers include the following instruments:

FACSCanto™ II
FACSAria™
FACSArray™
FACSVantage™ SE
FACSCalibur™ (all of the above are trade names ofBD Biosciences)
EPICS ALTRA HyPerSort
Cytomics FC 500
EPICS XL-MCL ADC EPICS XL ADC
Cell Lab Quanta / Cell Lab Quanta SC (all of the above are trade names of Beckman Coulter).

An example of a preferred method for measuring the activity of a test Claudin 3 antibody to bind to an antigen is set forth below. Staining is performed using an FITC-labeled secondary antibody that recognizes the test antibody which has been reacted with Claudin 3-expressing cells. The concentration of a test antibody used can be adjusted to a desired concentration by appropriately diluting the antibody in a suitable buffer. For example, the antibody can be used at any concentration between 10 μg/mL and 10 ng/mL. The fluorescence intensity and cell count are determined using FACSCalibur (BD). The amount of antibody bound to the cells is reflected in the fluorescence intensity, *i.e.*, the geometric mean value, which is obtained by an analysis using the CellQuest Software (BD). That is, the binding activity of an antibody, which is represented by the amount of antibody bound, can be determined by obtaining the geometric mean value.

In the present method, for example, "not substantially binding to 3/1 chimera-expressing cells" can be determined by the following method. First, a test antibody bound to the aforementioned 3/1 chimeric molecule-expressing cells is stained with a secondary antibody. For example, if the test antibody is a mouse antibody, an FITC-labeled anti-mouse immunoglobulin antibody can be used as the secondary antibody. Then, the fluorescence intensity of the cells is detected. When FACSCalibur is used as the flow cytometer for fluorescence detection, the obtained fluorescence intensity can be analyzed using the CellQuest Software. The rate of increase in fluorescence intensity as a result of the binding of a test antibody can be determined by using the equation below to calculate the Δ Geo-Mean ratio from the geometric mean values in the presence or absence of the test antibody.

$$\Delta \text{ Geo-Mean} = \text{geometric mean (in the presence of a test antibody) / geometric mean (in the absence of the test antibody)}$$

The geometric mean ratio that reflects the level of binding of a test antibody to 3/1 chimeric molecule-expressing cells (Δ Geo-Mean for 3/1 chimeric molecule) obtained by the analysis is compared with the Δ Geo-Mean ratio that reflects the level of binding of the test antibody to Claudin 3-expressing cells. In this case, it is particularly preferable to adjust the test antibody concentrations used for determining the Δ Geo-Mean ratios for 3/1 chimeric molecule-expressing cells and Claudin 3-expressing cells, to be identical or substantially identical to each other. A monoclonal antibody that has been confirmed in advance to recognize a conformational epitope of Claudin 3 can be used as a control antibody. For example, the monoclonal antibodies of the present invention shown below and in Fig. 9 can be used as antibodies that recognize a conformational epitope of Claudin 3:

CON01, CDN02, CDN03, CDN04, CDN05, CDN07, CDN08,
CDN16, CDN17, CDN24, CDN27, CDN28, CDN29,
CDN30, CDN31, CDN32, CDN33, CDN35, CDN36, CDN37, and CDN38.

In the present invention, if the Δ Geo-Mean ratio of a test antibody for 3/1 chimeric molecule-expressing cells is smaller than at least 80%, preferably 50%, more preferably 30%, and particularly preferably 15% of the Δ Geo-Mean ratio of the test antibody for Claudin 3-expressing cells, it is determined that the test antibody "does not substantially bind to 3/1 chimeric molecule-expressing cells". The equation for determining the geometric mean values is described in the CellQuest Software User's Guide (BD Biosciences). The activity to bind to 1/3 chimeric molecule-expressing cells can be evaluated similarly. For preparation of cells expressing the 3/1 chimera molecule, 1/3 chimera molecule, or Claudin 3 for evaluating the binding activity, it is preferable to use a common known expression vector and host cell, and to keep the expression levels in the respective cells the same.

Monoclonal antibodies of the present invention include monoclonal antibodies that bind to human Claudin 3-expressing cells, and whose activity of binding to cells expressing a chimeric molecule comprising a human Claudin 3 extracellular loop and a human Claudin 1 extracellular loop is lower than the activity of binding to the aforementioned human Claudin 3-expressing cells. Alternatively, preferred monoclonal antibodies of the present invention include monoclonal antibodies that bind to human Claudin 3-expresing cells, but do not substantially bind to cells expressing a chimeric molecule comprising a human Claudin 3 extracellular loop and a human Claudin 1 extracellular loop.

Production of anti-Claudin 3 monoclonal antibodies:

Monoclonal antibodies of the present invention can be obtained by DNA immunization. Mammal-derived monoclonal antibodies are particularly preferred as anti-Claudin 3 monoclonal antibodies of the present invention. The mammal-derived monoclonal antibodies include those produced by hybridomas, and those produced by hosts transformed with an expression vector containing an antibody gene using genetic engineering methods.

Monoclonal antibody-producing hybridomas of the present invention can be produced by DNA immunization as follows. DNA immunization is a method for providing immune stimulation by administering to an animal to be immunized, a vector DNA constructed so that a gene encoding an antigenic protein can be expressed in the immunized animal, and then expressing the immunogen in the body of the immunized animal. Compared to conventional immunization methods in which a protein antigen is administered, the following advantages can be expected from DNA immunization.

- Immune stimulation can be provided while maintaining the structure of a membrane protein such as Claudin 3.
- There is no need to purify an immunogen.

On the other hand, it is difficult to combine DNA immunization with the use of a means for immune stimulation such as an adjuvant. The identity between human and mouse Claudin 3 is particularly high in loop 1, which constitutes the extracellular region, 46/51. It was an unexpected achievement to obtain monoclonal antibodies that recognize proteins sharing such high interspecies identity by DNA immunization.

To obtain monoclonal antibodies of the present invention by DNA immunization, a DNA for expressing a Claudin 3 protein is administered to an animal to be immunized. A DNA encoding Claudin 3 can be synthesized by known methods such as PCR. The obtained DNA is inserted into a suitable expression vector, and then administered to an animal for immunization. Commercially available expression vectors such as pcDNA3.1 may be used as an expression vector. Conventional methods can be used to administer a vector to an organism. For example, gold particles adsorbed with an expression vector are shot into cells using a gene gun for DNA immunization.

According to the findings of the present inventors, hybridomas that produce Claudin 3-binding antibodies could not be obtained efficiently from mice immunized by intraperitoneal administration of cells forced to express Claudin 3. On the other hand, hybridomas that produce Claudin 3-binding antibodies could be obtained efficiently from mice immunized using DNA immunization. In particular, the hybridomas of interest could be readily obtained from mice to which cells forced to express Claudin 3 were administered after DNA immunization. That is, in a preferred method for obtaining the monoclonal antibodies of the present invention, a booster immunization using Claudin 3-expressing cells is performed after DNA immunization.

In the present invention, any non-human animal can be used as an animal for immunization. To obtain monoclonal antibodies by the cell fusion method, the animal to be immunized is preferably selected in consideration of its compatibility with the parental cell used for cell fusion. Generally, a rodent is preferred as the animal for immunization. More specifically, mice, rats, hamsters, or rabbits can be used as an animal for immunization. Alternatively, monkeys and such may be used as an animal for immunization.

Animals are immunized as described above. After confirming the increase in the titer of an antibody of interest in the serum, antibody-producing cells are collected from the immunized animals, and cloned. Preferred immunocytes (antibody-producing cells) are splenocytes.

A mammalian myeloma cell is used as a cell to be fused with the above-mentioned immunocyte. The myeloma cells preferably comprise a suitable selection marker for screening. A selection marker confers characteristics to cells for their survival (or failure to survive) under a specific culturing condition. Hypoxanthine-guanine phosphoribosyltransferase deficiency (hereinafter abbreviated as HGPRT deficiency), and thymidine kinase deficiency (hereinafter abbreviated as TK deficiency) are known as selection markers. Cells having HGPRT or TK deficiency have hypoxanthine-aminopterin-thymidine sensitivity (hereinafter abbreviated as HAT sensitivity). HAT-sensitive cells cannot carry out DNA synthesis in a HAT selection medium, and are thus killed. However, when the cells are fused with normal cells, they can continue to synthesize DNA using the salvage pathway of the normal cells, and therefore they can grow in the HAT selection medium.

HGPRT-deficient and TK-deficient cells can be selected in a medium containing 6-thioguanine or 8-azaguanine (hereinafter abbreviated as 8AG), and 5'-bromodeoxyuridine, respectively. Normal cells are killed since they incorporate these pyrimidine analogs into their DNA. On the other hand, cells that are deficient in these enzymes can survive in the selection medium, since they cannot incorporate these pyrimidine analogs. Alternatively, a selection marker referred to as G418 resistance provides resistance to 2-deoxystreptamine-type antibiotics (gentamycin analogs) from the neomycin-resistance gene. Various types of myeloma cells that are suitable for cell fusion are known. For example, myeloma cells including the following cells can be used to produce the monoclonal antibodies of the present invention:

P3 (P3x63Ag8.653) (J. Immunol. (1979) 123, 1548-1550);
P3x63Ag8U.1 (Current Topics in Microbiology and Immunology (1978) 81, 1-7);
NS-1 (Kohler. G. and Milstein, C. Eur. J. Immunol. (1976) 6, 511-519);
MPC-11 (Margulies. D.H. et al., Cell (1976) 8, 405-415);
SP2/0 (Shulman, M. et al., Nature (1978) 276, 269-270);
FO (de St. Groth, S. F. et al., J. Immunol. Methods (1980) 35, 1-21);
S194 (Trowbridge, 1. S. J. Exp. Med. (1978) 148, 313-323); and
R210 (Galfre, G. et al., Nature (1979) 277, 131-133).

Cell fusion of the above-mentioned immunocytes with myeloma cells is essentially performed according to a known method, for example, the method of Kohler and Milstein *et al.* (Kohler. G. and Milstein, C., Methods Enzymol. (1981) 73, 3-46).

More specifically, the above-mentioned cell fusion can be performed in a standard nutritional culture medium in the presence of, for example, a cell-fusion accelerator. A cell-fusion accelerator may be, for example, polyethylene glycol (PEG), Sendai virus (HVJ), or the like. If desired, an auxiliary agent such as dimethylsulfoxide can be added to further enhance fusion efficiency.

The ratio of immunocytes to myeloma cells used can be established at one's discretion. For example, the number of immunocytes is preferably set to one to ten times of that of myeloma cells. As a medium to be used for the above-mentioned cell fusion, for example, RPMI1640 medium and MEM medium, which are appropriate for the growth of the above-mentioned myeloma cell line, or other standard media that are used for this type of cell culture can be used. Moreover, a serum supplement solution such as fetal calf serum (FCS) can be added to the media.

Cell fusion is performed by thoroughly mixing predetermined amounts of the above-mentioned immunocytes and myeloma cells in the above-mentioned medium, adding and mixing with a PEG solution pre-heated to approximately 37°C, so as to form the desired fused cells (hybridomas). In the cell fusion method, for example, PEG with an average molecular weight of approximately 1000 to 6000 can generally be added at a concentration of 30 to 60% (w/v). Subsequently, the agent for cell fusion or the like which is unfavorable for the growth of hybridomas can be removed by successively adding an appropriate medium such as those listed above, removing the supernatant after centrifugation, and repeating these steps.

Hybridomas obtained in this manner can be selected using a selection medium appropriate for the selection markers carried by myelomas used for cell fusion. For example, cells that have HGPRT and TK deficiencies can be selected by culturing them in a HAT medium (a medium containing hypoxanthine, aminopterin, and thymidine). More specifically, when HAT-sensitive myeloma cells are used for cell fusion, cells that successfully fuse with normal cells can be selectively grown in the HAT medium. Culturing using the above-mentioned HAT medium is continued for a sufficient period of time to kill the cells other than the hybridoma of interest (non-fused cells). More specifically, the hybridoma of interest can be selected, typically by culturing for several days to several weeks. Subsequently, hybridomas that produce the antibody of interest can be screened and singly-cloned by carrying out a standard limiting dilution method. Alternatively, a Claudin 3-recognizing antibody can be prepared using the method described in International Patent Publication No. WO 03/104453.

An antibody of interest can be suitably screened and singly cloned by a screening method based on known antigen-antibody reaction. For example, preferred monoclonal antibodies of the present invention can bind to Claudin 3 expressed on the cell surface. Such monoclonal antibodies can be screened by fluorescence-activated cell sorting (FACS). FACS is a system that can be used to assess the binding of an antibody to the cell surface, by analyzing cells contacted with a fluorescent-labeled antibody using a laser beam, and measuring the fluorescence emitted by each cell.

To screen for hybridomas that produce monoclonal antibodies of the present invention by FACS, Claudin 3-expressing cells are prepared. Preferred cells for the screening are mammalian cells forced to express Claudin 3. By using untransformed mammalian host cells as the control, the activity of an antibody to bind to cell-surface Claudin 3 can be selectively detected. More specifically, hybridomas producing preferable monoclonal antibodies of the present invention can be obtained by selecting hybridomas that produce antibodies which do not bind to the untransformed host cells but bind to cells forced to express Claudin 3.

Alternatively, the activity of an antibody to bind to immobilized Claudin 3-expresing cells can be evaluated using the ELISA method. For example, Claudin 3-expressing cells are immobilized in the wells of an ELISA plate. A hybridoma culture supernatant is contacted with the immobilized cells in the wells, and the antibodies that bind to the immobilized cells are detected. If the monoclonal antibodies are derived from mice, the antibodies bound to the cells can be detected using anti-mouse immunoglobulin antibodies. Hybridomas selected by the screening, which produce the antibodies of interest having antigen-binding ability, can be cloned by the limiting dilution method or the like.

Alternatively, screening can be performed, for example, as follows to obtain monoclonal antibodies that recognize a conformational epitope of Claudin 3. Cells that express a chimeric molecule produced by linking one of the two extracellular loops of Claudin 3 with the other extracellular loop of a Claudin 3-like molecule are prepared. For example, human Claudin 1 can be used as the Claudin 3-like molecule. More specifically, cells forced to express the following chimeric molecules are produced.

| | 3/1 chimera | 1/3 chimera | Native |
|---|---|---|---|
| Loop 1 | Claudin 3 | Claudin 1 | Claudin 3 |
| Loop 2 | Claudin 1 | Claudin 3 | Claudin 3 |

Monoclonal antibodies that recognize a conformational epitope of Claudin 3 of the present invention can be obtained by contacting these forced expression cells with test antibodies, and selecting monoclonal antibodies having lower binding activity to both 3/1 chimera-expressing cells and 1/3 chimera-expressing cells, than to native-type Claudin 3-expressing cells. The preferred cells for the screening are mammalian cells. The reactivity of the monoclonal antibodies to these cells can be determined by ELISA or FACS using the cells as antigen.

The monoclonal antibody-producing hybridomas produced in this manner can be passaged and cultured in a standard medium. Alternatively, the hybridomas can be stored for a long period in liquid nitrogen.

The hybridomas can be cultured according to a standard method, and the monoclonal antibody of interest can be obtained from the culture supernatants. Alternatively, the hybridomas can be grown by administering them to a compatible mammal, and monoclonal antibodies can be obtained as its ascites. The former method is suitable for obtaining highly purified antibodies.

In the present invention, an antibody encoded by an antibody gene cloned from antibody-producing cells can be used. The cloned antibody gene can be incorporated into a suitable vector and then introduced into a host to express the antibody. Methods for isolating an antibody gene, introducing the gene into a vector, and transforming host cells have been established (see for example, Vandamme, A. M. et al., Eur. J. Biochem. (1990) 192, 767-775).

For example, a cDNA encoding the variable region (V region) of an anti-Claudin 3 antibody can be obtained from hybridoma cells producing the anti-Claudin 3 antibody. Usually, in order to accomplish this, first, total RNA is extracted from the hybridoma. For example, the following methods can be used as methods for extracting mRNA from cells:

the guanidine ultracentrifugation method (Chirgwin, J. M. et al., Biochemistry (1979) 18, 5294-5299); and
the AGPC method (Chomczynski, P. et al., Anal. Biochem. (1987) 162, 156-159).

The extracted mRNA can be purified using an mRNA purification kit (GE Healthcare Bio-Sciences) or the like. Alternatively, kits for directly extracting total mRNA from cells, such as the QuickPrep mRNA Purification Kit (GE Healthcare Bio-Sciences), are also commercially available. Total RNA can be obtained from the hybridoma by using such kits. A cDNA encoding the antibody V region can be synthesized from the obtained mRNA using reverse transcriptase. cDNA can be synthesized using the AMV Reverse Transcriptase First-strand cDNA Synthesis Kit (SEIKAGAKU CORPORATION) or the like. To synthesize and amplify cDNA, the SMART RACE cDNA Amplification Kit (Clontech) and the 5'-RACE method using PCR (Frohman, M. A. et al., Proc. Natl. Acad. Sci. USA (1988) 85, 8998-9002; Belyavsky, A. et al., Nucleic Acids Res. (1989) 17, 2919-2932) can be used. Furthermore, in the process of such cDNA synthesis, appropriate restriction enzyme sites, which will be described later, can be introduced into both ends of the cDNA.

The cDNA fragment of interest is purified from the obtained PCR product, and then ligated to a vector DNA. The recombinant vector is prepared in this manner and introduced into *Escherichia coli* or the like, and after colonies are selected, the desired recombinant vector can be prepared from the *E. coli* that formed the colonies. Whether or not the recombinant vector has the cDNA nucleotide sequence of interest can be confirmed by a known method, such as the dideoxynucleotide chain termination method.

To obtain a gene encoding a variable region, it is most convenient to use the 5'-RACE method which utilizes primers for amplifying the variable region gene. First, a 5'-RACE cDNA library is obtained by synthesizing cDNAs using RNAs extracted from hybridoma cells as template. To synthesize a 5'-RACE cDNA library, it is convenient to use commercially available kits such as the SMART RACE cDNA Amplification Kit.

The antibody genes are amplified by the PCR method, using the obtained 5'-RACE cDNA library as a template. Primers for amplification of mouse antibody genes can be designed based on known antibody gene sequences. The nucleotide sequences of these primers vary depending on the immunoglobulin subclass. Therefore, the subclasses are desirably determined in advance using a commercially available kit such as the IsoStrip Mouse Monoclonal Antibody Isotyping Kit (Roche Diagnostics).

More specifically, for example, when the objective is to obtain genes encoding mouse IgG, one may use primers that can amplify genes encoding $\gamma$1, $\gamma$2a, $\gamma$2b, and $\gamma$3 as the heavy chain and the $\kappa$ chain and $\lambda$ chain as the light chain. To amplify genes of the IgG variable region, generally, a primer that anneals to a portion corresponding to the constant region close to the variable region is used as the 3'-end primer. For the 5'-end primer, the primer included in a 5'-RACE cDNA library production kit can be used.

PCR products amplified in this manner can be used to reconstitute an immunoglobulin comprising a combination of heavy and light chains. Based on the binding activity of the reconstituted immunoglobulin to Claudin 3, one can screen for antibodies of interest.

For example, when the objective is to obtain an antibody against Claudin 3, preferably, the binding of the antibody to Claudin 3 is specific. One can screen for an antibody that binds to Claudin 3, for example, by the following steps:

(1) contacting an antibody comprising the V regions encoded by a cDNA obtained from a hybridoma with a Claudin 3-expressing cell;
(2) detecting binding between the Claudin 3-expressing cell and the antibody; and
(3) selecting the antibody that binds to the Claudin 3-expressing cell.

A method for detecting binding between an antibody and a Claudin 3-expressing cell is known. Specifically, the binding between an antibody and a Claudin 3-expressing cell can be detected by the aforementioned methods such as FACS. To evaluate the binding activity of an antibody, a fixed sample of a Claudin 3-expressing cell may also be used.

Alternatively, for an antibody screening method based on the binding activity, a phage vector-based panning method may be used. When the antibody genes are obtained as libraries of the heavy-chain and light-chain subclasses from polyclonal antibody-expressing cells, phage displaying methods are advantageous. Genes encoding variable regions of the heavy and light chains can be made into a single-chain Fv (scFv) gene by linking the genes via suitable linker sequences. Phages expressing an scFv on their surface can be obtained by inserting a gene encoding the scFv into a phagemid vector. DNA encoding an scFv having the binding activity of interest can be collected by contacting the phage with an antigen of interest, and then collecting antigen-bound phage. scFv having the binding activity of interest can be enriched by repeating this operation as necessary.

An antibody-encoding polynucleotide of the present invention may encode a full-length antibody or a portion of the antibody. "A portion of an antibody" refers to any portion of an antibody molecule. Hereinafter, the term "antibody fragment" may be used to refer to a portion of an antibody. A preferred antibody fragment of the present invention comprises the complementarity determination region (CDR) of an antibody. More preferably, an antibody fragment of the present invention comprises all of the three CDRs that constitute a variable region.

Once a cDNA encoding the V region of an anti-Claudin 3 antibody of interest is obtained, this cDNA is digested with restriction enzymes that recognize the restriction enzyme sites inserted to both ends of the cDNA. A preferred restriction enzyme recognizes and digests a nucleotide sequence that is less likely to appear in the nucleotide sequence constituting the antibody gene. Furthermore, to insert a single copy of the digested fragment into a vector in the correct direction, a restriction enzyme that provides sticky ends is preferred. A cDNA encoding the anti-Claudin 3 antibody V region, which has been digested as described above, is inserted into a suitable expression vector to obtain the antibody expression vector. In this step, a chimeric antibody can be obtained by fusing a gene encoding the antibody constant region (C region) with the above-mentioned gene encoding the V region in frame. Herein, "chimeric antibody" refers to an antibody whose constant and variable regions are derived from different origins. Therefore, in addition to interspecies chimeric antibodies such as mouse-human chimeric antibodies, human-human intraspecies chimeric antibodies are also included in the chimeric antibodies of the present invention. A chimeric antibody expression vector can also be constructed by inserting the aforementioned V-region gene into an expression vector into which a constant region gene has been introduced. More specifically, for example, the restriction enzyme recognition sequence for a restriction enzyme that digests the aforementioned V-region gene can be placed at the 5' end of a DNA encoding a desired antibody constant region (C region) in an expression vector. The chimeric antibody expression vector is constructed by digesting the two vectors using the same combination of restriction enzymes, and fusing them in frame.

To produce an anti-Claudin 3 monoclonal antibody of the present invention, the antibody gene can be incorporated into an expression vector so that it is expressed under the regulation of an expression control region. The expression regulatory region for antibody expression includes, for example, an enhancer or a promoter. Then, by transforming suitable host cells with this expression vector, recombinant cells that carry the DNA expressing the anti-Claudin 3 antibody can be obtained.

To express an antibody gene, a DNA encoding the antibody heavy chain (H-chain) and a DNA encoding the antibody light chain (L-chain) can be incorporated separately into expression vectors. An antibody molecule comprising the H chain and L chain can be expressed by simultaneously transfecting (co-transfecting) the H-chain and L-chain-incorporated vectors into the same host cell. Alternatively, DNAs encoding the H chain and L chain can be incorporated into a single expression vector to transform a host cell with the vector (see International Patent Publication No. WO 94/11523).

Many combinations of hosts and expression vectors for isolating an antibody gene and then introducing the gene into an appropriate host to produce the antibody are known. Any of these expression systems can be applied to the present invention. When using eukaryotic cells as a host, animal cells, plant cells, and fungal cells can be used. More specifically, animal cells that may be used in the present invention are, for example, the following cells:

(1) mammalian cells such as CHO, COS, myeloma, baby hamster kidney (BHK), HeLa, and Vero cells;
(2) amphibian cells such as Xenopus oocytes; and
(3) insect cells such as sf9, sf21, Tn5.

In addition, as a plant cell system, an antibody gene expression system using cells derived from the *Nicotiana* genus such as *Nicotiana tabacum* is known. Callus-cultured cells can be used to transform plant cells.

Furthermore, the following cells can be used as fungal cells; yeasts: the *Saceharomyces* genus, for example, *Saccharomyces cerevisiae*, and the *Pichia* genus, for example, *Pichia pastoris*; and filamentous fungi: the *Aspergillus* genus, for example, *Aspergillus niger*.

Antibody gene expression systems that utilize prokaryotic cells are also known. For example, when using bacterial cells, *E. coli* cells, *Bacillus subtilis* cells, and such may be used in the present invention.

Expression vectors comprising the antibody genes of interest are introduced into these cells by transformation. By culturing the transformed cells *in vitro,* the desired antibodies can be obtained from the transformed cell culture.

In addition to the above host cells, transgenic animals can also be used to produce a recombinant antibody. That is, the antibody can be obtained from an animal into which the gene encoding the antibody of interest is introduced. For example, the antibody gene can be inserted in frame into a gene that encodes a protein produced inherently in milk to construct a fused gene. Goat β-casein or such can be used, for example, as the protein secreted in milk. A DNA fragment containing the fused gene inserted with the antibody gene is injected into a goat embryo, and then this embryo is introduced into a female goat. Desired antibodies can be obtained as a protein fused with the milk protein from milk produced by the transgenic goat born from the goat that received the embryo (or progeny thereof). To increase the volume of milk containing the desired antibody produced by the transgenic goat, hormones can be used on the transgenic goat as necessary (Ebert, K. M. et al., Bio/Technology (1994) 12, 699-702).

Animal-derived antibody C regions can be used for the C regions of a recombinant antibody of the present invention. For example, Cγ1, Cγ2a, Cγ2b, Cγ3, Cμ, Cδ, Cα1, Cα2, and Cε can be used for the mouse antibody H-chain C-region, and Cκ and Cλ can be used for the L-chain C-region. In addition to mouse antibodies, antibodies of animals such as rats, rabbits, goat, sheep, camels, and monkeys can be used as animal antibodies. Their sequences are known. Furthermore, the C region can be modified to improve the stability of the antibodies or their production.

In the present invention, when administering antibodies to humans, genetically recombinant antibodies that have been artificially modified for the purpose of reducing xenoantigenicity against humans, or the like can be used. Examples of the genetically recombinant antibodies include chimeric antibodies and humanized antibodies. These modified antibodies can be produced using known methods.

A chimeric antibody is an antibody whose variable regions and constant regions are of different origins. For example, an antibody comprising the heavy-chain and light-chain variable regions of a mouse antibody and the heavy-chain and light-chain constant regions of a human antibody is a mouse-human interspecies chimeric antibody. A recombinant vector expressing a chimeric antibody can be produced by ligating a DNA encoding a mouse antibody variable region to a DNA encoding a human antibody constant region, and then inserting it into an expression vector. The recombinant cells that have been transformed with the vector are cultured, and the incorporated DNA is expressed to obtain the chimeric antibody produced in the culture. Human C regions are used for the C regions of chimeric antibodies and humanized antibodies.

For example, Cγ1, Cγ2, Cγ3, Cγ4, Cμ, Cδ, Cα1, Cα2, and Cε can be used as an H-chain C region. Cκ and Cλ can be used as an L-chain C region. The amino acid sequences of these C regions and the nucleotide sequences encoding them are known. Furthermore, the human antibody C region can be modified to improve the stability of an antibody or its production.

Generally, a chimeric antibody consists of the V region of an antibody derived from a non-human animal, and a C region derived from a human antibody. On the other hand, a humanized antibody consists of the complementarity determining region (CDR) of an antibody derived from a non-human animal, and the framework region (FR) and C region derived from a human antibody. Since the antigenicity of a humanized antibody in human body is reduced, a humanized antibody is useful as an active ingredient for therapeutic agents of the present invention.

For example, mouse-human chimeric antibodies obtained by linking the variable regions of an anti-Claudin 3 monoclonal antibody produced based on the present invention with amino acid sequences constituting the human constant regions are preferred as monoclonal antibodies of the present invention. More specifically, the present invention provides mouse-human chimeric monoclonal antibodies comprising an H-chain variable region and an L-chain variable region comprising the amino acid sequences of any of (1) to (6):

(1) CDN04

H chain: the amino acid sequence of SEQ ID NO: 18
L chain: the amino acid sequence of SEQ ID NO: 30

(2) CDN16

H chain: the amino acid sequence of SEQ ID NO: 42
L chain: the amino acid sequence of SEQ ID NO: 52

(3) CDN27

H chain: the amino acid sequence of SEQ ID NO: 62
L chain: the amino acid sequence of SEQ ID NO: 72

(4) CDN28

H chain: the amino acid sequence of SEQ ID NO: 82
L chain: the amino acid sequence of SEQ ID NO: 92

(5) CDN35

H chain: the amino acid sequence of SEQ ID NO: 102
L chain: the amino acid sequence of SEQ ID NO: 112

(6) CDN38

H chain: the amino acid sequence of SEQ ID NO: 165

L chain: the amino acid sequence of SEQ ID NO: 177

Furthermore, as an example of such mouse-human chimeric antibodies, the present invention provides a mouse-human chimeric antibody comprising an H chain comprising the amino acid sequence of SEQ ID NO: 175 and an L chain comprising the amino acid sequence of SEQ ID NO: 187, which is obtained by linking the CDN38 variable regions with the human constant regions.
The antibody variable region generally comprises three complementarity-determining regions (CDRs) separated by four framework regions (FRs). CDR is a region that substantially determines the binding specificity of an antibody. The amino acid sequences of CDRs are highly diverse. On the other hand, the FR-constituting amino acid sequences are often highly homologous even among antibodies with different binding specificities. Therefore, generally, the binding specificity of a certain antibody can be transferred to another antibody by CDR grafting.
A humanized antibody is also called a reshaped human antibody. Specifically, humanized antibodies prepared by grafting the CDR of a non-human animal antibody such as a mouse antibody to a human antibody and such are known. Common genetic engineering technologies for obtaining humanized antibodies are also known.
Specifically, for example, overlap extension PCR is known as a method for grafting a mouse antibody CDR to a human FR. In overlap extension PCR, a nucleotide sequence encoding a mouse antibody CDR to be grafted is added to the primers for synthesizing a human antibody FR. Primers are prepared for each of the four FRs. It is generally considered that when grafting a mouse CDR to a human FR, selecting a human FR that is highly homologous to a mouse FR is advantageous for maintaining the CDR function. That is, it is generally preferable to use a human FR comprising an amino acid sequence highly homologous to the amino acid sequence of the FR adjacent to the mouse CDR to be grafted.
Nucleotide sequences to be ligated are designed so that they will be connected to each other in frame. Human FRs are individually synthesized using the respective primers. As a result, products in which the mouse CDR-encoding DNA is attached to the individual FR-encoding DNAs are obtained. Nucleotide sequences encoding the mouse CDR of each product are designed so that they overlap with each other. Then, overlapping CDR regions of the products synthesized using a human antibody gene as the template are annealed for complementary strand synthesis reaction. By this reaction, human FRs are ligated through the mouse CDR sequences.
The full length of the V-region gene, in which three CDRs and four FRs are ultimately ligated, is amplified using primers that anneal to its 5' and 3' ends and which have suitable restriction enzyme recognition sequences. A vector for human antibody expression can be produced by inserting the DNA obtained as described above and a DNA that encodes a human antibody C region into an expression vector so that they will ligate in frame. After transfecting this integration vector into a host to establish recombinant cells, the recombinant cells are cultured, and the DNA encoding the humanized antibody is expressed to produce the humanized antibody in the cell culture (see, European Patent Publication No. EP 239,400, and International Patent Publication No. WO 96/02576).
By qualitatively or quantitatively measuring and evaluating the antigen-binding activity of the humanized antibody produced as described above, one can suitably select human antibody FRs that allow CDRs to form a favorable antigen-binding site when ligated through the CDRs. As necessary, amino acid residues in an FR may be substituted so that the CDRs of a reshaped human antibody form an appropriate antigen-binding site. For example, amino acid sequence mutations can be introduced into FRs by applying the PCR method used for fusing a mouse CDR with a human FR. More specifically, partial nucleotide sequence mutations can be introduced into primers that anneal to the FR sequence. Nucleotide sequence mutations are introduced into the FRs synthesized using such primers. Mutant FR sequences having the desired characteristics can be selected by measuring and evaluating the activity of the amino acid-substituted mutant antibody to bind to the antigen by the above-mentioned method (Sato, K. et al., Cancer Res. 1993, 53, 851-856).
Alternatively, a desired human antibody can be obtained by DNA immunization using a transgenic animal that comprises the entire repertoire of human antibody genes (see International Patent Publication Nos. WO 93/12227, WO 92/03918, WO 94/02602, WO 94/25585, WO 96/34096, and WO 96/33735) as an animal for immunization. Furthermore, technologies to obtain human antibodies by panning a human antibody library are also known. For example, the V region of a human antibody is expressed as a single chain antibody (scFv) on the phage surface using a phage display method, and phages that bind to the antigen can be selected. By analyzing the genes of selected phages, the DNA sequences encoding the V regions of human antibodies that bind to the antigen can be determined. After determining the DNA sequences of scFvs that bind to the antigen, the V region sequence is fused in frame with the desired human antibody C region sequence, and this is inserted into a suitable expression vector to produce an expression vector. This expression vector can be introduced into suitable expression cells such as those described above, and the human antibody-encoding gene can be expressed to obtain the human antibodies. Such methods are well known (International Patent Publication Nos. WO 92/01047, WO 92/20791, WO 93/06213, WO 93/11236, WO 93/19172, WO 95/01438, and WO 95/15388).
The monoclonal antibodies of the present invention are not limited to bivalent antibodies represented by IgG, but include monovalent antibodies and multivalent antibodies represented by IgM, as long as it binds to the Claudin 3 protein. The multivalent antibody of the present invention includes a multivalent antibody that has the same antigen binding sites, and a multivalent antibody that has partially or completely different antigen binding sites. The monoclonal antibody of the present invention is not limited to the whole antibody molecule, but includes minibodies and modified products thereof, as long as they bind to the Claudin 3 protein.

A minibody contains an antibody fragment lacking a portion of a whole antibody (for example, whole IgG). As long as it has the ability to bind the Claudin 3 antigen, partial deletions of an antibody molecule are permissible. Antibody fragments of the present invention preferably contain a heavy-chain variable region (VH) and/or a light-chain variable region (VL). The amino acid sequence of VH or VL may have substitutions, deletions, additions, and/or insertions. Furthermore, as long as it has the ability to bind the Claudin 3 antigen, VH and/or VL can be partially deleted. The variable region may be chimerized or humanized. Specific examples of the antibody fragments include Fab, Fab', F(ab') 2, and Fv. Specific examples of minibodies include Fab, Fab', F(ab')2, Fv, scFv (single chain Fv), diabody, and sc(Fv) 2 (single chain (Fv)2). Multimers of these antibodies (for example, dimers, trimers, tetramers, and polymers) are also included in the minibodies of the present invention.

Fragments of antibodies can be obtained by treating an antibody with an enzyme to produce antibody fragments. Known enzymes that produce antibody fragments are, for example, papain, pepsin, and plasmin. Alternatively, genes encoding these antibody fragments can be constructed, introduced into expression vectors, and then expressed in appropriate host cells (see, for example, Co, M. S. et al., J. Immunol. (1994) 152, 2968-2976; Better, M. and Horwitz, A. H., Methods in Enzymology (1989) 178, 476-496; Plueckthun, A. and Skerra, A., Methods in Enzymology (1989) 178, 476-496; Lamoyi, E., Methods in Enzymology (1989) 121, 652-663; Rousseaux, J. et al., Methods in Enzymology (1989) 121, 663-669; and Bird, R. E. et al., TIBTECH (1991) 9, 132-137).

Digestive enzymes cleave specific sites of an antibody fragment, and yield antibody fragments with the following specific structures. When genetic engineering technologies are used on such enzymatically obtained antibody fragments, any portion of the antibody can be deleted.

Papain digestion: F(ab)2 or Fab
Pepsin digestion: F(ab')2 or Fab'
Plasmin digestion: Facb

Therefore, minibodies of the present invention may be antibody fragments lacking any region, as long as they have binding affinity to Claudin 3. Furthermore, according to the present invention, the antibodies desirably maintain their effector activity, particularly in the treatment of cell proliferative diseases such as cancer. More specifically, preferred minibodies of the present invention have both binding affinity to Claudin 3 and effector function. The antibody effector function includes ADCC activity and CDC activity. Particularly preferably, therapeutic antibodies of the present invention have ADCC activity and/or CDC activity as effector function.

A diabody refers to a bivalent antibody fragment constructed by gene fusion (Hollinger P. et al., Proc. Natl. Acad. Sci. USA 90: 6444-6448 (1993); EP 404,097; WO 93/11161; and such). A diabody is a dimer composed of two polypeptide chains. Generally, in each polypeptide chain constituting the dimer, VL and VH are linked by a linker within the same chain. The linker in a diabody is generally short enough to prevent binding between VL and VH. Specifically, the amino acid residues constituting the linker are, for example, five residues or so. Therefore, VL and VH that are encoded by the same polypeptide chain cannot form a single-chain variable region fragment, and form a dimer with another single chain variable region fragment. As a result, diabodies have two antigen binding sites.

scFv can be obtained by ligating the H-chain V region and L-chain V region of an antibody. In scFv, the H-chain V region and L-chain V region are ligated via a linker, preferably a peptide linker (Huston, J. S. et al., Proc. Natl. Acad. Sci. U.S.A., 1988, 85, 5879-5883). The H-chain V region and L-chain V region of scFv may be derived from any of the antibodies described herein. The peptide linker for ligating the V regions is not particularly limited. For example, any single-chain peptide consisting of 3 to 25 residues or so can be used as the linker. More specifically, for example, peptide linkers described below or such can be used.

PCR methods such as those described above can be used for ligating the V regions. For ligation of the V regions by PCR methods, first, a whole DNA or a DNA encoding a desired partial amino acid sequence selected from the following DNAs can be used as a template:

a DNA sequence encoding the H chain or the H-chain V region of the above-mentioned antibody; and
a DNA sequence encoding the L chain or the L-chain V region of the above-mentioned antibody.

DNAs encoding the H-chain and L-chain V regions are individually amplified by PCR methods using a pair of primers that have sequences corresponding to the sequences of both ends of the DNA to be amplified. Then, a DNA encoding the peptide linker portion is prepared. The DNA encoding the peptide linker can also be synthesized using PCR. To the 5' end of the primers used, nucleotide sequences that can be ligated to each of the individually synthesized V-region amplification products are added. Then, PCR reaction is carried out using the "H-chain V region DNA", "peptide linker DNA", and "L-chain V region DNA", and the primers for assembly PCR.

The primers for assembly PCR consist of the combination of a primer that anneals to the 5' end of the "H-chain V region DNA" and a primer that anneals to the 3' end of the "L-chain V region DNA". That is, the primers for assembly PCR are a primer set that can amplify a DNA encoding the full-length sequence of scFv to be synthesized. On the other hand, nucleotide sequences that can be ligated to each V-region DNA are added to the "peptide linker DNA". Thus, these DNAs are ligated, and the full-length scFv is ultimately produced as an amplification product using the primers for assembly PCR. Once the scFv-encoding DNA is constructed, expression vectors containing the DNA, and recombinant cells transformed by these expression vectors can be obtained according to conventional methods. Furthermore, the scFvs can be obtained by culturing the resulting recombinant cells and expressing the scFv-encoding DNA.

sc(Fv)2 is a minibody prepared by ligating two VHs and two VLs with linkers or such to form a single chain (Hudson et al., J. Immunol. Methods 1999; 231: 177-189). sc(Fv)2 can be produced, for example, by joining scFvs with a linker.

Moreover, antibodies in which two VHs and two VLs are arranged in the order of VH, VL, VH, and VL ([VH]-linker-[VL]-linker-[VH]-linker-[VL]), starting from the N-terminal side of a single chain polypeptide, are preferred.

The order of the two VHs and the two VLs is not particularly limited to the above-mentioned arrangement, and they may be placed in any order. Examples include the following arrangements:

[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

Any arbitrary peptide linker can be introduced by genetic engineering, and synthetic linkers (see, for example, those disclosed in Protein Engineering, 9(3), 299-305, 1996) or such can be used as linkers for linking the antibody variable regions. In the present invention, peptide linkers are preferable. The length of the peptide linkers is not particularly limited, and can be suitably selected by those skilled in the art according to the purpose. The length of amino acid residues composing a peptide linker is generally 1 to 100 amino acids, preferably 3 to 50 amino acids, more preferably 5 to 30 amino acids, and particularly preferably 12 to 18 amino acids (for example, 15 amino acids).

Any amino acid sequences composing peptide linkers can be used, as long as they do not inhibit the binding activity of scFv. Examples of the amino acid sequences used in peptide linkers include:

Ser
Gly-Ser
Gly-Gly-Ser
Ser-Gly-Gly
Gly-Gly-Gly-Ser (SEQ ID NO: 149)
Ser-Gly-Gly-Gly (SEQ ID NO: 150)
Gly-Gly-Gly-Gly-Ser (SEQ ID NO: 151)
Ser-Gly-Gly-Gly-Gly (SEQ ID NO: 152)
Gly-Gly-Gly-Gly-Gly-Ser (SEQ ID NO: 153)
Ser-Gly-Gly-Gly-Gly-Gly (SEQ ID NO: 154)
Gly-Gly-Gly-Gly-Gly-Gly-Ser (SEQ ID NO: 155)
Ser-Gly-Gly-Gly-Gly-Gly-Gly (SEQ ID NO: 156)
(Gly-Gly-Gly-Gly-Ser (SEQ ID NO: 157))n
(Ser-Gly-Gly-Gly-Gly (SEQ ID NO: 158))n

in which n is an integer of 1 or larger.

The amino acid sequences of the peptide linkers can be selected appropriately by those skilled in the art according to the purpose. For example, n, which determines the length of the peptide linkers, is generally 1 to 5, preferably 1 to 3, more preferably 1 or 2.

Therefore, a particularly preferred embodiment of sc(Fv)2 in the present invention is, for example, the following sc(Fv)2:

[VH]-peptide linker (15 amino acids)-[VL]-peptide linker (15 amino acids)-[VH]-peptide linker (15 amino acids)-[VL]

Alternatively, synthetic chemical linkers (chemical crosslinking agents) can be used to link the V regions.

Crosslinking agents routinely used to crosslink peptide compounds and such can be used in the present invention. For example, the following chemical crosslinking agents are known. These crosslinking agents are commercially available:

N-hydroxy succinimide (NHS);
disuccinimidyl suberate (DSS);
bis(sulfosuccinimidyl) suberate (BS3);
dithiobis(succinimidyl propionate) (DSP);
dithiobis(sulfosuccinimidyl propionate) (DTSSP);
ethylene glycol bis(succinimidyl succinate) (EGS);
ethylene glycol bis(sulfosuccinimidyl succinate) (sulfo-EGS);
disuccinimidyl tartrate (DST);
disulfosuccinimidyl tartrate (sulfo-DST);
bis[2-(succinimidoxycarbonyloxy)ethyl] sulfone (BSOCOES); and
bis[2-(sulfosuccinimidoxycarbonyloxy)ethyl] sulfone (sulfo-BSOCOES).

Usually, three linkers are required to link four antibody variable regions. The multiple linkers to be used may all be of the same type or different types. In the present invention, a preferred minibody is a diabody or an sc(Fv)2. Such minibody can be obtained by treating an antibody with an enzyme, such as papain or pepsin, to generate antibody fragments, or by constructing DNAs that encode these antibody fragments, introducing them into expression vectors, and then expressing them in appropriate host cells (see, for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

Monoclonal antibodies of the present invention include any antibody that recognizes and binds to Claudin 3. For example, preferred antibodies include the antibodies of (1) to (61) shown below. These antibodies may be full-length antibodies, minibodies, animal antibodies, chimeric antibodies, humanized antibodies, or human antibodies.

(1) an antibody comprising an H chain having the amino acid sequence of SEQ ID NO: 12 as CDR1, the amino acid sequence of SEQ ID NO: 14 as CDR2, and the amino acid sequence of SEQ ID NO: 16 as CDR3;
(2) an antibody comprising the H chain of (1), wherein the H chain has the amino acid sequence of positions 139 to 462 in the amino acid sequence of SEQ ID NO: 20 as CH;
(3) an antibody comprising the H chain of (1), wherein the H chain has the amino acid sequence of SEQ ID NO: 22 as CH;
(4) an antibody comprising an L chain having the amino acid sequence of SEQ ID NO: 24 as CDR1, the amino acid sequence of SEQ ID NO: 26 as CDR2, and the amino acid sequence of SEQ ID NO: 28 as CDR3;
(5) an antibody comprising the L chain of (4), wherein the L chain has the amino acid sequence of positions 135 to 240 in the amino acid sequence of SEQ ID NO: 32 as CL;
(6) an antibody comprising the L chain of (4), wherein the L chain has the amino acid sequence of SEQ ID NO: 34 as CL;
(7) an antibody comprising the H chain of (1) and the L chain of (4);
(8) an antibody comprising the H chain of (2) and the L chain of (5);
(9) an antibody comprising the H chain of (3) and the L chain of (6);
(10) an antibody having one or more amino acid substitutions, deletions, additions, and/or insertions in the antibody of any of (1) to (9), which has equivalent activity as the antibody of any of (1) to (9);
(11) an antibody comprising an H chain having the amino acid sequence of SEQ ID NO: 36 as CDR1, the amino acid sequence of SEQ ID NO: 38 as CDR2, and the amino acid sequence of SEQ ID NO: 40 as CDR3;
(12) an antibody comprising the H chain of (11), wherein the H chain has the amino acid sequence of positions 140 to 476 in the amino acid sequence of SEQ ID NO: 44 as CH;
(13) an antibody comprising the H chain of (11), wherein the H chain has the amino acid sequence of SEQ ID NO: 22 as CH;
(14) an antibody comprising an L chain having the amino acid sequence of SEQ ID NO: 46 as CDR1, the amino acid sequence of SEQ ID NO: 48 as CDR2, and the amino acid sequence of SEQ ID NO: 50 as CDR3;
(15) an antibody comprising the L chain of (14), wherein the L chain has the amino acid sequence of positions 13 to 238 in the amino acid sequence of SEQ ID NO: 54 as CL;
(16) an antibody comprising the L chain of (14), wherein the L chain has the amino acid sequence of SEQ ID NO: 34 as CL;
(17) an antibody comprising the H chain of (11) and the L chain of (14);
(18) an antibody comprising the H chain of (12) and the L chain of (15);
(19) an antibody comprising the H chain of (13) and the L chain of (16);

(20) an antibody having one or more amino acid substitutions, deletions, additions, and/or insertions in the antibody of any of (11) to (19), which has equivalent activity as the antibody of any of (11) to (19);
(21) an antibody comprising an H chain having the amino acid sequence of SEQ ID NO: 56 as CDR1, the amino acid sequence of SEQ ID NO: 58 as CDR2, and the amino acid sequence of SEQ ID NO: 60 as CDR3;
(22) an antibody comprising the H chain of (21), wherein the H chain has the amino acid sequence of positions 137 to 471 in the amino acid sequence of SEQ ID NO: 64 as CH;
(23) an antibody comprising the H chain of (21), wherein the H chain has the amino acid sequence of SEQ ID NO: 22 as CH;
(24) an antibody comprising an L chain having the amino acid sequence of SEQ ID NO: 66 as CDR1, the amino acid sequence of SEQ ID NO: 68 as CDR2, and the amino acid sequence of SEQ ID NO: 70 as CDR3;
(25) an antibody comprising the L chain of (24), wherein the L chain has the amino acid sequence of positions 133 to 238 in the amino acid sequence of SEQ ID NO: 74 as CL;
(26) an antibody comprising the L chain of (24), wherein the L chain has the amino acid sequence of SEQ ID NO: 34 as CL;
(27) an antibody comprising the H chain of (21) and the L chain of (24);
(28) an antibody comprising the H chain of (22) and the L chain of (25);
(29) an antibody comprising the H chain of (23) and the L chain of (26);
(30) an antibody having one or more amino acid substitutions, deletions, additions, and/or insertions in the antibody of any of (21) to (29), which has equivalent activity as the antibody of any of (21) to (29);
(31) an antibody comprising an H chain having the amino acid sequence of SEQ ID NO: 76 as CDR1, the amino acid sequence of SEQ ID NO: 78 as CDR2, and the amino acid sequence of SEQ ID NO: 80 as CDR3;
(32) an antibody comprising the H chain of (31), wherein the H chain has the amino acid sequence of positions 140 to 463 in the amino acid sequence of SEQ ID NO: 84 as CH;
(33) an antibody comprising the H chain of (31), wherein the H chain has the amino acid sequence of SEQ ID NO: 22 as CH;
(34) an antibody comprising an L chain having the amino acid sequence of SEQ ID NO: 86 as CDR1, the amino acid sequence of SEQ ID NO: 88 as CDR2, and the amino acid sequence of SEQ ID NO: 90 as CDR3;
(35) an antibody comprising the L chain of (34), wherein the L chain has the amino acid sequence of positions 133 to 238 in the amino acid sequence of SEQ ID NO: 94 as CL;
(36) an antibody comprising the L chain of (34), wherein the L chain has the amino acid sequence of SEQ ID NO: 34 as CL;
(37) an antibody comprising the H chain of (31) and the L chain of (34);
(38) an antibody comprising the H chain of (32) and the L chain of (35);
(39) an antibody comprising the H chain of (33) and the L chain of (36);
(40) an antibody having one or more amino acid substitutions, deletions, additions, and/or insertions in the antibody of any of (31) to (39), which has equivalent activity as the antibody of any of (31) to (3 9);
(41) an antibody comprising an H chain having the amino acid sequence of SEQ ID NO: 96 as CDR1, the amino acid sequence of SEQ ID NO: 98 as CDR2, and the amino acid sequence of SEQ ID NO: 100 as CDR3;
(42) an antibody comprising the H chain of (41), wherein the H chain has the amino acid sequence of positions 140 to 474 in the amino acid sequence of SEQ ID NO: 104 as CH;
(43) an antibody comprising the H chain of (41), wherein the H chain has the amino acid sequence of SEQ ID NO: 22 as CH;
(44) an antibody comprising an L chain having the amino acid sequence of SEQ ID NO: 106 as CDR1, the amino acid sequence of SEQ ID NO: 108 as CDR2, and the amino acid sequence of SEQ ID NO: 110 as CDR3;
(45) an antibody comprising the L chain of (44), wherein the L chain has the amino acid sequence of positions 133 to 238 in the amino acid sequence of SEQ ID NO: 114 as CL;
(46) an antibody comprising the L chain of (44), wherein the L chain has the amino acid sequence of SEQ ID NO: 34 as CL;
(47) an antibody comprising the H chain of (41) and the L chain of (44);
(48) an antibody comprising the H chain of (42) and the L chain of (45);
(49) an antibody comprising the H chain of (43) and the L chain of (46);
(50) an antibody having one or more amino acid substitutions, deletions, additions, and/or insertions in the antibody of any of (41) to (49), which has equivalent activity as the antibody of any of (41) to (49);
(51) an antibody comprising an H chain having the amino acid sequence of SEQ ID NO: 167 as CDR1, the amino acid sequence of SEQ ID NO: 169 as CDR2, and the amino acid sequence of SEQ ID NO: 171 as CDR3;
(52) an antibody comprising the H chain of (51), wherein the H chain has the amino acid sequence of positions 118 to 447 in the amino acid sequence of SEQ ID NO: 173 as CH;
(53) an antibody comprising the H chain of (51), wherein the H chain has the amino acid sequence of SEQ ID NO:

22 as CH;

(54) an antibody comprising an L chain having the amino acid sequence of SEQ ID NO: 179 as CDR1, the amino acid sequence of SEQ ID NO: 181 as CDR2, and the amino acid sequence of SEQ ID NO: 183 as CDR3;

(55) an antibody comprising the L chain of (54), wherein the L chain has the amino acid sequence of positions 113 to 218 in the amino acid sequence of SEQ ID NO: 185 as CL;

(56) an antibody comprising the L chain of (54), wherein the L chain has the amino acid sequence of SEQ ID NO: 34 as CL;

(57) an antibody comprising the H chain of (51) and the L chain of (54);

(58) an antibody comprising the H chain of (52) and the L chain of (55);

(59) an antibody comprising the H chain of (53) and the L chain of (56);

(60) an antibody having one or more amino acid substitutions, deletions, additions, and/or insertions in the antibody of any of (51) to (59), which has equivalent activity as the antibody of any of (5 1) to (59);

(61) an antibody that binds to the same epitope as the Claudin 3 protein epitope bound by the antibody of any of (1) to (60).

In the present invention, preferred monoclonal antibodies comprise the amino acid sequences constituting CDR1, CDR2, and CDR3 of the heavy chains and light chains derived from any of CDN04, CDN16, CDN27, CDN28, CDN35, and CDN38 as the CDR amino acid sequences. The CDR amino acid sequences of these monoclonal antibodies are shown below. The monoclonal antibodies comprising in their variable regions, CDRs comprising the amino acid sequences shown in the sequence ID numbers indicated below, are preferred as the monoclonal antibodies of the present invention. For example, the V region sequences and full-length amino acid sequences of the monoclonal antibodies are shown in the sequence ID numbers indicated below. The monoclonal antibodies comprising these amino acid sequences in the V regions can be indicated as preferred monoclonal antibodies of the present invention.

| | Heavy chain | Light chain |
|---|---|---|
| CDR1 | SEQ ID NO: 12 | SEQ ID NO: 24 |
| CDN04 CDR2 | SEQ ID NO: 14 | SEQ ID NO: 26 |
| CDR3 | SEQ ID NO: 16 | SEQ ID NO: 28 |
| [CDN04 V region | SEQ ID NO: 18 | SEQ ID NO: 30] |
| [CDN04 C region | positions 139-462 of SEQ ID NO: 20 | positions 135-240 of SEQ ID NO: 32] |
| [CDN04 full length | SEQ ID NO: 20 | SEQ ID NO: 32] |
| CDR1 | SEQ ID NO: 36 | SEQ ID NO: 46 |
| CDN16 CDR2 | SEQ ID NO: 38 | SEQ ID NO: 48 |
| CDR3 | SEQ ID NO: 40 | SEQ ID NO: 50 |
| [CDN16 V region | SEQ ID NO: 42 | SEQ ID NO: 52] |
| [CDN16 C region | positions 140-476 of SEQ ID NO: 44 | positions 133-238 of SEQ ID NO: 54] |
| [CDN16 full length | SEQ ID NO: 44 | SEQ ID NO: 54] |
| CDR1 | SEQ ID NO: 56 | SEQ ID NO: 66 |
| CDN27 CDR2 | SEQ ID NO: 58 | SEQ ID NO: 68 |
| CDR3 | SEQ ID NO: 60 | SEQ ID NO: 70 |
| [CDN27 V region | SEQ ID NO: 62 | SEQ ID NO: 72] |
| [CDN27 C region | positions 137-471 of SEQ ID NO: 64 | positions 133-234 of SEQ ID NO: 74] |
| [CDN27 full length | SEQ ID NO: 64 | SEQ ID NO: 74] |
| CDR1 | SEQ ID NO: 76 | SEQ ID NO: 86 |
| CDN28 CDR2 | SEQ ID NO: 78 | SEQ ID NO: 88 |
| CDR3 | SEQ ID NO: 80 | SEQ ID NO: 90 |
| [CDN28 V region | SEQ ID NO: 82 | SEQ ID NO: 92] |
| [CDN28 C region | positions 140-463 of SEQ ID NO: 84 | positions 133-238 of SEQ ID NO: 94] |
| [CDN28 full length | SEQ ID NO: 84 | SEQ ID NO: 94] |

(continued)

| | Heavy chain | Light chain |
|---|---|---|
| CDR1 | SEQ ID NO: 96 | SEQ ID NO: 106 |
| CDN35 CDR2 | SEQ ID NO: 98 | SEQ ID NO: 108 |
| CDR3 | SEQ ID NO: 100 | SEQ ID NO: 110 |
| [CDN35 V region | SEQ ID NO: 102 | SEQ ID NO: 112] |
| [CDN35 C region | positions 140-474 of SEQ ID NO: 104 | positions 133-238 of SEQ ID NO: 114] |
| [CDN35 full length | SEQ ID NO: 104 | SEQ ID NO: 114] |
| CDR1 | SEQ ID NO: 167 | SEQ ID NO: 179 |
| CDN38 CDR2 | SEQ ID NO: 169 | SEQ ID NO: 181 |
| CDR3 | SEQ ID NO: 171 | SEQ ID NO: 183 |
| [CDN38 V region | SEQ ID NO: 165 | SEQ ID NO: 177] |
| [CDN38 C region | positions 118-447 of SEQ ID NO: 173 | positions 113-218 of SEQ ID NO: 185] |
| [CDN38 full length | SEQ ID NO: 173 | SEQ ID NO: 185] |

The monoclonal antibodies of the present invention may comprise a constant region in addition to a variable region comprising the aforementioned CDRs. The full-length sequences of the monoclonal antibodies including the constant regions are as shown above. Furthermore, the following human-derived amino acid sequences can be shown as examples of the constant regions comprised in the monoclonal antibodies of the present invention:

SEQ ID NO: 21 (human IgG1 CH sequence), SEQ ID NO: 33 (human IgG1 CL kappa sequence),
SEQ ID NO: 22 (human IgG1 CH sequence), SEQ ID NO: 34 (human IgG1 CL kappa sequence)

Therefore, the monoclonal antibodies produced by linking the constant regions comprising the human-derived amino acid sequences shown by the above-mentioned sequence ID numbers with the variable regions comprising the aforementioned CDRs 1, 2, and 3 are preferable monoclonal antibodies of the present invention. Examples of such monoclonal antibodies include the above-mentioned monoclonal antibodies of (3), (13), (23), (33), (43), and (53), and may include the above-mentioned light chains of (6), (16), (26), (36), (46), and (56), respectively, as the light chains.

A preferred embodiment of the above-mentioned antibody of (10), (20), (30), (40), (50), or (60) is an antibody in which the CDR has not been modified. For example, among the above-mentioned antibodies of (10), a preferred embodiment of "an antibody having one or more amino acid substitutions, deletions, additions, and/or insertions in the antibody of (1) and having an activity equivalent to that of the antibody of (1)" is "an antibody having an activity equivalent to that of the antibody of (1) and having one or more amino acid substitutions, deletions, additions, and/or insertions in the antibody of (1), and also comprising an H chain having the amino acid sequence of SEQ ID NO: 12 as CDR1, the amino acid sequence of SEQ ID NO: 14 as CDR2, and the amino acid sequence of SEQ ID NO: 16 as CDR3". Preferred embodiments of other antibodies included in the above-mentioned antibody of (10), (20), (30), (40), (50), or (60) can be expressed in a similar manner.

A method of introducing mutations into polypeptides is one of the methods well known to those skilled in the art for preparing polypeptides that are functionally equivalent to a certain polypeptide. For example, those skilled in the art can prepare an antibody functionally equivalent to an antibody of the present invention by introducing appropriate mutations into the antibody using site-directed mutagenesis (Hashimoto-Gotoh, T. et al. (1995) Gene 152, 271-275; Zoller, MJ, and Smith, M. (1983) Methods Enzymol. 100, 468-500; Kramer, W. et al. (1984) Nucleic Acids Res. 12, 9441-9456; Kramer W, and Fritz HJ (1987) Methods. Enzymol. 154, 350-367; Kunkel, TA (1985) Proc. Natl. Acad. Sci. USA. 82,488-492; Kunkel (1988) Methods Enzymol. 85, 2763-2766) and such. Amino acid mutations may also occur naturally. In this way, the antibodies of the present invention also comprise antibodies comprising amino acid sequences with one or more amino acid mutations in the amino acid sequences of the antibodies of the present invention, and which are functionally equivalent to the antibodies of the present invention.

The number of amino acids that are mutated in such mutants is generally considered to be 50 amino acids or less, preferably 30 amino acids or less, and more preferably 10 amino acids or less (for example, 5 amino acids or less).

It is desirable that the amino acid residues are mutated into amino acids in which the properties of the amino acid side chains are conserved. For example, the following categories have been established depending on the amino acid side chain properties:

hydrophobic amino acids (A, I, L, M, F, P, W, Y, and V);
hydrophilic amino acids (R, D, N, C, E, Q, G, H, K, S, and T);
amino acids having aliphatic side chains (G, A, V, L, I, and P);
amino acids having hydroxyl-containing side chains (S, T, and Y);
amino acids having sulfur-containing side chains (C and M);
amino acids having carboxylic acid- and amide-containing side chains (D, N, E, and Q);
amino acids having basic side chains (R, K, and H); and
amino acids having aromatic ring-containing side chains (H, F, Y, and W)
(amino acids are represented by one-letter codes in parentheses).

Polypeptides comprising a modified amino acid sequence, in which one or more amino acid residues in a certain amino acid sequence is deleted, added, and/or substituted with other amino acids, are known to retain their original biological activities (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413). That is, generally in an amino acid sequence constituting a certain polypeptide, the activity of the polypeptide is highly likely to be maintained when amino acids classified into the same group are mutually substituted. In the present invention, the above-mentioned substitution between amino acids within the same amino acid group is referred to as conservative substitution.

The present invention provides antibodies that bind to the same epitope as the monoclonal antibodies disclosed in the present application. More specifically, the present invention relates to antibodies that recognize the same epitope as the monoclonal antibodies of the present invention, and uses thereof. Such antibodies can be obtained, for example, by the following method.

Whether a test antibody binds to the same epitope as the epitope bound by a certain antibody; that is, whether a test antibody shares the epitope of a certain antibody can be confirmed by checking whether the two antibodies compete for the same epitope. In the present invention, competition between antibodies can be detected by FACS or cross-blocking assay. In FACS, first, a monoclonal antibody of the present invention is bound to Claudin 3-expressing cells, and the fluorescence signal is detected. Next, a candidate competitive antibody is reacted with the cells, then the monoclonal antibody of the present invention is reacted with the same cells, and this is analyzed similarly by FACS. Alternatively, a monoclonal antibody of the present invention and a test competitive antibody can be reacted with the same cells at the same time. If the pattern of FACS analysis of a monoclonal antibody of the present invention changes upon reaction with a competitive antibody, one can confirm that the competitive antibody recognizes the same epitope as the monoclonal antibody of the present invention.

Alternatively, for example, competitive ELISA assay is a preferred cross-blocking assay. Specifically, in a cross-blocking assay, Claudin 3 protein-expressing cells are immobilized onto the wells of a microtiter plate. After preincubation in the presence or absence of a candidate competitive antibody, a monoclonal antibody of the present invention is added. The amount of monoclonal antibody of the present invention that binds to the Claudin 3 protein-expressing cells in the wells inversely correlates with the binding ability of the candidate competitive antibody (test antibody) that competes for binding to the same epitope. That is, the greater the affinity the test antibody has for the same epitope, the lower the amount of the monoclonal antibody of the present invention bound to the wells onto which the Claudin 3 protein-expressing cells are immobilized. On the other hand, the greater the affinity the test antibody has for the same epitope, the greater the amount of the test antibody bound to the wells onto which the Claudin 3 protein-expressing cells are immobilized.

The amount of antibodies bound to the wells can be easily determined by labeling the antibodies in advance. For example, biotin-labeled antibodies can be detected using an avidin peroxidase conjugate and its suitable substrate. Cross-blocking assays that use the antibody labeled with an enzyme such as peroxidase are specifically called competitive ELISA assays. The antibodies can be labeled with other detectable or measurable substances. Specifically, radioactive labeling and fluorescent labeling are known.

Furthermore, when the test antibody has a constant region derived from a species different from that of the monoclonal antibody of the present invention, measurement can be done for either one of the antibodies bound to the wells using a labeled antibody that specifically recognizes the constant region derived from the species of the antibody to be detected. Alternatively, if the antibodies are derived from the same species but belong to different classes, the antibodies bound to the wells can be measured using antibodies that specifically distinguish individual classes.

If a candidate competing antibody can block binding of a monoclonal antibody of the present invention by at least 20%, preferably by at least 20% to 50%, and even more preferably, by at least 50%, as compared to the binding activity obtained in a control experiment performed in the absence of the candidate competing antibody, the candidate competing antibody is either an antibody that binds substantially to the same epitope or one that competes for binding to the same epitope as a monoclonal antibody of the present invention.

Antibodies that bind to the same epitope as the monoclonal antibodies include, for example, the above-mentioned antibody of (61).

As described above, the above-mentioned antibodies of (1) to (61) include not only monovalent antibodies but also multivalent antibodies. Multivalent antibodies of the present invention include multivalent antibodies whose antigen binding sites are all the same and multivalent antibodies whose antigen binding sites are partially or completely different.

Antibodies bound to various types of molecules such as polyethylene glycol (PEG) can also be used as modified antibodies. Moreover, chemotherapeutic agents, toxic peptides, or radioactive chemical substances can be bound to the antibodies. Such modified antibodies (hereinafter referred to as antibody conjugates) can be obtained by subjecting the obtained antibodies to chemical modification. Methods for modifying antibodies are already established in this field. Furthermore, as described below, such antibodies can also be obtained in the molecular form of a bispecific antibody designed using genetic engineering technologies to recognize not only Claudin 3 proteins, but also chemotherapeutic agents, toxic peptides, radioactive chemical compounds, or such. These antibodies are included in the "antibodies" of the present invention.

Chemotherapeutic agents that are bound to monoclonal antibodies of the present invention to drive the cytotoxic activity include the following:

azaribine, anastrozole, azacytidine, bleomycin, bortezomib,
bryostatin-1, busulfan, camptothecin, 10-hydroxycamptothecin, carmustine,
celebrex, chlorambucil, cisplatin, irinotecan, carboplatin, cladribine,
cyclophosphamide, cytarabine, dacarbazine, docetaxel, dactinomycin,
daunomycin glucuronide, daunorubicin, dexamethasone, diethylstilbestrol,
doxorubicin, doxorubicin glucuronide, epirubicin, ethinyl estradiol,
estramustine, etoposide, etoposide glucuronide, floxuridine, fludarabine,
flutamide, fluorouracil, fluoxymesterone, gemcitabine,
hydroxyprogesterone caproate, hydroxyurea, idarubicin, ifosfamide,
leucovorin, lomustine, mechlorethamine, medroxyprogesterone acetate,
megestrol acetate, melphalan, mercaptopurine, methotrexate, mitoxantrone,
mithramycin, mitomycin, mitotane, phenylbutyrate, prednisone, procarbazine,
paclitaxel, pentostatin, semustine streptozocin, tamoxifen, taxanes,
taxol, testosterone propionate, thalidomide, thioguanine,
thiotepa, teniposide, topotecan, uracil mustard, vinblastine,
vinorelbine, and vincristine.

In the present invention, preferred chemotherapeutic agents are low-molecular-weight chemotherapeutic agents. Low-molecular-weight chemotherapeutic agents are unlikely to interfere with antibody function even after binding to antibodies. In the present invention, low-molecular-weight chemotherapeutic agents usually have a molecular weight of 100 to 2000, preferably 200 to 1000. Examples of the chemotherapeutic agents demonstrated herein are all low-molecular-weight chemotherapeutic agents. The chemotherapeutic agents of the present invention include prodrugs that are converted to active chemotherapeutic agents *in vivo.* Prodrug activation may be enzymatic conversion or non-enzymatic conversion.

Furthermore, the antibodies can be modified using toxic peptides such as ricin, abrin, ribonuclease, onconase, DNase I, Staphylococcal enterotoxin-A, pokeweed antiviral protein, gelonin, diphtheria toxin, Pseudomonas exotoxin, Pseudomonas endotoxin, L-asparaginase, and PEG L-Asparaginase. In another embodiment, one or two or more of the low-molecular-weight chemotherapeutic agents and toxic peptides can be combined and used for antibody modification. The bonding between a monoclonal antibody and the above-mentioned low-molecular weight chemotherapeutic agent may be covalent bonding or non-covalent bonding. Methods for producing antibodies bound to these chemotherapeutic agents are known.

Furthermore, pharmacologically active proteins or peptide toxins can be bound to antibodies by gene recombination technologies. Specifically, for example, it is possible to construct a recombinant vector by fusing a DNA encoding the above-mentioned toxic peptide with a DNA encoding a monoclonal antibody of the present invention in frame, and inserting this into an expression vector. This vector is introduced into suitable host cells, the obtained transformed cells are cultured, and the incorporated DNA is expressed. Thus, an anti-Claudin 3 antibody bound to the toxic peptide can be obtained as a fusion protein. When obtaining an antibody as a fusion protein, the pharmacologically active protein or toxin is generally fused at the C terminus of the antibody. A peptide linker can be inserted between the antibody and the pharmacologically active protein or toxin.

Furthermore, the monoclonal antibody of the present invention may be a bispecific antibody. A bispecific antibody refers to an antibody that carries variable regions that recognize different epitopes within the same antibody molecule. The bispecific antibody may have antigen-binding sites that recognize different epitopes on a Claudin 3 molecule. Two molecules of such a bispecific antibody can bind to one molecule of Claudin 3. As a result, stronger cytotoxic action can be expected.

Alternatively, the bispecific antibody may be an antibody in which one antigen-binding site recognizes Claudin 3, and the other antigen-binding site recognizes a cytotoxic substance. Specifically, cytotoxic substances include chemotherapeutic agents, toxic peptides, and radioactive chemical substances. Such a bispecific antibody binds to Claudin 3-expressing cells, and at the same time, captures cytotoxic substances. This enables the cytotoxic substances to directly act on Claudin 3-expressing cells. Therefore, bispecific antibodies that recognize cytotoxic substances specifically injure tumor cells and suppress tumor cell proliferation.

Furthermore, in the present invention, bispecific antibodies that recognize antigens other than Claudin 3 may be combined. For example, it is possible to combine bispecific antibodies that recognize non-Claudin 3 antigens that are specifically expressed on the surface of target cancer cells like Claudin 3.

Methods for producing bispecific antibodies are known. For example, two types of antibodies recognizing different antigens may be linked to prepare a bispecific antibody. The antibodies to be linked may be half molecules each having an H chain or an L chain, or may be quarter molecules consisting of only an H chain. Alternatively, hybrid cells producing a bispecific antibody can be prepared by fusing hybridomas producing different monoclonal antibodies. Bispecific antibodies can also be prepared by genetic engineering technologies.

Antibody genes constructed as described above can be expressed and antibodies can be obtained by known methods. For mammalian cells, the antibody genes can be expressed by operatively placing the antibody gene just behind a commonly used effective promoter, and a polyA signal on the 3' downstream side of the antibody gene. An example of such promoter/enhancer is human cytomegalovirus immediate early promoter/enhancer.

Other promoters/enhancers that can be used for antibody expression include viral promoters/enhancers, or mammalian cell-derived promoters/enhancers such as human elongation factor 1$\alpha$ (HEF1$\alpha$). Specific examples of viruses whose promoters/enhancers may be used include retrovirus, polyoma virus, adenovirus, and simian virus 40 (SV40).

When an SV40 promoter/enhancer is used, the method of Mulligan et al. (Nature (1979) 277, 108) may be utilized. An HEF1$\alpha$, promoter/enhancer can be readily used for expressing a gene of interest by the method of Mizushima et al. (Nucleic Acids Res. (1990) 18, 5322).

In the case of *E. coli,* the antibody can be expressed by operatively placing the antibody gene with a signal sequence for secretion at downstream of a commonly used effective promoter. Examples of such promoter include the lacZ promoter and araB promoter. For the lacZ promoter, the method of Ward et al., (Nature (1989) 341, 544-546; FASEB J. (1992) 6, 2422-2427) may be used. Alternatively, the araB promoter can be used for expressing a gene of interest by the method of Better et al. (Science (1988) 240, 1041-1043).

The pelB signal sequence for secretion (Lei, S. P. et al., J. Bacteriol. (1987) 169, 4379) may be used for antibody production in the periplasm of *E. coli*. After isolation of the antibody produced in the periplasm, the antibody can be refolded by using a protein denaturant like guanidine hydrochloride or urea so that the antibody will have the desired binding activity.

The replication origin inserted into the expression vector includes, for example, those derived from SV40, polyoma virus, adenovirus, or bovine papilloma virus (BPV). In order to amplify the gene copy number in the host cell system, a selection marker can be inserted into the expression vector. Specifically, the following selection markers can be used:

the aminoglycoside transferase (APH) gene;
the thymidine kinase (TK) gene;
the *E. coli* xanthine guanine phosphoribosyltransferase (Ecogpt) gene;
the dihydrofolate reductase (dhfr) gene, etc.

Any expression system, for example, a eukaryotic cell system or a prokaryotic cell system can be used to produce monoclonal antibodies of the present invention. Examples of eukaryotic cells include animal cells such as established mammalian cell lines, insect cell lines, and filamentous fungus cells and yeast cells. Examples of prokaryotic cells include bacterial cells such as *E. coli* cells. Monoclonal antibodies of the present invention are preferably expressed in mammalian cells. For example, mammalian cells such as CHO, COS, myeloma, BHK, Vero, or HeLa cells can be used.

Then, the transformed cell is then cultured *in vitro* or *in vivo* to produce an antibody of interest. The cells are cultured according to known methods. For example, DMEM, MEM, RPMI 1640, or IMDM can be used as the culture medium. A serum such as fetal calf serum (FCS) can also be used as supplement.

Antibodies produced as described above can be purified by using a single or a suitable combination of known methods generally used for purifying proteins. Antibodies can be separated and purified by, for example, appropriately combining filtration, ultrafiltration, salt precipitation, dialysis, affinity chromatography using a protein A column, other chromatography, and such (Antibodies A Laboratory Manual. Ed Harlow, David Lane, Cold Spring Harbor Laboratory, 1988).

Known methods can be used to measure the antigen-binding activity of the antibodies (Antibodies A Laboratory Manual. Ed Harlow, David Lane, Cold Spring Harbor Laboratory, 1988). For example, an enzyme linked immunosorbent assay (ELISA), an enzyme immunoassay (EIA), a radioimmunoassay (RIA), or a fluoroimmunoassay can be used.

The monoclonal antibodies of the present invention may be antibodies with a modified sugar chain. It is known that the cytotoxic activity of an antibody can be increased by modifying its sugar chain. Known antibodies having modified sugar chains include the following:

antibodies with modified glycosylation (for example, WO 99/54342);
antibodies deficient in fucose attached to sugar chains (for example, WO 00/61739 and WO 02/31140);
antibodies having a sugar chain with bisecting GlcNAc (for example, WO 02/79255), etc.

The antibodies used in the present invention are preferably antibodies having cytotoxic activity.

In the present invention, the cytotoxic activity includes, for example, antibody-dependent cell-mediated cytotoxicity (ADCC) activity and complement-dependent cytotoxicity (CDC) activity. In the present invention, CDC activity refers to complement system-mediated cytotoxic activity. ADCC activity refers to the activity of injuring a target cell when a specific antibody attaches to its cell surface antigen. An Fcγ receptor-carrying cell (immune cell, or such) binds to the Fc portion of the antibody via the Fcγ receptor and the target cell is damaged.

A monoclonal antibody of the present invention can be tested to see whether it has ADCC activity or CDC activity using known methods (for example, Current Protocols in Immunology, Chapter 7. Immunologic studies in humans, Editor, John E. Coligan et al., John Wiley & Sons, Inc., (1993) and the like).

First, specifically, effector cells, complement solution, and target cells are prepared.

(1) Preparation of effector cells
Spleen is removed from a CBA/N mouse or the like, and spleen cells are isolated in RPMI1640 medium (manufactured by Invitrogen). After washing in the same medium containing 10% fetal bovine serum (FBS, manufactured by HyClone), the cell concentration is adjusted to 5 x $10^6$ /mL to prepare the effector cells.
(2) Preparation of complement solution
Baby Rabbit Complement (manufactured by CEDARLANE) is diluted 10-fold in a culture medium (manufactured by Invitrogen) containing 10% FBS to prepare a complement solution.
(3) Preparation of target cells
The target cells can be radioactively labeled by incubating cells expressing the Claudin 3 protein with 0.2 mCi of sodium chromate-$^{51}$Cr (manufactured by GE Healthcare Bio-Sciences) in a DMEM medium containing 10% FBS for one hour at 37°C. For Claudin 3 protein-expressing cells, one may use transformed cells with a Claudin 3 gene, ovarian cancer cells, prostate cancer cells, breast cancer cells, uterine cancer cells, liver cancer cells, lung cancer cells, pancreatic cancer cells, stomach cancer cells, bladder cancer cells, colon cancer cells, or such. After radioactive labeling, cells are washed three times in RPMI1640 medium with 10% FBS, and the target cells can be prepared by adjusting the cell concentration to 2 x $10^5$ /mL.

ADCC activity or CDC activity can be measured by the method described below. In the case of ADCC activity measurement, the target cell and anti-Claudin 3 antibody (50 μL each) are added to a 96-well U-bottom plate (manufactured by Becton Dickinson), and reacted for 15 minutes on ice. Thereafter, 100 μL of effector cells are added and incubated in a carbon dioxide incubator for four hours. The final concentration of the antibody is adjusted to 0 or 10 μg/mL. After culturing, 100 μL of the supernatant is collected, and the radioactivity is measured with a gamma counter (COBRAII AUTO-GAMMA, MODEL D5005, manufactured by Packard Instrument Company). The cytotoxic activity (%) can be calculated using the measured values according to the equation: (A - C) / (B - C) x 100, wherein A represents the radioactivity (cpm) in each sample, B represents the radioactivity (cpm) in a sample where 1% NP-40 (manufactured by Nacalai Tesque) has been added, and C represents the radioactivity (cpm) of a sample containing the target cells only.

Meanwhile, in the case of CDC activity measurement, 50 μL of target cell and 50 μL of an anti-Claudin 3 antibody are added to a 96-well flat-bottomed plate (manufactured by Becton Dickinson), and reacted for 15 minutes on ice. Thereafter, 100 μL of the complement solution is added, and incubated in a carbon dioxide incubator for four hours. The final concentration of the antibody is adjusted to 0 or 3 μg/mL. After incubation, 100 μL of supernatant is collected, and the radioactivity is measured with a gamma counter. The cytotoxic activity can be calculated in the same way as in the ADCC activity determination.

On the other hand, in the case of measuring the cytotoxic activity of an antibody conjugate, 50 μL of target cell and 50 μL of an anti-Claudin 3 antibody conjugate are added to a 96-well flat-bottomed plate (manufactured by Becton Dickinson), and reacted for 15 minutes on ice. This is then incubated in a carbon dioxide incubator for one to four hours. The final concentration of the antibody is adjusted to 0 or 3 μg/mL. After culturing, 100 μL of supernatant is collected, and the radioactivity is measured with a gamma counter. The cytotoxic activity can be calculated in the same way as in the ADCC activity determination.

In the present invention, the cells whose proliferation is suppressed by a monoclonal antibody are not particularly limited, as long as the cells express a Claudin 3 protein. Preferred Claudin 3-expressing cells are, for example, cancer cells. More preferably, the cells are ovarian cancer, prostate cancer, breast cancer, uterine cancer, liver cancer, lung cancer, pancreatic cancer, stomach cancer, bladder cancer, and colon cancer cells. Therefore, anti-Claudin 3 antibodies can be used for the purpose of treating or preventing cell proliferation-induced diseases such as ovarian cancer, prostate cancer, breast cancer, uterine cancer, liver cancer, lung cancer, pancreatic cancer, stomach cancer, bladder cancer, and colon cancer.

Pharmaceutical Compositions

In another aspect, the present invention provides pharmaceutical compositions comprising a monoclonal antibody that binds to a Claudin 3 protein as an active ingredient. Furthermore, the present invention relates to anticancer agents comprising a monoclonal antibody that binds to a Claudin 3 protein as an active ingredient. Cell proliferation inhibitors and anticancer agents of the present invention are preferably administered to subjects affected with cancer, or subjects with the likelihood of recurrence of cancer.

Furthermore, in the present invention, an anticancer agent comprising a monoclonal antibody that binds to a Claudin 3 protein as an active ingredient can also be described as a method for preventing or treating cancer which comprises the step of administering an antibody that binds to a Claudin 3 protein to a subject, or as use of a monoclonal antibody that binds to a Claudin 3 protein in the production of an anticancer agent.

In the present invention, the phrase "comprising a monoclonal antibody that binds to Claudin 3 as an active ingredient" means comprising an anti-Claudin 3 monoclonal antibody as the major active ingredient, and does not limit the content percentage of the monoclonal antibody.

Furthermore, multiple types of monoclonal antibodies can be mixed into the pharmaceutical compositions or anticancer agents of the present invention as necessary. For example, the cytotoxic effect against Claudin 3-expressing cells may be strengthened by producing a cocktail of multiple Claudin 3-binding monoclonal antibodies. Alternatively, the therapeutic effect can be enhanced by mixing a Claudin 3-binding antibody with an antibody that recognizes another tumor-related antigen.

The monoclonal antibody included in the pharmaceutical composition of the present invention (for example, cell proliferation inhibitor and anticancer agent; same hereinafter) is not particularly limited as long as it binds to a Claudin 3 protein, and examples include antibodies described herein.

The pharmaceutical compositions or anticancer agents of the present invention can be administered orally or parenterally to a patient. Preferably, the administration is parenteral administration. Specifically, the method of administration is, for example, administration by injection, transnasal administration, transpulmonary administration, or transdermal administration. Examples of administration by injection include systemic and local administrations of a pharmaceutical composition of the present invention by intravenous injection, intramuscular injection, intraperitoneal injection, subcutaneous injection, or such. A suitable administration method may be selected according to the age of the patient and symptoms. The dosage may be selected, for example, within the range of 0.0001 mg to 1000 mg per kg body weight in each administration. Alternatively, for example, the dosage for each patient may be selected within the range of 0.001 to 100,000 mg/body. However, the pharmaceutical composition of the present invention is not limited to these doses.

The pharmaceutical compositions of the present invention can be formulated according to conventional methods (for example, Remington's Pharmaceutical Science, latest edition, Mark Publishing Company, Easton, U.S.A), and may also contain pharmaceutically acceptable carriers and additives. Examples include, but are not limited to, surfactants, excipients, coloring agents, perfumes, preservatives, stabilizers, buffers, suspending agents, isotonization agents, binders, disintegrants, lubricants, fluidity promoting agents, and flavoring agents; and other commonly used carriers can be suitably used. Specific examples of the carriers include light anhydrous silicic acid, lactose, crystalline cellulose, mannitol, starch, carmellose calcium, carmellose sodium, hydroxypropyl cellulose, hydroxypropyl methyl cellulose, polyvinylacetal diethylaminoacetate, polyvinylpyrrolidone, gelatin, medium chain fatty acid triglyceride, polyoxyethylene hardened castor oil 60, saccharose, carboxymethyl cellulose, corn starch, inorganic salt, and such.

Furthermore, the present invention provides methods for inducing injury in Claudin 3-expressing cells or methods for suppressing cell proliferation by contacting the Claudin 3-expressing cells with monoclonal antibodies that bind to a Claudin 3 protein. The monoclonal antibodies that bind to a Claudin 3 protein are described above as Claudin 3 protein-binding antibodies contained in the cell proliferation inhibitors of the present invention. Cells to which the anti-Claudin 3 antibodies bind are not particularly limited, as long as the cells express Claudin 3. Preferred Claudin 3-expressing cells of the present invention are cancer cells. Specifically, ovarian cancer, prostate cancer, breast cancer, uterine cancer, liver cancer, lung cancer, pancreatic cancer, stomach cancer, bladder cancer, and colon cancer cells are suitable as Claudin 3-expressing cells of the present invention.

In the present invention "contacting" is accomplished, for example, by adding an antibody to a culture solution containing Claudin 3-expressing cells in a test tube. In this case, the antibody can be added in the form of, for example, a solution or a solid obtained by freeze-drying or the like. When adding the antibody as an aqueous solution, the aqueous solution used may purely contain only the antibody, or the solution may include, for example, the above-mentioned surfactants, excipients, coloring agents, perfumes, preservatives, stabilizers, buffers, suspending agents, isotonization agents, binders, disintegrants, lubricants, fluidity promoting agents, or flavoring agents. The concentration for addition is not particularly limited, but the final concentration in the culture that may be suitably used is preferably in the range of 1 pg/mL to 1 g/mL, more preferably 1 ng/mL to 1 mg/mL, and even more preferably 1 μg/mL to 1 mg/mL.

Furthermore, in another embodiment, "contacting" in the present invention is carried out by administration to a non-human animal to which a Claudin 3-expressing cell has been transplanted into the body, or to an animal carrying cancer cells endogenously expressing Claudin 3. The method of administration may be oral or parenteral administration. The method of administration is particularly preferably parenteral administration, and specifically, the method of administration is, for example, administration by injection, transnasal administration, transpulmonary administration, or transdermal administration. Examples of administration by injection include systemic and local administrations of pharmaceutical compositions, cell proliferation inhibitors and anticancer agents of the present invention by intravenous injection, intramuscular injection, intraperitoneal injection, subcutaneous injection, or such. A suitable administration method may be selected according to the age of the test animal and symptoms. When administering as an aqueous solution, the aqueous solution used may purely contain only the antibody, or the solution may include, for example, the above-mentioned surfactants, excipients, coloring agents, perfumes, preservatives, stabilizers, buffers, suspending agents, isotonization agents, binders, disintegrants, lubricants, fluidity promoting agents, or flavoring agents. The dosage may be selected, for example, within the range of 0.0001 mg to 1000 mg per kg body weight in each administration. Alternatively, for example, the dosage for each patient may be selected within the range of 0.001 to 100,000 mg/body. However, the antibody dose of the present invention is not limited to these doses.

The following method is suitably used as a method for evaluating or measuring cell damage induced by contacting Claudin 3-expressing cells with an anti-Claudin 3 antibody. Examples of a method for evaluating or measuring the cytotoxic activity in a test tube include methods for measuring the above-mentioned antibody-dependent cell-mediated cytotoxicity (ADCC) activity, complement-dependent cytotoxicity (CDC) activity, and such. Whether or not an anti-Claudin 3 antibody has ADCC activity or CDC activity can be measured by known methods (for example, Current protocols in Immunology, Chapter 7. Immunologic studies in humans, Editor, John E. Coligan et al., John Wiley & Sons, Inc., (1993) and the like). For activity measurements, an binding antibody having the same isotype as anti-Claudin 3 antibody but not having any cytotoxic activity can be used as a control antibody in the same manner as the anti-Claudin 3 antibody, and it can be determined that the activity is present when the anti-Claudin 3 antibody shows a stronger cytotoxic activity than the control antibody.

The isotype of an antibody is defined by the sequence of its H chain constant region in the antibody amino acid sequence. The isotype of an antibody is ultimately determined *in vivo* by class switching that arises from genetic recombinations in chromosomes which occur during maturation of antibody-producing B-cells. Difference in isotype is reflected in the difference of physiological and pathological functions of antibodies. Specifically, for example, the strength of cytotoxic activity is known to be influenced by antibody isotype in addition to the expression level of the antigen. Therefore, when measuring the above-described cell damaging activity, an antibody of the same isotype as the test antibody is preferably used as the control.

To evaluate or measure cell damaging activity *in vivo,* for example, Claudin 3-expressing cancer cells are intradermally or subcutaneously transplanted to a non-human test animal, and then a test antibody is intravenously or intraperitoneally administered daily or at the interval of few days, starting from the day of transplantation or the following day. Cytotoxicity can be defined by daily measurement of tumor size. In a similar manner to the evaluation in a test tube, cytotoxicity can be determined by administering a control antibody having the same isotype, and observing that the tumor size in the anti-Claudin 3 antibody-administered group is significantly smaller than the tumor size in the control antibody-administered group. When using a mouse as the non-human test animal, it is suitable to use a nude (nu/nu) mouse whose thymus has been made genetically defective so that its T lymphocyte function is lost. The use of such a mouse can eliminate the participation of T lymphocytes in the test animals when evaluating or measuring the cytotoxicity of the administered antibody.

As a method for evaluating or measuring the suppressive effect on proliferation of Claudin 3-expressing cells by contact with an anti-Claudin 3 antibody, a method of measuring the uptake of isotope-labeled thymidine into cells or the MTT method may be suitably used.

Furthermore, as a method for evaluating or measuring the cell proliferation-suppressing activity *in vivo,* the above-described method for evaluating or measuring the cytotoxic activity *in vivo* can be suitably used.

All prior art references cited herein are incorporated by reference into this description.

## Examples

Herein below, the present invention will be specifically described with reference to the Examples, but it is not to be construed as being limited thereto.

[Example 1] Gene cloning and production of forced expression cells

The genes encoding human Claudin 3, Claudin 1, Claudin 4, Claudin 6, and mouse Claudin 3 were cloned, their mammalian cell expression vectors were constructed, and their forced expression cells were established. PCR primers were designed based on the respective GenBank reference sequences, and PCR amplification of the respective genes was performed using gene-expressing tissue cDNA libraries as template, and the genes of interest were isolated. Sequences of the obtained genes were confirmed by DNA sequencing analysis. The primers and cDNA libraries (Marathon cDNA libraries from Clonetech) used for the gene cloning are shown in Table 1.

[Table 1]

| Species | Gene name (SEQ ID NO:) | Abbreviation | GenBank reference sequence ID | cDNA library for gene cloning | Cloning primer 1 | Cloning primer 2 |
|---|---|---|---|---|---|---|
| Human | claudin3 (SEQ ID NO:1) | hCLDN3 | NM_ 001306 | Human kidney | 5'-CGGCCACCATGTCCATGGGCCTGGAGATCA-3' (SEQ ID NO:119) | 5'-GTCTGTCCCTTAGACGTAGTCCTTGCGGTC-3' (SEQ ID NO:120) |
| Human | claudin1 (SEQ ID NO:5) | hCLDN1 | NM_ 021101 | Human liver | 5'-ATGGCCAACGGGGGGCTGCAGCTGTTGGGC-3' (SEQ ID NO:123) | 5'-TGTGTCACACGTAGTCTTTCCCGCTGGAAG-3' (SEQ ID NO:124) |
| Human | claudin4 (SEQ ID NO:7) | hCLDN4 | NM_ 001305 | Human fetal lung | 5'-GAACAATGGCCTCCATGGGGCTACAGG-3' (SEQ ID NO:125) | 5'-AGGAGGGTGGACTCTGTTCTTGCTAGCAG-3' (SEQ ID NO:126) |
| Human | claudin6 (SEQ ID NO:9) | hCLDN6 | NM_ 021195 | Human fetal lung | 5'-CATGGCCTCTGGCGGAATGGAGATCCT-3' (SEQ ID NO:127) | 5'-CCCAAAGCTGTTGGGCACTGCCACTTC-3' (SEQ ID NO:128) |
| Mouse | claudin3 (SEQ ID NO:3) | mCLDN3 | NM_ 009902 | Mouse liver | 5'-GCGAATTCCACCATGTCCATGGGCCTGGAGATCA-3' (SEQ ID NO:121) | 5'-GCGATATCTGTCCTCTTCCAGCCTAGCAAGCAG-3' (SEQ ID NO:122) |

The obtained genes were inserted into a mammalian expression verctor in which a gene was transcribed under the mouse CMV promoter. The nucleotide séquences of the inserted cDNAs used for the construction of expression vectors are shown in SEQ ID NOs: 1, 3, 5, 7, and 9. All of these expression vectors, except for the vectors for human and mouse Claudin 3, have a nucleotide sequence encoding a FLAG tag attached to the C terminus of the recombinant proteins. The expression vectors were introduced into the cell lines listed below by the electroporation method. Ba/F3 is a mouse lymphocyte-derived cancer cell line. The other, DG44 is a Chinese hamster ovary (CHO) cell-derived dihydrofolate reductase-deficient (dhfr$^-$) cell line. The respective transformed cells were selected on Geneticin (Invitrogen) resistance, which is conferred by the expression vectors.

Ba/F3 (RIKEN Biosource Center, Cell No. RCB0805)

DG44 (Invitrogen, 12613014)

The presence or absence of recombinant protein expression in Geneticin-resistant cell clones was judged by the SDS-PAGE/Western blotting method. The human Claudin 1, Claudin 4, and Claudin 6 proteins were detected by an anti-FLAG M2 antibody (Sigma), of which the C terminal FLAG expression tags were added by genetic engineering . The protein expression of human and mouse Claudin 3 was detected with an anti-Claudin 3 antibody (Zymed, 34-1700). Clones that showed highest expression level of each protein were selected, and the cell lines were cultured, maintained, and used for subsequent experiments.

[Example 2] Establishment of anti-Claudin 3 monoclonal antibody hybridomas

Mice were immunized by the DNA immunization method using the Helios gene gun system (Bio-Rad) to establish hybridomas producing anti-Claudin 3 monoclonal antibodies.

The expression vectors used for DNA immunization were constructed as follows. A cDNA of human Claudin 3 was amplified by PCR from a kidney cDNA library (Clontech). The PCR was conducted by using an LA Taq DNA polymerase reaction solution (Takara) with the cDNA amplification primers, cloning primer 1 and cloning primer 2 (shown in Table 1). The amplified cDNA fragments were cloned into the pGEM-T Easy vector, and the nucleotide sequences were determined. A cDNA fragment containing Claudin 3 was excised using EcoRI, and this fragment was inserted into the EcoRI site of pMC, which is a mammalian expression vector, to obtain an expression vector (full-length human Claudin 3 expression vector) for DNA immunization. The nucleotide sequence of the full-length human Claudin 3 expression vector is shown in SEQ ID NO: 159. In the nucleotide sequence of SEQ ID NO: 159, the nucleotide sequence of positions 836 to 1498 encodes the amino acid sequence of Claudin 3.

The cartridge tubing was coated with gold-DNA (full length human Claudin 3 expression vector) particles according to the Helios gene gun operation manual. 50 mg of 1.0-μm gold particles were weighed out, and suspended by mixing in 0.1 mL of 0.05 M spermidine solution. 0.1 mL of 1 mg/mL plasmid solution was added to this, and then vortexed. Subsequently, 0.1 mL of 1 M $CaCl_2$ was added, and this was left to stand for ten minutes. After brief centrifugation, the supernatant was removed, and the pellet was suspended in ethanol, and then this was centrifuged. After repeating the ethanol dehydration step three times, this was ultimately suspended in 6 mL of 0.05 mg/mL polyvinylpyrollidone/ethanol solution. This solution was drawn into the tubing for coating, and the tubing was coated, dried, and cut into 0.5-inch-long segments using a tube cutter.

DNA immunization was performed on four- to five-weeks-old mice (Charles River Japan, MRL/MpJ-Tnfrsf6$^{lpr}$/Crlj) (approximately 200 psi helium pressure) for one to three times per week, and the anti-Claudin 3 antibody titer in the serum was monitored intermittently during this period. Cells forced to express Claudin 3 (5 x $10^6$ cells/head) were administered intraperitoneally to individuals confirmed to have an increased serum antibody titer. After rearing for two to three days, the spleen was extirpated, and mononuclear cells containing antibody-producing cells were isolated. Spleen-derived cells were mixed with P3-X63Ag8U.1 (ATCC CRL-1597) at an approximately 2:1 ratio, and cell fusion was carried out by gradual addition of PEG 1500 (Roche Diagnostics). RPMI1640 medium (GIBCO BRL) was added carefully to dilute PEG 1500, and then PEG 1500 was removed by centrifugation. Then, the cells were seeded into a 96-well culture plate at 200 μL/well in RPMI1640 medium containing the following components (hereinafter referred to as HAT medium), and cultured at 37°C under 5% $CO_2$ for approximately one week:

10% FBS;
1x HAT media supplement (SIGMA); and
0.5x BM-Condimed H1 Hybridoma cloning supplement (Roche Diagnostics).

After confirming the formation of hybridoma colonies under a microscope, the presence or absence of Claudin 3-binding antibodies in the culture supernatant was screened by a flow cytometry method using cells forced to express Claudin 3. The mouse antibodies bound to the forced expression cells were measured by FACSCalibur (Becton Dickinson) using an FITC-labeled goat anti-mouse IgG antibody (Beckman Coulter) as secondary antibodies. The selective binding of the antibodies to Claudin 3 was judged by comparison of the forced expression cells and the non-recombinant parental cells, and the hybridomas from positive wells were cloned by the limiting dilution method.

Not many hybridomas producing Claudin 3-binding antibodies could be obtained from mice immunized by intraperitoneal administration of cells forced to express Claudin 3. In contrast, hybridomas producing Claudin 3-binding antibodies could be efficiently obtained from mice subjected to DNA immunization followed by intraperitoneal administration of cells forced to express Claudin 3.

The antibody isotype of the hybridoma clones was determined using the Mouse Monoclonal Antibody Isotyping Kit (Roche Diagnostics).

For purification of monoclonal antibodies, hybridomas were cultured in a HAT medium supplemented with Ultra low IgG FBS (Invitrogen), and the culture supernatants were harvested. Antibodies belonging to the IgG1, IgG2a, and IgG2b subtypes were purified using HiTrap Protein G HP (Amersham Biosciences) according to the manufacturer's instructions. Antibodies belonging to the IgG3 and IgM subtypes were purified using a Protein L Agarose (Sigma) column under conditions similar to those for Protein G. The solvent of the elution fractions was replaced with PBS using a PD-10 column (Amersham Bioscience). Then, the purified antibodies were concentrated by ultrafiltration, and stored at 4°C. The antibody concentration was determined by the Bradford method using mouse IgG as the standard.

[Example 3] Analysis of the binding specificity of the monoclonal antibodies

24 Claudin family genes are present on the human chromosome. Claudin 4 is highly homologous to Claudin 3 in the full-length amino acid sequence. To characterize the redundancy and specificity of cell surface epitopes recognized by monoclonal antibodies, a sequence alignment and clustering diagrams of the putative extracellular sequences of Claudin 3 and the corresponding sequences of highly homologous family molecules were depicted (Fig. 1). The family molecule showing the highest identity in the extracellular loop 1 is Claudin 4 (48 residues out of 51 residues are identical). In the second place, Claudin 6 and Claudin 9 have a high identity to Claudin 3 (41 residues out of 51 residues are identical). Compared to the extracellular loop 1 region, the sequences in the extracellular loop 2 region is not conserved (15 residues out of 22 residues are identical between Claudin 6 and Claudin 8). Conservation between the sequences of human and mouse Claudin 3 is high; 46 out of 51 residues are identical in the extracellular loop 1 region, and 22 out of 23 residues are identical in the extracellular loop 2 region (Fig. 2).

To verify the specificity of monoclonal antibodies and categorize the epitopes, the binding reactivity to cells forced to express Claudin 3 and cells forced to express the following highly homologous Claudin family molecules was evaluated by the flow cytometry method:

mouse Claudin 3;
human Claudin 1;
human Claudin 4; and
human Claudin 6.

A monoclonal antibody was added to the respective forced expression cells, and then incubated at 4°C for 30 minutes. After incubation, the cells were washed once with a PBS solution containing 1% fetal bovine serum, then a 150-fold dilution of an FITC-goat anti-mouse IgG (H+L) antibody (Beckman Coulter) was added, and this was incubated at 4°C for 30 minutes. The amount of antibodies bound per cell was measured using FACSCalibur, and the X geometric mean value, which is the geometric mean of the cell fluorescence intensity, was calculated using the accessory CellQuest Pro analysis software of FACSCalibur. The results are summarized in Table 2.

[Table 2]

| | Isotype | hCLN3/DG44 | | | MCF7 | | hCLN3 | mCLN3 | hCLN1 | hCLN4 | hCLN6 | Ba/F3 |
|---|---|---|---|---|---|---|---|---|---|---|---|---|
| | | 5μg/ml | 1μg/ml | 0.1μg/ml | 5μ/ml | 1μg/ml | 2μg/ml | | | | | |
| CDN01 | IgG2b/IgK | 456 | 176 | 27 | 24 | 10 | 197 | 23 | 9 | 10 | 8 | 10 |
| CDN02 | IgG2b/IgK | 1757 | 531 | 66 | 313 | 143 | 1464 | 15 | 14 | 137 | 8 | 10 |
| CDN03 | IgM/IgK | 724 | 542 | 94 | 35 | 29 | 474 | 348 | 10 | 17 | 9 | 11 |
| CDN04 | IgG1/IgK | 667 | 180 | 28 | 130 | 47 | 366 | 16 | 11 | 14 | 9 | 8 |
| CDN05 | IgM/IgK | 169 | 70 | 18 | 71 | 46 | 59 | 119 | 9 | 148 | 11 | 10 |
| CDN07 | IgG1/IgK | 351 | 109 | 26 | 79 | 34 | 238 | 76 | 12 | 14 | 9 | 10 |
| CDN08 | IgG2b/IgK | 2014 | 809 | 105 | 321 | 125 | 1380 | 12 | 14 | 47 | 8 | 10 |
| CDN16 | IgG2b/IgK | 2482 | 928 | 125 | 361 | 125 | 1975 | 1270 | 13 | 11 | 9 | 10 |
| CDN 17 | IgM/IgK | 866 | 567 | 88 | 299 | 173 | 1028 | 342 | 9 | 156 | 10 | 11 |
| CDN24 | IgM/IgK | 1129 | 694 | 101 | 382 | 286 | 1114 | 502 | 18 | 293 | 13 | 12 |
| CDN27 | IgG2a/IgK | 2696 | 1793 | 308 | 310 | 202 | 3344 | 50 | 42 | 302 | 25 | 48 |
| CDN28 | IgG1/IgK | 1990 | 1452 | 275 | 186 | 175 | 1427 | 82 | 12 | 27 | 9 | 11 |
| CDN29 | IgG1/IgK | 1643 | 851 | 126 | 87 | 60 | 1689 | 65 | 11 | 12 | 10 | 11 |
| CDN30 | IgG2a/IgK | 1663 | 615 | 93 | 58 | 29 | 1718 | 176 | 27 | 38 | 19 | 30 |
| CDN31 | IgG2a/IgK | 1917 | 649 | 86 | 231 | 101 | 1598 | 31 | 35 | 174 | 22 | 43 |
| CDN32 | IgG1/IgK | 951 | 354 | 63 | 80 | 49 | 818 | 27 | 11 | 11 | 10 | 10 |
| CDN33 | IgG3/IgK | 547 | 406 | 65 | 77 | 45 | 576 | 17 | 11 | 12 | 9 | 11 |
| CDN35 | IgG2a/IgK | 2297 | 869 | 120 | 380 | 176 | 2147 | 1310 | 69 | 59 | 36 | 44 |
| CDN36 | IgG1/IgK | 986 | 384 | 68 | 46 | 18 | 909 | 60 | 11 | 11 | 11 | 11 |
| CDN37 | IgG2a/IgK | 1134 | 544 | 90 | 52 | 21 | 388 | 44 | 26 | 66 | 22 | 31 |
| CDN38 | IgG3/IgK | 258 | 215 | 207 | 61 | 63 | 142 | 115 | 7 | 74 | 9 | 8 |
| Control | | 12 | | | 9 | | 28 | 12 | 10 | 9 | 8 | 13 |

When the antibodies were added to DG44 forced to express Human Claudin 3 at a final concentration of 5,1, or 0.1 μg/mL, the amounts of bound antibodies increased in a dose dependent manner for all of the antibodies. The cross reactivity for human Claudin 3, human Claudin1, human Claudin 4, human Claudin 6, and mousse Claudin 3 forcedly expressed in Ba/F3 was evaluated using at a final antibody concentration of 2 μg/mL. All of the isolated antibodies bound more strongly to cells forced to express human Claudin 3, as compared to the Ba/F3 cells which is a parental cell line accommodating the forced expression. On the other hand, all of the antibodies hardly bound to cells forced to express human Claudin 1 or human Claudin 6, which have lower sequence identity to Claudin 3. The antibodies that showed high and selective affinity to Claudin 3 are listed below:

CDN08, CDN16, CDN14, CDN28, CDN29,
CDN30, CDN32, CDN33, and CDN36.

CDN16 and CDN35 showed nearly equivalent high affinity to human and mouse Claudin 3. Specific antibodies recognizing a common epitope among animal species may be useful as tools for studying the difference between efficacy and toxicity in pathologic model animals. That is, when an antibody that specifically recognizes an epitope whose sequence is conserved among animal species is administered to pathologic model animals, the pharmacokinetics of the antibody is expected to show similar behavior in the animal species for which the disease is treated. CDN02, CDN05, CDN17, CDN24, CDN27, and CDN31, which bind to human Claudin 3, were also shown to bind to human Claudin 4, and this suggests that the antibodies recognize a sequence structure that is common or very similar between the two proteins.

The affinity of the antibodies to the MCF7 breast cancer cell line (ATCC, HTB-22), which endogenously expresses Claudin 3, was evaluated by the flow cytometry method. While the antibody concentration-dependent elevation of the binding level was observed as in the forced expression cells, the preference of binding to MCF7 did not necessarily correlate with the result for the forced expression cells (Fig. 3). This suggests that the manner in which the epitopes are exposed could be different between the forced expression cells and the cancer cell line.

[Example 4] Affinity of the monoclonal antibodies to the extracellular loop region sequence peptides

In general, it is said that even when an antibody can be successfully obtained by immunization with a short loop of a multi-transmembrane protein in the form of a linear peptide, such an antibody scarcely binds to the naturally-occurring protein with high affinity. On the other hand, an antibody that binds to a rigid portion in a tertiary structure may hardly bind to a linearized peptide. The isolated monoclonal antibodies bind to Claudin 3 expressed on cells, and their affinity for linarized peptides that correspond to the extracellular loop sequences was evaluated using GST/extracellular loop peptide fusion proteins. The portions predicted to be the extracellular regions of human Claudin 3 are shown below.

Loop 1: the sequence of amino acid residue numbers 30 to 80 in SEQ ID NO: 2
Loop 2: the sequence of amino acid residue numbers 137 to 159 in SEQ ID NO: 2

In the pGEX-4T2 *Escherichia coli* expression vector, an expression unit was engineered so that the GST protein is fused to the N terminus of the loop sequences, and a His tag is attached to the C terminus of the loop sequences. The protein expression was induced in *E. coli*, and the fusion proteins were purified. The amino acid sequences of the loop 1 and loop 2 fusion proteins are shown in SEQ ID NO: 116 and SEQ ID NO: 117, respectively. The loop 1 fusion protein was accumulated in *E. coli* as an insoluble protein. Thus, after disrupting the *E. coli,* the insoluble fraction was collected, solubilized in 7 M urea, and then the protein was purified using a nickel affinity column in the presence of urea. After elution by imidazole, urea was removed by dialysis against 50 mM Tris-HCl (pH 8). Since the loop 2 fusion protein was expressed in a soluble fraction, the fusion protein was purified by glutathione affinity chromatography. Nunc-Immuno plates were coated with the purified fusion proteins. After blocking with a solution containing BSA, the binding reactivity of the monoclonal antibodies was evaluated. An anti-His mouse monoclonal antibody (Santa Cruz) was used as a positive control antibody that binds to the fusion proteins.

After one hour of incubation, the plates were washed, and an alkaline phosphatase-labeled anti-mouse IgG (H+L) antibody was added and reacted. After washing, the amount of antibody bound was measured by adding the Sigma 104 detection reagent (Fig. 4). While the positive control anti-His antibody bound to the fusion proteins on the plates, the anti-Claudin 3 monoclonal antibodies hardly bound to the loop 1 or loop 2 peptide fragment. Some antibodies showed weak binding to the loop 1 and loop 2 fusion proteins, they bound to both the proteins with equivalent affinity, and no binding specificity was observed. The above-mentioned results suggest that all of the antibodies isolated in the present invention bind to a rigid portion of the tertiary structure of the Claudin 3 protein.

Considering that the only previously reported Claudin 3-binding polyclonal antibody was obtained by peptide immunization and peptide affinity purification, it is clear that the mode of binding to the antigen of the reported antibody differs from that of the antibodies isolated in the present invention. It was confirmed that the monoclonal antibodies of the present invention are useful in that the antibodies bind more efficiently to Claudin 3 expressed on cells.

[Example 5] Induction of cytotoxicity by the monoclonal antibodies

The selective complement-dependent cytotoxicity activity of the monoclonal antibodies in Claudin 3-expressing cells was evaluated using a baby rabbit complement. DG44 cells forced to express human Claudin 3 were used as human Claudin 3-expressing cells, and the parental DG44 cells were used as the control. After addition of a purified monoclonal antibody at a final reaction concentration of 5 μg/mL, the cells were incubated at 4°C for 30 minutes. Then, Baby Rabbit Complement (Cederlane, Cat. No. CL3441) was added at a final concentration of 1%, and this was incubated at 37°C under 5% $CO_2$ for 90 minutes.

After incubation, 7-aminoactinomycin D (7-AAD, Invitrogen), which is a DNA-binding fluorescent reagent, was added at a final concentration of 1 μg/mL, and this was left to stand in the dark for ten minutes. After centrifugation, the supernatant was removed, and the cells were suspended in PBS containing 1% fetal bovine serum, and the fluorescence intensity of the cells stained was measured using a flow cytometer. The instrument and gating measurement conditions were set in advance, so that the percentage of positive cells stained with 7-AAD will be 5% or less under the conditions without addition of an antibody or a complement. The complement-dependent cytotoxicity activity of the antibodies was measured.

As in the case without antibody addition, cell injury was hardly induced in the parental DG44 cells by the antibodies. In contrast, all of the antibodies, except for the antibodies of the IgG1 subtype, showed cytotoxic activity against DG44 cells forced to express human Claudin 3 (Fig. 5). Although the antibody subtype of CDN28 and CDN32 is IgG1, these antibodies induced cytotoxicity. As described above, many of the antibodies isolated in the present invention were shown to induce antigen expression-dependent and complement-dependent cytotoxicity.

The complement-dependent cytotoxicity activity of the monoclonal antibodies against the MCF7 breast cancer cell line was evaluated by the chromium release method. RPMI1640 medium (Invitrogen) containing 10% fetal bovine serum and 10 μg/mL human insulin was used to maintain MCF7. MCF7 cells were seeded onto a 96-well plate, and cultured overnight. Then, Chromium-51 (Code No. CJS4, Amersham Biosciences) was added, and the cells were incubated for a few more hours. After washing the cells with the medium, fresh medium was added. Then, the anti-Claudin 3 monoclonal antibodies and the control mouse IgG2a antibody were added to the wells. The final concentration of the antibodies was adjusted to 10 μg/mL. Subsequently, a baby rabbit complement was added at a final concentration of 2%, and then the plate was left to stand in a 5% carbon dioxide gas incubator at 37°C for 1.5 hours. Thereafter, the plate was centrifuged (1000 rpm for five minutes at 4°C), 100 μL of the supernatant was collected from each well, and its radioactivity was measured using a gamma counter (1480 WIZARD 3", Wallac). The specific chromium release rate was determined based on the following equation:

$$\text{Specific chromium release rate (\%)} = (A - C) \times 100/(B - C)$$

where A, B, and C show values for the following:

A- the radioactivity (cpm) in each well;
B- the mean value of radioactivity (cpm) in wells where 100 μL of 2% NP-40 solution (Nonidet P-40, Code No. 252-23, Nacalai Tesque) was added to 100 μL of cells; and
C- the mean value of radioactivity (cpm) in wells where 100 μL of the medium was added to 100 μL of cells.

The measurements were conducted in triplicate for each experimental condition, and the mean value and standard deviation were calculated for the specific chromium release rate (Fig. 6). The following monoclonal antibodies showed strong complement-dependent cytotoxicity activity against MCF7:

CDN27, CDN31, CDN35, CDN02,
CDN08, CDN16, CDN17, and CDN24.

The strength of cytotoxicity activity closely correlated with the amount of bound antibody as measured by the flow cytometry method. On the other hand, the control mouse IgG2a antibody did not show complement-dependent cytotoxicity activity at the same concentration.

Using MCF7 cells as the target, the antibody-dependent cytotoxicity activity was measured by the chromium release method. Cells were cultured in a 96-well flat-bottomed plate. After reaction with Chromium-51, the cells were washed with RPMI1640 medium, and 100 μL of fresh medium was added. Then, the anti-Claudin 3 monoclonal antibodies and the control (no antibody) were added at a final concentration of 0.1 μg/mL. Subsequently, a solution containing effector cells, the number of which is approximately 50-times that of MCF7, was added to each well, and the plate was incubated at 37°C in a 5% carbon dioxide gas incubator. For the effector cells, spleen cells of C3H/HeNCrlCrlj mice (Charles River Japan) cultured in a medium containing 50 ng/mL of recombinant interleukin-2 (Cat. No. 200-02, Pepro-Tech) were used. After letting the plate stand for six hours, the specific chromium release rate was measured, and the mean and standard deviation were calculated (Fig. 7).

Compared to no addition of antibody and addition of the control antibody (IgG2a subtype), addition of CDN04, CDN27, CDN35, and CDN16 induced chromium release. Thus, these antibodies were confirmed to have antibody-dependent cell-mediated cytotoxicity activity against Claudin 3-expressing cells.

[Example 6] Cloning of antibody variable regions and production of recombinant antibodies

cDNAs encoding the antibody variable regions were cloned using the SMART RACE cDNA Amplification kit (Clonetech), and the nucleotide sequences were determined. Total RNAs were purified using RNeasy Mini (Qiagen) from cultured hybridoma cells. From this RNA, cDNAs were synthesized according to the SMART RACE cDNA Amplification Kit manual, and the cDNAs of the antibody gene variable regions were amplified by PCR using subtype-specific primers. The subtype-specific primer sequences used for the amplification are shown in Table 3.

[Table 3]

| Antibody subtype | Primer sequence |
|---|---|
| IgG1 | 5'-CCATGGAGTTAGTTTOGGCAGCAGATCC-3' (SEQ ID NO: 129) |
| IgG2a | 5'-CAGGGGCCAGTGGATAGACCGATG-3' (SEQ ID NO: 130) |
| IgG2b | 5'-CAGGGGCCAGTGGATAGACTGATG-3' (SEQ ID NO: 131) |
| IgG3 | 5'-ATGTGTCACTGCAGCCAGGGACCAA-3' (SEQ ID NO: 188) |
| IgK | 5'-GGGACCTCCAGATGTTAACTGCTCACT-3' (SEQ ID NO: 132) |
| IgL | 5'-TCGAGCTCTTCAGAGGAAGGTGGAAAC-3' (SEQ ID NO: 133) |

The fragments were amplified using Takara Ex Taq DNA polymerase (Takara), and cloned into the pGEM-T Easy vector, and the nucleotide sequences were determined. Recombinant antibody expression vectors were constructed from the isolated antibody variable region sequences. In brief, individually-cloned heavy-chain and light-chain variable region sequences were linked in translational frame with the human antibody IgG1 constant region and human Igκ constant region sequences, respectively. In expression vectors constructed, the mouse-human chimeric antibody genes are transcribed under the mouse CMV promoter. Cells transiently-expressing recombinant antibodies were obtained by introducing the expression vectors into COS7 cells. Flow cytometric data using the supernatants obtained after two days of culturing and anti-human IgG (H+L)-FITC as the secondary antibody demonstrated that the recombinant antibodies bind specifically to cells forced to express Claudin 3 (Fig. 8).

[Example 7] Analysis of the binding of the monoclonal antibodies to the loops displayed on cells

As described above, the monoclonal antibodies of the present invention do not show affinity to the GST fusion protein, comprising linearized peptides that are putative extracellular loop. To obtain information on the epitopes of these monoclonal antibodies, each antibody was analyzed to determine whether it binds to loop 1 or loop 2. The monoclonal antibodies isolated in the present invention hardly bound to human Claudin 1. A chimeric molecule carrying loop 1 of Claudin 3 and loop 2 of Claudin 1 (CLD1/3), and a chimeric molecule carrying loop 1 of Claudin 1 and loop 2 of Claudin 3 (CLD3/1) were expressed in cells, and the affinity of the antibodies to the cells was evaluated by flow cytometry. If an antibody binds to CLD1/3-expressing cells but not to CLD3/1-expressing cells, this means that the antibody binds to loop 2. If an antibody binds to CLD3/1-expressing cells but not to CLD1/3-expressing cells, this means that the antibody binds to loop 1.

Comparison of the amino acid sequences of Claudin 3 and Claudin 1 shows that a common sequence motif "FLLA" is present in the third putative transmembrane region. This portion was used as the boundary to design chimeric constructs, in which the amino acid sequences before and after the boundary derived from different proteins are linked together. Information on the amino acid sequences of the designed chimeric molecules is set forth below:

CLD1/3 protein: positions 1-127 of the Claudin 1 amino acid sequence and positions 126-220 of the Claudin 3 amino acid sequence
CLD3/1 protein: positions 1-125 of the Claudin 3 amino acid sequence and positions 128-211 of the Claudin 1 amino acid sequence
The nucleotide sequence and amino acid sequence of each chimeric molecule are shown in the following sequence ID numbers:

| | Nucleotide sequence | Amino acid sequence |
|---|---|---|
| CLD1/3 protein: | SEQ ID NO: 160 | SEQ ID NO: 161 |
| CLD3/1 protein: | SEQ ID NO: 162 | SEQ ID NO: 163 |

In brief, the genes were constructed as follows. Using the Claudin 1 and 3 cDNA sequences as templates, partial gene fragments were amplified by PCR, and the gene fragments were linked by PCR assembly to produce a gene of chimeric molecule. The genes were inserted in translational frame into a mammalian cell expression vector designed for addition of a FLAG tag to the C terminus. The vector was introduced into Ba/F3 cells to obtain drug-resistant cell clones. Protein expression in the drug-resistant clone was confirmed by Western blotting using an anti-FLAG antibody, and chimeric molecule-expressing cells were established by selecting a clone with high expression level.

Epitope analysis of the monoclonal antibodies using the chimeric molecule-expressing cells showed unexpected results. Many of the monoclonal antibodies bound strongly to Claudin 3 (CLD3/3) having the naturally-occurring amino acid sequence. Specifically, in the results of FACS shown in Fig. 9, clear peaks with fluorescence signal were detected. On the other hand, most of the monoclonal antibodies did not bind at all to both the chimeric protein-expressing cells used in the experiment. Peaks with low fluorescence were observed for some of the antibodies, indicating only weak binding to the cells. For example, weak binding was observed between CDN16 and CLD1/3, and between CDN35 and CLD1/3. A similar tendency was also observed for anti-Claudin 3 mouse antiserum. It was presumed that the above results were not due to a screening bias at the establishment of hybridoma, but that both loop 1 and loop 2 are necessary for an antibody to strongly bind to Claudin 3 expressed on cells.

[Example 8] Production of cell lines stably expressing an anti-Claudin 3 chimeric antibody

DG44 cells were transformed with a human chimeric antibody expression vector by the electroporation method. Recombinant cell clones were selected based on the geneticin resistance acquired by a selection marker present on the human chimeric antibody expression vector. The antibodies in the culture supernatant of the recombinant clones was quantified by sandwich ELISA using anti-human antibodies, and recombinant antibody-expressing cells were selected. Human chimeric antibodies were purified from the culture supernatant of the selected recombinant cells using a HiTrap Protein A column (Amersham Bioscience) according to the attached manual.

The affinity of the human chimeric antibodies to DG44 cells forced to express Claudin 3 and Ba/F3 cells forced to express Claudin 3 was evaluated by flow cytometry. The chimeric antibodies were added to and reacted with the cells forced to express Claudin 3, and then the bound chimeric antibodies were detected using anti-human IgG (H+L)-FITC. As shown in Fig. 11, remarkable shifts by chimeric antibody addition were observed in the histograms, and thus the chimeric antibodies were confirmed to bind to Claudin 3.

[Example 9] Inhibition of colony formation in soft agar and cell migration by addition of anti-Claudin 3 antibodies

Agarwal and others reported that overexpression of Claudin 3 and Claudin 4 is involved in the enhancement of survival capacity and acquisition of invasion ability of ovarian cancer cells, from analyses using forced expression of Claudin 3 and 4 and small interfering RNAs against Claudin 3 and 4 (Agarwal et al. (2005) Cancer Res 65, 7378-7385). On the other hand, Michl and others reported that overexpression of Claudin 4 suppresses the metastatic and infiltration ability of pancreatic cancer cells (Michl et al. (2003) Cancer Res 63, 6265-6271).

The effect of the presence or absence of the expression, or increase or decrease in the expression was evaluated in the above-mentioned reports. No report has shown that cell functions can be modified by an antibody that binds to a Claudin 3 protein. To see whether the survival capacity or invasion ability of cancer cells can be altered by binding of anti-Claudin 3 antibodies, the effect of antibody addition on the ability of MCF7 cells to form colonies in soft agar and to migrate was assessed.

The effect of antibody addition on colony formation in soft agar was evaluated using CytoSelect 96-well In Vitro Tumor Sensitivity Assay (Cell Biolabs, Inc.). 5000 MCF7 cells per well were seeded into soft agar together with a mouse antibody, and cultured for seven days at 37°C under 5% $CO_2$. After culturing, the number of cells was quantified by the MTT method (Fig. 12). Colony formation was suppressed by addition of anti-Claudin 3 antibodies, and this effect was particularly strong with CDN04.

The effect of antibody addition on cell motility was evaluated by the following method (wound-healing assay). MCF7 cells were seeded into a 12-well plastic plate, and culturing was continued until the density of cells capable of attached growth became saturated. The cell monolayer was linearly scratched with the edge of a pipette tip. After replacement of medium, the antibodies were added at a final concentration of 10 μg/mL, and the cells were continuously cultured for four days. After incubation, cells migrated to cover the wounded region in the control wells without antibody addition. On the other hand, in the wells to which the CDN04 antibody (10 μg/mL) was added, cell migration to the wounded region was hardly observed (Fig. 13). No significant inhibition of cell migration was observed in the wells to which CDN16, CDN27, CDN28, CDN35, or CDN38 was added.

This example demonstrates for the first time that Claudin 3-binding antibodies can regulate cellular functions such as anchorage-independent proliferation and cell migration, which are characteristics of cancer cells.

Industrial Applicability

The present invention provides anti-Claudin 3 monoclonal antibodies. Since Claudin 3 shows high sequence identity among species, it was not easy to obtain such antibodies by conventional immunization methods. Therefore, it is highly significant that the present invention provides Claudin 3-recognizing antibodies. In particular, in a preferred embodiment, the monoclonal antibodies provided by the present invention can bind to Claudin 3 expressed on the cell surface, but no substantial reactivity to linear peptides comprising amino acid sequences of the extracellular domains of Claudin 3 was observed. That is, the monoclonal antibodies of the present invention are antibodies that cannot be obtained by domain peptide immunization using the amino acid sequences of the extracellular domains.

Many of the molecules belonging to the Claudin family are structurally similar. In addition, since the lengths of the extracellular domains are short, it was expected to be difficult to obtain antibodies that can distinguish individual Claudin family molecules expressed on cell surface. However, in a preferred embodiment, the monoclonal antibodies provided by the present invention can immunologically distinguish between Claudin 3 and Claudin 6. Among the 51 residues of the amino acid sequence constituting extracellular loop 1 of Claudin 3, 41 residues are shared with the amino acid sequence of extracellular loop 1 of Claudin 6. It can be said that antibodies that can immunologically distinguish molecules sharing high identity as such are antibodies with excellent specificity.

Therefore, the present invention provides antibodies that can specifically recognize and bind to Claudin 3 expressed on the surface of cancer cells. Antibodies of the present invention can detect cancers that overexpress Claudin 3. For example, the expression of Claudin 3 has been shown to be elevated in ovarian cancer, prostate cancer, breast cancer, uterine cancer, liver cancer, lung cancer, pancreatic cancer, stomach cancer, bladder cancer, colon cancer, and such. Thus, monoclonal antibodies of the present invention are useful for diagnosis of cancers that have enhanced expression of Claudin 3, such as ovarian cancer, prostate cancer, breast cancer, uterine cancer, liver cancer, lung cancer, pancreatic cancer, stomach cancer, bladder cancer, and colon cancer.

Furthermore, in a preferred embodiment, the monoclonal antibodies of the present invention were confirmed to show cytotoxic action against Claudin 3-expressing cells. Specifically, for example, monoclonal antibodies having CDC activity and ADCC activity against breast cancer are provided. Furthermore, in a preferred embodiment, the monoclonal antibodies of the present invention recognize not only Claudin 3 but also Claudin 4. The expression of the Claudin 4 gene was reported to be elevated at chemotherapeutic agent-resistant recurrent sites in uterine cancer patients.

Therefore, the monoclonal antibodies of the present invention were shown to be useful for treatment of cancers that overexpress Claudin 3 or Claudin 4. Furthermore, the monoclonal antibodies of the present invention retain the activity to bind to Claudin 3 even after chimerization by substituting the constant-region sequences with human-derived amino acid sequences. This confirms that the monoclonal antibodies provided by the present invention can be chimerized and made into cancer therapeutic antibodies that can be administered to humans. More specifically, monoclonal antibodies of the present invention are useful for treatment of cancers that have enhanced expression of either one or both of Claudin 3 and Claudin 4, such as ovarian cancer, prostate cancer, breast cancer, uterine cancer, liver cancer, lung cancer, pancreatic cancer, stomach cancer, bladder cancer, and colon cancer.

## Claims

**1.** A monoclonal antibody that binds to a Claudin 3 protein.

**2.** The monoclonal antibody of claim 1, wherein the antibody binds to a protein expressed on the cell membrane and comprising the amino acid sequence of SEQ ID NO: 2.

**3.** The monoclonal antibody of claim 2, wherein the antibody does not substantially cross-react with a peptide comprising the amino acid sequence of positions 30 to 80 or positions 137 to 159 in the amino acid sequence of SEQ ID NO: 2.

**4.** The monoclonal antibody of any one of claims 1 to 3, wherein the antibody binds to a protein expressed on the cell membrane and comprising the amino acid sequence of SEQ ID NO: 4.

**5.** The monoclonal antibody of any one of claims 1 to 3, wherein the antibody binds to a protein expressed on the cell membrane and comprising the amino acid sequence of SEQ ID NO: 8.

**6.** An antibody that binds to a protein expressed on the cell membrane and comprising the amino acid sequence of any one of SEQ ID NOs: 2, 4, and 8, by recognizing a conformation formed by two extracellular loops of the protein.

**7.** The antibody of claim 6, wherein the antibody does not substantially cross-react with a peptide comprising the amino acid sequence of positions 30 to 80 or positions 137 to 159 in the amino acid sequence of SEQ ID NO: 2.

**8.** The antibody of claim 6 or 7, wherein the antibody is a monoclonal antibody.

**9.** The antibody of any one of claims 1 to 8, wherein the antibody has cytotoxic activity.

**10.** The antibody of claim 9, wherein the cytotoxic activity is ADCC activity.

**11.** The antibody of claim 9, wherein the cytotoxic activity is CDC activity.

**12.** The antibody of any one of claims 1 to 11, wherein a chemotherapeutic agent or a toxic peptide is bound to the antibody.

**13.** An antibody that binds to a Claudin 3 protein, wherein a cytotoxic substance selected from the group consisting of a chemotherapeutic agent, toxic peptide, and radioisotope is bound to the antibody.

**14.** The antibody of any one of claims 1 to 13, which is an antibody described in any of (1) to (61) below:

(1) an antibody comprising an H chain having the amino acid sequence of SEQ ID NO: 12 as CDR1, the amino acid sequence of SEQ ID NO: 14 as CDR2, and the amino acid sequence of SEQ ID NO: 16 as CDR3;
(2) an antibody comprising the H chain of (1), wherein the H chain has the amino acid sequence of positions 139 to 462 in the amino acid sequence of SEQ ID NO: 20 as CH;
(3) an antibody comprising the H chain of (1), wherein the H chain has the amino acid sequence of SEQ ID NO: 22 as CH;
(4) an antibody comprising an L chain having the amino acid sequence of SEQ ID NO: 24 as CDR1, the amino acid sequence of SEQ ID NO: 26 as CDR2, and the amino acid sequence of SEQ ID NO: 28 as CDR3;
(5) an antibody comprising the L chain of (4), wherein the L chain has the amino acid sequence of positions 135 to 240 in the amino acid sequence of SEQ ID NO: 32 as CL;
(6) an antibody comprising the L chain of (4), wherein the L chain has the amino acid sequence of SEQ ID NO:

34 as CL;
(7) an antibody comprising the H chain of (1) and the L chain of (4);
(8) an antibody comprising the H chain of (2) and the L chain of (5);
(9) an antibody comprising the H chain of (3) and the L chain of (6);
(10) an antibody having one or more amino acid substitutions, deletions, additions, and/or insertions in the antibody of any of (1) to (9), which has equivalent activity as the antibody of any of (1) to (9);
(11) an antibody comprising an H chain having the amino acid sequence of SEQ ID NO: 36 as CDR1, the amino acid sequence of SEQ ID NO: 38 as CDR2, and the amino acid sequence of SEQ ID NO: 40 as CDR3;
(12) an antibody comprising the H chain of (11), wherein the H chain has the amino acid sequence of positions 140 to 476 in the amino acid sequence of SEQ ID NO: 44 as CH;
(13) an antibody comprising the H chain of (11), wherein the H chain has the amino acid sequence of SEQ ID NO: 22 as CH;
(14) an antibody comprising an L chain having the amino acid sequence of SEQ ID NO: 46 as CDR1, the amino acid sequence of SEQ ID NO: 48 as CDR2, and the amino acid sequence of SEQ ID NO: 50 as CDR3;
(15) an antibody comprising the L chain of (14), wherein the L chain has the amino acid sequence of positions 133 to 238 in the amino acid sequence of SEQ ID NO: 54 as CL;
(16) an antibody comprising the L chain of (14), wherein the L chain has the amino acid sequence of SEQ ID NO: 34 as CL;
(17) an antibody comprising the H chain of (11) and the L chain of (14);
(18) an antibody comprising the H chain of (12) and the L chain of (15);
(19) an antibody comprising the H chain of (13) and the L chain of (16);
(20) an antibody having one or more amino acid substitutions, deletions, additions, and/or insertions in the antibody of any of (11) to (19), which has equivalent activity as the antibody of any of (11) to (19);
(21) an antibody comprising an H chain having the amino acid sequence of SEQ ID NO: 56 as CDR1, the amino acid sequence of SEQ ID NO: 58 as CDR2, and the amino acid sequence of SEQ ID NO: 60 as CDR3;
(22) an antibody comprising the H chain of (21), wherein the H chain has the amino acid sequence of positions 137 to 471 in the amino acid sequence of SEQ ID NO: 64 as CH;
(23) an antibody comprising the H chain of (21), wherein the H chain has the amino acid sequence of SEQ ID NO: 22 as CH;
(24) an antibody comprising an L chain having the amino acid sequence of SEQ ID NO: 66 as CDR1, the amino acid sequence of SEQ ID NO: 68 as CDR2, and the amino acid sequence of SEQ ID NO: 70 as CDR3;
(25) an antibody comprising the L chain of (24), wherein the L chain has the amino acid sequence of positions 133 to 238 in the amino acid sequence of SEQ ID NO: 74 as CL;
(26) an antibody comprising the L chain of (24), wherein the L chain has the amino acid sequence of SEQ ID NO: 34 as CL;
(27) an antibody comprising the H chain of (21) and the L chain of (24);
(28) an antibody comprising the H chain of (22) and the L chain of (25);
(29) an antibody comprising the H chain of (23) and the L chain of (26);
(30) an antibody having one or more amino acid substitutions, deletions, additions, and/or insertions in the antibody of any of (21) to (29), which has equivalent activity as the antibody of any of (21) to (29);
(31) an antibody comprising an H chain having the amino acid sequence of SEQ ID NO: 76 as CDR1, the amino acid sequence of SEQ ID NO: 78 as CDR2, and the amino acid sequence of SEQ ID NO: 80 as CDR3;
(32) an antibody comprising the H chain of (31), wherein the H chain has the amino acid sequence of positions 140 to 463 in the amino acid sequence of SEQ ID NO: 84 as CH;
(33) an antibody comprising the H chain of (31), wherein the H chain has the amino acid sequence of SEQ ID NO: 22 as CH;
(34) an antibody comprising an L chain having the amino acid sequence of SEQ ID NO: 86 as CDR1, the amino acid sequence of SEQ ID NO: 88 as CDR2, and the amino acid sequence of SEQ ID NO: 90 as CDR3;
(35) an antibody comprising the L chain of (34), wherein the L chain has the amino acid sequence of positions 133 to 238 in the amino acid sequence of SEQ ID NO: 94 as CL;
(36) an antibody comprising the L chain of (34), wherein the L chain has the amino acid sequence of SEQ ID NO: 34 as CL;
(37) an antibody comprising the H chain of (31) and the L chain of (34);
(38) an antibody comprising the H chain of (32) and the L chain of (35);
(39) an antibody comprising the H chain of (33) and the L chain of (36);
(40) an antibody having one or more amino acid substitutions, deletions, additions, and/or insertions in the antibody of any of (31) to (39), which has equivalent activity as the antibody of any of (31) to (39);
(41) an antibody comprising an H chain having the amino acid sequence of SEQ ID NO: 96 as CDR1, the amino acid sequence of SEQ ID NO: 98 as CDR2, and the amino acid sequence of SEQ ID NO: 100 as CDR3;
(42) an antibody comprising the H chain of (41), wherein the H chain has the amino acid sequence of positions 140 to 474 in the amino acid sequence of SEQ ID NO: 104 as CH;
(43) an antibody comprising the H chain of (41), wherein the H chain has the amino acid sequence of SEQ ID NO: 22 as CH;
(44) an antibody comprising an L chain having the amino acid sequence of SEQ ID NO: 106 as CDR1, the amino acid sequence of SEQ ID NO: 108 as CDR2, and the amino acid sequence of SEQ ID NO: 110 as CDR3;
(45) an antibody comprising the L chain of (44), wherein the L chain has the amino acid sequence of positions 133 to 238 in the amino acid sequence of SEQ ID NO: 114 as CL;
(46) an antibody comprising the L chain of (44), wherein the L chain has the amino acid sequence of SEQ ID NO: 34 as CL;
(47) an antibody comprising the H chain of (41) and the L chain of (44);
(48) an antibody comprising the H chain of (42) and the L chain of (45);
(49) an antibody comprising the H chain of (43) and the L chain of (46);
(50) an antibody having one or more amino acid substitutions, deletions, additions, and/or insertions in the antibody of any of (41) to (49), which has equivalent activity as the antibody of any of (41) to (49);
(51) an antibody comprising an H chain having the amino acid sequence of SEQ ID NO: 167 as CDR1, the amino acid sequence of SEQ ID NO: 169 as CDR2, and the amino acid sequence of SEQ ID NO: 171 as CDR3;
(52) an antibody comprising the H chain of (51), wherein the H chain has the amino acid sequence of positions 118 to 447 in the amino acid sequence of SEQ ID NO: 173 as CH;
(53) an antibody comprising the H chain of (51), wherein the H chain has the amino acid sequence of SEQ ID NO: 22 as CH;
(54) an antibody comprising an L chain having the amino acid sequence of SEQ ID NO: 179 as CDR1, the amino acid sequence of SEQ ID NO: 181 as CDR2, and the amino acid sequence of SEQ ID NO: 183 as CDR3;
(55) an antibody comprising the L chain of (54), wherein the L chain has the amino acid sequence of positions 113 to 218 in the amino acid sequence of SEQ ID NO: 185 as CL;
(56) an antibody comprising the L chain of (54), wherein the L chain has the amino acid sequence of SEQ ID NO: 34 as CL;
(57) an antibody comprising the H chain of (51) and the L chain of (54);
(58) an antibody comprising the H chain of (52) and the L chain of (55);
(59) an antibody comprising the H chain of (53) and the L chain of (56);
(60) an antibody having one or more amino acid substitutions, deletions, additions, and/or insertions in the antibody of any of (51) to (59), which has equivalent activity as the antibody of any of (51) to (59);
(61) an antibody that binds to the same epitope as the Claudin 3 protein epitope bound by the antibody of any of (1) to (60).

**15.** A method for diagnosis of cancer, comprising the step of binding the antibody of any one of claims 1 to 14 to a Claudin 3 protein.

**16.** A method for diagnosis of cancer, comprising the steps of:

(a) collecting a sample from a subject; and
(b) detecting a Claudin 3 protein contained in the collected sample using an antibody that binds to the Claudin 3 protein.

**17.** A method for diagnosis of cancer, comprising the steps of: (1) administering to a subject a radioisotope-labeled antibody that binds to a Claudin 3 protein; and (2) detecting accumulation of said radioisotope.

**18.** The diagnostic method of claim 17, wherein the radioisotope is a positron-emitting nuclide.

**19.** The diagnostic method of claim 18, wherein the positron-emitting nuclide is any nuclide selected from the group consisting of $^{11}C$, $^{13}N$, $^{15}O$, $^{18}F$, $^{45}Ti$, $^{55}Co$, $^{64}Cu$, $^{66}Ga$, $^{68}Ga$, $^{76}Br$, $^{89}Zr$, and $^{124}I$.

**20.** The diagnostic method of any one of claims 15 to 19, wherein the cancer is any cancer selected from the group consisting of ovarian cancer, prostate cancer, breast cancer, uterine cancer, liver cancer, lung cancer, pancreatic cancer, stomach cancer, bladder cancer, and colon cancer.

**21.** The diagnostic method of claim 20, wherein the cancer is primary cancer.

**22.** The diagnostic method of claim 20, wherein the cancer is metastatic cancer.

**23.** A diagnostic agent for use in a method of cancer diagnosis, which comprises an antibody that binds to a Claudin 3 protein.

**24.** A kit for use in a method of cancer diagnosis, which comprises an antibody that binds to a Claudin 3 protein, and a biological sample comprising a Claudin 3 protein.

**25.** A pharmaceutical composition comprising an antibody that binds to a Claudin 3 protein as an active ingredient.

**26.** A cell proliferation inhibitor comprising an antibody that binds to a Claudin 3 protein as an active ingredient.

**27.** An anticancer agent comprising an antibody that binds to a Claudin 3 protein as an active ingredient.

**28.** The anticancer agent of claim 27, wherein the cancer is any cancer selected from the group consisting of ovarian cancer, prostate cancer, breast cancer, uterine cancer, liver cancer, lung cancer, pancreatic cancer, stomach cancer, bladder cancer, and colon cancer.

**29.** The anticancer agent of claim 28, wherein the cancer is primary cancer.

**30.** The anticancer agent of claim 28, wherein the cancer is metastatic cancer.

**31.** The anticancer agent of claim 28, wherein said antibody is the antibody of any one of claims 1 to 14.

**32.** Use of an antibody that binds to a Claudin 3 protein in the production of a cell proliferation inhibitor.

**33.** Use of an antibody that binds to a Claudin 3 protein in the production of an anticancer agent.

**34.** A method of suppressing cell proliferation, which comprises the step of administering to a subject an antibody that binds to a Claudin 3 protein.

**35.** A method of preventing or treating cancer, which comprises the step of administering to a subject an antibody that binds to a Claudin 3 protein.

**36.** A method of inducing cell injury in a cell expressing a Claudin 3 protein, which comprises the step of contacting a Claudin 3 protein-expressing cell with an antibody that binds to a Claudin 3 protein.

**37.** A method of suppressing the proliferation of a cell expressing a Claudin 3 protein, which comprises the step of contacting a Claudin 3 protein-expressing cell with an antibody that binds to a Claudin 3 protein.

EXTRACELLULAR LOOP 1

CLD3 RVSAFIGSNIITSQNIWEGLWMNCVVQSTGQMQCKVYDSLLALPQDLQAAR(SEQ ID NO:2 30-80)
CLD4 RVTAFIGSNIVTSQTIWEGLWMNCVVQSTGQMQCKVYDSLLALPQDLQAAR(SEQ ID NO:134)
CLD9 KVTAFIGNSIVVAQVVWEGLWMSCVVQSTGQMQCKVYDSLLALPQDLQAAR(SEQ ID NO:135)
CLD6 KVTAFIGNSIVVAQVVWEGLWMSCVVQSTGQMQCKVYDSLLALPQDLQAAR(SEQ ID NO:136)
CLD8 RVSAFIENNIVVFENFWEGLWMNCVRQANIRMQCKIYDSLLALSPDLQAAR(SEQ ID NO:137)
CLD5 QVTAFLDHNIVTAQTTWKGLWMSCVVQSTGHMQCKVYDSVLALSTEVQAAR(SEQ ID NO:138)
CLD17 RVSAFVGSNIIVFERLWEGLWMNCIRQARVRLQCKFYSSLLALPPALETAR(SEQ ID NO:139)
CLD1 RIYSYAGDNIVTAQAMYEGLWMSCVSQSTGQIQCKVFDSLLNLSSTLQATR(SEQ ID NO:140)
CLD7 QMSSYAGDNIITAQAMYKGLWMDCVTQSTGMMSCKMYDSVLALSAALQATR(SEQ ID NO:141)
CLD14 RRTAHVGTNILTAVSYLKGLWMECVWHSTGIYQCQIYRSLLALPQDLQAAR(SEQ ID NO:142)
CLD19 --SSYAGDAIITAVGLYEGLWMSCASQSTGQVQCKLYDSLLALDGHIQSAR(SEQ ID NO:143)
CLD2 KTSSYVGASIVTAVGFSKGLWMECATHSTGITQCDIYSTLLGLPADIQAAQ(SEQ ID NO:144)
CLD20 KVNVDVDSNIITAIVQLHGLWMDCTWYSTGMFSCALKHSILSLPIHVQAAR(SEQ ID NO:145)
. .. . .*.... ..**** *. .... ..*. ....*.*. ......

EXTRACELLULAR LOOP 2

CLD3 SANTIIRDFYNPVVPEAQKREM (SEQ ID NO:2 138-159)
CLD6 TAHAVIRDFYNPLVAEAQKREL (SEQ ID NO:10 139-160)
CLD8 VANAIIRDFYNSIVNVAQKREL(SEQ ID NO:146)
CLD4 TAHNIIQDFYNPLVASGQKREM (SEQ ID NO:8 139-160)
CLD9 TAHAIIQDFYNPLVAEALKREL(SEQ ID NO:147)
CLD17 TANIIIRDFYNPAIHIGQKREL(SEQ ID NO:148)
.* .*.****. . ..***.

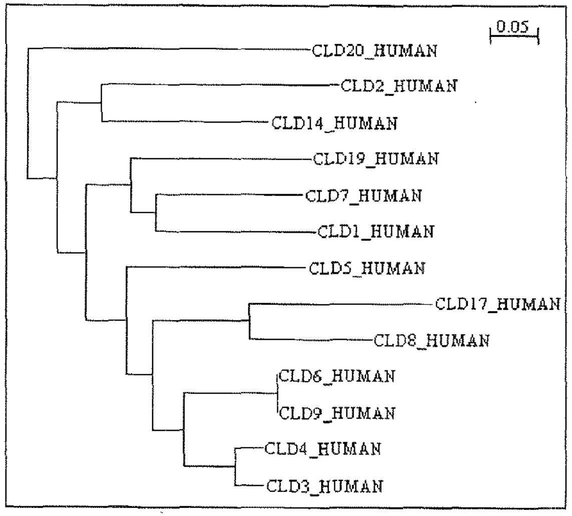


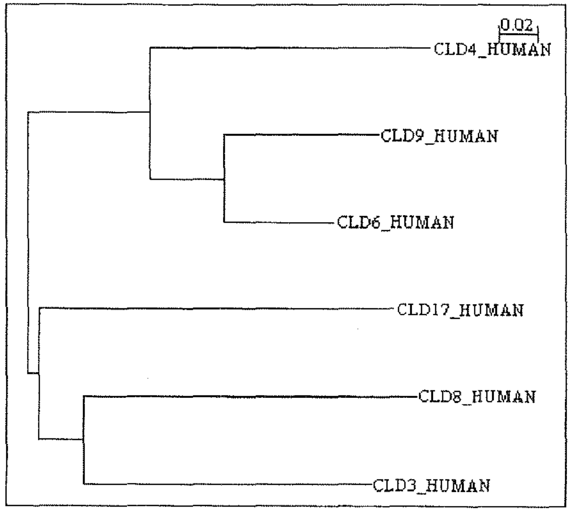


FIG. 1

```
CLD3_HUMAN    1:MSMGLEIT GTALAVLGWLGTIVCCALPMW RVSAFIGSNIITSQNIWEGLWMNCVVQSTGQ   60
CLD3_MOUSE    1:MSMGLEIT GTSLAVLGWLCTIVCCALPMW RVSAFIGSSIITAQITWEGLWMNCVVQSTGQ   60
                ******** ** ******* ********** ******** *** *  ***************

CLD3_HUMAN   61:MQCKVYDSLLALPQDLQAAR ALIVVAILLAAFGLLVALVGA QCTNCVQDDTAKAK ITIVA 120
CLD3_MOUSE   61:MQCKMYDSLLALPQDLQAAR ALIVVSILLAAFGLLVALVGA QCTNCVQDETAKAK ITIVA 120
                **** *************** ***** *************** ******** ***** *****

CLD3_HUMAN  121:GVLFLLAALLTLVPVS WSANTIIRDFYNPVVPEAQKREM GAGLYVGWAAAALQLLGGALL 180
CLD3_MOUSE  121:GVLFLLAALLTLVPVS WSANTIIRDFYNPLVPEAQKREM GAGLYVGWAAAALQLLGGALL 180
                **************** ************* ********* *********************

CLD3_HUMAN  181:CCSCPPREKKYTATKVVYSAPRSTGPGASLGTGYDRKDYV (SEQ ID NO: 2)    220
CLD3_MOUSE  181:CCSCPPRDK-YAPTKILYSAPRSTGPGTGTGTAYDRKDYV (SEQ ID NO: 4)    219
```

FIG. 2

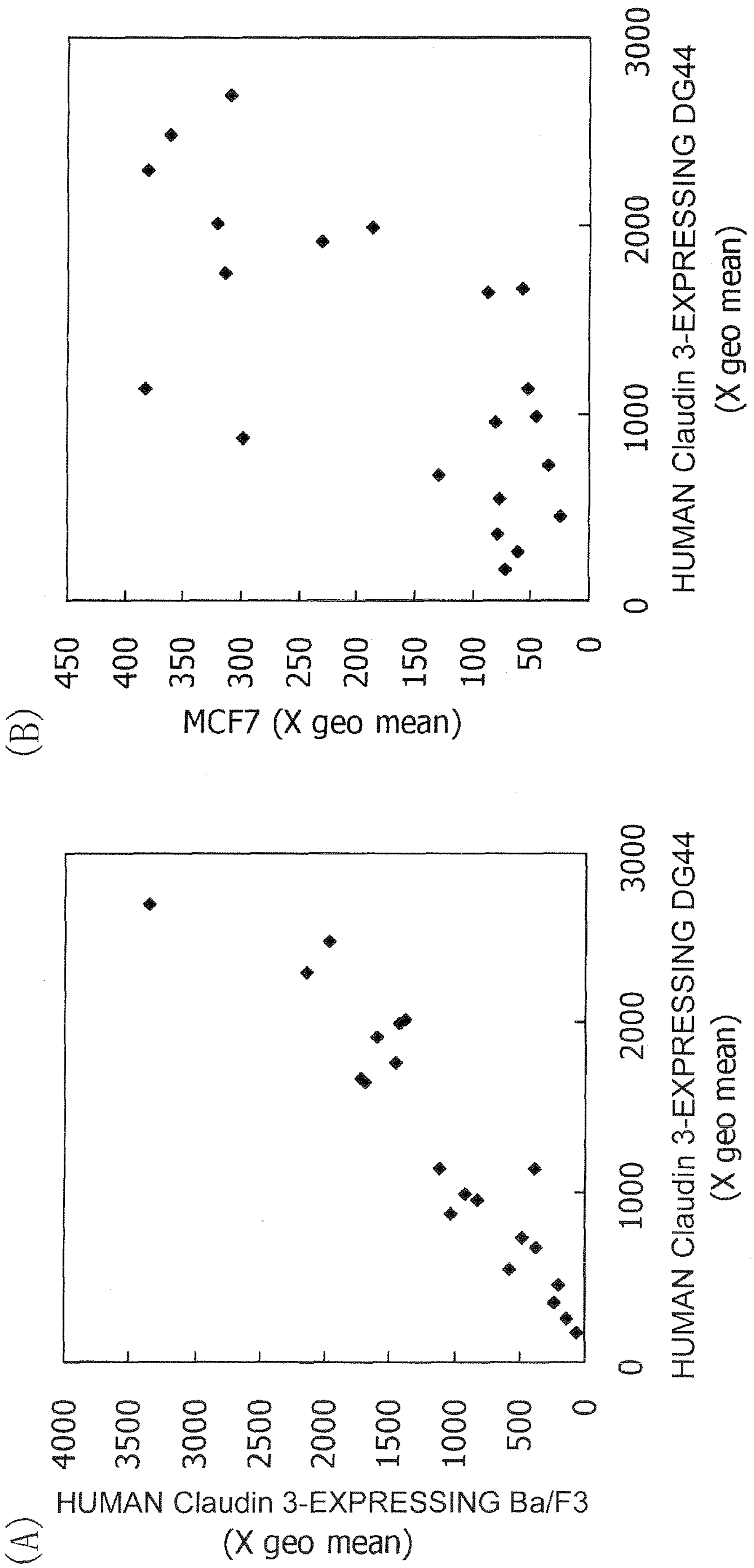
(A)
HUMAN Claudin 3-EXPRESSING Ba/F3
(X geo mean)
0
500
1000
1500
2000
2500
3000
3500
4000
HUMAN Claudin 3-EXPRESSING DG44
(X geo mean)
0
1000
2000
3000
(B)
MCF7 (X geo mean)
0
50
100
150
200
250
300
350
400
450
HUMAN Claudin 3-EXPRESSING DG44
(X geo mean)
0
1000
2000
3000

FIG. 3

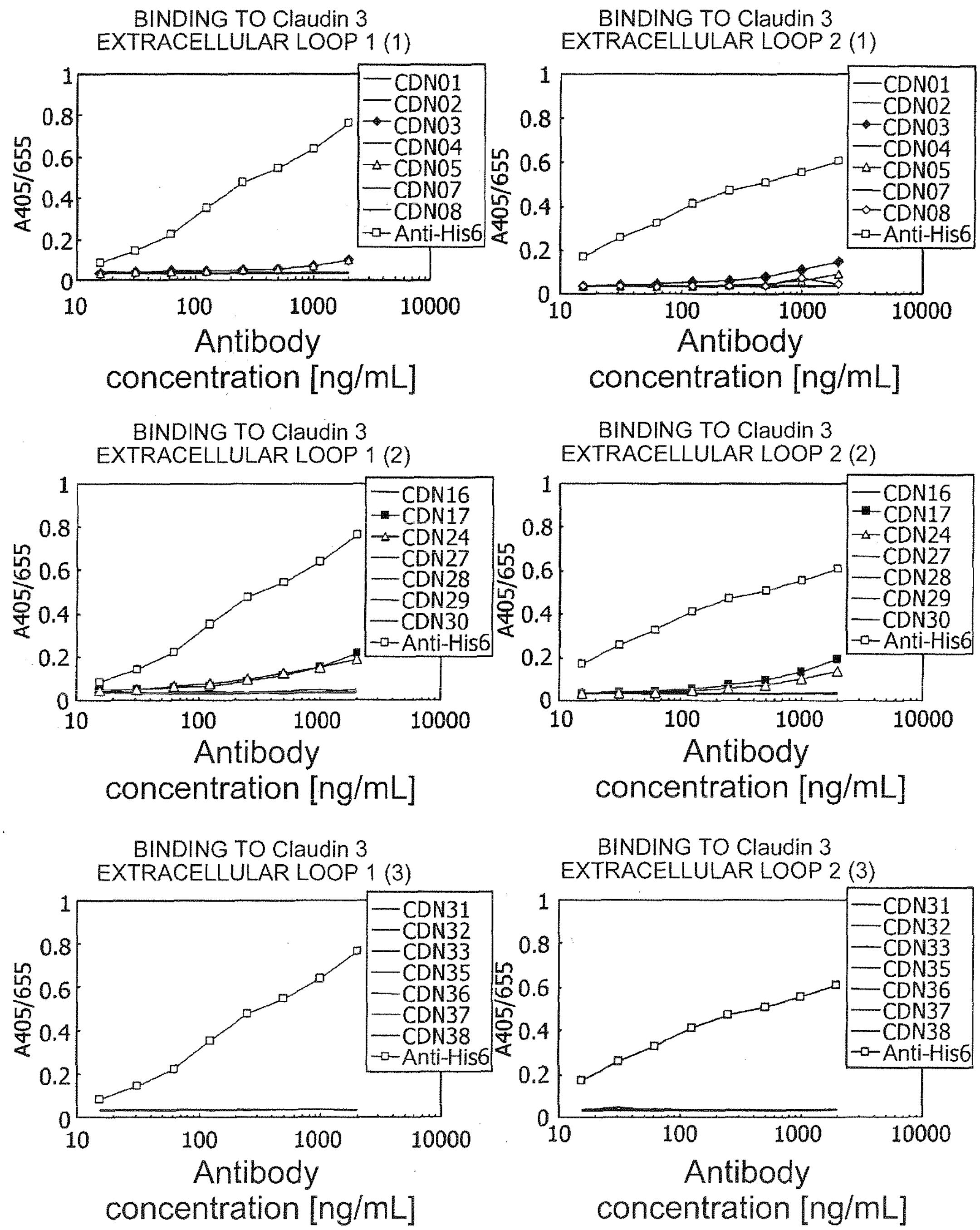
BINDING TO Claudin 3
EXTRACELLULAR LOOP 1 (1)
CDN01
CDN02
CDN03
CDN04
CDN05
CDN07
CDN08
Anti-His6
A405/655
Antibody concentration [ng/mL]
BINDING TO Claudin 3
EXTRACELLULAR LOOP 2 (1)
BINDING TO Claudin 3
EXTRACELLULAR LOOP 1 (2)
CDN16
CDN17
CDN24
CDN27
CDN28
CDN29
CDN30
BINDING TO Claudin 3
EXTRACELLULAR LOOP 2 (2)
BINDING TO Claudin 3
EXTRACELLULAR LOOP 1 (3)
CDN31
CDN32
CDN33
CDN35
CDN36
CDN37
CDN38
BINDING TO Claudin 3
EXTRACELLULAR LOOP 2 (3)

FIG. 4

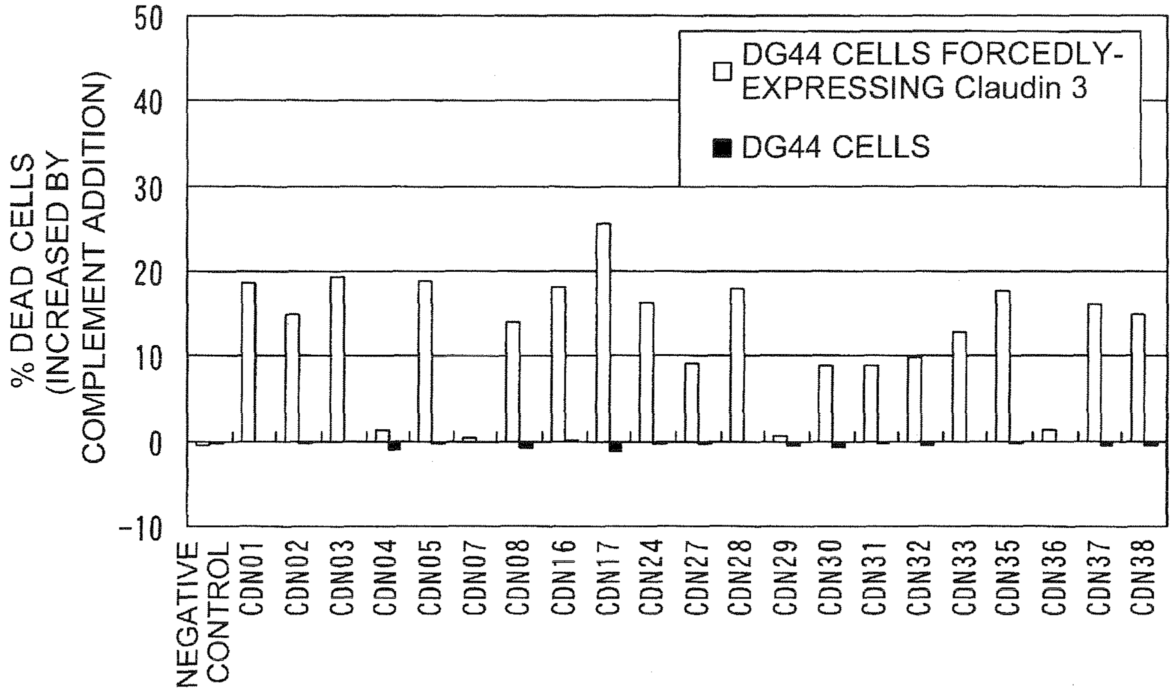
50
40
30
20
10
0
-10
% DEAD CELLS (INCREASED BY COMPLEMENT ADDITION)
DG44 CELLS FORCEDLY-EXPRESSING Claudin 3
DG44 CELLS
NEGATIVE CONTROL
CDN01
CDN02
CDN03
CDN04
CDN05
CDN07
CDN08
CDN16
CDN17
CDN24
CDN27
CDN28
CDN29
CDN30
CDN31
CDN32
CDN33
CDN35
CDN36
CDN37
CDN38

FIG. 5

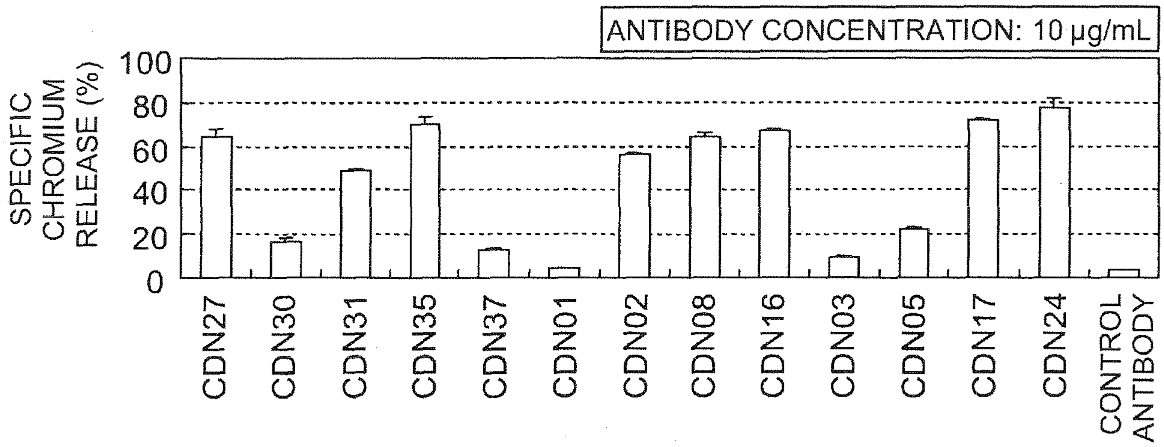
ANTIBODY CONCENTRATION: 10 µg/mL
SPECIFIC CHROMIUM RELEASE (%)
100
80
60
40
20
0
CDN27
CDN30
CDN31
CDN35
CDN37
CDN01
CDN02
CDN08
CDN16
CDN03
CDN05
CDN17
CDN24
CONTROL ANTIBODY

FIG. 6

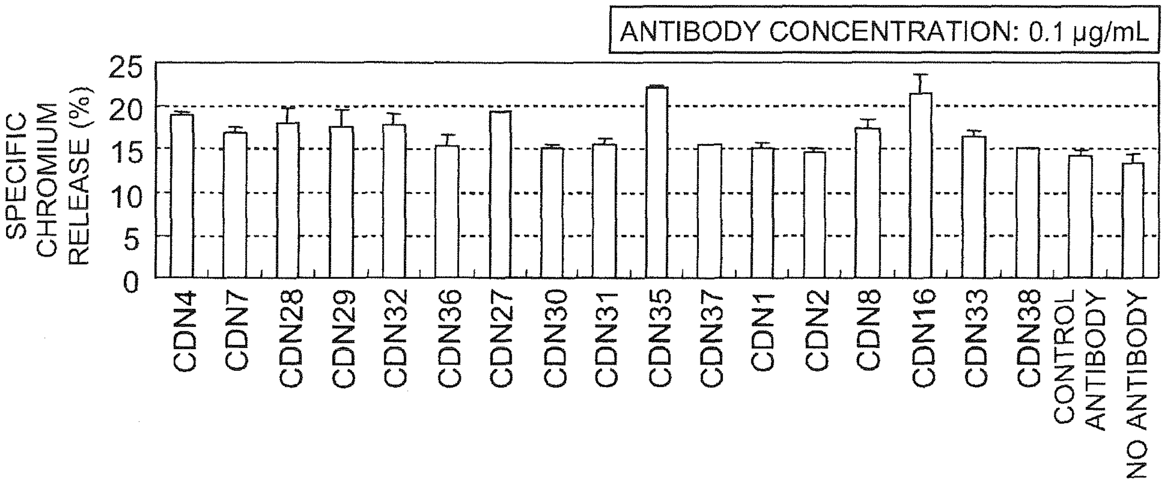
ANTIBODY CONCENTRATION: 0.1 μg/mL
SPECIFIC CHROMIUM RELEASE (%)
0
5
10
15
20
25
CDN4
CDN7
CDN28
CDN29
CDN32
CDN36
CDN27
CDN30
CDN31
CDN35
CDN37
CDN1
CDN2
CDN8
CDN16
CDN33
CDN38
CONTROL ANTIBODY
NO ANTIBODY

FIG. 7

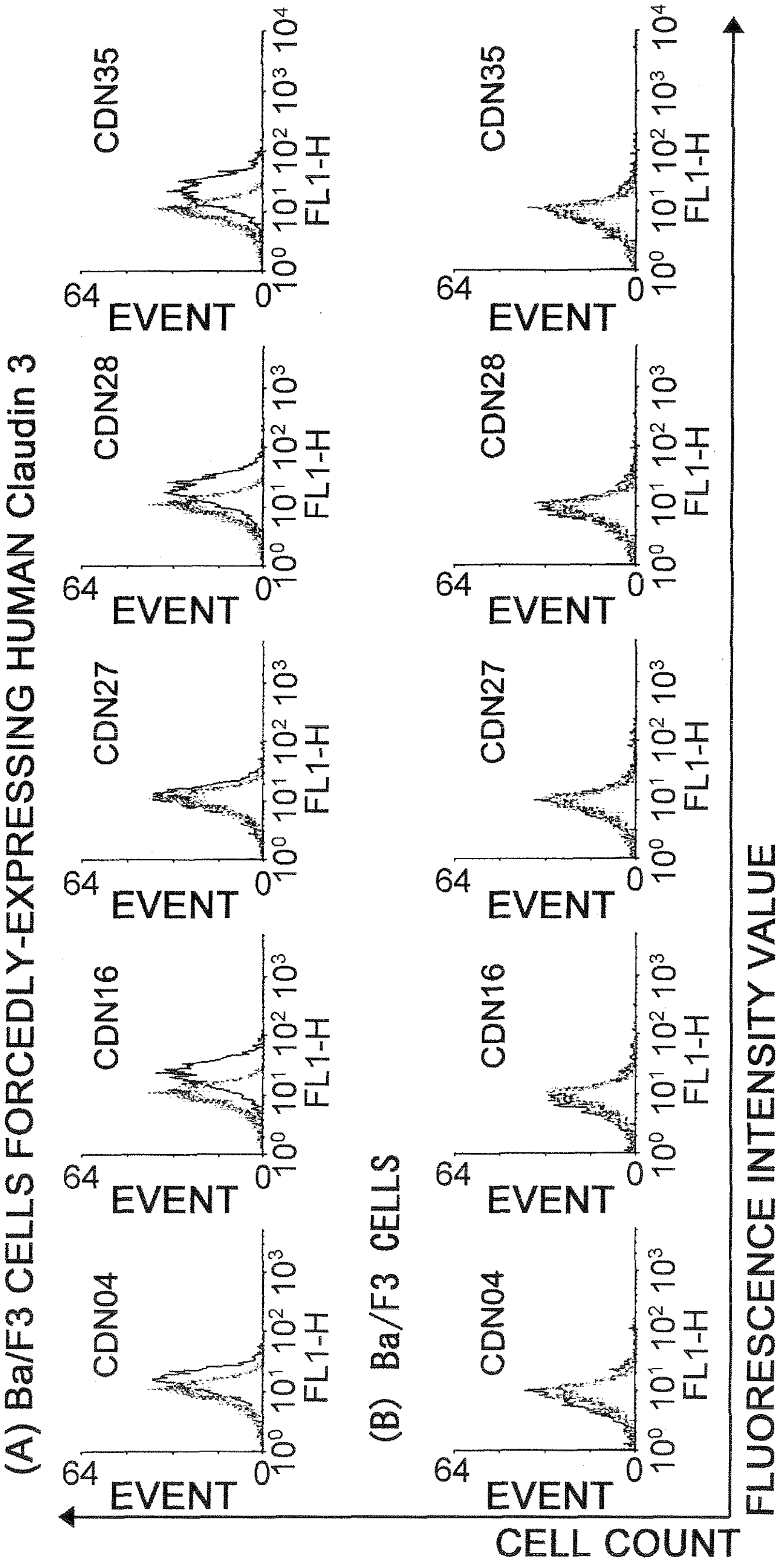
(A) Ba/F3 CELLS FORCEDLY-EXPRESSING HUMAN Claudin 3
(B) Ba/F3 CELLS
CDN04
CDN16
CDN27
CDN28
CDN35
EVENT
64
0
FL1-H
$10^0$ $10^1$ $10^2$ $10^3$ $10^4$
CELL COUNT
FLUORESCENCE INTENSITY VALUE

FIG. 8

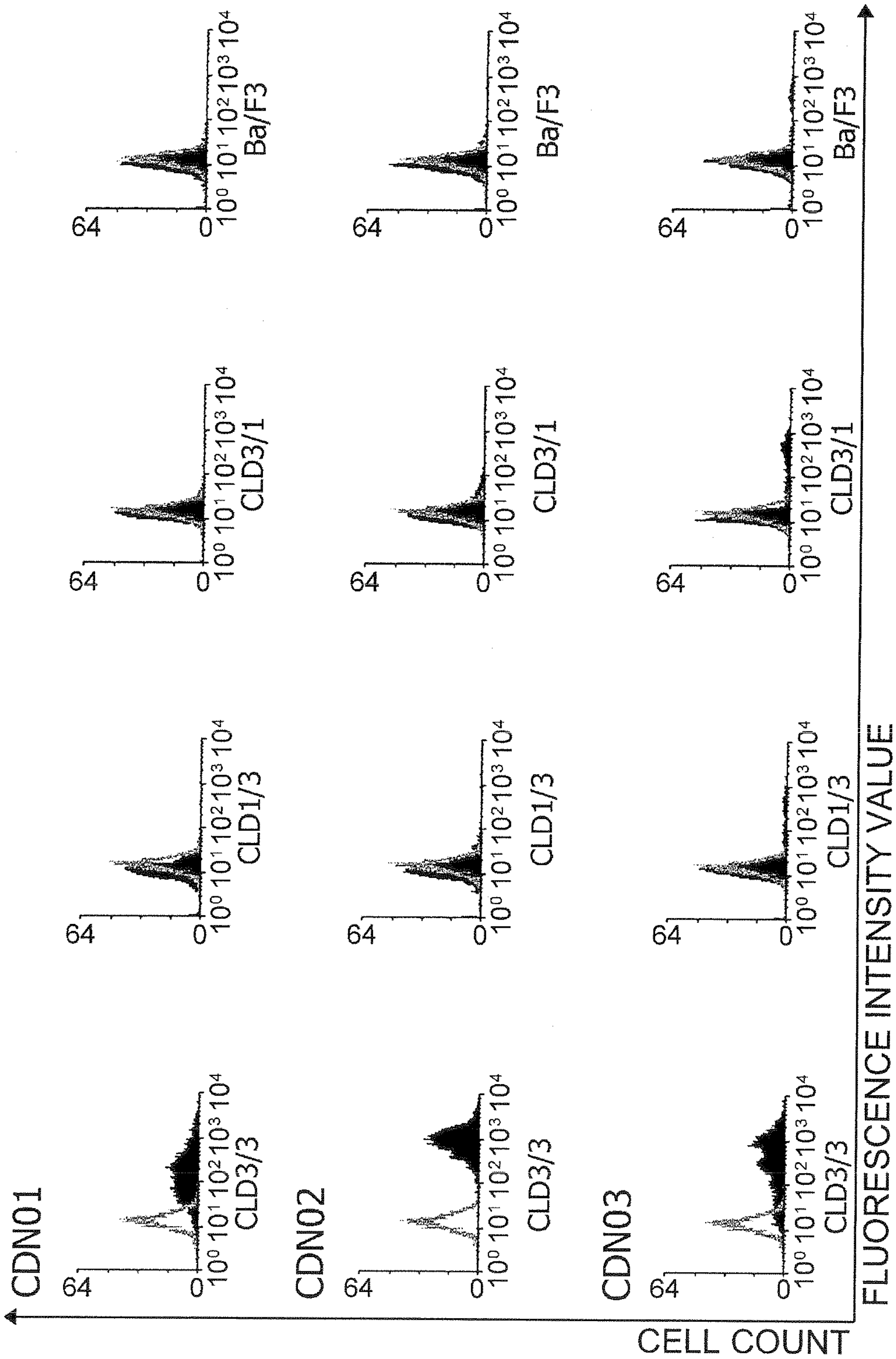
CDN01
CDN02
CDN03
64
0
$10^0$ $10^1$ $10^2$ $10^3$ $10^4$
CLD3/3
CLD1/3
CLD3/1
Ba/F3
CELL COUNT
FLUORESCENCE INTENSITY VALUE

FIG. 9-1

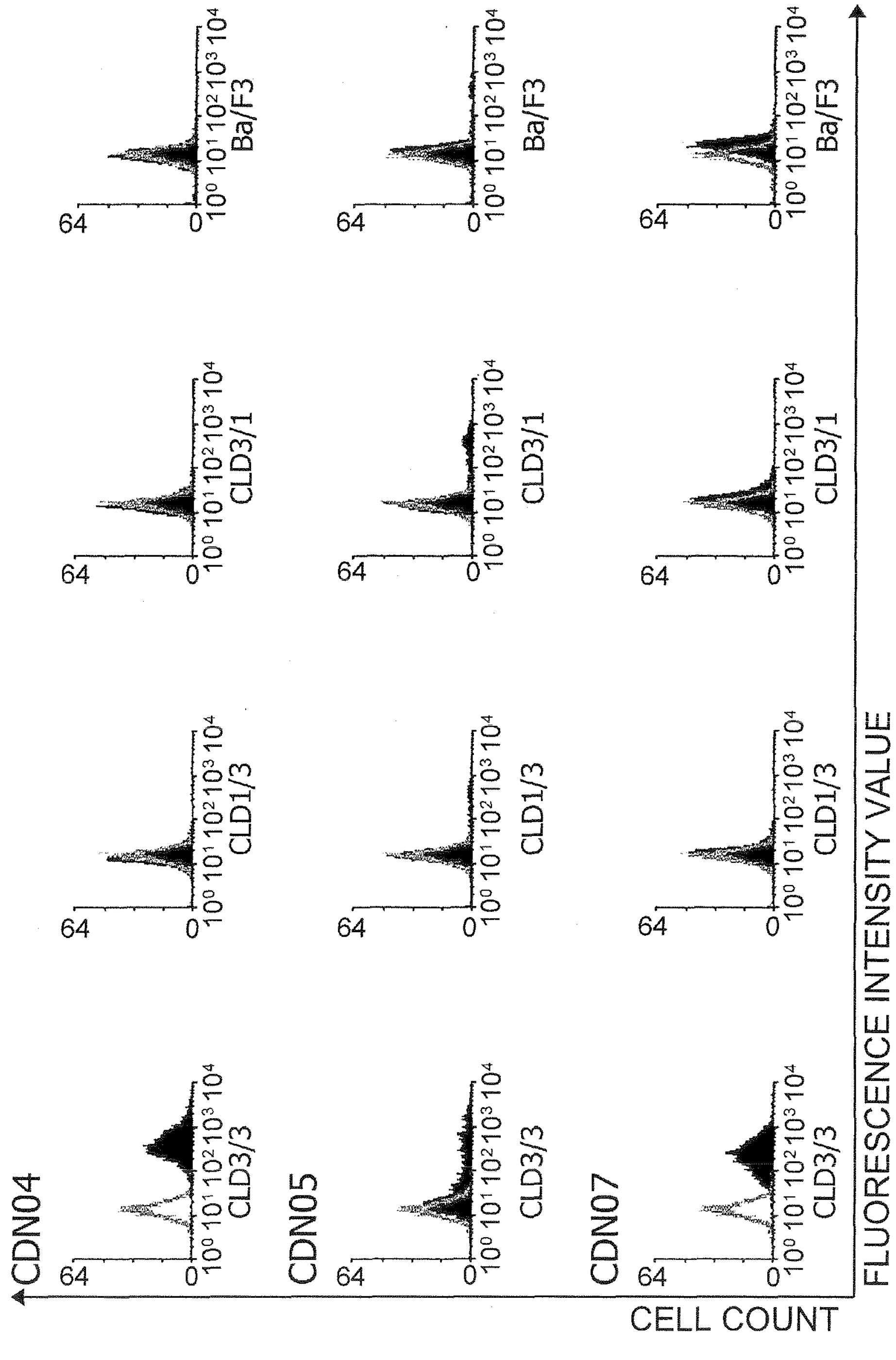
CDN04
CDN05
CDN07
CLD3/3
CLD1/3
CLD3/1
Ba/F3
64
0
$10^0$ $10^1$ $10^2$ $10^3$ $10^4$
CELL COUNT
FLUORESCENCE INTENSITY VALUE

FIG. 9-2

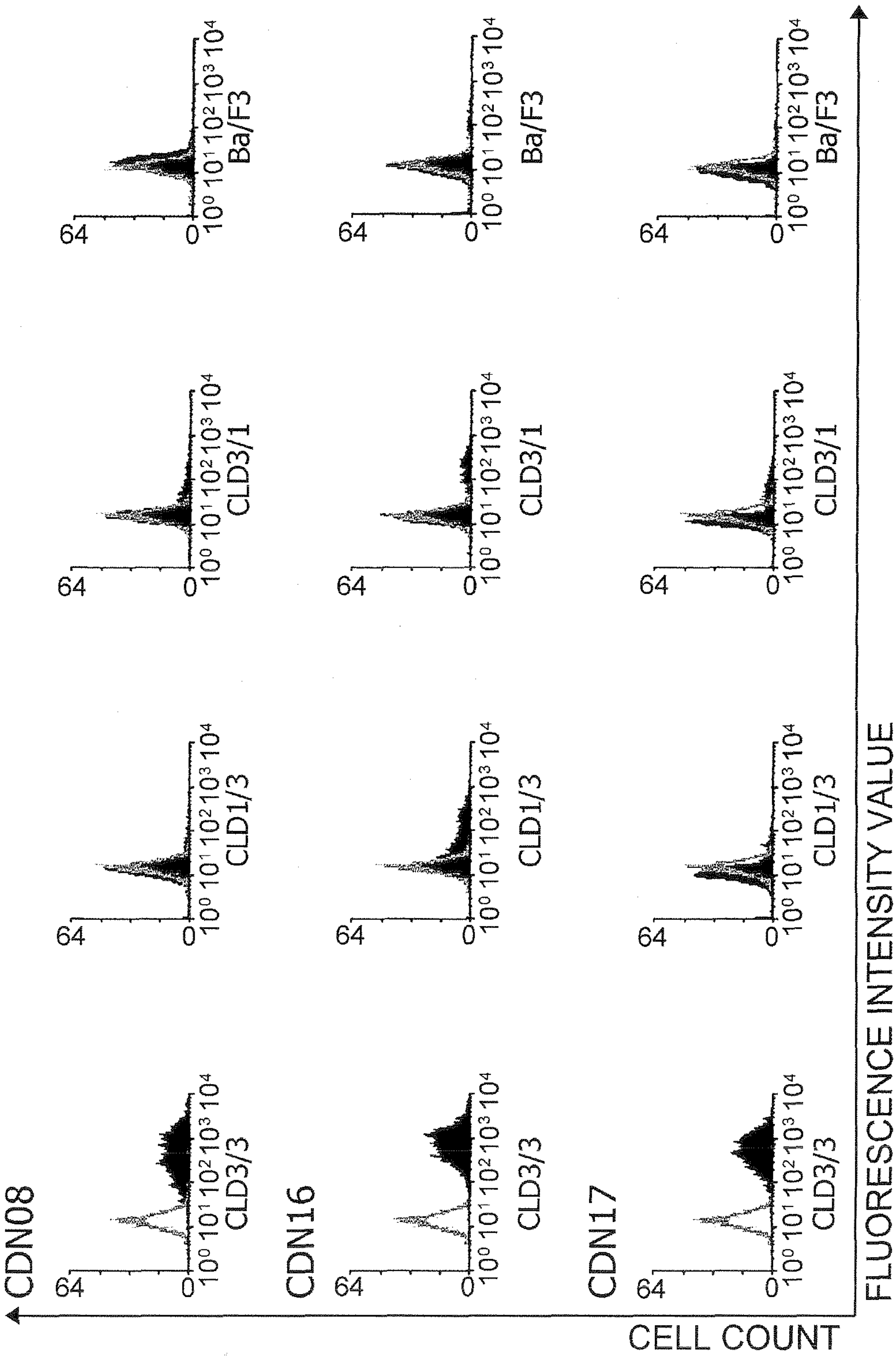
CDN08
CDN16
CDN17
CLD3/3
CLD1/3
CLD3/1
Ba/F3
64
0
$10^0$ $10^1$ $10^2$ $10^3$ $10^4$
CELL COUNT
FLUORESCENCE INTENSITY VALUE

FIG. 9-3

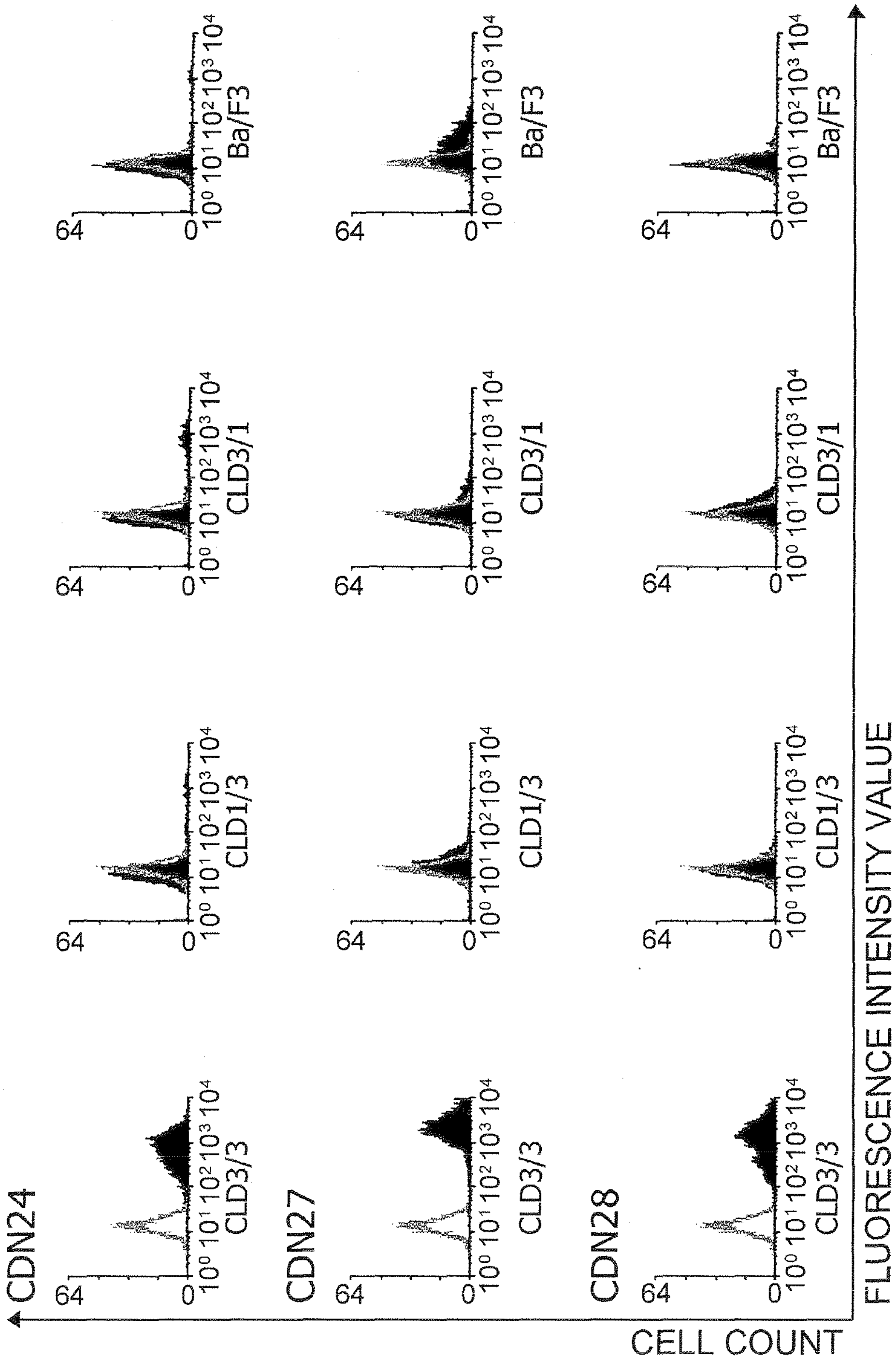
CDN24
CDN27
CDN28
CLD3/3
CLD1/3
CLD3/1
Ba/F3
64
0
$10^0$ $10^1$ $10^2$ $10^3$ $10^4$
CELL COUNT
FLUORESCENCE INTENSITY VALUE

FIG. 9-4

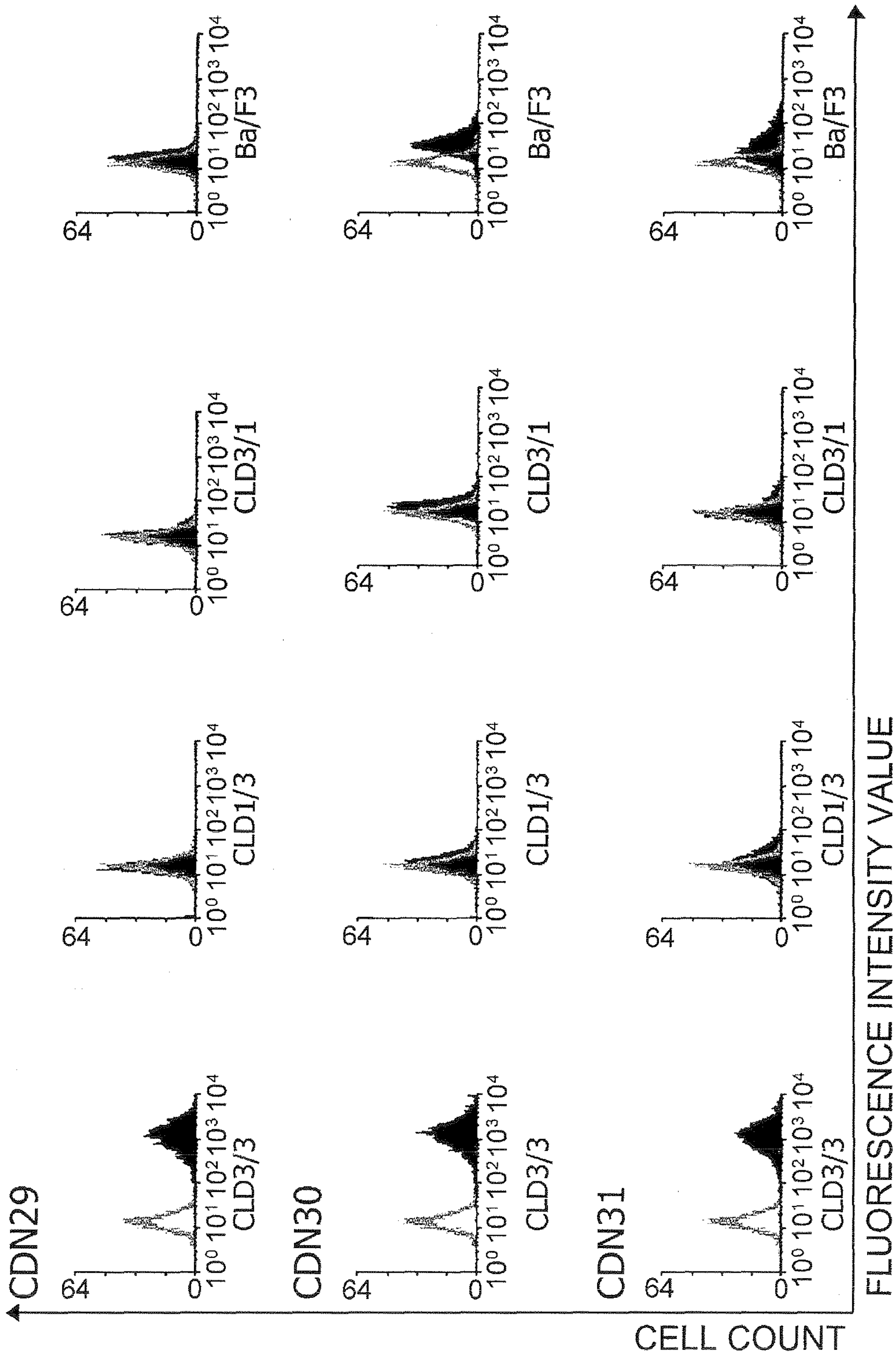
CDN29
CDN30
CDN31
CLD3/3
CLD1/3
CLD3/1
Ba/F3
64
0
$10^0$ $10^1$ $10^2$ $10^3$ $10^4$
CELL COUNT
FLUORESCENCE INTENSITY VALUE

FIG. 9-5

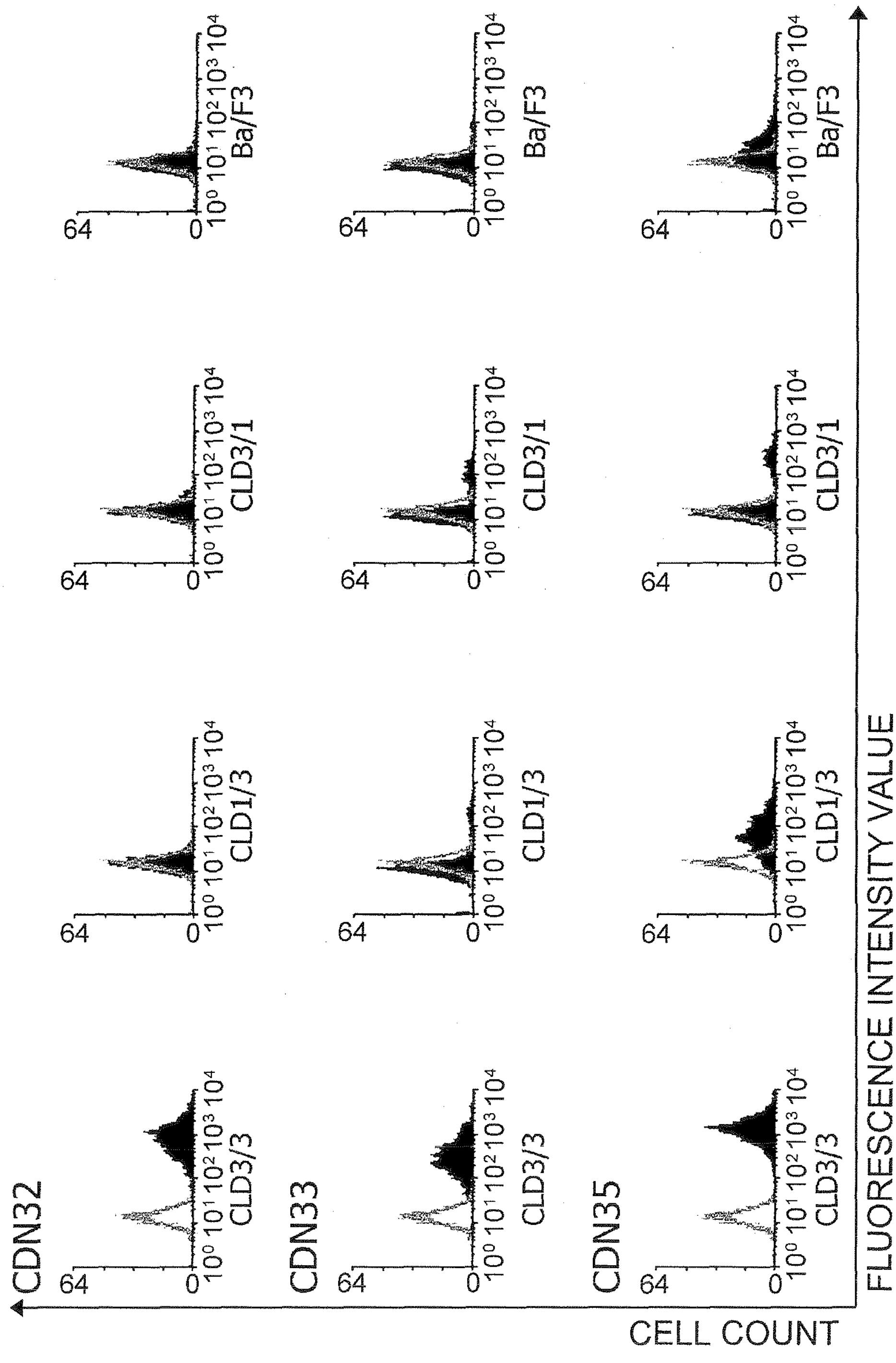
CDN32
CDN33
CDN35
64
0
$10^0$ $10^1$ $10^2$ $10^3$ $10^4$
CLD3/3
CLD1/3
CLD3/1
Ba/F3
CELL COUNT
FLUORESCENCE INTENSITY VALUE

FIG. 9-6

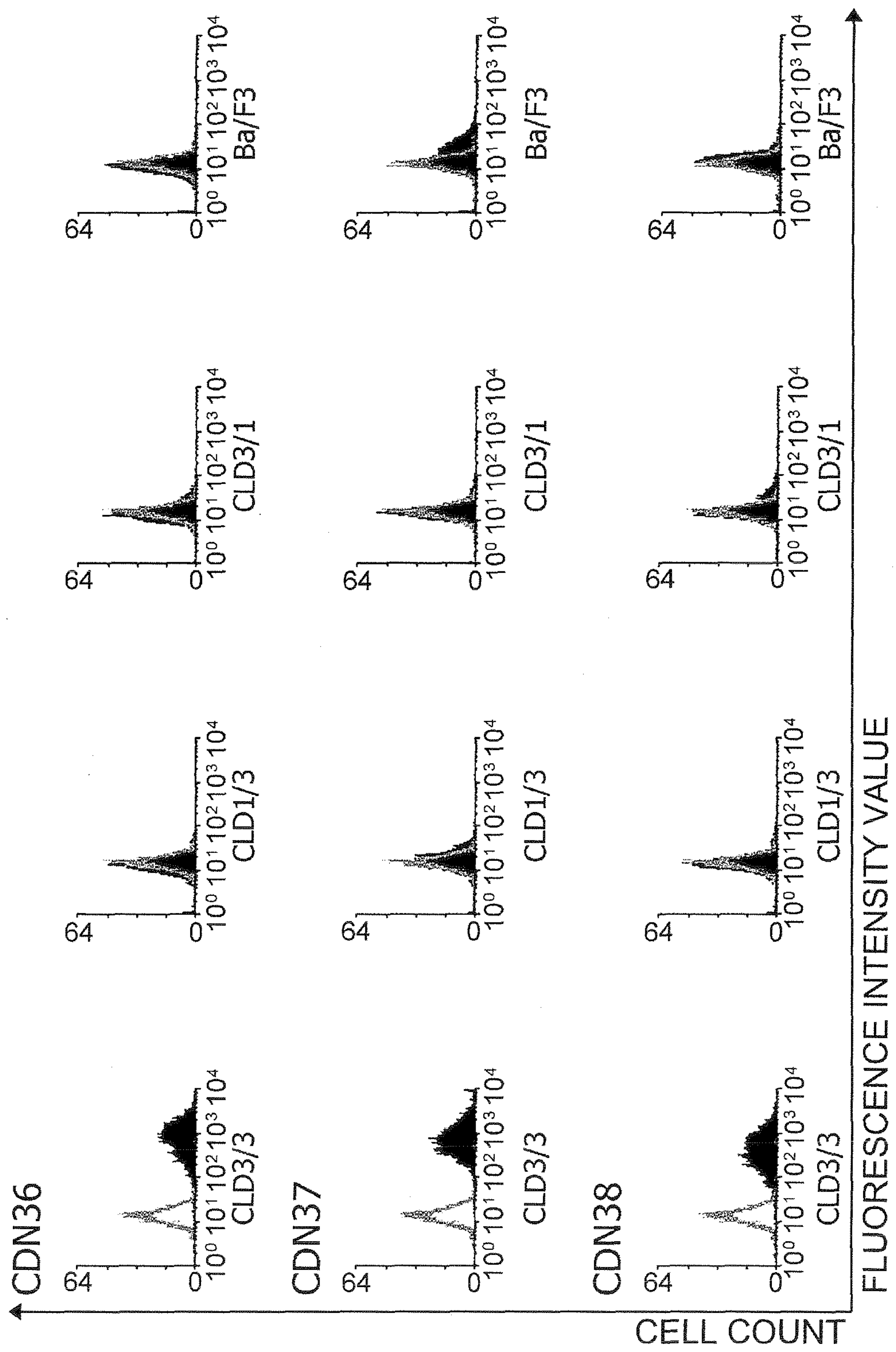
CDN36
CDN37
CDN38
CLD3/3
CLD1/3
CLD3/1
Ba/F3
64
0
$10^0$ $10^1$ $10^2$ $10^3$ $10^4$
CELL COUNT
FLUORESCENCE INTENSITY VALUE

FIG. 9-7

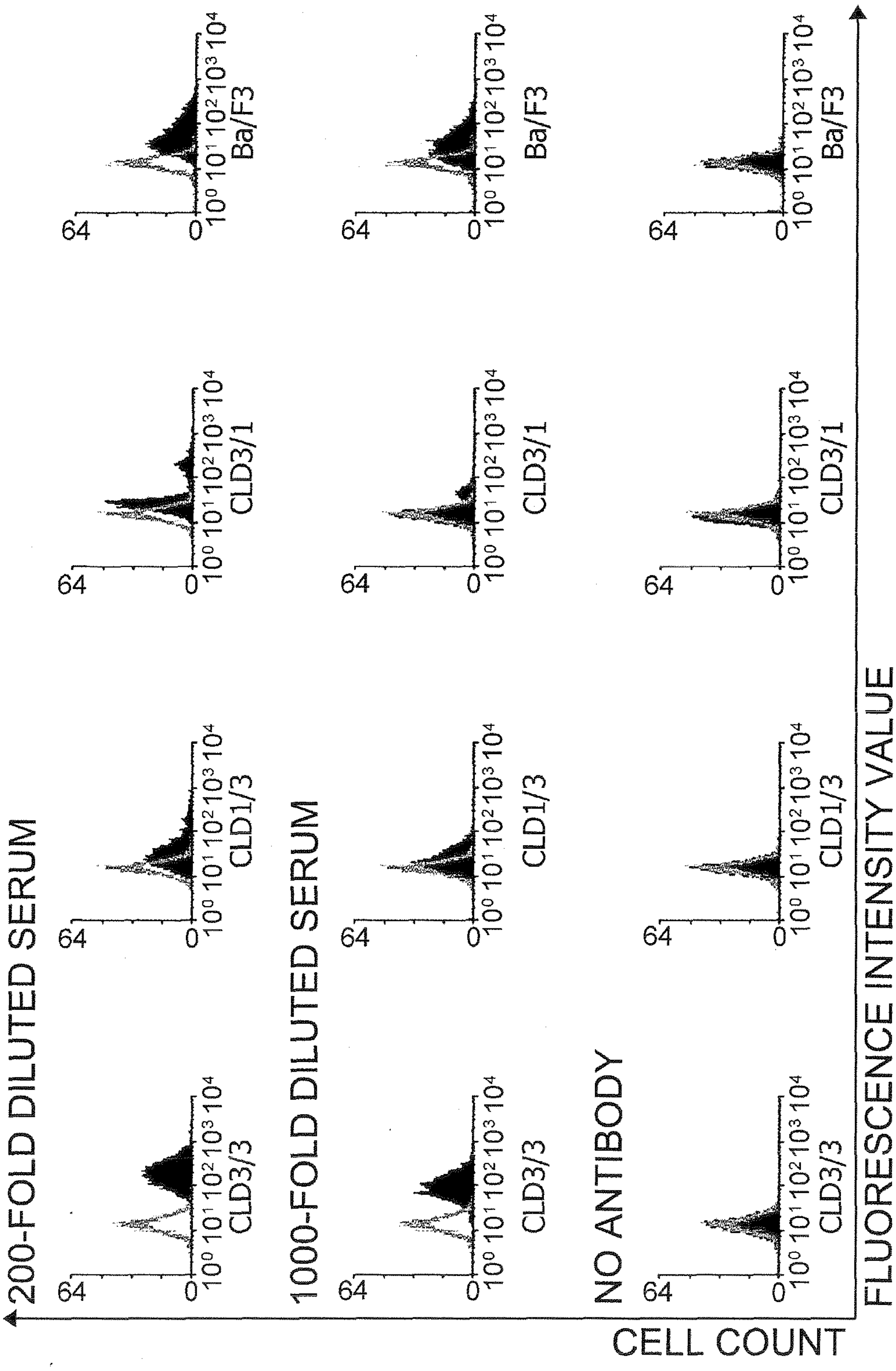
CELL COUNT
FLUORESCENCE INTENSITY VALUE
200-FOLD DILUTED SERUM
1000-FOLD DILUTED SERUM
NO ANTIBODY
CLD3/3
CLD1/3
CLD3/1
Ba/F3
64
0
10⁰ 10¹ 10² 10³ 10⁴

FIG. 9-8

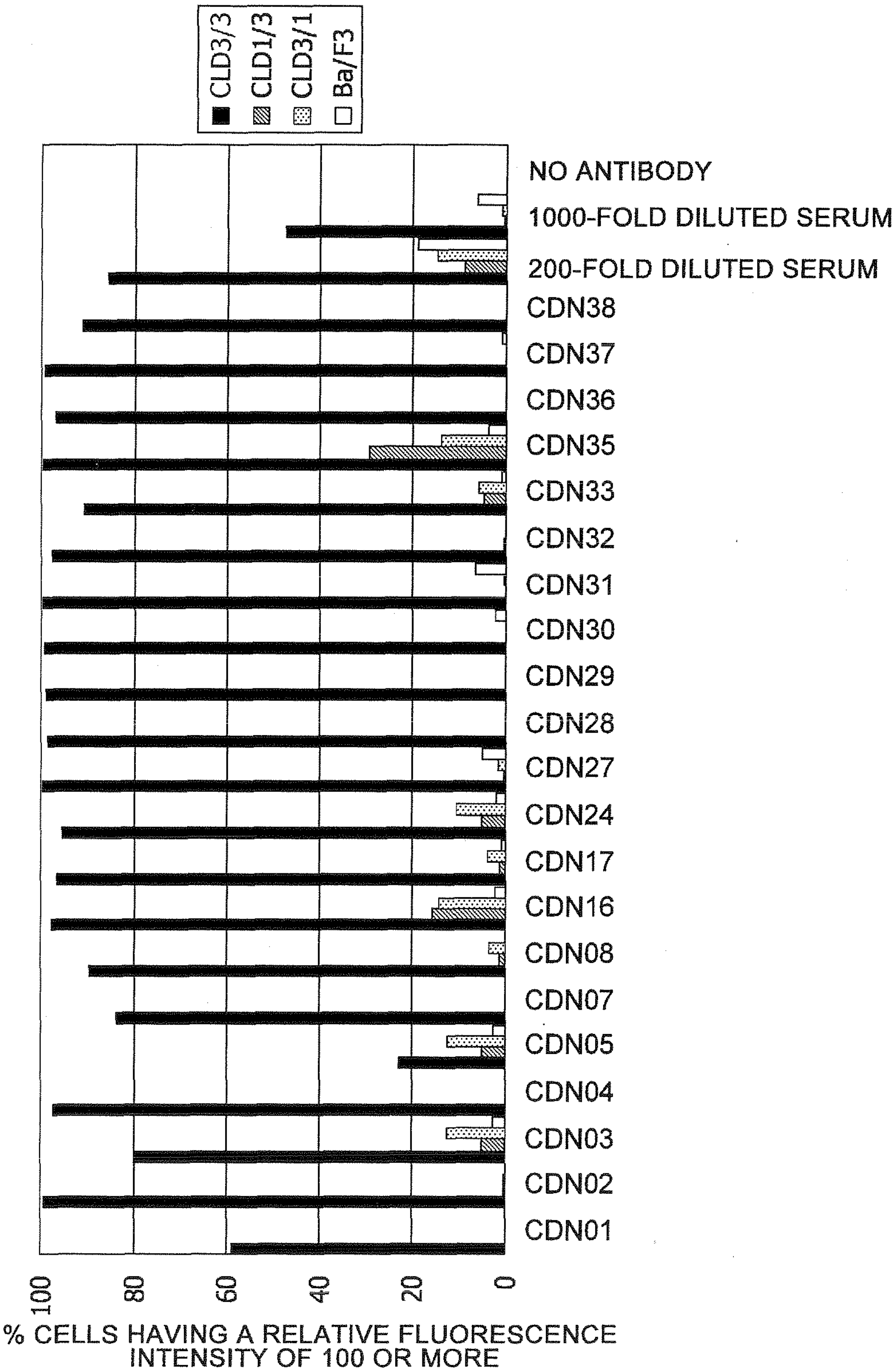
CLD3/3
CLD1/3
CLD3/1
Ba/F3
NO ANTIBODY
1000-FOLD DILUTED SERUM
200-FOLD DILUTED SERUM
CDN38
CDN37
CDN36
CDN35
CDN33
CDN32
CDN31
CDN30
CDN29
CDN28
CDN27
CDN24
CDN17
CDN16
CDN08
CDN07
CDN05
CDN04
CDN03
CDN02
CDN01
100
80
60
40
20
0
% CELLS HAVING A RELATIVE FLUORESCENCE INTENSITY OF 100 OR MORE

FIG. 10

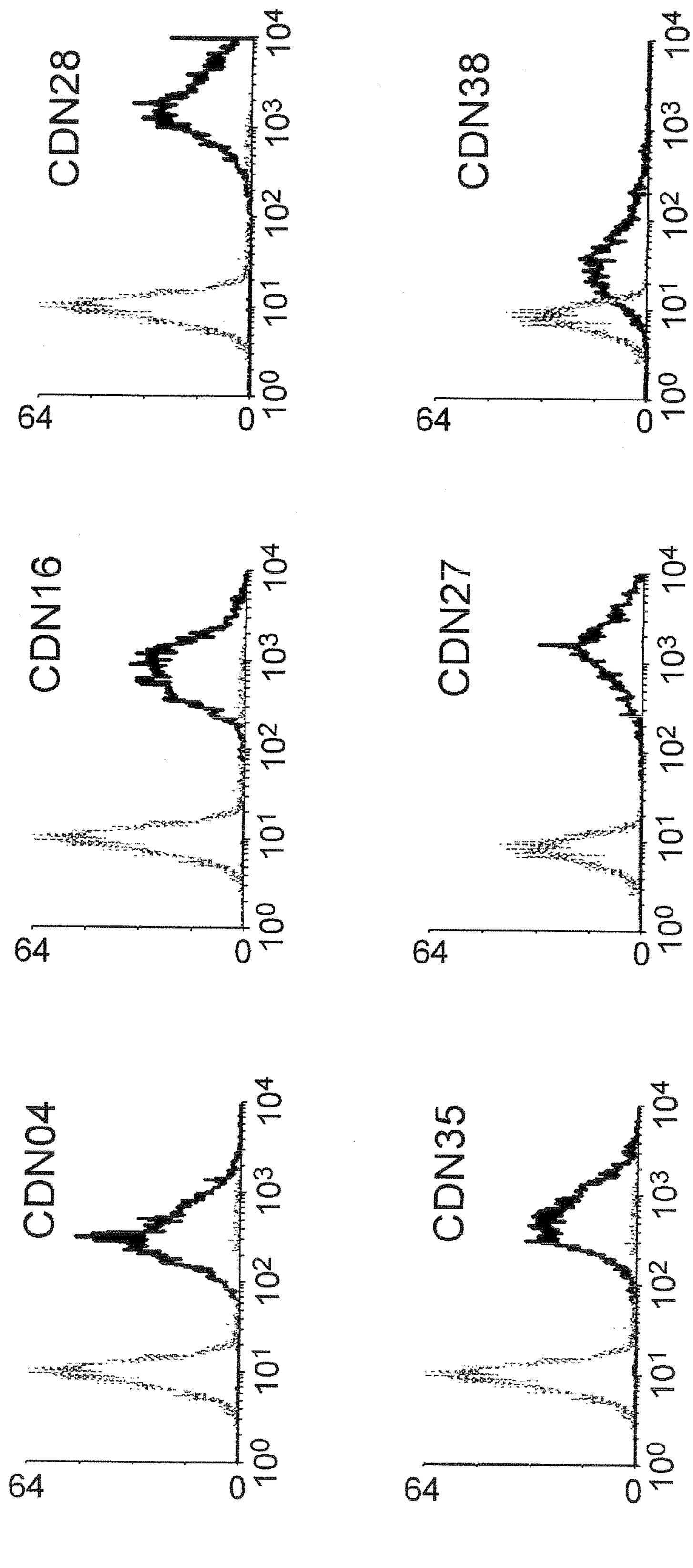


FIG. 11

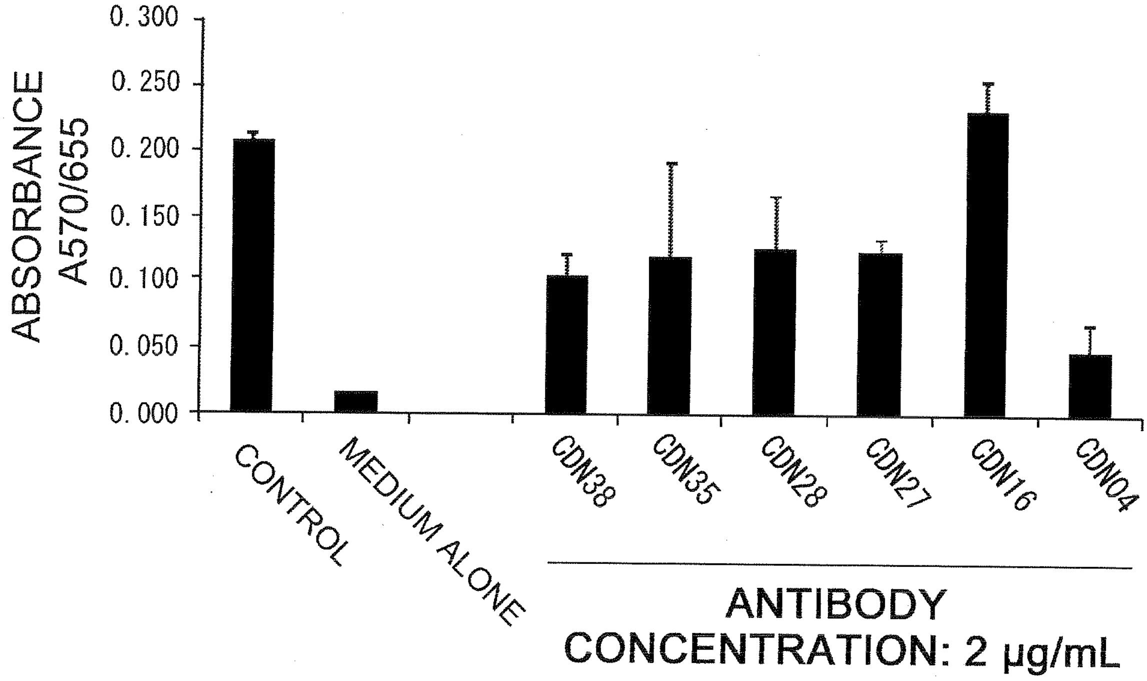
ABSORBANCE
A570/655
0. 300
0. 250
0. 200
0. 150
0. 100
0. 050
0. 000
CONTROL
MEDIUM ALONE
CDN38
CDN35
CDN28
CDN27
CDN16
CDN04
ANTIBODY
CONCENTRATION: 2 µg/mL

FIG. 12

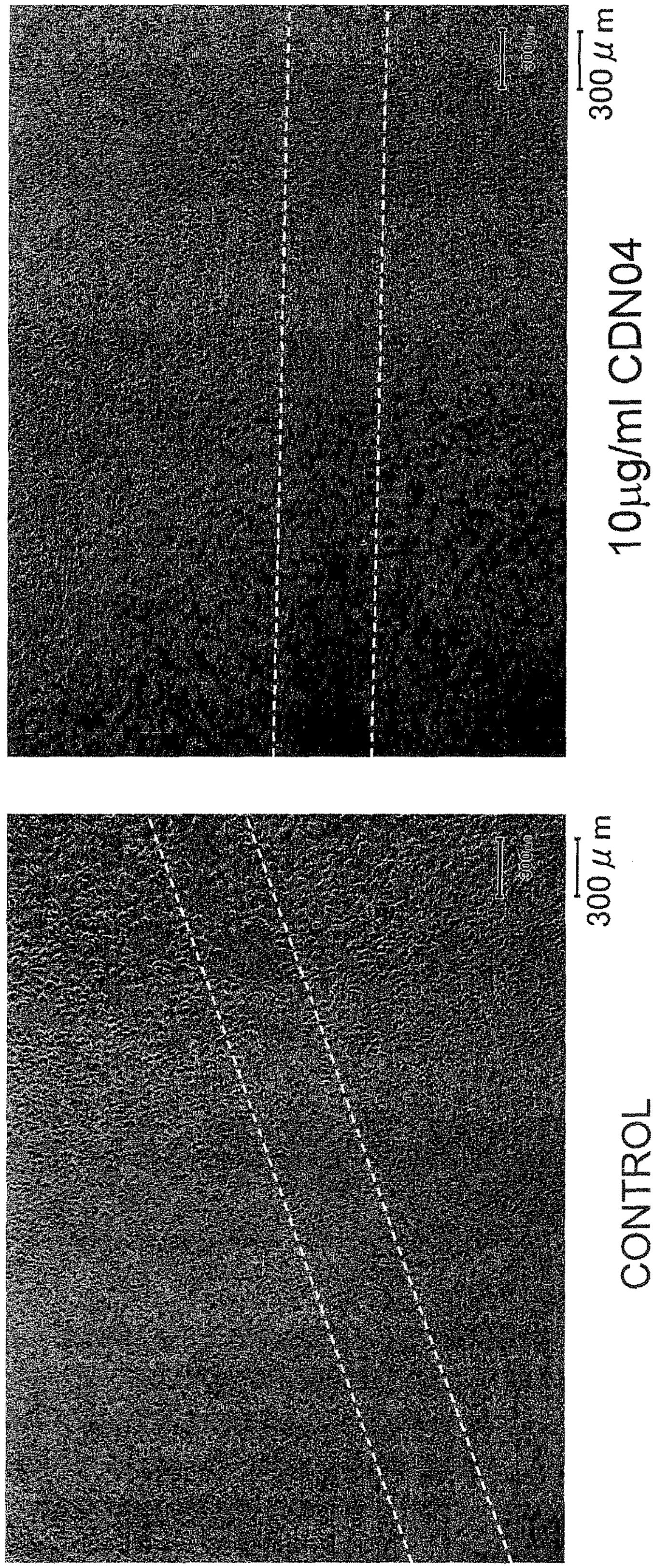


FIG. 13

**INTERNATIONAL SEARCH REPORT**

International application No.
PCT/JP2007/074081

A. CLASSIFICATION OF SUBJECT MATTER
*C07K16/28*(2006.01)i, *A61K39/395*(2006.01)i, *A61P35/00*(2006.01)i, *A61P35/04*(2006.01)i, *A61P43/00*(2006.01)i, *C07K16/46*(2006.01)i, *G01N33/574*(2006.01)i, *C12N15/13*(2006.01)n, *C12P21/08*(2006.01)n
According to International Patent Classification (IPC) or to both national classification and IPC

B. FIELDS SEARCHED
Minimum documentation searched (classification system followed by classification symbols)
C07K16/28, A61K39/395, A61P35/00, A61P35/04, A61P43/00, C07K16/46, G01N33/574, C12N15/13, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS/MEDLINE/WPIDS(STN), CAplus(STN), JMEDPlus(JDream2), JST7580(JDream2), JSTPlus(JDream2), Igaku·Yakugaku Yokoshu Zenbun Database

C. DOCUMENTS CONSIDERED TO BE RELEVANT

| Category* | Citation of document, with indication, where appropriate, of the relevant passages | Relevant to claim No. |
|---|---|---|
| Y | Morin P., Claudin proteins in human cancer: promising new targets for diagnosis and therapy., Cancer Res., 2005, 65(21), p.9603-9606 | 1-14,23-33, 36-37 |
| Y | WO 2005/121338 A1 (Kyowa Hakko Kogyo Co., Ltd.), 22 December, 2005 (22.12.05), Par. Nos. [0008], [0060], [0084] to [0087], [0197] to [0226], [0254] to [0262] & EP 1767627 A1 & AU 2005252521 A1 & CA 2573430 A1 | 1-14,23-33 36-37 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 February, 2008 (27.02.08) | 11 March, 2008 (11.03.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2007/074081

C (Continuation). DOCUMENTS CONSIDERED TO BE RELEVANT

| Category* | Citation of document, with indication, where appropriate, of the relevant passages | Relevant to claim No. |
|---|---|---|
| Y | Database GenBank, [online], Accession No.NM_001306.2, <http://www.ncbi.nlm.nih.gov/entrez/viewer.fcgi?21536298:OLD11:596053>, 21-Jun-2002 uploaded, [retrieved on 26-Feb-2008] Definition: Homo sapiens claudin 3 (CLDN3), mRNA | 1-14,23-33 36-37 |
| X | Offner S. et al., Epithelial tight junction proteins as potential antibody targets for pancarcinoma therapy., Cancer Immunol. Immunother., 2005, 54(5), p.431-445 | 1-2,4-6, 8-13,23-33 36-37 |
| X | Morita K. et al., Claudin multigene family encoding four-transmembrane domain protein components of tight junction strands., Proc. Natl. Acad. USA, 1999, 96(2), p.511-516 | 1 |
| A | Hoevel T. et al., Expression and targeting of the tight junction protein CLDN1 in CLDN1-negative human breast tumor cells., Journal of Cellular Physiology, 2002, 191(1), p.60-68 | 1-14,23-33 36-37 |
| A | Nakamura N. et al., Establishment of a chicken monoclonal antibody panel against mammalian prion protein., J. Vet. Med. Sci., 2004, 66(7), p.807-814 | 1-14,23-33 36-37 |
| A | Nishinaka S. et al., Two chiken B cell lines resistant to ouabain for the production of chicken monoclonal antibodies., J. Vet. Med. Sci., 1996, 58(11), p.1053-1056 | 1-14,23-33 36-37 |
| A | Asaoka H. & Matsuda H., Detection of N-Glycolylneuraminic acid-containing glycoprotein from various animal erythrocytes by chicken monoclonal antibody against Hanganutziu-Deicher antigens., J. Vet. Med. Sci., 1994, 56(2), p.375-377 | 1-14,23-33 36-37 |

Form PCT/ISA/210 (continuation of second sheet) (April 2007)

**INTERNATIONAL SEARCH REPORT**

International application No.
PCT/JP2007/074081

**Box No. II Observations where certain claims were found unsearchable (Continuation of item 2 of first sheet)**

This international search report has not been established in respect of certain claims under Article 17(2)(a) for the following reasons:

1. [X] Claims Nos.: 15-22, 34-35
because they relate to subject matter not required to be searched by this Authority, namely:
The inventions in the above claims pertain to diagnostic methods to be practiced on the human body or methods for treatment of the human body by therapy, and thus relate to a subject matter which this International Searching Authority is not required to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. [ ] As all required additional search fees were timely paid by the applicant, this international search report covers all searchable claims.
2. [X] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:
4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2007)

**INTERNATIONAL SEARCH REPORT**

International application No.
PCT/JP2007/074081

Continuation of Box No.III of continuation of first sheet(2)

A common matter among claims 1-14, 23-33 and 36-37 appears to be an antibody capable of binding to Claudin-3 protein or a matter relating to the antibody.

However, an antibody capable of binding to Claudin-3 protein is already known at the time of the priority date of the present application (see Cancer Immunol. Immunother., 2005, 54(5), p.431-445). Therefore, the common matter cannot be regarded as a special technical feature in the meaning within PCT Rule 13.2, second sentence.

Further, with regard to the inventions of claims 6-8, an antibody capable of recognizing a conformation formed by two extracellular loops of a protein comprising the amino acid sequence depicted in SEQ ID NO:2 expressed on a cell membrane and binding to the conformation is also already known at the time of the priority date of the present application (see Cancer Immunol. Immunother., 2005, 54, p.431-445).

Consequently, claims 1-14, 23-33 and 36-37 include the following six groups of inventions.

A. The inventions of claims 1-5 and parts of the inventions of claims 9-12 and 14 which relate to claims 1-5.

B. Parts of the inventions of claims 6-12 and 14 which relate to an antibody capable of recognizing a conformation formed by two extracellular loops of a protein comprising the amino acid sequence depicted in SEQ ID NO:2 expressed on a cell membrane and binding to the conformation.

C. Parts of the inventions of claims 6-12 and 14 which relate to an antibody capable of recognizing a conformation formed by two extracellular loops of a protein comprising the amino acid sequence depicted in SEQ ID NO:4 expressed on a cell membrane and binding to the conformation.

D. Parts of the inventions of claims 6-12 and 14 which relate to an antibody capable of recognizing a conformation formed by two extracellular loops of a protein comprising the amino acid sequence depicted in SEQ ID NO:8 expressed on a cell membrane and binding to the conformation.

E. The invention of claim 13 and a part of the invention of claim 14 which relates to claim 13.

F. The inventions of claims 23-33 and 36-37.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03104453 A
- WO 9411523 A
- EP 239400 A
- WO 9602576 A
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- EP 404097 A
- WO 9311161 A
- WO 9954342 A
- WO 0061739 A
- WO 0231140 A
- WO 0279255 A


### Non-patent literature cited in the description


- **Soini.** *Histopathology,* 2005, vol. 46, 551
- **Santin et al.** *Br. J. Cancer,* 2005, vol. 92, 1561
- **Offner et al.** *Cancer Immunol. Immunother.,* 2005, vol. 54, 431
- **Long et al.** *Cancer Res.,* 2001, vol. 61, 7878
- **Rangel et al.** *Clin. Cancer Res.,* 2003, vol. 9, 2567
- **Kominsky et al.** *Am. J. Path.,* 2004, vol. 164, 1627
- **Santin et al.** *Cancer Res.,* 2005, vol. 65, 4334
- **Hoevel et al.** *J. Cell. Physiol.,* 2002, vol. 191, 60
- Antibodies A Laboratory Manual. Cold Spring Harbor Laboratory, 1988, 359-420
- *J. Immunol.,* 1979, vol. 123, 1548-1550
- *Current Topics in Microbiology and Immunology,* 1978, vol. 81, 1-7
- **Kohler. G. ; Milstein, C.** *Eur. J. Immunol.,* 1976, vol. 6, 511-519
- **Margulies. D.H. et al.** *Cell,* 1976, vol. 8, 405-415
- **Shulman, M. et al.** *Nature,* 1978, vol. 276, 269-270
- **de St. Groth, S. F. et al.** *J. Immunol. Methods,* 1980, vol. 35, 1-21
- **Trowbridge, 1. S.** *J. Exp. Med.,* 1978, vol. 148, 313-323
- **Galfre, G. et al.** *Nature,* 1979, vol. 277, 131-133
- **Kohler. G. ; Milstein, C.** *Methods Enzymol.,* 1981, vol. 73, 3-46
- **Vandamme, A. M. et al.** *Eur. J. Biochem.,* 1990, vol. 192, 767-775
- **Chirgwin, J. M. et al.** *Biochemistry,* 1979, vol. 18, 5294-5299
- **Chomczynski, P. et al.** *Anal. Biochem.,* 1987, vol. 162, 156-159
- **Frohman, M. A. et al.** *Proc. Natl. Acad. Sci. USA,* 1988, vol. 85, 8998-9002
- **Belyavsky, A. et al.** *Nucleic Acids Res.,* 1989, vol. 17, 2919-2932
- **Ebert, K. M. et al.** *Bio/Technology,* 1994, vol. 12, 699-702
- **Sato, K. et al.** *Cancer Res.,* 1993, vol. 53, 851-856
- **Co, M. S. et al.** *J. Immunol.,* 1994, vol. 152, 2968-2976
- **Better, M. ; Horwitz, A. H.** *Methods in Enzymology,* 1989, vol. 178, 476-496
- **Plueckthun, A. ; Skerra, A.** *Methods in Enzymology,* 1989, vol. 178, 476-496
- **Lamoyi, E.** *Methods in Enzymology,* 1989, vol. 121, 652-663
- **Rousseaux, J. et al.** *Methods in Enzymology,* 1989, vol. 121, 663-669
- **Bird, R. E. et al.** *TIBTECH,* 1991, vol. 9, 132-137
- **Hollinger P. et al.** *Proc. Natl. Acad. Sci. USA,* 1993, vol. 90, 6444-6448
- **Huston, J. S. et al.** *Proc. Natl. Acad. Sci. U.S.A.,* 1988, vol. 85, 5879-5883
- **Hudson et al.** *J. Immunol. Methods,* 1999, vol. 231, 177-189
- *Protein Engineering,* 1996, vol. 9 (3), 299-305
- **Better, M. ; Horwitz, A. H.** *Methods Enzymol.,* 1989, vol. 178, 476-496

- **Pluckthun, A. ; Skerra, A.** *Methods Enzymol.,* 1989, vol. 178, 497-515
- **Lamoyi, E.** *Methods Enzymol.,* 1986, vol. 121, 652-663
- **Rousseaux, J. et al.** *Methods Enzymol.,* 1986, vol. 121, 663-669
- **Bird, R. E. ; Walker, B. W.** *Trends Biotechnol.,* 1991, vol. 9, 132-137
- **Hashimoto-Gotoh, T. et al.** *Gene,* 1995, vol. 152, 271-275
- **Zoller, MJ ; Smith, M.** *Methods Enzymol.,* 1983, vol. 100, 468-500
- **Kramer, W. et al.** *Nucleic Acids Res.,* 1984, vol. 12, 9441-9456
- **Kramer W ; Fritz HJ.** *Methods. Enzymol.,* 1987, vol. 154, 350-367
- **Kunkel, TA.** *Proc. Natl. Acad. Sci. USA.,* 1985, vol. 82, 488-492
- **Kunkel.** *Methods Enzymol.,* 1988, vol. 85, 2763-2766
- **Mark, D. F. et al.** *Proc. Natl. Acad. Sci. USA,* 1984, vol. 81, 5662-5666
- **Zoller, M. J. ; Smith, M.** *Nucleic Acids Research,* 1982, vol. 10, 6487-6500
- **Wang, A. et al.** *Science,* vol. 224, 1431-1433
- **Dalbadie-McFarland, G. et al.** *Proc. Natl. Acad. Sci. USA,* 1982, vol. 79, 6409-6413
- **Mulligan et al.** *Nature,* 1979, vol. 277, 108
- **Mizushima et al.** *Nucleic Acids Res.,* 1990, vol. 18, 5322
- **Ward et al.** *Nature,* 1989, vol. 341, 544-546
- *FASEB J.,* 1992, vol. 6, 2422-2427
- **Better et al.** *Science,* 1988, vol. 240, 1041-1043
- **Lei, S. P. et al.** *J. Bacteriol.,* 1987, vol. 169, 4379
- Antibodies A Laboratory Manual. Cold Spring Harbor Laboratory, 1988
- Current Protocols in Immunology. Immunologic studies in humans. John Wiley & Sons, Inc, 1993
- Remington's Pharmaceutical Science. Mark Publishing Company
- Current protocols in Immunology. Immunologic studies in humans. John Wiley & Sons, Inc, 1993
- **Agarwal et al.** *Cancer Res,* 2005, vol. 65, 7378-7385
- **Michl et al.** *Cancer Res,* 2003, vol. 63, 6265-6271

SEQUENCE LISTING

<110> FORERUNNER PHARMA RESEARCH CO., LTD.

<120> Anti-Claudin 3 monoclonal antibody and treatment and diagnosis of cancer using the same

<130> R2075 EP S3

<140> EP 07 85 0593.0
<141> 2007-12-14

<150> JP 2006-336555
<151> 2006-12-14

<160> 188

<170> PatentIn version 3.3

<210> 1
<211> 663
<212> DNA
<213> Homo sapiens

<400> 1
atgtccatgg gcctggagat cacgggcacc gcgctggccg tgctgggctg gctgggcacc 60

atcgtgtgct gcgcgttgcc catgtggcgc gtgtcggcct tcatcggcag caacatcatc 120

acgtcgcaga acatctggga gggcctgtgg atgaactgcg tggtgcagag caccggccag 180

atgcagtgca aggtgtacga ctcgctgctg gcactgccac aggaccttca ggcggcccgc 240

gccctcatcg tggtggccat cctgctggcc gccttcgggc tgctagtggc gctggtgggc 300

gcccagtgca ccaactgcgt gcaggacgac acggccaagg ccaagatcac catcgtggca 360

ggcgtgctgt tccttctcgc cgccctgctc accctcgtgc cggtgtcctg gtcggccaac 420

accattatcc gggacttcta caaccccgtg gtgcccgagg cgcagaagcg cgagatgggc 480

gcgggcctgt acgtgggctg ggcggccgcg gcgctgcagc tgctgggggg cgcgctgctc 540

tgctgctcgt gtcccccacg cgagaagaag tacacggcca ccaaggtcgt ctactccgcg 600

ccgcgctcca ccggcccggg agccagcctg ggcacaggct acgaccgcaa ggactacgtc 660

taa 663

<210> 2
<211> 220
<212> PRT
<213> Homo sapiens

<400> 2

Met Ser Met Gly Leu Glu Ile Thr Gly Thr Ala Leu Ala Val Leu Gly
1               5                   10                  15

```
Trp Leu Gly Thr Ile Val Cys Cys Ala Leu Pro Met Trp Arg Val Ser
            20                  25                  30

Ala Phe Ile Gly Ser Asn Ile Ile Thr Ser Gln Asn Ile Trp Glu Gly
        35                  40                  45

Leu Trp Met Asn Cys Val Val Gln Ser Thr Gly Gln Met Gln Cys Lys
    50                  55                  60

Val Tyr Asp Ser Leu Leu Ala Leu Pro Gln Asp Leu Gln Ala Ala Arg
65                  70                  75                  80

Ala Leu Ile Val Val Ala Ile Leu Leu Ala Ala Phe Gly Leu Leu Val
                85                  90                  95

Ala Leu Val Gly Ala Gln Cys Thr Asn Cys Val Gln Asp Asp Thr Ala
            100                 105                 110

Lys Ala Lys Ile Thr Ile Val Ala Gly Val Leu Phe Leu Leu Ala Ala
        115                 120                 125

Leu Leu Thr Leu Val Pro Val Ser Trp Ser Ala Asn Thr Ile Ile Arg
    130                 135                 140

Asp Phe Tyr Asn Pro Val Val Pro Glu Ala Gln Lys Arg Glu Met Gly
145                 150                 155                 160

Ala Gly Leu Tyr Val Gly Trp Ala Ala Ala Ala Leu Gln Leu Leu Gly
                165                 170                 175

Gly Ala Leu Leu Cys Cys Ser Cys Pro Pro Arg Glu Lys Lys Tyr Thr
            180                 185                 190

Ala Thr Lys Val Val Tyr Ser Ala Pro Arg Ser Thr Gly Pro Gly Ala
        195                 200                 205

Ser Leu Gly Thr Gly Tyr Asp Arg Lys Asp Tyr Val
    210                 215                 220
```

<210> 3
<211> 660
<212> DNA
<213> Mus musculus

<400> 3
atgtccatgg gcctggagat caccggcacg tcgctggccg tgctgggctg gctgtgcacc 60

atcgtgtgct gcgcccttcc catgtggcgc gtttcggcct tcatcggcag cagcatcatc 120

acggcgcaga tcacctggga gggcctgtgg atgaactgcg tggtgcagag caccggtcag 180

atgcagtgca aaatgtacga ctcgctgctg gccctgccgc aggacctgca ggccgcccga 240

gccctcatcg tggtgtccat cctgctggcc gccttcgggc tcctcgtggc gctcgtgggc 300

gcccagtgta ccaactgcgt acaagacgag acggccaagg ccaagatcac catcgtggcg 360

ggagtgcttt tcctgttggc ggctctgctc accttagtac cggtgtcctg gtcggccaac 420

accatcatca gggatttcta taacccgttg gtgcccgagg cccagaagcg ggagatggga 480

gctgggttgt acgtgggctg ggctgccgcc gcgctgcagt tgctaggggg cgccttgctg 540

tgttgctcct gcccaccgcg cgacaagtat gcacccacca agatcctcta ttctgcgccg 600

cgatccaccg gccctggcac cggtaccggc accgcctacg accgcaagga ctacgtctga 660

<210> 4
<211> 219
<212> PRT
<213> Mus musculus

<400> 4

Met Ser Met Gly Leu Glu Ile Thr Gly Thr Ser Leu Ala Val Leu Gly
1               5                   10                  15

Trp Leu Cys Thr Ile Val Cys Cys Ala Leu Pro Met Trp Arg Val Ser
            20                  25                  30

Ala Phe Ile Gly Ser Ser Ile Ile Thr Ala Gln Ile Thr Trp Glu Gly
        35                  40                  45

Leu Trp Met Asn Cys Val Val Gln Ser Thr Gly Gln Met Gln Cys Lys
    50                  55                  60

Met Tyr Asp Ser Leu Leu Ala Leu Pro Gln Asp Leu Gln Ala Ala Arg
65                  70                  75                  80

Ala Leu Ile Val Val Ser Ile Leu Leu Ala Ala Phe Gly Leu Leu Val
                85                  90                  95

Ala Leu Val Gly Ala Gln Cys Thr Asn Cys Val Gln Asp Glu Thr Ala
            100                 105                 110

Lys Ala Lys Ile Thr Ile Val Ala Gly Val Leu Phe Leu Leu Ala Ala
        115                 120                 125

```
Leu Leu Thr Leu Val Pro Val Ser Trp Ser Ala Asn Thr Ile Ile Arg
    130                 135                 140

Asp Phe Tyr Asn Pro Leu Val Pro Glu Ala Gln Lys Arg Glu Met Gly
145                 150                 155                 160

Ala Gly Leu Tyr Val Gly Trp Ala Ala Ala Ala Leu Gln Leu Leu Gly
                165                 170                 175

Gly Ala Leu Leu Cys Cys Ser Cys Pro Pro Arg Asp Lys Tyr Ala Pro
            180                 185                 190

Thr Lys Ile Leu Tyr Ser Ala Pro Arg Ser Thr Gly Pro Gly Thr Gly
        195                 200                 205

Thr Gly Thr Ala Tyr Asp Arg Lys Asp Tyr Val
    210                 215


<210>  5
<211>  681
<212>  DNA
<213>  Homo sapiens

<400>  5
atggccaacg cggggctgca gctgttgggc ttcattctcg ccttcctggg atggatcggc      60

gccatcgtca gcactgccct gccccagtgg aggatttact cctatgccgg cgacaacatc     120

gtgaccgccc aggccatgta cgaggggctg tggatgtcct gcgtgtcgca gagcaccggg     180

cagatccagt gcaaagtctt tgactccttg ctgaatctga gcagcacatt gcaagcaacc     240

cgtgccttga tggtggttgg catcctcctg ggagtgatag caatctttgt ggccaccgtt     300

ggcatgaagt gtatgaagtg cttggaagac gatgaggtgc agaagatgag gatggctgtc     360

attgggggcg cgatatttct tcttgcaggt ctggctattt tagttgccac agcatggtat     420

ggcaatagaa tcgttcaaga attctatgac cctatgaccc cagtcaatgc caggtacgaa     480

tttggtcagg ctctcttcac tggctgggct gctgcttctc tctgccttct gggaggtgcc     540

ctactttgct gttcctgtcc ccgaaaaaca acctcttacc caacaccaag gccctatcca     600

aaacctgcac cttccagcgg gaaagactac gtggctagcg atatcgcggc cgctgactac     660

aaagacgatg acgacaagtg a                                               681


<210>  6
<211>  226
<212>  PRT
<213>  Homo sapiens
```

```
Met Ala Asn Ala Gly Leu Gln Leu Leu Gly Phe Ile Leu Ala Phe Leu
1               5                   10                  15

Gly Trp Ile Gly Ala Ile Val Ser Thr Ala Leu Pro Gln Trp Arg Ile
            20                  25                  30

Tyr Ser Tyr Ala Gly Asp Asn Ile Val Thr Ala Gln Ala Met Tyr Glu
        35                  40                  45

Gly Leu Trp Met Ser Cys Val Ser Gln Ser Thr Gly Gln Ile Gln Cys
    50                  55                  60

Lys Val Phe Asp Ser Leu Leu Asn Leu Ser Ser Thr Leu Gln Ala Thr
65                  70                  75                  80

Arg Ala Leu Met Val Val Gly Ile Leu Leu Gly Val Ile Ala Ile Phe
                85                  90                  95

Val Ala Thr Val Gly Met Lys Cys Met Lys Cys Leu Glu Asp Asp Glu
            100                 105                 110

Val Gln Lys Met Arg Met Ala Val Ile Gly Gly Ala Ile Phe Leu Leu
        115                 120                 125

Ala Gly Leu Ala Ile Leu Val Ala Thr Ala Trp Tyr Gly Asn Arg Ile
    130                 135                 140

Val Gln Glu Phe Tyr Asp Pro Met Thr Pro Val Asn Ala Arg Tyr Glu
145                 150                 155                 160

Phe Gly Gln Ala Leu Phe Thr Gly Trp Ala Ala Ala Ser Leu Cys Leu
                165                 170                 175

Leu Gly Gly Ala Leu Leu Cys Cys Ser Cys Pro Arg Lys Thr Thr Ser
            180                 185                 190

Tyr Pro Thr Pro Arg Pro Tyr Pro Lys Pro Ala Pro Ser Ser Gly Lys
        195                 200                 205

Asp Tyr Val Ala Ser Asp Ile Ala Ala Ala Asp Tyr Lys Asp Asp Asp
    210                 215                 220

Asp Lys
```

225

<210> 7
<211> 675
<212> DNA
<213> Homo sapiens

<400> 7
atggcctcca tggggctaca ggtaatgggc atcgcgctgg ccgtcctggg ctggctggcc 60

gtcatgctgt gctgcgcgct gcccatgtgg cgcgtgacgg ccttcatcgg cagcaacatt 120

gtcacctcgc agaccatctg ggagggccta tggatgaact gcgtggtgca gagcaccggc 180

cagatgcagt gcaaggtgta cgactcgctg ctggcactgc cgcaggacct gcaggcggcc 240

cgcgccctcg tcatcatcag catcatcgtg gctgctctgg gcgtgctgct gtccgtggtg 300

gggggcaagt gtaccaactg cctggaggat gaaagcgcca aggccaagac catgatcgtg 360

gcgggcgtgg tgttcctgtt ggccggcctt atggtgatag tgccggtgtc ctggacggcc 420

cacaacatca tccaagactt ctacaatccg ctggtggcct ccgggcagaa gcgggagatg 480

ggtgcctcgc tctacgtcgg ctgggccgcc tccggcctgc tgctccttgg cggggggctg 540

ctttgctgca actgtccacc ccgcacagac aagccttact ccgccaagta ttctgctgcc 600

cgctctgctg ctgccagcaa ctacgtggct agcgatatcg cggccgctga ctacaaagac 660

gatgacgaca agtga 675

<210> 8
<211> 224
<212> PRT
<213> Homo sapiens

<400> 8

Met Ala Ser Met Gly Leu Gln Val Met Gly Ile Ala Leu Ala Val Leu
1 5 10 15

Gly Trp Leu Ala Val Met Leu Cys Cys Ala Leu Pro Met Trp Arg Val
20 25 30

Thr Ala Phe Ile Gly Ser Asn Ile Val Thr Ser Gln Thr Ile Trp Glu
35 40 45

Gly Leu Trp Met Asn Cys Val Val Gln Ser Thr Gly Gln Met Gln Cys
50 55 60

Lys Val Tyr Asp Ser Leu Leu Ala Leu Pro Gln Asp Leu Gln Ala Ala
65 70 75 80

```
Arg Ala Leu Val Ile Ile Ser Ile Ile Val Ala Ala Leu Gly Val Leu
                85                  90                  95

Leu Ser Val Val Gly Gly Lys Cys Thr Asn Cys Leu Glu Asp Glu Ser
            100                 105                 110

Ala Lys Ala Lys Thr Met Ile Val Ala Gly Val Val Phe Leu Leu Ala
        115                 120                 125

Gly Leu Met Val Ile Val Pro Val Ser Trp Thr Ala His Asn Ile Ile
    130                 135                 140

Gln Asp Phe Tyr Asn Pro Leu Val Ala Ser Gly Gln Lys Arg Glu Met
145                 150                 155                 160

Gly Ala Ser Leu Tyr Val Gly Trp Ala Ala Ser Gly Leu Leu Leu Leu
                165                 170                 175

Gly Gly Gly Leu Leu Cys Cys Asn Cys Pro Pro Arg Thr Asp Lys Pro
            180                 185                 190

Tyr Ser Ala Lys Tyr Ser Ala Ala Arg Ser Ala Ala Ala Ser Asn Tyr
        195                 200                 205

Val Ala Ser Asp Ile Ala Ala Ala Asp Tyr Lys Asp Asp Asp Asp Lys
    210                 215                 220
```

<210> 9
<211> 708
<212> DNA
<213> Homo sapiens

<400> 9

```
atggcctctg ccggaatgca gatcctggga gtcgtcctga cactgctggg ctgggtgaat      60

ggcctggtct cctgtgccct gcccatgtgg aaggtgaccg ctttcatcgg caacagcatc     120

gtggtggccc aggtggtgtg ggagggcctg tggatgtcct gcgtggtgca gagcaccggc     180

cagatgcagt gcaaggtgta cgactcactg ctggcgctgc cacaggacct gcaggctgca     240

cgtgccctct gtgtcatcgc cctccttgtg gccctgttcg gcttgctggt ctaccttgct     300

ggggccaagt gtaccacctg tgtggaggag aaggattcca aggcccgcct ggtgctcacc     360

tctgggattg tctttgtcat ctcaggggtc ctgacgctaa tccccgtgtg ctggacggcg     420

catgccgtca tccgggactt ctataacccc ctggtggctg aggcccaaaa gcgggagctg     480

ggggcctccc tctacttggg ctgggcggcc tcaggccttt tgttgctggg tggggggttg     540
```

```
ctgtgctgca cttgcccctc ggggggggtcc cagggcccca gccattacat ggcccgctac    600

tcaacatctg cccctgccat ctctcggggg ccctctgagt accctaccaa gaattacgtc    660

gctagcgata tcgcggccgc tgactacaaa gacgatgacg acaagtga                 708


<210>  10
<211>  235
<212>  PRT
<213>  Homo sapiens

<400>  10

Met Ala Ser Ala Gly Met Gln Ile Leu Gly Val Val Leu Thr Leu Leu
1               5                   10                  15


Gly Trp Val Asn Gly Leu Val Ser Cys Ala Leu Pro Met Trp Lys Val
            20                  25                  30


Thr Ala Phe Ile Gly Asn Ser Ile Val Val Ala Gln Val Val Trp Glu
        35                  40                  45


Gly Leu Trp Met Ser Cys Val Val Gln Ser Thr Gly Gln Met Gln Cys
    50                  55                  60


Lys Val Tyr Asp Ser Leu Leu Ala Leu Pro Gln Asp Leu Gln Ala Ala
65                  70                  75                  80


Arg Ala Leu Cys Val Ile Ala Leu Leu Val Ala Leu Phe Gly Leu Leu
                85                  90                  95


Val Tyr Leu Ala Gly Ala Lys Cys Thr Thr Cys Val Glu Glu Lys Asp
            100                 105                 110


Ser Lys Ala Arg Leu Val Leu Thr Ser Gly Ile Val Phe Val Ile Ser
        115                 120                 125


Gly Val Leu Thr Leu Ile Pro Val Cys Trp Thr Ala His Ala Val Ile
    130                 135                 140


Arg Asp Phe Tyr Asn Pro Leu Val Ala Glu Ala Gln Lys Arg Glu Leu
145                 150                 155                 160


Gly Ala Ser Leu Tyr Leu Gly Trp Ala Ala Ser Gly Leu Leu Leu Leu
                165                 170                 175


Gly Gly Gly Leu Leu Cys Cys Thr Cys Pro Ser Gly Gly Ser Gln Gly
```

180 185 190

Pro Ser His Tyr Met Ala Arg Tyr Ser Thr Ser Ala Pro Ala Ile Ser
195 200 205

Arg Gly Pro Ser Glu Tyr Pro Thr Lys Asn Tyr Val Ala Ser Asp Ile
210 215 220

Ala Ala Ala Asp Tyr Lys Asp Asp Asp Asp Lys
225 230 235

<210> 11
<211> 15
<212> DNA
<213> Mus musculus

<400> 11
ggctacacca tgaac 15

<210> 12
<211> 5
<212> PRT
<213> Mus musculus

<400> 12

Gly Tyr Thr Met Asn
1 5

<210> 13
<211> 51
<212> DNA
<213> Mus musculus

<400> 13
cttattaatc cttacaatgg tggtactagc tacaaccaga agttcaagga c 51

<210> 14
<211> 17
<212> PRT
<213> Mus musculus

<400> 14

Leu Ile Asn Pro Tyr Asn Gly Gly Thr Ser Tyr Asn Gln Lys Phe Lys
1 5 10 15

Asp

<210> 15

<211> 30
<212> DNA
<213> Mus musculus

<400> 15
gggtcctacg gtagtagcta ctttgactac 30

<210> 16
<211> 10
<212> PRT
<213> Mus musculus

<400> 16

Gly Ser Tyr Gly Ser Ser Tyr Phe Asp Tyr
1 5 10

<210> 17
<211> 330
<212> DNA
<213> Mus musculus

<400> 17
gagctggtga agcctggagc ttcaatgaag atatcctgca aggcttctgg ttactcattc 60

actggctaca ccatgaactg gatgaagcag ggccatggaa agaaccttga gtggattgga 120

cttattaatc cttacaatgg tggtactagc tacaaccaga agttcaagga caaggccaca 180

ttaactttag acaagtcatc cagttcagcc tacatggagc tcctcagtct gacatctgag 240

gactctgcag tctattactg tgcaagaggg tcctacggta gtagctactt tgactactgg 300

ggccaaggca ccactctcac agtctcctca 330

<210> 18
<211> 110
<212> PRT
<213> Mus musculus

<400> 18

Glu Leu Val Lys Pro Gly Ala Ser Met Lys Ile Ser Cys Lys Ala Ser
1 5 10 15

Gly Tyr Ser Phe Thr Gly Tyr Thr Met Asn Trp Met Lys Gln Gly His
20 25 30

Gly Lys Asn Leu Glu Trp Ile Gly Leu Ile Asn Pro Tyr Asn Gly Gly
35 40 45

Thr Ser Tyr Asn Gln Lys Phe Lys Asp Lys Ala Thr Leu Thr Leu Asp
50 55 60

Lys Ser Ser Ser Ser Ala Tyr Met Glu Leu Leu Ser Leu Thr Ser Glu
65 70 75 80

Asp Ser Ala Val Tyr Tyr Cys Ala Arg Gly Ser Tyr Gly Ser Ser Tyr
85 90 95

Phe Asp Tyr Trp Gly Gln Gly Thr Thr Leu Thr Val Ser Ser
100 105 110

<210> 19
<211> 1389
<212> DNA
<213> Mus musculus

<220>
<221> sig_peptide
<222> (1)..(84)

<400> 19
atgggatgga gctggatctt tctcttcctc ctgtcaggaa ctgcaggtgt ccactctgag 60
gtccagctgc aacagtctgg acctgagctg gtgaagcctg gagcttcaat gaagatatcc 120
tgcaaggctt ctggttactc attcactggc tacaccatga actggatgaa gcagggccat 180
ggaaagaacc ttgagtggat tggacttatt aatccttaca atggtggtac tagctacaac 240
cagaagttca aggacaaggc cacattaact ttagacaagt catccagttc agcctacatg 300
gagctcctca gtctgacatc tgaggactct gcagtctatt actgtgcaag agggtcctac 360
ggtagtagct actttgacta ctggggccaa ggcaccactc tcacagtctc ctcagccaaa 420
acgacacccc catctgtcta tccactggcc cctggatctg ctgcccaaac taactccatg 480
gtgaccctgg gatgcctggt caagggctat ttccctgagc cagtgacagt gacctggaac 540
tctggatccc tgtccagcgg tgtgcacacc ttcccagctg tcctgcagtc tgacctctac 600
actctgagca gctcagtgac tgtcccctcc agcacctggc ccagcgagac cgtcacctgc 660
aacgttgccc acccggccag cagcaccaag gtggacaaga aaattgtgcc cagggattgt 720
ggttgtaagc cttgcatatg tacagtccca gaagtatcat ctgtcttcat cttcccccca 780
aagcccaagg atgtgctcac cattactctg actcctaagg tcacgtgtgt tgtggtagac 840
atcagcaagg atgatcccga ggtccagttc agctggtttg tagatgatgt ggaggtgcac 900
acagctcaga cgcaaccccg ggaggagcag ttcaacagca ctttccgctc agtcagtgaa 960
cttcccatca tgcaccagga ctggctcaat ggcaaggagt tcaaatgcag ggtcaacagt 1020
gcagctttcc ctgcccccat cgagaaaacc atctccaaaa ccaaaggcag accgaaggct 1080

```
ccacaggtgt acaccattcc acctcccaag gagcagatgg ccaaggataa agtcagtctg   1140

acctgcatga taacagactt cttccctgaa gacattactg tggagtggca gtggaatggg   1200

cagccagcgg agaactacaa gaacactcag cccatcatga acacgaatgg ctcttacttc   1260

gtctacagca agctcaatgt gcagaagagc aactgggagg caggaaatac tttcacctgc   1320

tctgtcttac atgagggcct gcacaaccac catactgaga agagcctctc ccactctcct   1380

ggtaaataa                                                           1389


<210>  20
<211>  462
<212>  PRT
<213>  Mus musculus


<220>
<221>  SIGNAL
<222>  (1)..(28)

<400>  20

Met Gly Trp Ser Trp Ile Phe Leu Phe Leu Leu Ser Gly Thr Ala Gly
1               5                   10                  15


Val His Ser Glu Val Gln Leu Gln Gln Ser Gly Pro Glu Leu Val Lys
            20                  25                  30


Pro Gly Ala Ser Met Lys Ile Ser Cys Lys Ala Ser Gly Tyr Ser Phe
        35                  40                  45


Thr Gly Tyr Thr Met Asn Trp Met Lys Gln Gly His Gly Lys Asn Leu
    50                  55                  60


Glu Trp Ile Gly Leu Ile Asn Pro Tyr Asn Gly Gly Thr Ser Tyr Asn
65                  70                  75                  80


Gln Lys Phe Lys Asp Lys Ala Thr Leu Thr Leu Asp Lys Ser Ser Ser
                85                  90                  95


Ser Ala Tyr Met Glu Leu Leu Ser Leu Thr Ser Glu Asp Ser Ala Val
            100                 105                 110


Tyr Tyr Cys Ala Arg Gly Ser Tyr Gly Ser Ser Tyr Phe Asp Tyr Trp
        115                 120                 125


Gly Gln Gly Thr Thr Leu Thr Val Ser Ser Ala Lys Thr Thr Pro Pro
    130                 135                 140
```

```
Ser Val Tyr Pro Leu Ala Pro Gly Ser Ala Ala Gln Thr Asn Ser Met
145                 150                 155                 160

Val Thr Leu Gly Cys Leu Val Lys Gly Tyr Phe Pro Glu Pro Val Thr
                165                 170                 175

Val Thr Trp Asn Ser Gly Ser Leu Ser Ser Gly Val His Thr Phe Pro
            180                 185                 190

Ala Val Leu Gln Ser Asp Leu Tyr Thr Leu Ser Ser Ser Val Thr Val
        195                 200                 205

Pro Ser Ser Thr Trp Pro Ser Glu Thr Val Thr Cys Asn Val Ala His
    210                 215                 220

Pro Ala Ser Ser Thr Lys Val Asp Lys Lys Ile Val Pro Arg Asp Cys
225                 230                 235                 240

Gly Cys Lys Pro Cys Ile Cys Thr Val Pro Glu Val Ser Ser Val Phe
                245                 250                 255

Ile Phe Pro Pro Lys Pro Lys Asp Val Leu Thr Ile Thr Leu Thr Pro
            260                 265                 270

Lys Val Thr Cys Val Val Val Asp Ile Ser Lys Asp Asp Pro Glu Val
        275                 280                 285

Gln Phe Ser Trp Phe Val Asp Asp Val Glu Val His Thr Ala Gln Thr
    290                 295                 300

Gln Pro Arg Glu Glu Gln Phe Asn Ser Thr Phe Arg Ser Val Ser Glu
305                 310                 315                 320

Leu Pro Ile Met His Gln Asp Trp Leu Asn Gly Lys Glu Phe Lys Cys
                325                 330                 335

Arg Val Asn Ser Ala Ala Phe Pro Ala Pro Ile Glu Lys Thr Ile Ser
            340                 345                 350

Lys Thr Lys Gly Arg Pro Lys Ala Pro Gln Val Tyr Thr Ile Pro Pro
        355                 360                 365

Pro Lys Glu Gln Met Ala Lys Asp Lys Val Ser Leu Thr Cys Met Ile
    370                 375                 380
```

```
Thr Asp Phe Phe Pro Glu Asp Ile Thr Val Glu Trp Gln Trp Asn Gly
385                 390                 395                 400

Gln Pro Ala Glu Asn Tyr Lys Asn Thr Gln Pro Ile Met Asn Thr Asn
                405                 410                 415

Gly Ser Tyr Phe Val Tyr Ser Lys Leu Asn Val Gln Lys Ser Asn Trp
            420                 425                 430

Glu Ala Gly Asn Thr Phe Thr Cys Ser Val Leu His Glu Gly Leu His
        435                 440                 445

Asn His His Thr Glu Lys Ser Leu Ser His Ser Pro Gly Lys
    450                 455                 460


<210>  21
<211>  993
<212>  DNA
<213>  Homo sapiens

<400>  21
gctagcacca agggcccatc ggtcttcccc ctggcaccct cctccaagag cacctctggg      60

ggcacagcgg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacccagacc     240

tacatctgca acgtgaatca caagcccagc aacaccaagg tggacaagaa agttgagccc     300

aaatcttgtg acaaaactca cacatgccca ccgtgcccag cacctgaact cctgggggga     360

ccgtcagtct tcctcttccc cccaaaaccc aaggacaccc tcatgatctc ccggacccct     420

gaggtcacat gcgtggtggt ggacgtgagc cacgaagacc ctgaggtcaa gttcaactgg     480

tacgtggacg gcgtggaggt gcataatgcc aagacaaagc cgcgggagga gcagtacaac     540

agcacgtacc gtgtggtcag cgtcctcacc gtcctgcacc aggactggct gaatggcaag     600

gagtacaagt gcaaggtctc caacaaagcc ctcccagccc ccatcgagaa aaccatctcc     660

aaagccaaag ggcagccccg agaaccacag gtgtacaccc tgcccccatc ccgggatgag     720

ctgaccaaga accaggtcag cctgacctgc ctggtcaaag gcttctatcc cagcgacatc     780

gccgtggagt gggagagcaa tgggcagccg gagaacaact acaagaccac gcctcccgtg     840

ctggactccg acggctcctt cttcctctac agcaagctca ccgtggacaa gagcaggtgg     900

cagcagggga acgtcttctc atgctccgtg atgcatgagg ctctgcacaa ccactacacg     960

cagaagagcc tctccctgtc tccgggtaaa tga                                  993
```

```
<210>  22
<211>  330
<212>  PRT
<213>  Homo sapiens

<400>  22

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205
```

```
Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330


<210>  23
<211>  51
<212>  DNA
<213>  Mus musculus

<400>  23
aagtccagtc agagcctttt atacggtagc aatcaaaaga actacttggc c              51


<210>  24
<211>  17
<212>  PRT
<213>  Mus musculus

<400>  24

Lys Ser Ser Gln Ser Leu Leu Tyr Gly Ser Asn Gln Lys Asn Tyr Leu
1               5                   10                  15

Ala


<210>  25
<211>  21
<212>  DNA
```

<213> Mus musculus

<400> 25
tgggcatcca ctagggaatc t 21

<210> 26
<211> 7
<212> PRT
<213> Mus musculus

<400> 26

Trp Ala Ser Thr Arg Glu Ser
1 5

<210> 27
<211> 27
<212> DNA
<213> Mus musculus

<400> 27
caacaatatt ataactttcc gtacacg 27

<210> 28
<211> 9
<212> PRT
<213> Mus musculus

<400> 28

Gln Gln Tyr Tyr Asn Phe Pro Tyr Thr
1 5

<210> 29
<211> 342
<212> DNA
<213> Mus musculus

<400> 29
gacattgtga tgtcacagtc tccatcctcc ctagctgtgt cagttggaga gaaggttact 60

atgagttgta agtccagtca gagcctttta tacggtagca atcaaaagaa ctacttggcc 120

tggtaccagc agaaaccagg gcagtctcct aaactgctga tttactgggc atccactagg 180

gaatctgggg tccctgatcg cttcacaggc agtggatctg ggacagattt cactctcacc 240

atcagcagtg tgaaggctga agacctggca gtttattact gtcaacaata ttataacttt 300

ccgtacacgt tcggaggggg gaccaagctg gaaataaaac gg 342

<210> 30
<211> 114
<212> PRT
<213> Mus musculus

<400> 30

Asp Ile Val Met Ser Gln Ser Pro Ser Ser Leu Ala Val Ser Val Gly
1 5 10 15

Glu Lys Val Thr Met Ser Cys Lys Ser Ser Gln Ser Leu Leu Tyr Gly
20 25 30

Ser Asn Gln Lys Asn Tyr Leu Ala Trp Tyr Gln Gln Lys Pro Gly Gln
35 40 45

Ser Pro Lys Leu Leu Ile Tyr Trp Ala Ser Thr Arg Glu Ser Gly Val
50 55 60

Pro Asp Arg Phe Thr Gly Ser Gly Ser Gly Thr Asp Phe Thr Leu Thr
65 70 75 80

Ile Ser Ser Val Lys Ala Glu Asp Leu Ala Val Tyr Tyr Cys Gln Gln
85 90 95

Tyr Tyr Asn Phe Pro Tyr Thr Phe Gly Gly Gly Thr Lys Leu Glu Ile
100 105 110

Lys Arg

<210> 31
<211> 723
<212> DNA
<213> Mus musculus

<220>
<221> sig_peptide
<222> (1)..(60)

<400> 31
atggattcac aggcccaggt tcttatgtta ctgctgctat gggtatctgg tacctgtggg 60

gacattgtga tgtcacagtc tccatcctcc ctagctgtgt cagttggaga gaaggttact 120

atgagttgta agtccagtca gagcctttta tacggtagca atcaaaagaa ctacttggcc 180

tggtaccagc agaaaccagg gcagtctcct aaactgctga tttactgggc atccactagg 240

gaatctgggg tccctgatcg cttcacaggc agtggatctg ggacagattt cactctcacc 300

atcagcagtg tgaaggctga agacctggca gtttattact gtcaacaata ttataacttt 360

ccgtacacgt tcggaggggg gaccaagctg gaaataaaac gggctgatgc tgcaccaact 420

gtatccatct tcccaccatc cagtgagcag ttaacatctg gaggtgcctc agtcgtgtgc 480

ttcttgaaca acttctaccc caaagacatc aatgtcaagt ggaagattga tggcagtgaa 540

cgacaaaatg gcgtcctgaa cagttggact gatcaggaca gcaaagacag cacctacagc 600

atgagcagca ccctcacgtt gaccaaggac gagtatgaac gacataacag ctatacctgt 660

gaggccactc acaagacatc aacttcaccc attgtcaaga gcttcaacag gaatgagtgt 720

tag 723

<210> 32
<211> 240
<212> PRT
<213> Mus musculus

<220>
<221> SIGNAL
<222> (1)..(20)

<400> 32

Met Asp Ser Gln Ala Gln Val Leu Met Leu Leu Leu Leu Trp Val Ser
1 5 10 15

Gly Thr Cys Gly Asp Ile Val Met Ser Gln Ser Pro Ser Ser Leu Ala
20 25 30

Val Ser Val Gly Glu Lys Val Thr Met Ser Cys Lys Ser Ser Gln Ser
35 40 45

Leu Leu Tyr Gly Ser Asn Gln Lys Asn Tyr Leu Ala Trp Tyr Gln Gln
50 55 60

Lys Pro Gly Gln Ser Pro Lys Leu Leu Ile Tyr Trp Ala Ser Thr Arg
65 70 75 80

Glu Ser Gly Val Pro Asp Arg Phe Thr Gly Ser Gly Ser Gly Thr Asp
85 90 95

Phe Thr Leu Thr Ile Ser Ser Val Lys Ala Glu Asp Leu Ala Val Tyr
100 105 110

Tyr Cys Gln Gln Tyr Tyr Asn Phe Pro Tyr Thr Phe Gly Gly Gly Thr
115 120 125

Lys Leu Glu Ile Lys Arg Ala Asp Ala Ala Pro Thr Val Ser Ile Phe
130 135 140

Pro Pro Ser Ser Glu Gln Leu Thr Ser Gly Gly Ala Ser Val Val Cys
145 150 155 160

Phe Leu Asn Asn Phe Tyr Pro Lys Asp Ile Asn Val Lys Trp Lys Ile
165 170 175

Asp Gly Ser Glu Arg Gln Asn Gly Val Leu Asn Ser Trp Thr Asp Gln
180 185 190

Asp Ser Lys Asp Ser Thr Tyr Ser Met Ser Ser Thr Leu Thr Leu Thr
195 200 205

Lys Asp Glu Tyr Glu Arg His Asn Ser Tyr Thr Cys Glu Ala Thr His
210 215 220

Lys Thr Ser Thr Ser Pro Ile Val Lys Ser Phe Asn Arg Asn Glu Cys
225 230 235 240

<210> 33
<211> 321
<212> DNA
<213> Homo sapiens

<400> 33
acggtggctg caccatctgt cttcatcttc ccgccatctg atgagcagtt gaaatctgga 60

actgcctctg ttgtgtgcct gctgaataac ttctatccca gagaggccaa agtacagtgg 120

aaggtggata acgccctcca atcgggtaac tcccaggaga gtgtcacaga gcaggacagc 180

aaggacagca cctacagcct cagcagcacc ctgacgctga gcaaagcaga ctacgagaaa 240

cacaaagtct acgcctgcga agtcacccat cagggcctga gctcgcccgt cacaaagagc 300

ttcaacaggg gagagtgttg a 321

<210> 34
<211> 106
<212> PRT
<213> Homo sapiens

<400> 34

Thr Val Ala Ala Pro Ser Val Phe Ile Phe Pro Pro Ser Asp Glu Gln
1 5 10 15

Leu Lys Ser Gly Thr Ala Ser Val Val Cys Leu Leu Asn Asn Phe Tyr
20 25 30

Pro Arg Glu Ala Lys Val Gln Trp Lys Val Asp Asn Ala Leu Gln Ser

35 40 45

Gly Asn Ser Gln Glu Ser Val Thr Glu Gln Asp Ser Lys Asp Ser Thr
50 55 60

Tyr Ser Leu Ser Ser Thr Leu Thr Leu Ser Lys Ala Asp Tyr Glu Lys
65 70 75 80

His Lys Val Tyr Ala Cys Glu Val Thr His Gln Gly Leu Ser Ser Pro
85 90 95

Val Thr Lys Ser Phe Asn Arg Gly Glu Cys
100 105

<210> 35
<211> 15
<212> DNA
<213> Mus musculus

<400> 35
gactactaca tgaac 15

<210> 36
<211> 5
<212> PRT
<213> Mus musculus

<400> 36

Asp Tyr Tyr Met Asn
1 5

<210> 37
<211> 51
<212> DNA
<213> Mus musculus

<400> 37
cgtgttaatc ctagtaatgg tggtactagc tacaaccaga agttcaaggg c 51

<210> 38
<211> 17
<212> PRT
<213> Mus musculus

<400> 38

Arg Val Asn Pro Ser Asn Gly Gly Thr Ser Tyr Asn Gln Lys Phe Lys
1 5 10 15

Gly

<210> 39
<211> 33
<212> DNA
<213> Mus musculus

<400> 39
ggcctagcct actatagtaa ctcctttgtt tac 33

<210> 40
<211> 11
<212> PRT
<213> Mus musculus

<400> 40

Gly Leu Ala Tyr Tyr Ser Asn Ser Phe Val Tyr
1 5 10

<210> 41
<211> 360
<212> DNA
<213> Mus musculus

<400> 41
gaggtccagc tgcaacagtc tggacctgag ctggtgaagc ctggggcttc agtgaagatg 60

tcctgtaagg cttctggata cacattcact gactactaca tgaactgggt gaagcagagt 120

catggaaaga gccttgagtg gattggacgt gttaatccta gtaatggtgg tactagctac 180

aaccagaagt tcaagggcaa ggccacattg acagtagaca aatccctcag cacagcctac 240

atgcagctca acagcctgac atctgaggac tctgcggtct attactgtgc aagaggccta 300

gcctactata gtaactcctt tgtttactgg ggccaaggga ctctggtcac tgtctctgca 360

<210> 42
<211> 120
<212> PRT
<213> Mus musculus

<400> 42

Glu Val Gln Leu Gln Gln Ser Gly Pro Glu Leu Val Lys Pro Gly Ala
1 5 10 15

Ser Val Lys Met Ser Cys Lys Ala Ser Gly Tyr Thr Phe Thr Asp Tyr
20 25 30

Tyr Met Asn Trp Val Lys Gln Ser His Gly Lys Ser Leu Glu Trp Ile
35 40 45

```
Gly Arg Val Asn Pro Ser Asn Gly Gly Thr Ser Tyr Asn Gln Lys Phe
    50                  55                  60

Lys Gly Lys Ala Thr Leu Thr Val Asp Lys Ser Leu Ser Thr Ala Tyr
65                  70                  75                  80

Met Gln Leu Asn Ser Leu Thr Ser Glu Asp Ser Ala Val Tyr Tyr Cys
                85                  90                  95

Ala Arg Gly Leu Ala Tyr Tyr Ser Asn Ser Phe Val Tyr Trp Gly Gln
            100                 105                 110

Gly Thr Leu Val Thr Val Ser Ala
        115                 120


<210>  43
<211>  1431
<212>  DNA
<213>  Mus musculus


<220>
<221>  sig_peptide
<222>  (1)..(57)

<400>  43
atgggatgga gctgggtctt tctcttcctc ctgtcaggaa ctgcaggtgt ccactctgag     60

gtccagctgc aacagtctgg acctgagctg gtgaagcctg gggcttcagt gaagatgtcc    120

tgtaaggctt ctggatacac attcactgac tactacatga actgggtgaa gcagagtcat    180

ggaaagagcc ttgagtggat tggacgtgtt aatcctagta atggtggtac tagctacaac    240

cagaagttca agggcaaggc cacattgaca gtagacaaat ccctcagcac agcctacatg    300

cagctcaaca gcctgacatc tgaggactct gcggtctatt actgtgcaag aggcctagcc    360

tactatagta actcctttgt ttactggggc caagggactc tggtcactgt ctctgcagcc    420

aaaacaacac ccccatcagt ctatccactg gcccctgggt gtggagatac aactggttcc    480

tccgtgacct ctgggtgcct ggtcaagggc tacttccctg agtcagtgac tgtgacttgg    540

aactctggat ccctgtccag cagtgtgcac accctctccc aggctctcct gcagtctgga    600

ctctacacta tgagcagctc agtgactgtc ccctccagca cctggccaag tcagaccgtc    660

acctgcagcg ttgctcaccc agccagcagc accacggtgg acaaaaaact tgagcccagc    720

gggcccattt caacaatcaa cccctgtcct ccatgcaagg agtgtcacaa atgcccagct    780

cctaacctcg agggtggacc atccgtcttc atcttccctc caaatatcaa ggatgtactc    840
```

atgatctccc tgacacccaa ggtcacgtgt gtggtggtgg atgtgagcga ggatgaccca 900

gacgtccaga tcagctggtt tgtgaacaac gtggaagtac acacagctca gacacaaacc 960

catagagagg attacaacag tactatccgg gtggtcagca ccctccccat ccagcaccag 1020

gactggatga gtggcaagga gttcaaatgc aaggtcaaca acaaagacct cccatcaccc 1080

atcgagagaa ccatctcaaa aattaaaggg ctagtcagag ctccacaagt atacatcttg 1140

ccgccaccag cagagcagtt gtccaggaaa gatgtcagtc tcacttgcct ggtcgtgggc 1200

ttcaaccctg gagacatcag tgtggagtgg accagcaatg ggcatacaga ggagaactac 1260

aaggacaccg caccagtcct ggactctgac ggttcttact tcatatatag caagctcaat 1320

atgaaaacaa gcaagtggga gaaaacagat tccttctcat gcaacgtgag acacgagggt 1380

ctgaaaaatt actacctgaa gaagaccatc tcccggtctc cgggtaaatg a 1431

<210> 44
<211> 476
<212> PRT
<213> Mus musculus

<220>
<221> SIGNAL
<222> (1)..(19)

<400> 44

Met Gly Trp Ser Trp Val Phe Leu Phe Leu Leu Ser Gly Thr Ala Gly
1 5 10 15

Val His Ser Glu Val Gln Leu Gln Gln Ser Gly Pro Glu Leu Val Lys
20 25 30

Pro Gly Ala Ser Val Lys Met Ser Cys Lys Ala Ser Gly Tyr Thr Phe
35 40 45

Thr Asp Tyr Tyr Met Asn Trp Val Lys Gln Ser His Gly Lys Ser Leu
50 55 60

Glu Trp Ile Gly Arg Val Asn Pro Ser Asn Gly Gly Thr Ser Tyr Asn
65 70 75 80

Gln Lys Phe Lys Gly Lys Ala Thr Leu Thr Val Asp Lys Ser Leu Ser
85 90 95

Thr Ala Tyr Met Gln Leu Asn Ser Leu Thr Ser Glu Asp Ser Ala Val
100 105 110

```
Tyr Tyr Cys Ala Arg Gly Leu Ala Tyr Tyr Ser Asn Ser Phe Val Tyr
        115                 120                 125

Trp Gly Gln Gly Thr Leu Val Thr Val Ser Ala Ala Lys Thr Thr Pro
    130                 135                 140

Pro Ser Val Tyr Pro Leu Ala Pro Gly Cys Gly Asp Thr Thr Gly Ser
145                 150                 155                 160

Ser Val Thr Ser Gly Cys Leu Val Lys Gly Tyr Phe Pro Glu Ser Val
                165                 170                 175

Thr Val Thr Trp Asn Ser Gly Ser Leu Ser Ser Ser Val His Thr Leu
            180                 185                 190

Ser Gln Ala Leu Leu Gln Ser Gly Leu Tyr Thr Met Ser Ser Ser Val
        195                 200                 205

Thr Val Pro Ser Ser Thr Trp Pro Ser Gln Thr Val Thr Cys Ser Val
    210                 215                 220

Ala His Pro Ala Ser Ser Thr Thr Val Asp Lys Lys Leu Glu Pro Ser
225                 230                 235                 240

Gly Pro Ile Ser Thr Ile Asn Pro Cys Pro Pro Cys Lys Glu Cys His
                245                 250                 255

Lys Cys Pro Ala Pro Asn Leu Glu Gly Gly Pro Ser Val Phe Ile Phe
            260                 265                 270

Pro Pro Asn Ile Lys Asp Val Leu Met Ile Ser Leu Thr Pro Lys Val
        275                 280                 285

Thr Cys Val Val Val Asp Val Ser Glu Asp Asp Pro Asp Val Gln Ile
    290                 295                 300

Ser Trp Phe Val Asn Asn Val Glu Val His Thr Ala Gln Thr Gln Thr
305                 310                 315                 320

His Arg Glu Asp Tyr Asn Ser Thr Ile Arg Val Val Ser Thr Leu Pro
                325                 330                 335

Ile Gln His Gln Asp Trp Met Ser Gly Lys Glu Phe Lys Cys Lys Val
            340                 345                 350
```

```
Asn Asn Lys Asp Leu Pro Ser Pro Ile Glu Arg Thr Ile Ser Lys Ile
        355                 360                 365

Lys Gly Leu Val Arg Ala Pro Gln Val Tyr Ile Leu Pro Pro Pro Ala
    370                 375                 380

Glu Gln Leu Ser Arg Lys Asp Val Ser Leu Thr Cys Leu Val Val Gly
385                 390                 395                 400

Phe Asn Pro Gly Asp Ile Ser Val Glu Trp Thr Ser Asn Gly His Thr
                405                 410                 415

Glu Glu Asn Tyr Lys Asp Thr Ala Pro Val Leu Asp Ser Asp Gly Ser
            420                 425                 430

Tyr Phe Ile Tyr Ser Lys Leu Asn Met Lys Thr Ser Lys Trp Glu Lys
        435                 440                 445

Thr Asp Ser Phe Ser Cys Asn Val Arg His Glu Gly Leu Lys Asn Tyr
    450                 455                 460

Tyr Leu Lys Lys Thr Ile Ser Arg Ser Pro Gly Lys
465                 470                 475
```

<210> 45
<211> 45
<212> DNA
<213> Mus musculus

<400> 45
agagccagtg aaagtgttga tagttatggc aatagtttta tgcac 45

<210> 46
<211> 15
<212> PRT
<213> Mus musculus

<400> 46

```
Arg Ala Ser Glu Ser Val Asp Ser Tyr Gly Asn Ser Phe Met His
1               5                   10                  15
```

<210> 47
<211> 21
<212> DNA
<213> Mus musculus

<400> 47
cgtgcatcca acctagaatc t 21

<210> 48
<211> 7
<212> PRT
<213> Mus musculus

<400> 48

Arg Ala Ser Asn Leu Glu Ser
1               5

<210> 49
<211> 27
<212> DNA
<213> Mus musculus

<400> 49
cagcaaaata atgaggatcc gtggacg 27

<210> 50
<211> 9
<212> PRT
<213> Mus musculus

<400> 50

Gln Gln Asn Asn Glu Asp Pro Trp Thr
1               5

<210> 51
<211> 336
<212> DNA
<213> Mus musculus

<400> 51
aaaattgtgc tgacccaatc tccagcttct ttggctgtgt ctctaaggca gagggccacc 60

atatcctgca gagccagtga aagtgttgat agttatggca atagttttat gcactggtac 120

cagcagaaac caggacagcc acccaaactc ctcatctatc gtgcatccaa cctagaatct 180

ggggtccctg ccaggttcag tggcagtggg tctaggacag acttcaccct caccattgat 240

cctgtggagg ctgatgatgc tgcaacctat tactgtcagc aaaataatga ggatccgtgg 300

acgttcggtg gaggcaccaa gctggaaatc aaacgg 336

<210> 52
<211> 112
<212> PRT
<213> Mus musculus

<400> 52

Lys Ile Val Leu Thr Gln Ser Pro Ala Ser Leu Ala Val Ser Leu Arg

```
1               5                   10                  15

Gln Arg Ala Thr Ile Ser Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Arg Ala Ser Asn Leu Glu Ser Gly Val Pro Ala
    50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
                85                  90                  95

Glu Asp Pro Trp Thr Phe Gly Gly Gly Thr Lys Leu Glu Ile Lys Arg
            100                 105                 110
```

<210>  53
<211>  717
<212>  DNA
<213>  Mus musculus

<220>
<221>  sig_peptide
<222>  (1)..(60)

<400>  53

```
atggagacag agacactcct gctatgggtg ctactgctct gggttccagg ttccacaggt     60

aaaattgtgc tgacccaatc tccagcttct ttggctgtgt ctctaaggca gagggccacc    120

atatcctgca gagccagtga aagtgttgat agttatggca atagttttat gcactggtac    180

cagcagaaac caggacagcc acccaaactc ctcatctatc gtgcatccaa cctagaatct    240

ggggtccctg ccaggttcag tggcagtggg tctaggacag acttcaccct caccattgat    300

cctgtggagg ctgatgatgc tgcaacctat tactgtcagc aaaataatga ggatccgtgg    360

acgttcggtg gaggcaccaa gctggaaatc aaacgggctg atgctgcacc aactgtatcc    420

atcttcccac catccagtga gcagttaaca tctggaggtg cctcagtcgt gtgcttcttg    480

aacaacttct accccaaaga catcaatgtc aagtggaaga ttgatggcag tgaacgacaa    540

aatggcgtcc tgaacagttg gactgatcag gacagcaaag acagcaccta cagcatgagc    600

agcaccctca cgttgaccaa ggacgagtat gaacgacata acagctatac ctgtgaggcc    660
```

actcacaaga catcaacttc acccattgtc aagagcttca acaggaatga gtgttag 717

<210> 54
<211> 238
<212> PRT
<213> Mus musculus

<220>
<221> SIGNAL
<222> (1)..(20)

<400> 54

```
Met Glu Thr Glu Thr Leu Leu Leu Trp Val Leu Leu Leu Trp Val Pro
1               5                   10                  15

Gly Ser Thr Gly Lys Ile Val Leu Thr Gln Ser Pro Ala Ser Leu Ala
            20                  25                  30

Val Ser Leu Arg Gln Arg Ala Thr Ile Ser Cys Arg Ala Ser Glu Ser
        35                  40                  45

Val Asp Ser Tyr Gly Asn Ser Phe Met His Trp Tyr Gln Gln Lys Pro
    50                  55                  60

Gly Gln Pro Pro Lys Leu Leu Ile Tyr Arg Ala Ser Asn Leu Glu Ser
65                  70                  75                  80

Gly Val Pro Ala Arg Phe Ser Gly Ser Gly Ser Arg Thr Asp Phe Thr
                85                  90                  95

Leu Thr Ile Asp Pro Val Glu Ala Asp Asp Ala Ala Thr Tyr Tyr Cys
            100                 105                 110

Gln Gln Asn Asn Glu Asp Pro Trp Thr Phe Gly Gly Gly Thr Lys Leu
        115                 120                 125

Glu Ile Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
```

```
            180                 185                 190

Lys Asp Ser Thr Tyr Ser Met Ser Ser Thr Leu Thr Leu Thr Lys Asp
        195                 200                 205

Glu Tyr Glu Arg His Asn Ser Tyr Thr Cys Glu Ala Thr His Lys Thr
    210                 215                 220

Ser Thr Ser Pro Ile Val Lys Ser Phe Asn Arg Asn Glu Cys
225                 230                 235


<210>  55
<211>  15
<212>  DNA
<213>  Mus musculus

<400>  55
ggctacttta tgaac                                                    15


<210>  56
<211>  5
<212>  PRT
<213>  Mus musculus

<400>  56

Gly Tyr Phe Met Asn
1               5


<210>  57
<211>  51
<212>  DNA
<213>  Mus musculus

<400>  57
cgtattaatc cttacaatgg tgatactttc tacaaccaga agttcaaggg c            51


<210>  58
<211>  17
<212>  PRT
<213>  Mus musculus

<400>  58

Arg Ile Asn Pro Tyr Asn Gly Asp Thr Phe Tyr Asn Gln Lys Phe Lys
1               5                   10                  15

Gly


<210>  59
```

<211> 24
<212> DNA
<213> Mus musculus

<400> 59
agtggtgact ggtacttcga tgtc 24

<210> 60
<211> 8
<212> PRT
<213> Mus musculus

<400> 60

Ser Gly Asp Trp Tyr Phe Asp Val
1 5

<210> 61
<211> 351
<212> DNA
<213> Mus musculus

<400> 61
gaggttcagc tgcagcagtc tggacctgag ctggtgaagc ctggggcttc agtgaagata 60

tcctgcaagg cttctggtta ctcatttact ggctacttta tgaactgggt gaagcagagc 120

catggaaaga gccttgagtg gattggacgt attaatcctt acaatggtga tactttctac 180

aaccagaagt tcaagggcaa ggccacatta actgtagaca aatcctctag cacagcccac 240

atggagctcc ggagcctgac atctgaggac tctgcagtct attattgtgc aagaagtggt 300

gactggtact tcgatgtctg gggcgcaggg accacggtca ccgtctcctc a 351

<210> 62
<211> 117
<212> PRT
<213> Mus musculus

<400> 62

Glu Val Gln Leu Gln Gln Ser Gly Pro Glu Leu Val Lys Pro Gly Ala
1 5 10 15

Ser Val Lys Ile Ser Cys Lys Ala Ser Gly Tyr Ser Phe Thr Gly Tyr
20 25 30

Phe Met Asn Trp Val Lys Gln Ser His Gly Lys Ser Leu Glu Trp Ile
35 40 45

Gly Arg Ile Asn Pro Tyr Asn Gly Asp Thr Phe Tyr Asn Gln Lys Phe
50 55 60

```
Lys Gly Lys Ala Thr Leu Thr Val Asp Lys Ser Ser Ser Thr Ala His
65                  70                  75                  80

Met Glu Leu Arg Ser Leu Thr Ser Glu Asp Ser Ala Val Tyr Tyr Cys
                85                  90                  95

Ala Arg Ser Gly Asp Trp Tyr Phe Asp Val Trp Gly Ala Gly Thr Thr
            100                 105                 110

Val Thr Val Ser Ser
        115
```

```
<210>  63
<211>  1416
<212>  DNA
<213>  Mus musculus

<220>
<221>  sig_peptide
<222>  (1)..(57)

<400>  63
atgggatgga gctggatctt tctctttctc ctgtcagtaa ctgcaggtgt gttctctgag     60

gttcagctgc agcagtctgg acctgagctg gtgaagcctg gggcttcagt gaagatatcc    120

tgcaaggctt ctggttactc atttactggc tactttatga actgggtgaa gcagagccat    180

ggaaagagcc ttgagtggat tggacgtatt aatccttaca atggtgatac tttctacaac    240

cagaagttca agggcaaggc cacattaact gtagacaaat cctctagcac agcccacatg    300

gagctccgga gcctgacatc tgaggactct gcagtctatt attgtgcaag aagtggtgac    360

tggtacttcg atgtctgggg cgcagggacc acggtcaccg tctcctcagc caaaacaaca    420

gccccatcgg tctatccact ggcccctgtg tgtggaggta caactggctc ctcggtgact    480

ctaggatgcc tggtcaaggg ttatttccct gagccagtga ccttgacctg gaactctgga    540

tccctgtcca gtggtgtgca caccttccca gctctcctgc agtctggcct ctacaccctc    600

agcagctcag tgactgtaac ctcgaacacc tggcccagcc agaccatcac ctgcaatgtg    660

gcccacccgg caagcagcac caaagtggac aagaaaattg agcccagagt gcccataaca    720

cagaacccct gtcctccact caaagagtgt cccccatgcg cagctccaga cctcttgggt    780

ggaccatccg tcttcatctt ccctccaaag atcaaggatg tactcatgat ctccctgagc    840

cccatggtca catgtgtggt ggtggatgtg agcgaggatg acccagacgt ccagatcagc    900

tggtttgtga acaacgtgga agtacacaca gctcagacac aaacccatag agaggattac    960
```

```
aacagtactc tccgggtggt cagtgccctc cccatccagc accaggactg gatgagtggc   1020

aaggagttca aatgcaaggt caacaacaga gccctcccat cccccatcga gaaaaccatc   1080

tcaaaaccca gagggccagt aagagctcca caggtatatg tcttgcctcc accagcagaa   1140

gagatgacta agaaagagtt cagtctgacc tgcatgatca caggcttctt acctgccgaa   1200

attgctgtgg actggaccag caatgggcgt acagagcaaa actacaagaa caccgcaaca   1260

gtcctggact ctgatggttc ttacttcatg tacagcaagc tcagagtaca aaagagcact   1320

tgggaaagag gaagtctttt cgcctgctca gtggtccacg agggtctgca caatcacctt   1380

acgactaaga ccatctcccg gtctctgggt aaatga                              1416
```

<210>  64
<211>  471
<212>  PRT
<213>  Mus musculus

<220>
<221>  SIGNAL
<222>  (1)..(19)

<400>  64

```
Met Gly Trp Ser Trp Ile Phe Leu Phe Leu Leu Ser Val Thr Ala Gly
1               5                   10                  15

Val Phe Ser Glu Val Gln Leu Gln Gln Ser Gly Pro Glu Leu Val Lys
            20                  25                  30

Pro Gly Ala Ser Val Lys Ile Ser Cys Lys Ala Ser Gly Tyr Ser Phe
        35                  40                  45

Thr Gly Tyr Phe Met Asn Trp Val Lys Gln Ser His Gly Lys Ser Leu
    50                  55                  60

Glu Trp Ile Gly Arg Ile Asn Pro Tyr Asn Gly Asp Thr Phe Tyr Asn
65                  70                  75                  80

Gln Lys Phe Lys Gly Lys Ala Thr Leu Thr Val Asp Lys Ser Ser Ser
                85                  90                  95

Thr Ala His Met Glu Leu Arg Ser Leu Thr Ser Glu Asp Ser Ala Val
            100                 105                 110

Tyr Tyr Cys Ala Arg Ser Gly Asp Trp Tyr Phe Asp Val Trp Gly Ala
        115                 120                 125
```

```
Gly Thr Thr Val Thr Val Ser Ser Ala Lys Thr Thr Ala Pro Ser Val
    130                 135                 140

Tyr Pro Leu Ala Pro Val Cys Gly Gly Thr Thr Gly Ser Ser Val Thr
145                 150                 155                 160

Leu Gly Cys Leu Val Lys Gly Tyr Phe Pro Glu Pro Val Thr Leu Thr
                165                 170                 175

Trp Asn Ser Gly Ser Leu Ser Ser Gly Val His Thr Phe Pro Ala Leu
            180                 185                 190

Leu Gln Ser Gly Leu Tyr Thr Leu Ser Ser Ser Val Thr Val Thr Ser
        195                 200                 205

Asn Thr Trp Pro Ser Gln Thr Ile Thr Cys Asn Val Ala His Pro Ala
    210                 215                 220

Ser Ser Thr Lys Val Asp Lys Lys Ile Glu Pro Arg Val Pro Ile Thr
225                 230                 235                 240

Gln Asn Pro Cys Pro Pro Leu Lys Glu Cys Pro Pro Cys Ala Ala Pro
                245                 250                 255

Asp Leu Leu Gly Gly Pro Ser Val Phe Ile Phe Pro Pro Lys Ile Lys
            260                 265                 270

Asp Val Leu Met Ile Ser Leu Ser Pro Met Val Thr Cys Val Val Val
        275                 280                 285

Asp Val Ser Glu Asp Asp Pro Asp Val Gln Ile Ser Trp Phe Val Asn
    290                 295                 300

Asn Val Glu Val His Thr Ala Gln Thr Gln Thr His Arg Glu Asp Tyr
305                 310                 315                 320

Asn Ser Thr Leu Arg Val Val Ser Ala Leu Pro Ile Gln His Gln Asp
                325                 330                 335

Trp Met Ser Gly Lys Glu Phe Lys Cys Lys Val Asn Asn Arg Ala Leu
            340                 345                 350

Pro Ser Pro Ile Glu Lys Thr Ile Ser Lys Pro Arg Gly Pro Val Arg
        355                 360                 365
```

```
Ala Pro Gln Val Tyr Val Leu Pro Pro Pro Ala Glu Glu Met Thr Lys
    370                 375                 380

Lys Glu Phe Ser Leu Thr Cys Met Ile Thr Gly Phe Leu Pro Ala Glu
385                 390                 395                 400

Ile Ala Val Asp Trp Thr Ser Asn Gly Arg Thr Glu Gln Asn Tyr Lys
                405                 410                 415

Asn Thr Ala Thr Val Leu Asp Ser Asp Gly Ser Tyr Phe Met Tyr Ser
            420                 425                 430

Lys Leu Arg Val Gln Lys Ser Thr Trp Glu Arg Gly Ser Leu Phe Ala
        435                 440                 445

Cys Ser Val Val His Glu Gly Leu His Asn His Leu Thr Thr Lys Thr
    450                 455                 460

Ile Ser Arg Ser Leu Gly Lys
465                 470
```

<210> 65
<211> 45
<212> DNA
<213> Mus musculus

<400> 65
agggccagca aaagtgtcag tacatctagc tatagttaca tgcac 45

<210> 66
<211> 15
<212> PRT
<213> Mus musculus

<400> 66

```
Arg Ala Ser Lys Ser Val Ser Thr Ser Ser Tyr Ser Tyr Met His
1               5                   10                  15
```

<210> 67
<211> 21
<212> DNA
<213> Mus musculus

<400> 67
tttgcatcct acctagaatc t 21

<210> 68
<211> 7

<212> PRT
<213> Mus musculus

<400> 68

Phe Ala Ser Tyr Leu Glu Ser
1 5

<210> 69
<211> 27
<212> DNA
<213> Mus musculus

<400> 69
caacacagta gggagtttcc tcggacg 27

<210> 70
<211> 9
<212> PRT
<213> Mus musculus

<400> 70

Pro Val Glu Glu Glu Phe Pro Arg Thr
1 5

<210> 71
<211> 336
<212> DNA
<213> Mus musculus

<400> 71
gacattgtgc tgacacagtc tcctgcttcc ttagctgtat ctctggggca gagggccacc 60

atctcctgca gggccagcaa aagtgtcagt acatctagct atagttacat gcactggtac 120

caacagaaac caggacagcc acccaaactc ctcatcaagt ttgcatccta cctagaatct 180

ggggttcctg ccaggttcag tggcagtggg tctgggacag acttcaccct caacatccat 240

cctgtggagg aggaggatgc tgcaacatat tactgtcaac acagtaggga gtttcctcgg 300

acgttcggtg gaggcaccaa gctggaaatc aaacgg 336

<210> 72
<211> 112
<212> PRT
<213> Mus musculus

<400> 72

Asp Ile Val Leu Thr Gln Ser Pro Ala Ser Leu Ala Val Ser Leu Gly
1 5 10 15

Gln Arg Ala Thr Ile Ser Cys Arg Ala Ser Lys Ser Val Ser Thr Ser

20 25 30

Ser Tyr Ser Tyr Met His Trp Tyr Gln Gln Lys Pro Gly Gln Pro Pro
35 40 45

Lys Leu Leu Ile Lys Phe Ala Ser Tyr Leu Glu Ser Gly Val Pro Ala
50 55 60

Arg Phe Ser Gly Ser Gly Ser Gly Thr Asp Phe Thr Leu Asn Ile His
65 70 75 80

Pro Val Glu Glu Glu Asp Ala Ala Thr Tyr Tyr Cys Gln His Ser Arg
85 90 95

Glu Phe Pro Arg Thr Phe Gly Gly Gly Thr Lys Leu Glu Ile Lys Arg
100 105 110

<210> 73
<211> 717
<212> DNA
<213> Mus musculus

<220>
<221> sig_peptide
<222> (1)..(60)

<400> 73
atggagacag acacactcct gctatgggtg ctgctgctct gggttccagg ttccactggt 60
gacattgtgc tgacacagtc tcctgcttcc ttagctgtat ctctggggca gagggccacc 120
atctcctgca gggccagcaa aagtgtcagt acatctagct atagttacat gcactggtac 180
caacagaaac caggacagcc acccaaactc ctcatcaagt ttgcatccta cctagaatct 240
ggggttcctg ccaggttcag tggcagtggg tctgggacag acttcaccct caacatccat 300
cctgtggagg aggaggatgc tgcaacatat tactgtcaac acagtaggga gtttcctcgg 360
acgttcggtg gaggcaccaa gctggaaatc aaacgggctg atgctgcacc aactgtatcc 420
atcttcccac catccagtga gcagttaaca tctggaggtg cctcagtcgt gtgcttcttg 480
aacaacttct accccaaaga catcaatgtc aagtggaaga ttgatggcag tgaacgacaa 540
aatggcgtcc tgaacagttg gactgatcag gacagcaaag acagcaccta cagcatgagc 600
agcaccctca cgttgaccaa ggacgagtat gaacgacata acagctatac ctgtgaggcc 660
actcacaaga catcaacttc acccattgtc aagagcttca acaggaatga gtgttag 717

<210> 74

<211> 238
<212> PRT
<213> Mus musculus

<220>
<221> SIGNAL
<222> (1)..(20)

<400> 74

```
Met Glu Thr Asp Thr Leu Leu Leu Trp Val Leu Leu Leu Trp Val Pro
1               5                   10                  15

Gly Ser Thr Gly Asp Ile Val Leu Thr Gln Ser Pro Ala Ser Leu Ala
            20                  25                  30

Val Ser Leu Gly Gln Arg Ala Thr Ile Ser Cys Arg Ala Ser Lys Ser
        35                  40                  45

Val Ser Thr Ser Ser Tyr Ser Tyr Met His Trp Tyr Gln Gln Lys Pro
    50                  55                  60

Gly Gln Pro Pro Lys Leu Leu Ile Lys Phe Ala Ser Tyr Leu Glu Ser
65                  70                  75                  80

Gly Val Pro Ala Arg Phe Ser Gly Ser Gly Ser Gly Thr Asp Phe Thr
                85                  90                  95

Leu Asn Ile His Pro Val Glu Glu Glu Asp Ala Ala Thr Tyr Tyr Cys
            100                 105                 110

Gln His Ser Arg Glu Phe Pro Arg Thr Phe Gly Gly Gly Thr Lys Leu
        115                 120                 125

Glu Ile Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
            180                 185                 190

Lys Asp Ser Thr Tyr Ser Met Ser Ser Thr Leu Thr Leu Thr Lys Asp
```

195 200 205

Glu Tyr Glu Arg His Asn Ser Tyr Thr Cys Glu Ala Thr His Lys Thr
210 215 220

Ser Thr Ser Pro Ile Val Lys Ser Phe Asn Arg Asn Glu Cys
225 230 235

<210> 75
<211> 15
<212> DNA
<213> Mus musculus

<400> 75
ggctacttta tgaac 15

<210> 76
<211> 5
<212> PRT
<213> Mus musculus

<400> 76

Gly Tyr Phe Met Asn
1 5

<210> 77
<211> 51
<212> DNA
<213> Mus musculus

<400> 77
cgtattaatc cttacaatgg tgatactttc tacaaccaga agttcaaggg c 51

<210> 78
<211> 17
<212> PRT
<213> Mus musculus

<400> 78

Arg Ile Asn Pro Tyr Asn Gly Asp Thr Phe Tyr Asn Gln Lys Phe Lys
1 5 10 15

Gly

<210> 79
<211> 33
<212> DNA
<213> Mus musculus

<400> 79
ggagatggtt actacgtgac gagtcttgct tac 33

<210> 80
<211> 11
<212> PRT
<213> Mus musculus

<400> 80

Gly Asp Gly Tyr Tyr Val Thr Ser Leu Ala Tyr
1 5 10

<210> 81
<211> 360
<212> DNA
<213> Mus musculus

<400> 81
gaggttcagc tgcagcagtc tggacctgag ctggtgaagc ctggggcttc agtgaagata 60

tcctgcaagg cttctggtta ctcatttact ggctacttta tgaactgggt gaagcagagc 120

catggaaaga gccttgagtg gcttggacgt attaatcctt acaatggtga tactttctac 180

aaccagaagt tcaagggcaa ggccacatta actgtagaca aatcctctaa cacagcccac 240

atggagctcc ggagcctgac atctgaggac tctgcagtct attattgtgc aagaggagat 300

ggttactacg tgacgagtct tgcttactgg ggccaaggga ctctggtcac tgtctctgca 360

<210> 82
<211> 120
<212> PRT
<213> Mus musculus

<400> 82

Glu Val Gln Leu Gln Gln Ser Gly Pro Glu Leu Val Lys Pro Gly Ala
1 5 10 15

Ser Val Lys Ile Ser Cys Lys Ala Ser Gly Tyr Ser Phe Thr Gly Tyr
20 25 30

Phe Met Asn Trp Val Lys Gln Ser His Gly Lys Ser Leu Glu Trp Leu
35 40 45

Gly Arg Ile Asn Pro Tyr Asn Gly Asp Thr Phe Tyr Asn Gln Lys Phe
50 55 60

Lys Gly Lys Ala Thr Leu Thr Val Asp Lys Ser Ser Asn Thr Ala His
65 70 75 80

```
Met Glu Leu Arg Ser Leu Thr Ser Glu Asp Ser Ala Val Tyr Tyr Cys
                85                  90                  95

Ala Arg Gly Asp Gly Tyr Tyr Val Thr Ser Leu Ala Tyr Trp Gly Gln
            100                 105                 110

Gly Thr Leu Val Thr Val Ser Ala
        115                 120


<210>  83
<211>  1392
<212>  DNA
<213>  Mus musculus


<220>
<221>  sig_peptide
<222>  (1)..(57)

<400>  83
atgggatgga gctggatctt tctctttctc ctgtcagtaa ctgcaggtgt gttctctgag      60

gttcagctgc agcagtctgg acctgagctg gtgaagcctg gggcttcagt gaagatatcc     120

tgcaaggctt ctggttactc atttactggc tactttatga actgggtgaa gcagagccat     180

ggaaagagcc ttgagtggct tggacgtatt aatccttaca atggtgatac tttctacaac     240

cagaagttca agggcaaggc cacattaact gtagacaaat cctctaacac agcccacatg     300

gagctccgga gcctgacatc tgaggactct gcagtctatt attgtgcaag aggagatggt     360

tactacgtga cgagtcttgc ttactggggc caagggactc tggtcactgt ctctgcagcc     420

aaaacgacac ccccatctgt ctatccactg gcccctggat ctgctgccca aactaactcc     480

atggtgaccc tgggatgcct ggtcaagggc tatttccctg agccagtgac agtgacctgg     540

aactctggat ccctgtccag cggtgtgcac accttcccag ctgtcctgca gtctgacctc     600

tacactctga gcagctcagt gactgtcccc tccagcacct ggcccagcga gaccgtcacc     660

tgcaacgttg cccacccggc cagcagcacc aaggtggaca agaaaattgt gcccagggat     720

tgtggttgta agccttgcat atgtacagtc ccagaagtat catctgtctt catcttcccc     780

ccaaagccca aggatgtgct caccattact ctgactccta aggtcacgtg tgttgtggta     840

gacatcagca aggatgatcc cgaggtccag ttcagctggt ttgtagatga tgtggaggtg     900

cacacagctc agacgcaacc ccgggaggag cagttcaaca gcactttccg ctcagtcagt     960

gaacttccca tcatgcacca ggactggctc aatggcaagg agttcaaatg cagggtcaac    1020

agtgcagctt tccctgcccc catcgagaaa accatctcca aaaccaaagg cagaccgaag    1080
```

```
gctccacagg tgtacaccat tccacctccc aaggagcaga tggccaagga taaagtcagt   1140

ctgacctgca tgataacaga cttcttccct gaagacatta ctgtggagtg gcagtggaat   1200

gggcagccag cggagaacta caagaacact cagcccatca tgaacacgaa tggctcttac   1260

ttcgtctaca gcaagctcaa tgtgcagaag agcaactggg aggcaggaaa tactttcacc   1320

tgctctgtct tacatgaggg cctgcacaac caccatactg agaagagcct ctcccactct   1380

cctggtaaat aa                                                       1392


<210>  84
<211>  463
<212>  PRT
<213>  Mus musculus


<220>
<221>  SIGNAL
<222>  (1)..(19)

<400>  84

Met Gly Trp Ser Trp Ile Phe Leu Phe Leu Leu Ser Val Thr Ala Gly
1               5                   10                  15


Val Phe Ser Glu Val Gln Leu Gln Gln Ser Gly Pro Glu Leu Val Lys
            20                  25                  30


Pro Gly Ala Ser Val Lys Ile Ser Cys Lys Ala Ser Gly Tyr Ser Phe
        35                  40                  45


Thr Gly Tyr Phe Met Asn Trp Val Lys Gln Ser His Gly Lys Ser Leu
    50                  55                  60


Glu Trp Leu Gly Arg Ile Asn Pro Tyr Asn Gly Asp Thr Phe Tyr Asn
65                  70                  75                  80


Gln Lys Phe Lys Gly Lys Ala Thr Leu Thr Val Asp Lys Ser Ser Asn
                85                  90                  95


Thr Ala His Met Glu Leu Arg Ser Leu Thr Ser Glu Asp Ser Ala Val
            100                 105                 110


Tyr Tyr Cys Ala Arg Gly Asp Gly Tyr Tyr Val Thr Ser Leu Ala Tyr
        115                 120                 125


Trp Gly Gln Gly Thr Leu Val Thr Val Ser Ala Ala Lys Thr Thr Pro
    130                 135                 140
```

```
Pro Ser Val Tyr Pro Leu Ala Pro Gly Ser Ala Ala Gln Thr Asn Ser
145                 150                 155                 160

Met Val Thr Leu Gly Cys Leu Val Lys Gly Tyr Phe Pro Glu Pro Val
                165                 170                 175

Thr Val Thr Trp Asn Ser Gly Ser Leu Ser Ser Gly Val His Thr Phe
            180                 185                 190

Pro Ala Val Leu Gln Ser Asp Leu Tyr Thr Leu Ser Ser Ser Val Thr
        195                 200                 205

Val Pro Ser Ser Thr Trp Pro Ser Glu Thr Val Thr Cys Asn Val Ala
    210                 215                 220

His Pro Ala Ser Ser Thr Lys Val Asp Lys Lys Ile Val Pro Arg Asp
225                 230                 235                 240

Cys Gly Cys Lys Pro Cys Ile Cys Thr Val Pro Glu Val Ser Ser Val
                245                 250                 255

Phe Ile Phe Pro Pro Lys Pro Lys Asp Val Leu Thr Ile Thr Leu Thr
            260                 265                 270

Pro Lys Val Thr Cys Val Val Val Asp Ile Ser Lys Asp Asp Pro Glu
        275                 280                 285

Val Gln Phe Ser Trp Phe Val Asp Asp Val Glu Val His Thr Ala Gln
    290                 295                 300

Thr Gln Pro Arg Glu Glu Gln Phe Asn Ser Thr Phe Arg Ser Val Ser
305                 310                 315                 320

Glu Leu Pro Ile Met His Gln Asp Trp Leu Asn Gly Lys Glu Phe Lys
                325                 330                 335

Cys Arg Val Asn Ser Ala Ala Phe Pro Ala Pro Ile Glu Lys Thr Ile
            340                 345                 350

Ser Lys Thr Lys Gly Arg Pro Lys Ala Pro Gln Val Tyr Thr Ile Pro
        355                 360                 365

Pro Pro Lys Glu Gln Met Ala Lys Asp Lys Val Ser Leu Thr Cys Met
    370                 375                 380
```

Ile Thr Asp Phe Phe Pro Glu Asp Ile Thr Val Glu Trp Gln Trp Asn
385 390 395 400

Gly Gln Pro Ala Glu Asn Tyr Lys Asn Thr Gln Pro Ile Met Asn Thr
405 410 415

Asn Gly Ser Tyr Phe Val Tyr Ser Lys Leu Asn Val Gln Lys Ser Asn
420 425 430

Trp Glu Ala Gly Asn Thr Phe Thr Cys Ser Val Leu His Glu Gly Leu
435 440 445

His Asn His His Thr Glu Lys Ser Leu Ser His Ser Pro Gly Lys
450 455 460

<210> 85
<211> 33
<212> DNA
<213> Mus musculus

<400> 85
aaggccagtg agaatgtggt tagttatgta tcc 33

<210> 86
<211> 11
<212> PRT
<213> Mus musculus

<400> 86

Lys Ala Ser Glu Asn Val Val Ser Tyr Val Ser
1 5 10

<210> 87
<211> 21
<212> DNA
<213> Mus musculus

<400> 87
ggggcatcca accggtacac t 21

<210> 88
<211> 7
<212> PRT
<213> Mus musculus

<400> 88

Gly Ala Ser Asn Arg Tyr Thr
1 5

<210> 89
<211> 27
<212> DNA
<213> Mus musculus

<400> 89
ggacagagtt acagctatcc tctcacg 27

<210> 90
<211> 9
<212> PRT
<213> Mus musculus

<400> 90

Gly Gln Ser Tyr Ser Tyr Pro Leu Thr
1 5

<210> 91
<211> 324
<212> DNA
<213> Mus musculus

<400> 91
aacattgtaa tgacccaatc tcccaaatcc atgtccatgt cagtaggaga gagggtcacc 60

ttgagctgca aggccagtga gaatgtggtt agttatgtat cctggtttca acagaaacca 120

gagcagtctc ctaaactgct gatatatggg gcatccaacc ggtacactgg ggtccccgat 180

cgcttcacag gcagtggatc tgcaacagat ttcactctga ccatcagcag tgtgcaggct 240

gaagaccttg cagattatta ctgtggacag agttacagct atcctctcac gttcggtgct 300

gggaccaagc tggagctgaa acgg 324

<210> 92
<211> 108
<212> PRT
<213> Mus musculus

<400> 92

Asn Ile Val Met Thr Gln Ser Pro Lys Ser Met Ser Met Ser Val Gly
1 5 10 15

Glu Arg Val Thr Leu Ser Cys Lys Ala Ser Glu Asn Val Val Ser Tyr
20 25 30

Val Ser Trp Phe Gln Gln Lys Pro Glu Gln Ser Pro Lys Leu Leu Ile
35 40 45

Tyr Gly Ala Ser Asn Arg Tyr Thr Gly Val Pro Asp Arg Phe Thr Gly

50 55 60

Ser Gly Ser Ala Thr Asp Phe Thr Leu Thr Ile Ser Ser Val Gln Ala
65 70 75 80

Glu Asp Leu Ala Asp Tyr Tyr Cys Gly Gln Ser Tyr Ser Tyr Pro Leu
85 90 95

Thr Phe Gly Ala Gly Thr Lys Leu Glu Leu Lys Arg
100 105

<210> 93
<211> 717
<212> DNA
<213> Mus musculus

<220>
<221> sig_peptide
<222> (1)..(72)

<400> 93
atgggcatca agatggaatc acagactctg gtcttcatat ccatactgct ctggttatat 60

ggtgctgatg ggaacattgt aatgacccaa tctcccaaat ccatgtccat gtcagtagga 120

gagagggtca ccttgagctg caaggccagt gagaatgtgg ttagttatgt atcctggttt 180

caacagaaac cagagcagtc tcctaaactg ctgatatatg gggcatccaa ccggtacact 240

ggggtccccg atcgcttcac aggcagtgga tctgcaacag atttcactct gaccatcagc 300

agtgtgcagg ctgaagacct tgcagattat tactgtggac agagttacag ctatcctctc 360

acgttcggtg ctgggaccaa gctggagctg aaacgggctg atgctgcacc aactgtatcc 420

atcttcccac catccagtga gcagttaaca tctggaggtg cctcagtcgt gtgcttcttg 480

aacaacttct accccaaaga catcaatgtc aagtggaaga ttgatggcag tgaacgacaa 540

aatggcgtcc tgaacagttg gactgatcag gacagcaaag acagcaccta cagcatgagc 600

agcaccctca cgttgaccaa ggacgagtat gaacgacata acagctatac ctgtgaggcc 660

actcacaaga catcaacttc acccattgtc aagagcttca acaggaatga gtgttag 717

<210> 94
<211> 238
<212> PRT
<213> Mus musculus

<220>
<221> SIGNAL
<222> (1)..(24)

<400> 94

Met Gly Ile Lys Met Glu Ser Gln Thr Leu Val Phe Ile Ser Ile Leu
1               5                   10                  15

Leu Trp Leu Tyr Gly Ala Asp Gly Asn Ile Val Met Thr Gln Ser Pro
            20                  25                  30

Lys Ser Met Ser Met Ser Val Gly Glu Arg Val Thr Leu Ser Cys Lys
        35                  40                  45

Ala Ser Glu Asn Val Val Ser Tyr Val Ser Trp Phe Gln Gln Lys Pro
    50                  55                  60

Glu Gln Ser Pro Lys Leu Leu Ile Tyr Gly Ala Ser Asn Arg Tyr Thr
65                  70                  75                  80

Gly Val Pro Asp Arg Phe Thr Gly Ser Gly Ser Ala Thr Asp Phe Thr
                85                  90                  95

Leu Thr Ile Ser Ser Val Gln Ala Glu Asp Leu Ala Asp Tyr Tyr Cys
            100                 105                 110

Gly Gln Ser Tyr Ser Tyr Pro Leu Thr Phe Gly Ala Gly Thr Lys Leu
        115                 120                 125

Glu Leu Lys Arg Ala Asp Ala Ala Pro Thr Val Ser Ile Phe Pro Pro
    130                 135                 140

Ser Ser Glu Gln Leu Thr Ser Gly Gly Ala Ser Val Val Cys Phe Leu
145                 150                 155                 160

Asn Asn Phe Tyr Pro Lys Asp Ile Asn Val Lys Trp Lys Ile Asp Gly
                165                 170                 175

Ser Glu Arg Gln Asn Gly Val Leu Asn Ser Trp Thr Asp Gln Asp Ser
            180                 185                 190

Lys Asp Ser Thr Tyr Ser Met Ser Ser Thr Leu Thr Leu Thr Lys Asp
        195                 200                 205

Glu Tyr Glu Arg His Asn Ser Tyr Thr Cys Glu Ala Thr His Lys Thr
    210                 215                 220

Ser Thr Ser Pro Ile Val Lys Ser Phe Asn Arg Asn Glu Cys

225 230 235

<210> 95
<211> 15
<212> DNA
<213> Mus musculus

<400> 95
gactactaca tgaac 15

<210> 96
<211> 5
<212> PRT
<213> Mus musculus

<400> 96

Asp Tyr Tyr Met Asn
1 5

<210> 97
<211> 51
<212> DNA
<213> Mus musculus

<400> 97
cgtgttaatc ctagcaatgg tggtactagc tacaaccaga agttcaaggg c 51

<210> 98
<211> 17
<212> PRT
<213> Mus musculus

<400> 98

Arg Val Asn Pro Ser Asn Gly Gly Thr Ser Tyr Asn Gln Lys Phe Lys
1 5 10 15

Gly

<210> 99
<211> 33
<212> DNA
<213> Mus musculus

<400> 99
ggcctagcct actatagtaa ctcctttact tac 33

<210> 100
<211> 11
<212> PRT
<213> Mus musculus

<400>  100

```
Gly Leu Ala Tyr Tyr Ser Asn Ser Phe Thr Tyr
1               5                   10
```

<210>  101
<211>  360
<212>  DNA
<213>  Mus musculus

<400>  101

```
gaggtccagc tgcaacagtc tggacctgag ctggtgaagc ctggggcttc agtgaagatg     60

tcctgtaagg cttctggata cacattcact gactactaca tgaactgggt gaagcagagt    120

catggaaaga gccttgagtg gattggacgt gttaatccta gcaatggtgg tactagctac    180

aaccagaagt tcaagggcaa ggccacattg acagtagaca aatccctcag cacagcctac    240

atgcagctca acagcctgac atctgaggac tctgcggtct attactgtgc aagaggccta    300

gcctactata gtaactcctt tacttactgg ggccaaggga ctctggtcac tgtctctgca    360
```

<210>  102
<211>  120
<212>  PRT
<213>  Mus musculus

<400>  102

```
Glu Val Gln Leu Gln Gln Ser Gly Pro Glu Leu Val Lys Pro Gly Ala
1               5                   10                  15

Ser Val Lys Met Ser Cys Lys Ala Ser Gly Tyr Thr Phe Thr Asp Tyr
            20                  25                  30

Tyr Met Asn Trp Val Lys Gln Ser His Gly Lys Ser Leu Glu Trp Ile
        35                  40                  45

Gly Arg Val Asn Pro Ser Asn Gly Gly Thr Ser Tyr Asn Gln Lys Phe
    50                  55                  60

Lys Gly Lys Ala Thr Leu Thr Val Asp Lys Ser Leu Ser Thr Ala Tyr
65                  70                  75                  80

Met Gln Leu Asn Ser Leu Thr Ser Glu Asp Ser Ala Val Tyr Tyr Cys
                85                  90                  95

Ala Arg Gly Leu Ala Tyr Tyr Ser Asn Ser Phe Thr Tyr Trp Gly Gln
            100                 105                 110
```

```
Gly Thr Leu Val Thr Val Ser Ala
        115                 120


<210>  103
<211>  1425
<212>  DNA
<213>  Mus musculus


<220>
<221>  sig_peptide
<222>  (1)..(57)

<400>  103
atgggatgga gctgggtctt tctcttcctc ctgtcaggaa ctgcaggtgt ccactctgag      60

gtccagctgc aacagtctgg acctgagctg gtgaagcctg gggcttcagt gaagatgtcc     120

tgtaaggctt ctggatacac attcactgac tactacatga actgggtgaa gcagagtcat     180

ggaaagagcc ttgagtggat tggacgtgtt aatcctagca atggtggtac tagctacaac     240

cagaagttca agggcaaggc cacattgaca gtagacaaat ccctcagcac agcctacatg     300

cagctcaaca gcctgacatc tgaggactct gcggtctatt actgtgcaag aggcctagcc     360

tactatagta actcctttac ttactggggc caagggactc tggtcactgt ctctgcagcc     420

aaaacaacag ccccatcggt ctatccactg gcccctgtgt gtggaggtac aactggctcc     480

tcggtgactc taggatgcct ggtcaagggt tatttccctg agccagtgac cttgacctgg     540

aactctggat ccctgtccag tggtgtgcac accttcccag ctctcctgca gtctggcctc     600

tacaccctca gcagctcagt gactgtaacc tcgaacacct ggcccagcca gaccatcacc     660

tgcaatgtgg cccacccggc aagcagcacc aaagtggaca agaaaattga gcccagagtg     720

cccataacac agaacccctg tcctccactc aaagagtgtc cccatgcgc agctccagac      780

ctcttgggtg gaccatccgt cttcatcttc cctccaaaga tcaaggatgt actcatgatc     840

tccctgagcc ccatggtcac atgtgtggtg gtggatgtga gcgaggatga cccagacgtc     900

cagatcagct ggtttgtgaa caacgtggaa gtacacacag ctcagacaca aacccataga     960

gaggattaca acagtactct ccgggtggtc agtgccctcc ccatccagca ccaggactgg    1020

atgagtggca aggagttcaa atgcaaggtc aacaacagag ccctcccatc ccccatcgag    1080

aaaaccatct caaaacccag agggccagta agagctccac aggtatatgt cttgcctcca    1140

ccagcagaag agatgactaa gaaagagttc agtctgacct gcatgatcac aggcttctta    1200

cctgccgaaa ttgctgtgga ctggaccagc aatgggcgta cagagcaaaa ctacaagaac    1260

accgcaacag tcctggactc tgatggttct tacttcatgt acagcaagct cagagtacaa    1320
```

aagagcactt gggaaagagg aagtcttttc gcctgctcag tggtccacga gggtctgcac 1380

aatcacctta cgactaagac catctcccgg tctctgggta aatga 1425

<210> 104
<211> 474
<212> PRT
<213> Mus musculus

<220>
<221> SIGNAL
<222> (1)..(19)

<400> 104

```
Met Gly Trp Ser Trp Val Phe Leu Phe Leu Leu Ser Gly Thr Ala Gly
1               5                   10                  15

Val His Ser Glu Val Gln Leu Gln Gln Ser Gly Pro Glu Leu Val Lys
            20                  25                  30

Pro Gly Ala Ser Val Lys Met Ser Cys Lys Ala Ser Gly Tyr Thr Phe
        35                  40                  45

Thr Asp Tyr Tyr Met Asn Trp Val Lys Gln Ser His Gly Lys Ser Leu
    50                  55                  60

Glu Trp Ile Gly Arg Val Asn Pro Ser Asn Gly Gly Thr Ser Tyr Asn
65                  70                  75                  80

Gln Lys Phe Lys Gly Lys Ala Thr Leu Thr Val Asp Lys Ser Leu Ser
                85                  90                  95

Thr Ala Tyr Met Gln Leu Asn Ser Leu Thr Ser Glu Asp Ser Ala Val
            100                 105                 110

Tyr Tyr Cys Ala Arg Gly Leu Ala Tyr Tyr Ser Asn Ser Phe Thr Tyr
        115                 120                 125

Trp Gly Gln Gly Thr Leu Val Thr Val Ser Ala Ala Lys Thr Thr Ala
    130                 135                 140

Pro Ser Val Tyr Pro Leu Ala Pro Val Cys Gly Gly Thr Thr Gly Ser
145                 150                 155                 160

Ser Val Thr Leu Gly Cys Leu Val Lys Gly Tyr Phe Pro Glu Pro Val
                165                 170                 175
```

```
Thr Leu Thr Trp Asn Ser Gly Ser Leu Ser Ser Gly Val His Thr Phe
            180                 185                 190

Pro Ala Leu Leu Gln Ser Gly Leu Tyr Thr Leu Ser Ser Ser Val Thr
        195                 200                 205

Val Thr Ser Asn Thr Trp Pro Ser Gln Thr Ile Thr Cys Asn Val Ala
    210                 215                 220

His Pro Ala Ser Ser Thr Lys Val Asp Lys Lys Ile Glu Pro Arg Val
225                 230                 235                 240

Pro Ile Thr Gln Asn Pro Cys Pro Pro Leu Lys Glu Cys Pro Pro Cys
                245                 250                 255

Ala Ala Pro Asp Leu Leu Gly Gly Pro Ser Val Phe Ile Phe Pro Pro
            260                 265                 270

Lys Ile Lys Asp Val Leu Met Ile Ser Leu Ser Pro Met Val Thr Cys
        275                 280                 285

Val Val Val Asp Val Ser Glu Asp Asp Pro Asp Val Gln Ile Ser Trp
    290                 295                 300

Phe Val Asn Asn Val Glu Val His Thr Ala Gln Thr Gln Thr His Arg
305                 310                 315                 320

Glu Asp Tyr Asn Ser Thr Leu Arg Val Val Ser Ala Leu Pro Ile Gln
                325                 330                 335

His Gln Asp Trp Met Ser Gly Lys Glu Phe Lys Cys Lys Val Asn Asn
            340                 345                 350

Arg Ala Leu Pro Ser Pro Ile Glu Lys Thr Ile Ser Lys Pro Arg Gly
        355                 360                 365

Pro Val Arg Ala Pro Gln Val Tyr Val Leu Pro Pro Pro Ala Glu Glu
    370                 375                 380

Met Thr Lys Lys Glu Phe Ser Leu Thr Cys Met Ile Thr Gly Phe Leu
385                 390                 395                 400

Pro Ala Glu Ile Ala Val Asp Trp Thr Ser Asn Gly Arg Thr Glu Gln
                405                 410                 415
```

```
Asn Tyr Lys Asn Thr Ala Thr Val Leu Asp Ser Asp Gly Ser Tyr Phe
            420                 425                 430

Met Tyr Ser Lys Leu Arg Val Gln Lys Ser Thr Trp Glu Arg Gly Ser
        435                 440                 445

Leu Phe Ala Cys Ser Val Val His Glu Gly Leu His Asn His Leu Thr
    450                 455                 460

Thr Lys Thr Ile Ser Arg Ser Leu Gly Lys
465                 470


<210>  105
<211>  45
<212>  DNA
<213>  Mus musculus

<400>  105
agagccagtg aaagtgttga tagttatggc aatagtttta tgcac                     45


<210>  106
<211>  15
<212>  PRT
<213>  Mus musculus

<400>  106

Arg Ala Ser Glu Ser Val Asp Ser Tyr Gly Asn Ser Phe Met His
1               5                   10                  15


<210>  107
<211>  21
<212>  DNA
<213>  Mus musculus

<400>  107
cgtgcatcca acctagaatc t                                               21


<210>  108
<211>  7
<212>  PRT
<213>  Mus musculus

<400>  108

Arg Ala Ser Asn Leu Glu Ser
1               5


<210>  109
<211>  27
<212>  DNA
```

<213>  Mus musculus

<400>  109
cagcaaaata atgaggatcc gtggacg     27

<210>  110
<211>  9
<212>  PRT
<213>  Mus musculus

<400>  110

Gln Gln Asn Asn Glu Asp Pro Trp Thr
1               5

<210>  111
<211>  336
<212>  DNA
<213>  Mus musculus

<400>  111
aaaattgtgc tgacccaatc tccagcttct ttggctgtgt ctctaaggca gagggccacc     60

atatcctgca gagccagtga aagtgttgat agttatggca atagttttat gcactggtac     120

cagcagaaac caggacagcc acccaaactc ctcatctatc gtgcatccaa cctagaatct     180

ggggtccctg ccaggttcag tggcagtggg tctaggacag acttcaccct caccattgat     240

cctgtggagg ctgatgatgc tgcaacctat tactgtcagc aaaataatga ggatccgtgg     300

acgttcggtg gaggcaccaa gctggaaatc aaacgg     336

<210>  112
<211>  112
<212>  PRT
<213>  Mus musculus

<400>  112

Lys Ile Val Leu Thr Gln Ser Pro Ala Ser Leu Ala Val Ser Leu Arg
1               5                   10                  15

Gln Arg Ala Thr Ile Ser Cys Arg Ala Ser Glu Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Arg Ala Ser Asn Leu Glu Ser Gly Val Pro Ala
    50                  55                  60

Arg Phe Ser Gly Ser Gly Ser Arg Thr Asp Phe Thr Leu Thr Ile Asp

65 70 75 80

Pro Val Glu Ala Asp Asp Ala Ala Thr Tyr Tyr Cys Gln Gln Asn Asn
85 90 95

Glu Asp Pro Trp Thr Phe Gly Gly Gly Thr Lys Leu Glu Ile Lys Arg
100 105 110

<210> 113
<211> 717
<212> DNA
<213> Mus musculus

<220>
<221> sig_peptide
<222> (1)..(60)

<400> 113
atggagacag agacactcct gctatgggtg ctactgctct gggttccagg ttccacaggt 60

aaaattgtgc tgacccaatc tccagcttct ttggctgtgt ctctaaggca gagggccacc 120

atatcctgca gagccagtga aagtgttgat agttatggca atagttttat gcactggtac 180

cagcagaaac caggacagcc acccaaactc ctcatctatc gtgcatccaa cctagaatct 240

ggggtccctg ccaggttcag tggcagtggg tctaggacag acttcaccct caccattgat 300

cctgtggagg ctgatgatgc tgcaacctat tactgtcagc aaaataatga ggatccgtgg 360

acgttcggtg gaggcaccaa gctggaaatc aaacgggctg atgctgcacc aactgtatcc 420

atcttcccac catccagtga gcagttaaca tctggaggtg cctcagtcgt gtgcttcttg 480

aacaacttct accccaaaga catcaatgtc aagtggaaga ttgatggcag tgaacgacaa 540

aatggcgtcc tgaacagttg gactgatcag gacagcaaag acagcaccta cagcatgagc 600

agcaccctca cgttgaccaa ggacgagtat gaacgacata acagctatac ctgtgaggcc 660

actcacaaga catcaacttc acccattgtc aagagcttca acaggaatga gtgttag 717

<210> 114
<211> 238
<212> PRT
<213> Mus musculus

<220>
<221> SIGNAL
<222> (1)..(20)

<400> 114

Met Glu Thr Glu Thr Leu Leu Leu Trp Val Leu Leu Leu Trp Val Pro

1 5 10 15

Gly Ser Thr Gly Lys Ile Val Leu Thr Gln Ser Pro Ala Ser Leu Ala
20 25 30

Val Ser Leu Arg Gln Arg Ala Thr Ile Ser Cys Arg Ala Ser Glu Ser
35 40 45

Val Asp Ser Tyr Gly Asn Ser Phe Met His Trp Tyr Gln Gln Lys Pro
50 55 60

Gly Gln Pro Pro Lys Leu Leu Ile Tyr Arg Ala Ser Asn Leu Glu Ser
65 70 75 80

Gly Val Pro Ala Arg Phe Ser Gly Ser Gly Ser Arg Thr Asp Phe Thr
85 90 95

Leu Thr Ile Asp Pro Val Glu Ala Asp Asp Ala Ala Thr Tyr Tyr Cys
100 105 110

Gln Gln Asn Asn Glu Asp Pro Trp Thr Phe Gly Gly Gly Thr Lys Leu
115 120 125

Glu Ile Lys Arg Ala Asp Ala Ala Pro Thr Val Ser Ile Phe Pro Pro
130 135 140

Ser Ser Glu Gln Leu Thr Ser Gly Gly Ala Ser Val Val Cys Phe Leu
145 150 155 160

Asn Asn Phe Tyr Pro Lys Asp Ile Asn Val Lys Trp Lys Ile Asp Gly
165 170 175

Ser Glu Arg Gln Asn Gly Val Leu Asn Ser Trp Thr Asp Gln Asp Ser
180 185 190

Lys Asp Ser Thr Tyr Ser Met Ser Ser Thr Leu Thr Leu Thr Lys Asp
195 200 205

Glu Tyr Glu Arg His Asn Ser Tyr Thr Cys Glu Ala Thr His Lys Thr
210 215 220

Ser Thr Ser Pro Ile Val Lys Ser Phe Asn Arg Asn Glu Cys
225 230 235

<210> 115

<211> 867
<212> DNA
<213> Artificial

<220>
<221> source
<223> /note= "Description of artificial sequence: An artificially synthesized nucleotide sequence"

<400> 115
atgtccccta tactaggtta ttggaaaatt aagggccttg tgcaacccac tcgacttctt 60

ttggaatatc ttgaagaaaa atatgaagag catttgtatg agcgcgatga aggtgataaa 120

tggcgaaaca aaaagtttga attgggtttg gagtttccca atcttcctta ttatattgat 180

ggtgatgtta aattaacaca gtctatggcc atcatacgtt atatagctga caagcacaac 240

atgttgggtg gttgtccaaa agagcgtgca gagatttcaa tgcttgaagg agcggttttg 300

gatattagat acggtgtttc gagaattgca tatagtaaag actttgaaac tctcaaagtt 360

gattttctta gcaagctacc tgaaatgctg aaaatgttcg aagatcgttt atgtcataaa 420

acatatttaa atggtgatca tgtaacccat cctgacttca tgttgtatga cgctcttgat 480

gttgttttat acatggaccc aatgtgcctg gatgcgttcc caaaattagt ttgttttaaa 540

aaacgtattg aagctatccc acaaattgat aagtacttga aatccagcaa gtatatagca 600

tggcctttgc agggctggca agccacgttt ggtggtggcg accatcctcc aaaatcggat 660

ctggttccgc gtggatcccc aggaattcgc gtgtcggcct tcatcggcag caacatcatc 720

acgtcgcaga acatctggga gggcctgtgg atgaactgcg tggtgcagag caccggccag 780

atgcagtgca aggtgtacga ctcgctgctg gcactgccac aggaccttca ggcggcccgg 840

tcgactcacc atcatcatca tcattaa 867

<210> 116
<211> 288
<212> PRT
<213> Artificial

<220>
<221> source
<223> /note= "Description of artificial sequence: An artificially synthesized peptide sequence"

<400> 116

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

```
Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Pro Gly Ile Arg Val Ser Ala Phe Ile Gly Ser Asn Ile Ile
225                 230                 235                 240

Thr Ser Gln Asn Ile Trp Glu Gly Leu Trp Met Asn Cys Val Val Gln
                245                 250                 255

Ser Thr Gly Gln Met Gln Cys Lys Val Tyr Asp Ser Leu Leu Ala Leu
            260                 265                 270
```

```
Pro Gln Asp Leu Gln Ala Ala Arg Ser Thr His His His His His His
        275                 280                 285


<210>  117
<211>  261
<212>  PRT
<213>  Artificial

<220>
<221>  source
<223>  /note= "Description of artificial sequence: An artificially
       synthesized peptide sequence"

<400>  117

Met Ser Pro Ile Leu Gly Tyr Trp Lys Ile Lys Gly Leu Val Gln Pro
1               5                   10                  15


Thr Arg Leu Leu Leu Glu Tyr Leu Glu Glu Lys Tyr Glu Glu His Leu
            20                  25                  30


Tyr Glu Arg Asp Glu Gly Asp Lys Trp Arg Asn Lys Lys Phe Glu Leu
        35                  40                  45


Gly Leu Glu Phe Pro Asn Leu Pro Tyr Tyr Ile Asp Gly Asp Val Lys
    50                  55                  60


Leu Thr Gln Ser Met Ala Ile Ile Arg Tyr Ile Ala Asp Lys His Asn
65                  70                  75                  80


Met Leu Gly Gly Cys Pro Lys Glu Arg Ala Glu Ile Ser Met Leu Glu
                85                  90                  95


Gly Ala Val Leu Asp Ile Arg Tyr Gly Val Ser Arg Ile Ala Tyr Ser
            100                 105                 110


Lys Asp Phe Glu Thr Leu Lys Val Asp Phe Leu Ser Lys Leu Pro Glu
        115                 120                 125


Met Leu Lys Met Phe Glu Asp Arg Leu Cys His Lys Thr Tyr Leu Asn
    130                 135                 140


Gly Asp His Val Thr His Pro Asp Phe Met Leu Tyr Asp Ala Leu Asp
145                 150                 155                 160


Val Val Leu Tyr Met Asp Pro Met Cys Leu Asp Ala Phe Pro Lys Leu
                165                 170                 175
```

```
Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Pro Gly Ile Pro Trp Ser Ala Asn Thr Ile Ile Arg Asp Phe
225                 230                 235                 240

Tyr Asn Pro Val Val Pro Glu Ala Gln Lys Arg Glu Met Ser Thr His
                245                 250                 255

His His His His His
            260

<210>  118
<211>  786
<212>  DNA
<213>  Artificial

<220>
<221>  source
<223>  /note= "Description of artificial sequence: An artificially
       synthesized nucleotide sequence"

<400>  118
atgtccccta tactaggtta ttggaaaatt aagggccttg tgcaacccac tcgacttctt     60

ttggaatatc ttgaagaaaa atatgaagag catttgtatg agcgcgatga aggtgataaa    120

tggcgaaaca aaaagtttga attgggtttg gagtttccca atcttcctta ttatattgat    180

ggtgatgtta aattaacaca gtctatggcc atcatacgtt atatagctga caagcacaac    240

atgttgggtg gttgtccaaa agagcgtgca gagatttcaa tgcttgaagg agcggttttg    300

gatattagat acggtgtttc gagaattgca tatagtaaag actttgaaac tctcaaagtt    360

gattttctta gcaagctacc tgaaatgctg aaaatgttcg aagatcgttt atgtcataaa    420

acatatttaa atggtgatca tgtaacccat cctgacttca tgttgtatga cgctcttgat    480

gttgttttat acatggaccc aatgtgcctg gatgcgttcc caaaattagt ttgttttaaa    540

aaacgtattg aagctatccc acaaattgat aagtacttga aatccagcaa gtatatagca    600

tggcctttgc agggctggca agccacgttt ggtggtggcg accatcctcc aaaatcggat    660

ctggttccgc gtggatcccc aggaattccc tggtcggcca acaccattat ccgggacttc    720
```

tacaaccccg tggtgcccga ggcgcagaag cgcgagatgt cgactcacca tcatcatcat 780

cattaa 786

<210> 119
<211> 30
<212> DNA
<213> Homo sapiens

<400> 119
cggccaccat gtccatgggc ctggagatca 30

<210> 120
<211> 30
<212> DNA
<213> Homo sapiens

<400> 120
gtctgtccct tagacgtagt ccttgcggtc 30

<210> 121
<211> 34
<212> DNA
<213> Mus musculus

<400> 121
gcgaattcca ccatgtccat gggcctggag atca 34

<210> 122
<211> 33
<212> DNA
<213> Mus musculus

<400> 122
gcgatatctg tcctcttcca gcctagcaag cag 33

<210> 123
<211> 30
<212> DNA
<213> Homo sapiens

<400> 123
atggccaacg cggggctgca gctgttgggc 30

<210> 124
<211> 30
<212> DNA
<213> Homo sapiens

<400> 124
tgtgtcacac gtagtctttc ccgctggaag 30

<210> 125

<211> 27
<212> DNA
<213> Homo sapiens

<400> 125
gaacaatggc ctccatgggg ctacagg 27

<210> 126
<211> 29
<212> DNA
<213> Homo sapiens

<400> 126
aggagggtgg actctgttct tgctagcag 29

<210> 127
<211> 27
<212> DNA
<213> Homo sapiens

<400> 127
catggcctct gccggaatgc agatcct 27

<210> 128
<211> 27
<212> DNA
<213> Homo sapiens

<400> 128
cccaaagctg ttgggcactg ccacttc 27

<210> 129
<211> 28
<212> DNA
<213> Mus musculus

<400> 129
ccatggagtt agtttgggca gcagatcc 28

<210> 130
<211> 24
<212> DNA
<213> Mus musculus

<400> 130
caggggccag tggatagacc gatg 24

<210> 131
<211> 24
<212> DNA
<213> Mus musculus

<400> 131
caggggccag tggatagact gatg 24

<210> 132
<211> 27
<212> DNA
<213> Mus musculus

<400> 132
ggcacctcca gatgttaact gctcact 27

<210> 133
<211> 27
<212> DNA
<213> Mus musculus

<400> 133
tcgagctctt cagaggaagg tggaaac 27

<210> 134
<211> 51
<212> PRT
<213> Homo sapiens

<400> 134

```
Arg Val Thr Ala Phe Ile Gly Ser Asn Ile Val Thr Ser Gln Thr Ile
1               5                   10                  15

Trp Glu Gly Leu Trp Met Asn Cys Val Val Gln Ser Thr Gly Gln Met
            20                  25                  30

Gln Cys Lys Val Tyr Asp Ser Leu Leu Ala Leu Pro Gln Asp Leu Gln
        35                  40                  45

Ala Ala Arg
    50
```

<210> 135
<211> 51
<212> PRT
<213> Homo sapiens

<400> 135

```
Lys Val Thr Ala Phe Ile Gly Asn Ser Ile Val Val Ala Gln Val Val
1               5                   10                  15

Trp Glu Gly Leu Trp Met Ser Cys Val Val Gln Ser Thr Gly Gln Met
            20                  25                  30

Gln Cys Lys Val Tyr Asp Ser Leu Leu Ala Leu Pro Gln Asp Leu Gln
        35                  40                  45
```

Ala Ala Arg
50

<210> 136
<211> 51
<212> PRT
<213> Homo sapiens

<400> 136

Lys Val Thr Ala Phe Ile Gly Asn Ser Ile Val Val Ala Gln Val Val
1 5 10 15

Trp Glu Gly Leu Trp Met Ser Cys Val Val Gln Ser Thr Gly Gln Met
20 25 30

Gln Cys Lys Val Tyr Asp Ser Leu Leu Ala Leu Pro Gln Asp Leu Gln
35 40 45

Ala Ala Arg
50

<210> 137
<211> 51
<212> PRT
<213> Homo sapiens

<400> 137

Arg Val Ser Ala Phe Ile Glu Asn Asn Ile Val Val Phe Glu Asn Phe
1 5 10 15

Trp Glu Gly Leu Trp Met Asn Cys Val Arg Gln Ala Asn Ile Arg Met
20 25 30

Gln Cys Lys Ile Tyr Asp Ser Leu Leu Ala Leu Ser Pro Asp Leu Gln
35 40 45

Ala Ala Arg
50

<210> 138
<211> 51
<212> PRT
<213> Homo sapiens

<400> 138

Gln Val Thr Ala Phe Leu Asp His Asn Ile Val Thr Ala Gln Thr Thr

```
1               5                   10                  15

Trp Lys Gly Leu Trp Met Ser Cys Val Val Gln Ser Thr Gly His Met
            20                  25                  30

Gln Cys Lys Val Tyr Asp Ser Val Leu Ala Leu Ser Thr Glu Val Gln
        35                  40                  45

Ala Ala Arg
    50


<210>  139
<211>  51
<212>  PRT
<213>  Homo sapiens

<400>  139

Arg Val Ser Ala Phe Val Gly Ser Asn Ile Ile Val Phe Glu Arg Leu
1               5                   10                  15

Trp Glu Gly Leu Trp Met Asn Cys Ile Arg Gln Ala Arg Val Arg Leu
            20                  25                  30

Gln Cys Lys Phe Tyr Ser Ser Leu Leu Ala Leu Pro Pro Ala Leu Glu
        35                  40                  45

Thr Ala Arg
    50


<210>  140
<211>  51
<212>  PRT
<213>  Homo sapiens

<400>  140

Arg Ile Tyr Ser Tyr Ala Gly Asp Asn Ile Val Thr Ala Gln Ala Met
1               5                   10                  15

Tyr Glu Gly Leu Trp Met Ser Cys Val Ser Gln Ser Thr Gly Gln Ile
            20                  25                  30

Gln Cys Lys Val Phe Asp Ser Leu Leu Asn Leu Ser Ser Thr Leu Gln
        35                  40                  45

Ala Thr Arg
    50
```

```
<210>  141
<211>  51
<212>  PRT
<213>  Homo sapiens

<400>  141

Gln Met Ser Ser Tyr Ala Gly Asp Asn Ile Ile Thr Ala Gln Ala Met
1               5                   10                  15

Tyr Lys Gly Leu Trp Met Asp Cys Val Thr Gln Ser Thr Gly Met Met
            20                  25                  30

Ser Cys Lys Met Tyr Asp Ser Val Leu Ala Leu Ser Ala Ala Leu Gln
        35                  40                  45

Ala Thr Arg
    50


<210>  142
<211>  51
<212>  PRT
<213>  Homo sapiens

<400>  142

Arg Arg Thr Ala His Val Gly Thr Asn Ile Leu Thr Ala Val Ser Tyr
1               5                   10                  15

Leu Lys Gly Leu Trp Met Glu Cys Val Trp His Ser Thr Gly Ile Tyr
            20                  25                  30

Gln Cys Gln Ile Tyr Arg Ser Leu Leu Ala Leu Pro Gln Asp Leu Gln
        35                  40                  45

Ala Ala Arg
    50


<210>  143
<211>  49
<212>  PRT
<213>  Homo sapiens

<400>  143

Ser Ser Tyr Ala Gly Asp Ala Ile Ile Thr Ala Val Gly Leu Tyr Glu
1               5                   10                  15

Gly Leu Trp Met Ser Cys Ala Ser Gln Ser Thr Gly Gln Val Gln Cys
            20                  25                  30
```

```
Lys Leu Tyr Asp Ser Leu Leu Ala Leu Asp Gly His Ile Gln Ser Ala
        35                  40                  45

Arg
```

<210> 144
<211> 51
<212> PRT
<213> Homo sapiens

<400> 144

```
Lys Thr Ser Ser Tyr Val Gly Ala Ser Ile Val Thr Ala Val Gly Phe
1               5                   10                  15

Ser Lys Gly Leu Trp Met Glu Cys Ala Thr His Ser Thr Gly Ile Thr
            20                  25                  30

Gln Cys Asp Ile Tyr Ser Thr Leu Leu Gly Leu Pro Ala Asp Ile Gln
        35                  40                  45

Ala Ala Gln
    50
```

<210> 145
<211> 51
<212> PRT
<213> Homo sapiens

<400> 145

```
Lys Val Asn Val Asp Val Asp Ser Asn Ile Ile Thr Ala Ile Val Gln
1               5                   10                  15

Leu His Gly Leu Trp Met Asp Cys Thr Trp Tyr Ser Thr Gly Met Phe
            20                  25                  30

Ser Cys Ala Leu Lys His Ser Ile Leu Ser Leu Pro Ile His Val Gln
        35                  40                  45

Ala Ala Arg
    50
```

<210> 146
<211> 22
<212> PRT
<213> Homo sapiens

<400> 146

Val Ala Asn Ala Ile Ile Arg Asp Phe Tyr Asn Ser Ile Val Asn Val
1               5                   10                  15

Ala Gln Lys Arg Glu Leu
            20

<210> 147
<211> 22
<212> PRT
<213> Homo sapiens

<400> 147

Thr Ala His Ala Ile Ile Gln Asp Phe Tyr Asn Pro Leu Val Ala Glu
1               5                   10                  15

Ala Leu Lys Arg Glu Leu
            20

<210> 148
<211> 22
<212> PRT
<213> Homo sapiens

<400> 148

Thr Ala Asn Ile Ile Ile Arg Asp Phe Tyr Asn Pro Ala Ile His Ile
1               5                   10                  15

Gly Gln Lys Arg Glu Leu
            20

<210> 149
<211> 4
<212> PRT
<213> Artificial

<220>
<221> source
<223> /note= "Description of artificial sequence: An artificially synthesized peptide sequence"

<400> 149

Gly Gly Gly Ser
1

<210> 150
<211> 4
<212> PRT

<213> Artificial

<220>
<221> source
<223> /note= "Description of artificial sequence: An artificially synthesized peptide sequence"

<400> 150

Ser Gly Gly Gly
1

<210> 151
<211> 5
<212> PRT
<213> Artificial

<220>
<221> source
<223> /note= "Description of artificial sequence: An artificially synthesized peptide sequence"

<400> 151

Gly Gly Gly Gly Ser
1 5

<210> 152
<211> 5
<212> PRT
<213> Artificial

<220>
<221> source
<223> /note= "Description of artificial sequence: An artificially synthesized peptide sequence"

<400> 152

Ser Gly Gly Gly Gly
1 5

<210> 153
<211> 6
<212> PRT
<213> Artificial

<220>
<221> source
<223> /note= "Description of artificial sequence: An artificially synthesized peptide sequence"

<400> 153

Gly Gly Gly Gly Gly Ser
1 5

<210> 154
<211> 6
<212> PRT
<213> Artificial

<220>
<221> source
<223> /note= "Description of artificial sequence: An artificially synthesized peptide sequence"

<400> 154

Ser Gly Gly Gly Gly Gly
1               5

<210> 155
<211> 7
<212> PRT
<213> Artificial

<220>
<221> source
<223> /note= "Description of artificial sequence: An artificially synthesized peptide sequence"

<400> 155

Gly Gly Gly Gly Gly Gly Ser
1               5

<210> 156
<211> 7
<212> PRT
<213> Artificial

<220>
<221> source
<223> /note= "Description of artificial sequence: An artificially synthesized peptide sequence"

<400> 156

Ser Gly Gly Gly Gly Gly Gly
1               5

<210> 157
<211> 5
<212> PRT
<213> Artificial

<220>
<221> source
<223> /note= "Description of artificial sequence: An artificially synthesized peptide sequence"

<400> 157

Gly Gly Gly Gly Ser
1               5

<210>  158
<211>  5
<212>  PRT
<213>  Artificial

<220>
<221>  source
<223>  /note= "Description of artificial sequence: An artificially synthesized peptide sequence"

<400>  158

Ser Gly Gly Gly Gly
1               5

<210>  159
<211>  3876
<212>  DNA
<213>  Artificial

<220>
<221>  source
<223>  /note= "Description of artificial sequence: An artificially synthesized nucleotide sequence"

<220>
<221>  CDS
<222>  (836)..(1498)

<400>  159
tcgcgcgttt cggtgatgac ggtgaaaacc tctgacacat gcagctcccg gagacggtca     60

cagcttgtct gtaagcggat gccgggagca gacaagcccg tcagggcgcg tcagcgggtg    120

ttggcgggtg tcggggctgg cttaactatg cggcatcaga gcagattgta ctgagagtgc    180

accatatgtg agtcattagg gactttccaa tgggttttgc ccagtacata aggtcaatgg    240

gaggtaagcc aatgggtttt tcccattact ggcacctagc tgagtcatta gggactttcc    300

aatgggtttt gcccagtaca taaggtcaat aggggtgaat caacaggaaa gtcccattgg    360

agccaagtac actgagtcaa tagggacttt ccattgggtt ttgcccagta caaaaggtca    420

ataggggtg agtcaatggg tttttcccat tattggcacg tacataaggt caataggggt    480

gagtcattgg gtttttccag ccaatttaat taaaacgcca tgtactttcc caccattgac    540

gtcaatgggc tattgaaact aatgcaacgt gacctttaaa cggtactttc ccatagctga    600

ttaatgggaa agtaccgttc tcgagccaat acacgtcaat gggaagtgaa agggcagcca    660

aaacgtaaca ccgccccggt tttcccctgg aaattccata ttggcacgca ttctattggc    720

```
tgagctgcgt tctacgtggg tataagaggc gcgaccagcg tcggtaccgt cgcagtcttc      780

ggtctgacca ccgtagaacg caaagcttgc ccgggcgaat tcgattcggg ccacc atg       838
                                                             Met
                                                             1

tcc atg ggc ctg gag atc acg ggc acc gcg ctg gcc gtg ctg ggc tgg        886
Ser Met Gly Leu Glu Ile Thr Gly Thr Ala Leu Ala Val Leu Gly Trp
            5                   10                  15

ctg ggc acc atc gtg tgc tgc gcg ttg ccc atg tgg cgc gtg tcg gcc        934
Leu Gly Thr Ile Val Cys Cys Ala Leu Pro Met Trp Arg Val Ser Ala
        20                  25                  30

ttc atc ggc agc aac atc atc acg tcg cag aac atc tgg gag ggc ctg        982
Phe Ile Gly Ser Asn Ile Ile Thr Ser Gln Asn Ile Trp Glu Gly Leu
    35                  40                  45

tgg atg aac tgc gtg gtg cag agc acc ggc cag atg cag tgc aag gtg       1030
Trp Met Asn Cys Val Val Gln Ser Thr Gly Gln Met Gln Cys Lys Val
50                  55                  60                  65

tac gac tcg ctg ctg gca ctg cca cag gac ctt cag gcg gcc cgc gcc       1078
Tyr Asp Ser Leu Leu Ala Leu Pro Gln Asp Leu Gln Ala Ala Arg Ala
                70                  75                  80

ctc atc gtg gtg gcc atc ctg ctg gcc gcc ttc ggg ctg cta gtg gcg       1126
Leu Ile Val Val Ala Ile Leu Leu Ala Ala Phe Gly Leu Leu Val Ala
            85                  90                  95

ctg gtg ggc gcc cag tgc acc aac tgc gtg cag gac gac acg gcc aag       1174
Leu Val Gly Ala Gln Cys Thr Asn Cys Val Gln Asp Asp Thr Ala Lys
        100                 105                 110

gcc aag atc acc atc gtg gca ggc gtg ctg ttc ctt ctc gcc gcc ctg       1222
Ala Lys Ile Thr Ile Val Ala Gly Val Leu Phe Leu Leu Ala Ala Leu
    115                 120                 125

ctc acc ctc gtg ccg gtg tcc tgg tcg gcc aac acc att atc cgg gac       1270
Leu Thr Leu Val Pro Val Ser Trp Ser Ala Asn Thr Ile Ile Arg Asp
130                 135                 140                 145

ttc tac aac ccc gtg gtg ccc gag gcg cag aag cgc gag atg ggc gcg       1318
Phe Tyr Asn Pro Val Val Pro Glu Ala Gln Lys Arg Glu Met Gly Ala
                150                 155                 160

ggc ctg tac gtg ggc tgg gcg gcc gcg gcg ctg cag ctg ctg ggg ggc       1366
Gly Leu Tyr Val Gly Trp Ala Ala Ala Ala Leu Gln Leu Leu Gly Gly
            165                 170                 175

gcg ctg ctc tgc tgc tcg tgt ccc cca cgc gag aag aag tac acg gcc       1414
Ala Leu Leu Cys Cys Ser Cys Pro Pro Arg Glu Lys Lys Tyr Thr Ala
        180                 185                 190

acc aag gtc gtc tac tcc gcg ccg cgc tcc acc ggc ccg gga gcc agc       1462
Thr Lys Val Val Tyr Ser Ala Pro Arg Ser Thr Gly Pro Gly Ala Ser
    195                 200                 205

ctg ggc aca ggc tac gac cgc aag gac tac gtc taa gggtcagaca            1508
```

```
Leu Gly Thr Gly Tyr Asp Arg Lys Asp Tyr Val
210                 215                 220

atcactagtg aattcgtcga cgctagcgat atcgcggccg ctctagagtc ggggcggccg   1568
gccgcttcga gcagacatga taagatacat tgatgagttt ggacaaacca caactagaat   1628
gcagtgaaaa aaatgcttta tttgtgaaat ttgtgatgct attgctttat ttgtaaccat   1688
tataagctgc aataaacaag ttaacaacaa caattgcatt cattttatgt ttcaggttca   1748
gggggaggtg tgggaggttt tttaaagcaa gtaaaacctc tacaaatgtg gtaaaatcga   1808
taaggatccc agctgcatta atgaatcggc caacgcgcgg ggagaggcgg tttgcgtatt   1868
gggcgctctt ccgcttcctc gctcactgac tcgctgcgct cggtcgttcg gctgcggcga   1928
gcggtatcag ctcactcaaa ggcggtaata cggttatcca cagaatcagg ggataacgca   1988
ggaaagaaca tgtgagcaaa aggccagcaa aaggccagga accgtaaaaa ggccgcgttg   2048
ctggcgtttt tccataggct ccgcccccct gacgagcatc acaaaaatcg acgctcaagt   2108
cagaggtggc gaaacccgac aggactataa agataccagg cgtttccccc tggaagctcc   2168
ctcgtgcgct ctcctgttcc gaccctgccg cttaccggat acctgtccgc ctttctccct   2228
tcgggaagcg tggcgctttc tcatagctca cgctgtaggt atctcagttc ggtgtaggtc   2288
gttcgctcca agctgggctg tgtgcacgaa ccccccgttc agcccgaccg ctgcgcctta   2348
tccggtaact atcgtcttga gtccaacccg gtaagacacg acttatcgcc actggcagca   2408
gccactggta acaggattag cagagcgagg tatgtaggcg gtgctacaga gttcttgaag   2468
tggtggccta actacggcta cactagaaga acagtatttg gtatctgcgc tctgctgaag   2528
ccagttacct tcggaaaaag agttggtagc tcttgatccg gcaaacaaac caccgctggt   2588
agcggtggtt tttttgtttg caagcagcag attacgcgca gaaaaaaagg atctcaagaa   2648
gatcctttga tcttttctac ggggtctgac gctcagtgga acgaaaactc acgttaaggg   2708
attttggtca tgagattatc aaaaaggatc ttcacctaga tccttttaaa ttaaaaatga   2768
agttttaaat caatctaaag tatatatgag taaacttggt ctgacagtta ccaatgctta   2828
atcagtgagg cacctatctc agcgatctgt ctatttcgtt catccatagt tgcctgactc   2888
cccgtcgtgt agataactac gatacgggag ggcttaccat ctggccccag tgctgcaatg   2948
ataccgcgag acccacgctc accggctcca gatttatcag caataaacca gccagccgga   3008
agggccgagc gcagaagtgg tcctgcaact ttatccgcct ccatccagtc tattaattgt   3068
tgccgggaag ctagagtaag tagttcgcca gttaatagtt tgcgcaacgt tgttgccatt   3128
gctacaggca tcgtggtgtc acgctcgtcg tttggtatgg cttcattcag ctccggttcc   3188
caacgatcaa ggcgagttac atgatccccc atgttgtgca aaaaagcggt tagctccttc   3248
```

```
ggtcctccga tcgttgtcag aagtaagttg gccgcagtgt tatcactcat ggttatggca   3308

gcactgcata attctcttac tgtcatgcca tccgtaagat gcttttctgt gactggtgag   3368

tactcaacca agtcattctg agaatagtgt atgcggcgac cgagttgctc ttgcccggcg   3428

tcaatacggg ataataccgc gccacatagc agaactttaa aagtgctcat cattggaaaa   3488

cgttcttcgg ggcgaaaact ctcaaggatc ttaccgctgt tgagatccag ttcgatgtaa   3548

cccactcgtg cacccaactg atcttcagca tcttttactt tcaccagcgt ttctgggtga   3608

gcaaaaacag gaaggcaaaa tgccgcaaaa aagggaataa gggcgacacg gaaatgttga   3668

atactcatac tcttcctttt tcaatattat tgaagcattt atcagggtta ttgtctcatg   3728

agcggataca tatttgaatg tatttagaaa aataaacaaa taggggttcc gcgcacattt   3788

ccccgaaaag tgccacctga cgtctaagaa accattatta tcatgacatt aacctataaa   3848

aataggcgta tcacgaggcc ctttcgtc                                      3876


<210>  160
<211>  714
<212>  DNA
<213>  Artificial

<220>
<221>  source
<223>  /note= "Description of artificial sequence: An artificially
       synthesized nucleotide sequence"


<220>
<221>  CDS
<222>  (1)..(714)

<400>  160
atg gcc aac gcg ggg ctg cag ctg ttg ggc ttc att ctc gcc ttc ctg        48
Met Ala Asn Ala Gly Leu Gln Leu Leu Gly Phe Ile Leu Ala Phe Leu
1               5                   10                  15

gga tgg atc ggc gcc atc gtc agc act gcc ctg ccc cag tgg agg att        96
Gly Trp Ile Gly Ala Ile Val Ser Thr Ala Leu Pro Gln Trp Arg Ile
            20                  25                  30

tac tcc tat gcc ggc gac aac atc gtg acc gcc cag gcc atg tac gag       144
Tyr Ser Tyr Ala Gly Asp Asn Ile Val Thr Ala Gln Ala Met Tyr Glu
        35                  40                  45

ggg ctg tgg atg tcc tgc gtg tcg cag agc acc ggg cag atc cag tgc       192
Gly Leu Trp Met Ser Cys Val Ser Gln Ser Thr Gly Gln Ile Gln Cys
    50                  55                  60

aaa gtc ttt gac tcc ttg ctg aat ctg agc agc aca ttg caa gca acc       240
Lys Val Phe Asp Ser Leu Leu Asn Leu Ser Ser Thr Leu Gln Ala Thr
65                  70                  75                  80
```

```
cgt gcc ttg atg gtg gtt ggc atc ctc ctg gga gtg ata gca atc ttt      288
Arg Ala Leu Met Val Val Gly Ile Leu Leu Gly Val Ile Ala Ile Phe
                85                  90                  95

gtg gcc acc gtt ggc atg aag tgt atg aag tgc ttg gaa gac gat gag      336
Val Ala Thr Val Gly Met Lys Cys Met Lys Cys Leu Glu Asp Asp Glu
            100                 105                 110

gtg cag aag atg agg atg gct gtc att ggg ggc gcg ata ttt ctt ctc      384
Val Gln Lys Met Arg Met Ala Val Ile Gly Gly Ala Ile Phe Leu Leu
        115                 120                 125

gcc gcc ctg ctc acc ctc gtg ccg gtg tcc tgg tcg gcc aac acc att      432
Ala Ala Leu Leu Thr Leu Val Pro Val Ser Trp Ser Ala Asn Thr Ile
    130                 135                 140

atc cgg gac ttc tac aac ccc gtg gtg ccc gag gcg cag aag cgc gag      480
Ile Arg Asp Phe Tyr Asn Pro Val Val Pro Glu Ala Gln Lys Arg Glu
145                 150                 155                 160

atg ggc gcg ggc ctg tac gtg ggc tgg gcg gcc gcg gcg ctg cag ctg      528
Met Gly Ala Gly Leu Tyr Val Gly Trp Ala Ala Ala Ala Leu Gln Leu
                165                 170                 175

ctg ggg ggc gcg ctg ctc tgc tgc tcg tgt ccc cca cgc gag aag aag      576
Leu Gly Gly Ala Leu Leu Cys Cys Ser Cys Pro Pro Arg Glu Lys Lys
            180                 185                 190

tac acg gcc acc aag gtc gtc tac tcc gcg ccg cgc tcc acc ggc ccg      624
Tyr Thr Ala Thr Lys Val Val Tyr Ser Ala Pro Arg Ser Thr Gly Pro
        195                 200                 205

gga gcc agc ctg ggc aca ggc tac gac cgc aag gac tac gtc gct agc      672
Gly Ala Ser Leu Gly Thr Gly Tyr Asp Arg Lys Asp Tyr Val Ala Ser
    210                 215                 220

gat atc gcg gcc gct gac tac aaa gac gat gac gac aag tga              714
Asp Ile Ala Ala Ala Asp Tyr Lys Asp Asp Asp Asp Lys
225                 230                 235
```

```
<210>  161
<211>  237
<212>  PRT
<213>  Artificial

<220>
<221>  source
<223>  /note= "Description of artificial sequence: Synthetic
       Construct"

<400>  161

Met Ala Asn Ala Gly Leu Gln Leu Leu Gly Phe Ile Leu Ala Phe Leu
1               5                   10                  15

Gly Trp Ile Gly Ala Ile Val Ser Thr Ala Leu Pro Gln Trp Arg Ile
            20                  25                  30
```

```
Tyr Ser Tyr Ala Gly Asp Asn Ile Val Thr Ala Gln Ala Met Tyr Glu
        35                  40                  45

Gly Leu Trp Met Ser Cys Val Ser Gln Ser Thr Gly Gln Ile Gln Cys
    50                  55                  60

Lys Val Phe Asp Ser Leu Leu Asn Leu Ser Ser Thr Leu Gln Ala Thr
65                  70                  75                  80

Arg Ala Leu Met Val Val Gly Ile Leu Leu Gly Val Ile Ala Ile Phe
                85                  90                  95

Val Ala Thr Val Gly Met Lys Cys Met Lys Cys Leu Glu Asp Asp Glu
            100                 105                 110

Val Gln Lys Met Arg Met Ala Val Ile Gly Gly Ala Ile Phe Leu Leu
        115                 120                 125

Ala Ala Leu Leu Thr Leu Val Pro Val Ser Trp Ser Ala Asn Thr Ile
    130                 135                 140

Ile Arg Asp Phe Tyr Asn Pro Val Val Pro Glu Ala Gln Lys Arg Glu
145                 150                 155                 160

Met Gly Ala Gly Leu Tyr Val Gly Trp Ala Ala Ala Ala Leu Gln Leu
                165                 170                 175

Leu Gly Gly Ala Leu Leu Cys Cys Ser Cys Pro Pro Arg Glu Lys Lys
            180                 185                 190

Tyr Thr Ala Thr Lys Val Val Tyr Ser Ala Pro Arg Ser Thr Gly Pro
        195                 200                 205

Gly Ala Ser Leu Gly Thr Gly Tyr Asp Arg Lys Asp Tyr Val Ala Ser
    210                 215                 220

Asp Ile Ala Ala Ala Asp Tyr Lys Asp Asp Asp Asp Lys
225                 230                 235


<210>  162
<211>  675
<212>  DNA
<213>  Artificial

<220>
<221>  source
```

```
<223>  /note= "Description of artificial sequence: An artificially
       synthesized nucleotide sequence"

<220>
<221>  CDS
<222>  (1)..(675)

<400>  162
atg tcc atg ggc ctg gag atc acg ggc acc gcg ctg gcc gtg ctg ggc      48
Met Ser Met Gly Leu Glu Ile Thr Gly Thr Ala Leu Ala Val Leu Gly
1               5                   10                  15

tgg ctg ggc acc atc gtg tgc tgc gcg ttg ccc atg tgg cgc gtg tcg      96
Trp Leu Gly Thr Ile Val Cys Cys Ala Leu Pro Met Trp Arg Val Ser
            20                  25                  30

gcc ttc atc ggc agc aac atc atc acg tcg cag aac atc tgg gag ggc     144
Ala Phe Ile Gly Ser Asn Ile Ile Thr Ser Gln Asn Ile Trp Glu Gly
        35                  40                  45

ctg tgg atg aac tgc gtg gtg cag agc acc ggc cag atg cag tgc aag     192
Leu Trp Met Asn Cys Val Val Gln Ser Thr Gly Gln Met Gln Cys Lys
    50                  55                  60

gtg tac gac tcg ctg ctg gca ctg cca cag gac ctt cag gcg gcc cgc     240
Val Tyr Asp Ser Leu Leu Ala Leu Pro Gln Asp Leu Gln Ala Ala Arg
65                  70                  75                  80

gcc ctc atc gtg gtg gcc atc ctg ctg gcc gcc ttc ggg ctg cta gtg     288
Ala Leu Ile Val Val Ala Ile Leu Leu Ala Ala Phe Gly Leu Leu Val
                85                  90                  95

gcg ctg gtg ggc gcc cag tgc acc aac tgc gtg cag gac gac acg gcc     336
Ala Leu Val Gly Ala Gln Cys Thr Asn Cys Val Gln Asp Asp Thr Ala
            100                 105                 110

aag gcc aag atc acc atc gtg gca ggc gtg ctg ttc ctt ctt gca ggt     384
Lys Ala Lys Ile Thr Ile Val Ala Gly Val Leu Phe Leu Leu Ala Gly
        115                 120                 125

ctg gct att tta gtt gcc aca gca tgg tat ggc aat aga atc gtt caa     432
Leu Ala Ile Leu Val Ala Thr Ala Trp Tyr Gly Asn Arg Ile Val Gln
    130                 135                 140

gaa ttc tat gac cct atg acc cca gtc aat gcc agg tac gaa ttt ggt     480
Glu Phe Tyr Asp Pro Met Thr Pro Val Asn Ala Arg Tyr Glu Phe Gly
145                 150                 155                 160

cag gct ctc ttc act ggc tgg gct gct gct tct ctc tgc ctt ctg gga     528
Gln Ala Leu Phe Thr Gly Trp Ala Ala Ala Ser Leu Cys Leu Leu Gly
                165                 170                 175

ggt gcc cta ctt tgc tgt tcc tgt ccc cga aaa aca acc tct tac cca     576
Gly Ala Leu Leu Cys Cys Ser Cys Pro Arg Lys Thr Thr Ser Tyr Pro
            180                 185                 190

aca cca agg ccc tat cca aaa cct gca cct tcc agc ggg aaa gac tac     624
Thr Pro Arg Pro Tyr Pro Lys Pro Ala Pro Ser Ser Gly Lys Asp Tyr
        195                 200                 205
```

```
gtg gct agc gat atc gcg gcc gct gac tac aaa gac gat gac gac aag      672
Val Ala Ser Asp Ile Ala Ala Ala Asp Tyr Lys Asp Asp Asp Asp Lys
    210                 215                 220

tga                                                                  675


<210>  163
<211>  224
<212>  PRT
<213>  Artificial

<220>
<221>  source
<223>  /note= "Description of artificial sequence: Synthetic
       Construct"

<400>  163

Met Ser Met Gly Leu Glu Ile Thr Gly Thr Ala Leu Ala Val Leu Gly
1               5                   10                  15


Trp Leu Gly Thr Ile Val Cys Cys Ala Leu Pro Met Trp Arg Val Ser
            20                  25                  30


Ala Phe Ile Gly Ser Asn Ile Ile Thr Ser Gln Asn Ile Trp Glu Gly
        35                  40                  45


Leu Trp Met Asn Cys Val Val Gln Ser Thr Gly Gln Met Gln Cys Lys
    50                  55                  60


Val Tyr Asp Ser Leu Leu Ala Leu Pro Gln Asp Leu Gln Ala Ala Arg
65                  70                  75                  80


Ala Leu Ile Val Val Ala Ile Leu Leu Ala Ala Phe Gly Leu Leu Val
                85                  90                  95


Ala Leu Val Gly Ala Gln Cys Thr Asn Cys Val Gln Asp Asp Thr Ala
            100                 105                 110


Lys Ala Lys Ile Thr Ile Val Ala Gly Val Leu Phe Leu Leu Ala Gly
        115                 120                 125


Leu Ala Ile Leu Val Ala Thr Ala Trp Tyr Gly Asn Arg Ile Val Gln
    130                 135                 140


Glu Phe Tyr Asp Pro Met Thr Pro Val Asn Ala Arg Tyr Glu Phe Gly
145                 150                 155                 160
```

```
Gln Ala Leu Phe Thr Gly Trp Ala Ala Ala Ser Leu Cys Leu Leu Gly
                165                 170                 175

Gly Ala Leu Leu Cys Cys Ser Cys Pro Arg Lys Thr Thr Ser Tyr Pro
            180                 185                 190

Thr Pro Arg Pro Tyr Pro Lys Pro Ala Pro Ser Ser Gly Lys Asp Tyr
        195                 200                 205

Val Ala Ser Asp Ile Ala Ala Ala Asp Tyr Lys Asp Asp Asp Asp Lys
    210                 215                 220


<210>  164
<211>  351
<212>  DNA
<213>  Mus musculus


<220>
<221>  CDS
<222>  (1)..(351)

<400>  164
gag gtc cag ctg cag cag tct gga cct gag ctg gtg aag cct ggg gct       48
Glu Val Gln Leu Gln Gln Ser Gly Pro Glu Leu Val Lys Pro Gly Ala
1               5                   10                  15

tca gtg aag atg tcc tgc aag gct tct ggt tac tcc ttt act ggc tac       96
Ser Val Lys Met Ser Cys Lys Ala Ser Gly Tyr Ser Phe Thr Gly Tyr
            20                  25                  30

ttt atg aac tgg gtg aag cag agc cat gga aag agc ctt gag tgg att      144
Phe Met Asn Trp Val Lys Gln Ser His Gly Lys Ser Leu Glu Trp Ile
        35                  40                  45

gga cgt att aat cct tac aat ggt gat act ttc tac aac cag aag ttc      192
Gly Arg Ile Asn Pro Tyr Asn Gly Asp Thr Phe Tyr Asn Gln Lys Phe
    50                  55                  60

aag ggc aag gcc aca ttg act gta gac aaa tcc tct agc aca gcc cac      240
Lys Gly Lys Ala Thr Leu Thr Val Asp Lys Ser Ser Ser Thr Ala His
65                  70                  75                  80

atg gag ctc cgg agc ctg aca tct gag gac tct gca ctc tat tat tgt      288
Met Glu Leu Arg Ser Leu Thr Ser Glu Asp Ser Ala Leu Tyr Tyr Cys
                85                  90                  95

gca aga tct ggt aac tat gtt atg gac tac tgg ggt caa gga acc tca      336
Ala Arg Ser Gly Asn Tyr Val Met Asp Tyr Trp Gly Gln Gly Thr Ser
            100                 105                 110

gtc acc gtc tcc tca                                                  351
Val Thr Val Ser Ser
        115
```

<210> 165
<211> 117
<212> PRT
<213> Mus musculus

<400> 165

Glu Val Gln Leu Gln Gln Ser Gly Pro Glu Leu Val Lys Pro Gly Ala
1               5                   10                  15

Ser Val Lys Met Ser Cys Lys Ala Ser Gly Tyr Ser Phe Thr Gly Tyr
            20                  25                  30

Phe Met Asn Trp Val Lys Gln Ser His Gly Lys Ser Leu Glu Trp Ile
        35                  40                  45

Gly Arg Ile Asn Pro Tyr Asn Gly Asp Thr Phe Tyr Asn Gln Lys Phe
    50                  55                  60

Lys Gly Lys Ala Thr Leu Thr Val Asp Lys Ser Ser Ser Thr Ala His
65                  70                  75                  80

Met Glu Leu Arg Ser Leu Thr Ser Glu Asp Ser Ala Leu Tyr Tyr Cys
                85                  90                  95

Ala Arg Ser Gly Asn Tyr Val Met Asp Tyr Trp Gly Gln Gly Thr Ser
            100                 105                 110

Val Thr Val Ser Ser
        115

<210> 166
<211> 15
<212> DNA
<213> Mus musculus

<220>
<221> CDS
<222> (1)..(15)

<400> 166
ggc tac ttt atg aac 15
Gly Tyr Phe Met Asn
1               5

<210> 167
<211> 5
<212> PRT
<213> Mus musculus

<400> 167

Gly Tyr Phe Met Asn
1 5

<210> 168
<211> 51
<212> DNA
<213> Mus musculus

<220>
<221> CDS
<222> (1)..(51)

<400> 168
cgt att aat cct tac aat ggt gat act ttc tac aac cag aag ttc aag 48
Arg Ile Asn Pro Tyr Asn Gly Asp Thr Phe Tyr Asn Gln Lys Phe Lys
1 5 10 15

ggc 51
Gly

<210> 169
<211> 17
<212> PRT
<213> Mus musculus

<400> 169

Arg Ile Asn Pro Tyr Asn Gly Asp Thr Phe Tyr Asn Gln Lys Phe Lys
1 5 10 15

Gly

<210> 170
<211> 24
<212> DNA
<213> Mus musculus

<220>
<221> CDS
<222> (1)..(24)

<400> 170
tct ggt aac tat gtt atg gac tac 24
Ser Gly Asn Tyr Val Met Asp Tyr
1 5

<210> 171
<211> 8
<212> PRT

<213>  Mus musculus

<400>  171

```
Ser Gly Asn Tyr Val Met Asp Tyr
1               5
```

<210>  172
<211>  1401
<212>  DNA
<213>  Mus musculus

<220>
<221>  CDS
<222>  (1)..(1398)

<220>
<221>  sig_peptide
<222>  (1)..(57)

<220>
<221>  mat_peptide
<222>  (58)..(1398)

<400>  172

```
atg gga tgg agc tgg atc ttt ctc ttc ctt atg tca gga acg gca ggt       48
Met Gly Trp Ser Trp Ile Phe Leu Phe Leu Met Ser Gly Thr Ala Gly
                -15                 -10                 -5

gtc ctc tct gag gtc cag ctg cag cag tct gga cct gag ctg gtg aag       96
Val Leu Ser Glu Val Gln Leu Gln Gln Ser Gly Pro Glu Leu Val Lys
        -1  1               5                   10

cct ggg gct tca gtg aag atg tcc tgc aag gct tct ggt tac tcc ttt      144
Pro Gly Ala Ser Val Lys Met Ser Cys Lys Ala Ser Gly Tyr Ser Phe
    15                  20                  25

act ggc tac ttt atg aac tgg gtg aag cag agc cat gga aag agc ctt      192
Thr Gly Tyr Phe Met Asn Trp Val Lys Gln Ser His Gly Lys Ser Leu
30                  35                  40                  45

gag tgg att gga cgt att aat cct tac aat ggt gat act ttc tac aac      240
Glu Trp Ile Gly Arg Ile Asn Pro Tyr Asn Gly Asp Thr Phe Tyr Asn
                50                  55                  60

cag aag ttc aag ggc aag gcc aca ttg act gta gac aaa tcc tct agc      288
Gln Lys Phe Lys Gly Lys Ala Thr Leu Thr Val Asp Lys Ser Ser Ser
            65                  70                  75

aca gcc cac atg gag ctc cgg agc ctg aca tct gag gac tct gca ctc      336
Thr Ala His Met Glu Leu Arg Ser Leu Thr Ser Glu Asp Ser Ala Leu
        80                  85                  90

tat tat tgt gca aga tct ggt aac tat gtt atg gac tac tgg ggt caa      384
Tyr Tyr Cys Ala Arg Ser Gly Asn Tyr Val Met Asp Tyr Trp Gly Gln
    95                  100                 105

gga acc tca gtc acc gtc tcc tca gct aca aca aca gcc cca tct gtc      432
```

```
Gly Thr Ser Val Thr Val Ser Ser Ala Thr Thr Thr Ala Pro Ser Val
110                 115                 120                 125

tat ccc ttg gtc cct ggc tgc agt gac aca tct gga tcc tcg gtg aca      480
Tyr Pro Leu Val Pro Gly Cys Ser Asp Thr Ser Gly Ser Ser Val Thr
                130                 135                 140

ctg gga tgc ctt gtc aaa ggc tac ttc cct gag ccg gta act gta aaa      528
Leu Gly Cys Leu Val Lys Gly Tyr Phe Pro Glu Pro Val Thr Val Lys
            145                 150                 155

tgg aac tat gga gcc ctg tcc agc ggt gtg cgc aca gtc tca tct gtc      576
Trp Asn Tyr Gly Ala Leu Ser Ser Gly Val Arg Thr Val Ser Ser Val
        160                 165                 170

ctg cag tct ggg ttc tat tcc ctc agc agc ttg gtg act gta ccc tcc      624
Leu Gln Ser Gly Phe Tyr Ser Leu Ser Ser Leu Val Thr Val Pro Ser
    175                 180                 185

agc acc tgg ccc agc cag act gtc atc tgc aac gta gcc cac cca gcc      672
Ser Thr Trp Pro Ser Gln Thr Val Ile Cys Asn Val Ala His Pro Ala
190                 195                 200                 205

agc aag act gag ttg atc aag aga atc gag cct aga ata ccc aag ccc      720
Ser Lys Thr Glu Leu Ile Lys Arg Ile Glu Pro Arg Ile Pro Lys Pro
                210                 215                 220

agt acc ccc cca ggt tct tca tgc cca cct ggt aac atc ttg ggt gga      768
Ser Thr Pro Pro Gly Ser Ser Cys Pro Pro Gly Asn Ile Leu Gly Gly
            225                 230                 235

cca tcc gtc ttc atc ttc ccc cca aag ccc aag gat gca ctc atg atc      816
Pro Ser Val Phe Ile Phe Pro Pro Lys Pro Lys Asp Ala Leu Met Ile
        240                 245                 250

tcc cta acc ccc aag gtt acg tgt gtg gtg gtg gat gtg agc gag gat      864
Ser Leu Thr Pro Lys Val Thr Cys Val Val Val Asp Val Ser Glu Asp
    255                 260                 265

gac cca gat gtc cat gtc agc tgg ttt gtg gac aac aaa gaa gta cac      912
Asp Pro Asp Val His Val Ser Trp Phe Val Asp Asn Lys Glu Val His
270                 275                 280                 285

aca gcc tgg aca cag ccc cgt gaa gct cag tac aac agt acc ttc cga      960
Thr Ala Trp Thr Gln Pro Arg Glu Ala Gln Tyr Asn Ser Thr Phe Arg
                290                 295                 300

gtg gtc agt gcc ctc ccc atc cag cac cag gac tgg atg agg ggc aag     1008
Val Val Ser Ala Leu Pro Ile Gln His Gln Asp Trp Met Arg Gly Lys
            305                 310                 315

gag ttc aaa tgc aag gtc aac aac aaa gcc ctc cca gcc ccc atc gag     1056
Glu Phe Lys Cys Lys Val Asn Asn Lys Ala Leu Pro Ala Pro Ile Glu
        320                 325                 330

aga acc atc tca aaa ccc aaa gga aga gcc cag aca cct caa gta tac     1104
Arg Thr Ile Ser Lys Pro Lys Gly Arg Ala Gln Thr Pro Gln Val Tyr
    335                 340                 345

acc ata ccc cca cct cgt gaa caa atg tcc aag aag aag gtt agt ctg     1152
```

```
Thr Ile Pro Pro Pro Arg Glu Gln Met Ser Lys Lys Lys Val Ser Leu
350                 355                 360                 365

acc tgc ctg gtc acc aac ttc ttc tct gaa gcc atc agt gtg gag tgg      1200
Thr Cys Leu Val Thr Asn Phe Phe Ser Glu Ala Ile Ser Val Glu Trp
                370                 375                 380

gaa agg aac gga gaa ctg gag cag gat tac aag aac act cca ccc atc      1248
Glu Arg Asn Gly Glu Leu Glu Gln Asp Tyr Lys Asn Thr Pro Pro Ile
            385                 390                 395

ctg gac tca gat ggg acc tac ttc ctc tac agc aag ctc act gtg gat      1296
Leu Asp Ser Asp Gly Thr Tyr Phe Leu Tyr Ser Lys Leu Thr Val Asp
        400                 405                 410

aca gac agt tgg ttg caa gga gaa att ttt acc tgc tcc gtg gtg cat      1344
Thr Asp Ser Trp Leu Gln Gly Glu Ile Phe Thr Cys Ser Val Val His
    415                 420                 425

gag gct ctc cat aac cac cac aca cag aag aac ctg tct cgc tcc cct      1392
Glu Ala Leu His Asn His His Thr Gln Lys Asn Leu Ser Arg Ser Pro
430                 435                 440                 445

ggt aaa tga                                                          1401
Gly Lys


<210>  173
<211>  466
<212>  PRT
<213>  Mus musculus

<400>  173

Met Gly Trp Ser Trp Ile Phe Leu Phe Leu Met Ser Gly Thr Ala Gly
                -15                 -10                 -5

Val Leu Ser Glu Val Gln Leu Gln Gln Ser Gly Pro Glu Leu Val Lys
        -1  1               5                   10

Pro Gly Ala Ser Val Lys Met Ser Cys Lys Ala Ser Gly Tyr Ser Phe
    15                  20                  25

Thr Gly Tyr Phe Met Asn Trp Val Lys Gln Ser His Gly Lys Ser Leu
30                  35                  40                  45

Glu Trp Ile Gly Arg Ile Asn Pro Tyr Asn Gly Asp Thr Phe Tyr Asn
                50                  55                  60

Gln Lys Phe Lys Gly Lys Ala Thr Leu Thr Val Asp Lys Ser Ser Ser
            65                  70                  75

Thr Ala His Met Glu Leu Arg Ser Leu Thr Ser Glu Asp Ser Ala Leu
```

```
        80                  85                  90

Tyr Tyr Cys Ala Arg Ser Gly Asn Tyr Val Met Asp Tyr Trp Gly Gln
    95                  100                 105

Gly Thr Ser Val Thr Val Ser Ser Ala Thr Thr Thr Ala Pro Ser Val
110                 115                 120                 125

Tyr Pro Leu Val Pro Gly Cys Ser Asp Thr Ser Gly Ser Ser Val Thr
                130                 135                 140

Leu Gly Cys Leu Val Lys Gly Tyr Phe Pro Glu Pro Val Thr Val Lys
            145                 150                 155

Trp Asn Tyr Gly Ala Leu Ser Ser Gly Val Arg Thr Val Ser Ser Val
        160                 165                 170

Leu Gln Ser Gly Phe Tyr Ser Leu Ser Ser Leu Val Thr Val Pro Ser
    175                 180                 185

Ser Thr Trp Pro Ser Gln Thr Val Ile Cys Asn Val Ala His Pro Ala
190                 195                 200                 205

Ser Lys Thr Glu Leu Ile Lys Arg Ile Glu Pro Arg Ile Pro Lys Pro
                210                 215                 220

Ser Thr Pro Pro Gly Ser Ser Cys Pro Pro Gly Asn Ile Leu Gly Gly
            225                 230                 235

Pro Ser Val Phe Ile Phe Pro Pro Lys Pro Lys Asp Ala Leu Met Ile
        240                 245                 250

Ser Leu Thr Pro Lys Val Thr Cys Val Val Val Asp Val Ser Glu Asp
    255                 260                 265

Asp Pro Asp Val His Val Ser Trp Phe Val Asp Asn Lys Glu Val His
270                 275                 280                 285

Thr Ala Trp Thr Gln Pro Arg Glu Ala Gln Tyr Asn Ser Thr Phe Arg
                290                 295                 300

Val Val Ser Ala Leu Pro Ile Gln His Gln Asp Trp Met Arg Gly Lys
            305                 310                 315

Glu Phe Lys Cys Lys Val Asn Asn Lys Ala Leu Pro Ala Pro Ile Glu
```

```
        320                 325                 330

Arg Thr Ile Ser Lys Pro Lys Gly Arg Ala Gln Thr Pro Gln Val Tyr
    335                 340                 345

Thr Ile Pro Pro Pro Arg Glu Gln Met Ser Lys Lys Lys Val Ser Leu
350                 355                 360                 365

Thr Cys Leu Val Thr Asn Phe Phe Ser Glu Ala Ile Ser Val Glu Trp
                370                 375                 380

Glu Arg Asn Gly Glu Leu Glu Gln Asp Tyr Lys Asn Thr Pro Pro Ile
            385                 390                 395

Leu Asp Ser Asp Gly Thr Tyr Phe Leu Tyr Ser Lys Leu Thr Val Asp
        400                 405                 410

Thr Asp Ser Trp Leu Gln Gly Glu Ile Phe Thr Cys Ser Val Val His
    415                 420                 425

Glu Ala Leu His Asn His His Thr Gln Lys Asn Leu Ser Arg Ser Pro
430                 435                 440                 445

Gly Lys


<210>  174
<211>  1401
<212>  DNA
<213>  Artificial

<220>
<221>  source
<223>  /note= "Description of artificial sequence: An artificially
       synthesized nucleotide sequence"


<220>
<221>  CDS
<222>  (1)..(1398)

<220>
<221>  sig_peptide
<222>  (1)..(57)

<220>
<221>  mat_peptide
<222>  (58)..(1398)

<400>  174
atg gga tgg agc tgg atc ttt ctc ttc ctt atg tca gga acg gca ggt       48
```

```
Met Gly Trp Ser Trp Ile Phe Leu Phe Leu Met Ser Gly Thr Ala Gly
                -15                -10                 -5

gtc ctc tct gag gtc cag ctg cag cag tct gga cct gag ctg gtg aag      96
Val Leu Ser Glu Val Gln Leu Gln Gln Ser Gly Pro Glu Leu Val Lys
        -1  1               5                  10

cct ggg gct tca gtg aag atg tcc tgc aag gct tct ggt tac tcc ttt     144
Pro Gly Ala Ser Val Lys Met Ser Cys Lys Ala Ser Gly Tyr Ser Phe
    15                  20                  25

act ggc tac ttt atg aac tgg gtg aag cag agc cat gga aag agc ctt     192
Thr Gly Tyr Phe Met Asn Trp Val Lys Gln Ser His Gly Lys Ser Leu
30                  35                  40                  45

gag tgg att gga cgt att aat cct tac aat ggt gat act ttc tac aac     240
Glu Trp Ile Gly Arg Ile Asn Pro Tyr Asn Gly Asp Thr Phe Tyr Asn
                50                  55                  60

cag aag ttc aag ggc aag gcc aca ttg act gta gac aaa tcc tct agc     288
Gln Lys Phe Lys Gly Lys Ala Thr Leu Thr Val Asp Lys Ser Ser Ser
            65                  70                  75

aca gcc cac atg gag ctc cgg agc ctg aca tct gag gac tct gca ctc     336
Thr Ala His Met Glu Leu Arg Ser Leu Thr Ser Glu Asp Ser Ala Leu
        80                  85                  90

tat tat tgt gca aga tct ggt aac tat gtt atg gac tac tgg ggt caa     384
Tyr Tyr Cys Ala Arg Ser Gly Asn Tyr Val Met Asp Tyr Trp Gly Gln
    95                  100                 105

gga acc tca gtc acc gtc tcc tca gct agc acc aag ggc cca tcg gtc     432
Gly Thr Ser Val Thr Val Ser Ser Ala Ser Thr Lys Gly Pro Ser Val
110                 115                 120                 125

ttc ccc ctg gca ccc tcc tcc aag agc acc tct ggg ggc aca gcg gcc     480
Phe Pro Leu Ala Pro Ser Ser Lys Ser Thr Ser Gly Gly Thr Ala Ala
                130                 135                 140

ctg ggc tgc ctg gtc aag gac tac ttc ccc gaa ccg gtg acg gtg tcg     528
Leu Gly Cys Leu Val Lys Asp Tyr Phe Pro Glu Pro Val Thr Val Ser
            145                 150                 155

tgg aac tca ggc gcc ctg acc agc ggc gtg cac acc ttc ccg gct gtc     576
Trp Asn Ser Gly Ala Leu Thr Ser Gly Val His Thr Phe Pro Ala Val
        160                 165                 170

cta cag tcc tca gga ctc tac tcc ctc agc agc gtg gtg acc gtg ccc     624
Leu Gln Ser Ser Gly Leu Tyr Ser Leu Ser Ser Val Val Thr Val Pro
    175                 180                 185

tcc agc agc ttg ggc acc cag acc tac atc tgc aac gtg aat cac aag     672
Ser Ser Ser Leu Gly Thr Gln Thr Tyr Ile Cys Asn Val Asn His Lys
190                 195                 200                 205

ccc agc aac acc aag gtg gac aag aaa gtt gag ccc aaa tct tgt gac     720
Pro Ser Asn Thr Lys Val Asp Lys Lys Val Glu Pro Lys Ser Cys Asp
                210                 215                 220

aaa act cac aca tgc cca ccg tgc cca gca cct gaa ctc ctg ggg gga     768
```

```
Lys Thr His Thr Cys Pro Pro Cys Pro Ala Pro Glu Leu Leu Gly Gly
            225                 230                 235

ccg tca gtc ttc ctc ttc ccc cca aaa ccc aag gac acc ctc atg atc      816
Pro Ser Val Phe Leu Phe Pro Pro Lys Pro Lys Asp Thr Leu Met Ile
        240                 245                 250

tcc cgg acc cct gag gtc aca tgc gtg gtg gtg gac gtg agc cac gaa      864
Ser Arg Thr Pro Glu Val Thr Cys Val Val Val Asp Val Ser His Glu
    255                 260                 265

gac cct gag gtc aag ttc aac tgg tac gtg gac ggc gtg gag gtg cat      912
Asp Pro Glu Val Lys Phe Asn Trp Tyr Val Asp Gly Val Glu Val His
270                 275                 280                 285

aat gcc aag aca aag ccg cgg gag gag cag tac aac agc acg tac cgt      960
Asn Ala Lys Thr Lys Pro Arg Glu Glu Gln Tyr Asn Ser Thr Tyr Arg
                290                 295                 300

gtg gtc agc gtc ctc acc gtc ctg cac cag gac tgg ctg aat ggc aag     1008
Val Val Ser Val Leu Thr Val Leu His Gln Asp Trp Leu Asn Gly Lys
            305                 310                 315

gag tac aag tgc aag gtc tcc aac aaa gcc ctc cca gcc ccc atc gag     1056
Glu Tyr Lys Cys Lys Val Ser Asn Lys Ala Leu Pro Ala Pro Ile Glu
        320                 325                 330

aaa acc atc tcc aaa gcc aaa ggg cag ccc cga gaa cca cag gtg tac     1104
Lys Thr Ile Ser Lys Ala Lys Gly Gln Pro Arg Glu Pro Gln Val Tyr
    335                 340                 345

acc ctg ccc cca tcc cgg gat gag ctg acc aag aac cag gtc agc ctg     1152
Thr Leu Pro Pro Ser Arg Asp Glu Leu Thr Lys Asn Gln Val Ser Leu
350                 355                 360                 365

acc tgc ctg gtc aaa ggc ttc tat ccc agc gac atc gcc gtg gag tgg     1200
Thr Cys Leu Val Lys Gly Phe Tyr Pro Ser Asp Ile Ala Val Glu Trp
                370                 375                 380

gag agc aat ggg cag ccg gag aac aac tac aag acc acg cct ccc gtg     1248
Glu Ser Asn Gly Gln Pro Glu Asn Asn Tyr Lys Thr Thr Pro Pro Val
            385                 390                 395

ctg gac tcc gac ggc tcc ttc ttc ctc tac agc aag ctc acc gtg gac     1296
Leu Asp Ser Asp Gly Ser Phe Phe Leu Tyr Ser Lys Leu Thr Val Asp
        400                 405                 410

aag agc agg tgg cag cag ggg aac gtc ttc tca tgc tcc gtg atg cat     1344
Lys Ser Arg Trp Gln Gln Gly Asn Val Phe Ser Cys Ser Val Met His
    415                 420                 425

gag gct ctg cac aac cac tac acg cag aag agc ctc tcc ctg tct ccg     1392
Glu Ala Leu His Asn His Tyr Thr Gln Lys Ser Leu Ser Leu Ser Pro
430                 435                 440                 445

ggt aaa tga                                                         1401
Gly Lys
```

```
Lys Ser Arg Trp Gln Gln Gly Asn Val Phe Ser Cys Ser Val Met His
    415                 420                 425

Glu Ala Leu His Asn His Tyr Thr Gln Lys Ser Leu Ser Leu Ser Pro
430                 435                 440                 445

Gly Lys


<210>  176
<211>  336
<212>  DNA
<213>  Mus musculus


<220>
<221>  CDS
<222>  (1)..(336)

<400>  176
gac att gtg ctc acc caa tct cca gct tct ttg gct gtg tct cta ggg      48
Asp Ile Val Leu Thr Gln Ser Pro Ala Ser Leu Ala Val Ser Leu Gly
1               5                   10                  15

cag agt gtc acc atc tcc tgc aga gcc agt gaa agt gtt gaa tat tat      96
Gln Ser Val Thr Ile Ser Cys Arg Ala Ser Glu Ser Val Glu Tyr Tyr
            20                  25                  30

ggc act agt tta atg cag tgg tac caa cag aaa cca gga cag cca ccc     144
Gly Thr Ser Leu Met Gln Trp Tyr Gln Gln Lys Pro Gly Gln Pro Pro
        35                  40                  45

aaa ctc ctc atc tat ggt gca tcc aac gta gaa tct ggg gtc cct gcc     192
Lys Leu Leu Ile Tyr Gly Ala Ser Asn Val Glu Ser Gly Val Pro Ala
    50                  55                  60

agg ttt agt ggc agt ggg tct ggg aca gac ttc agc ctc aac atc cat     240
Arg Phe Ser Gly Ser Gly Ser Gly Thr Asp Phe Ser Leu Asn Ile His
65                  70                  75                  80

cct gtg gag gag gat gat att gca atg tat ttc tgt cag caa agt agg     288
Pro Val Glu Glu Asp Asp Ile Ala Met Tyr Phe Cys Gln Gln Ser Arg
                85                  90                  95

aag gtt ccg tgg acg ttc ggt gga ggc acc aag ctg gaa atc aaa cgg     336
Lys Val Pro Trp Thr Phe Gly Gly Gly Thr Lys Leu Glu Ile Lys Arg
            100                 105                 110


<210>  177
<211>  112
<212>  PRT
<213>  Mus musculus

<400>  177
```

```
Leu Gln Ser Ser Gly Leu Tyr Ser Leu Ser Ser Val Val Thr Val Pro
    175                 180                 185

Ser Ser Ser Leu Gly Thr Gln Thr Tyr Ile Cys Asn Val Asn His Lys
190                 195                 200                 205

Pro Ser Asn Thr Lys Val Asp Lys Lys Val Glu Pro Lys Ser Cys Asp
                210                 215                 220

Lys Thr His Thr Cys Pro Pro Cys Pro Ala Pro Glu Leu Leu Gly Gly
            225                 230                 235

Pro Ser Val Phe Leu Phe Pro Pro Lys Pro Lys Asp Thr Leu Met Ile
        240                 245                 250

Ser Arg Thr Pro Glu Val Thr Cys Val Val Val Asp Val Ser His Glu
    255                 260                 265

Asp Pro Glu Val Lys Phe Asn Trp Tyr Val Asp Gly Val Glu Val His
270                 275                 280                 285

Asn Ala Lys Thr Lys Pro Arg Glu Glu Gln Tyr Asn Ser Thr Tyr Arg
                290                 295                 300

Val Val Ser Val Leu Thr Val Leu His Gln Asp Trp Leu Asn Gly Lys
            305                 310                 315

Glu Tyr Lys Cys Lys Val Ser Asn Lys Ala Leu Pro Ala Pro Ile Glu
        320                 325                 330

Lys Thr Ile Ser Lys Ala Lys Gly Gln Pro Arg Glu Pro Gln Val Tyr
    335                 340                 345

Thr Leu Pro Pro Ser Arg Asp Glu Leu Thr Lys Asn Gln Val Ser Leu
350                 355                 360                 365

Thr Cys Leu Val Lys Gly Phe Tyr Pro Ser Asp Ile Ala Val Glu Trp
                370                 375                 380

Glu Ser Asn Gly Gln Pro Glu Asn Asn Tyr Lys Thr Thr Pro Pro Val
            385                 390                 395

Leu Asp Ser Asp Gly Ser Phe Phe Leu Tyr Ser Lys Leu Thr Val Asp
        400                 405                 410
```

```
Lys Ser Arg Trp Gln Gln Gly Asn Val Phe Ser Cys Ser Val Met His
    415                 420                 425

Glu Ala Leu His Asn His Tyr Thr Gln Lys Ser Leu Ser Leu Ser Pro
430                 435                 440                 445

Gly Lys


<210>  176
<211>  336
<212>  DNA
<213>  Mus musculus


<220>
<221>  CDS
<222>  (1)..(336)

<400>  176
gac att gtg ctc acc caa tct cca gct tct ttg gct gtg tct cta ggg       48
Asp Ile Val Leu Thr Gln Ser Pro Ala Ser Leu Ala Val Ser Leu Gly
1               5                   10                  15

cag agt gtc acc atc tcc tgc aga gcc agt gaa agt gtt gaa tat tat       96
Gln Ser Val Thr Ile Ser Cys Arg Ala Ser Glu Ser Val Glu Tyr Tyr
            20                  25                  30

ggc act agt tta atg cag tgg tac caa cag aaa cca gga cag cca ccc      144
Gly Thr Ser Leu Met Gln Trp Tyr Gln Gln Lys Pro Gly Gln Pro Pro
        35                  40                  45

aaa ctc ctc atc tat ggt gca tcc aac gta gaa tct ggg gtc cct gcc      192
Lys Leu Leu Ile Tyr Gly Ala Ser Asn Val Glu Ser Gly Val Pro Ala
    50                  55                  60

agg ttt agt ggc agt ggg tct ggg aca gac ttc agc ctc aac atc cat      240
Arg Phe Ser Gly Ser Gly Ser Gly Thr Asp Phe Ser Leu Asn Ile His
65                  70                  75                  80

cct gtg gag gag gat gat att gca atg tat ttc tgt cag caa agt agg      288
Pro Val Glu Glu Asp Asp Ile Ala Met Tyr Phe Cys Gln Gln Ser Arg
                85                  90                  95

aag gtt ccg tgg acg ttc ggt gga ggc acc aag ctg gaa atc aaa cgg      336
Lys Val Pro Trp Thr Phe Gly Gly Gly Thr Lys Leu Glu Ile Lys Arg
            100                 105                 110


<210>  177
<211>  112
<212>  PRT
<213>  Mus musculus

<400>  177

Asp Ile Val Leu Thr Gln Ser Pro Ala Ser Leu Ala Val Ser Leu Gly
```

```
1               5                   10                  15

Gln Ser Val Thr Ile Ser Cys Arg Ala Ser Glu Ser Val Glu Tyr Tyr
            20                  25                  30

Gly Thr Ser Leu Met Gln Trp Tyr Gln Gln Lys Pro Gly Gln Pro Pro
        35                  40                  45

Lys Leu Leu Ile Tyr Gly Ala Ser Asn Val Glu Ser Gly Val Pro Ala
    50                  55                  60

Arg Phe Ser Gly Ser Gly Ser Gly Thr Asp Phe Ser Leu Asn Ile His
65                  70                  75                  80

Pro Val Glu Glu Asp Asp Ile Ala Met Tyr Phe Cys Gln Gln Ser Arg
                85                  90                  95

Lys Val Pro Trp Thr Phe Gly Gly Gly Thr Lys Leu Glu Ile Lys Arg
            100                 105                 110


<210>  178
<211>  45
<212>  DNA
<213>  Mus musculus


<220>
<221>  CDS
<222>  (1)..(45)

<400>  178
aga gcc agt gaa agt gtt gaa tat tat ggc act agt tta atg cag           45
Arg Ala Ser Glu Ser Val Glu Tyr Tyr Gly Thr Ser Leu Met Gln
1               5                   10                  15


<210>  179
<211>  15
<212>  PRT
<213>  Mus musculus

<400>  179

Arg Ala Ser Glu Ser Val Glu Tyr Tyr Gly Thr Ser Leu Met Gln
1               5                   10                  15


<210>  180
<211>  21
<212>  DNA
<213>  Mus musculus
```

<220>
<221> CDS
<222> (1)..(21)

<400> 180
ggt gca tcc aac gta gaa tct 21
Gly Ala Ser Asn Val Glu Ser
1 5

<210> 181
<211> 7
<212> PRT
<213> Mus musculus

<400> 181

Gly Ala Ser Asn Val Glu Ser
1 5

<210> 182
<211> 27
<212> DNA
<213> Mus musculus

<220>
<221> CDS
<222> (1)..(27)

<400> 182
cag caa agt agg aag gtt ccg tgg acg 27
Gln Gln Ser Arg Lys Val Pro Trp Thr
1 5

<210> 183
<211> 9
<212> PRT
<213> Mus musculus

<400> 183

Gln Gln Ser Arg Lys Val Pro Trp Thr
1 5

<210> 184
<211> 717
<212> DNA
<213> Mus musculus

<220>
<221> CDS
<222> (1)..(714)

<220>
<221> sig_peptide

```
<222>  (1)..(60)

<220>
<221>  mat_peptide
<222>  (61)..(714)

<400>  184
atg gag aca gac aca ctc ctg cta tgg gtg ctg ctg ctc tgg gtt cca      48
Met Glu Thr Asp Thr Leu Leu Leu Trp Val Leu Leu Leu Trp Val Pro
-20                 -15                 -10                 -5

ggc tcc act ggt gac att gtg ctc acc caa tct cca gct tct ttg gct      96
Gly Ser Thr Gly Asp Ile Val Leu Thr Gln Ser Pro Ala Ser Leu Ala
            -1  1               5                   10

gtg tct cta ggg cag agt gtc acc atc tcc tgc aga gcc agt gaa agt     144
Val Ser Leu Gly Gln Ser Val Thr Ile Ser Cys Arg Ala Ser Glu Ser
        15                  20                  25

gtt gaa tat tat ggc act agt tta atg cag tgg tac caa cag aaa cca     192
Val Glu Tyr Tyr Gly Thr Ser Leu Met Gln Trp Tyr Gln Gln Lys Pro
    30                  35                  40

gga cag cca ccc aaa ctc ctc atc tat ggt gca tcc aac gta gaa tct     240
Gly Gln Pro Pro Lys Leu Leu Ile Tyr Gly Ala Ser Asn Val Glu Ser
45                  50                  55                  60

ggg gtc cct gcc agg ttt agt ggc agt ggg tct ggg aca gac ttc agc     288
Gly Val Pro Ala Arg Phe Ser Gly Ser Gly Ser Gly Thr Asp Phe Ser
                65                  70                  75

ctc aac atc cat cct gtg gag gag gat gat att gca atg tat ttc tgt     336
Leu Asn Ile His Pro Val Glu Glu Asp Asp Ile Ala Met Tyr Phe Cys
            80                  85                  90

cag caa agt agg aag gtt ccg tgg acg ttc ggt gga ggc acc aag ctg     384
Gln Gln Ser Arg Lys Val Pro Trp Thr Phe Gly Gly Gly Thr Lys Leu
        95                  100                 105

gaa atc aaa cgg gct gat gct gca cca act gta tcc atc ttc cca cca     432
Glu Ile Lys Arg Ala Asp Ala Ala Pro Thr Val Ser Ile Phe Pro Pro
    110                 115                 120

tcc agt gag cag tta aca tct gga ggt gcc tca gtc gtg tgc ttc ttg     480
Ser Ser Glu Gln Leu Thr Ser Gly Gly Ala Ser Val Val Cys Phe Leu
125                 130                 135                 140

aac aac ttc tac ccc aaa gac atc aat gtc aag tgg aag att gat ggc     528
Asn Asn Phe Tyr Pro Lys Asp Ile Asn Val Lys Trp Lys Ile Asp Gly
                145                 150                 155

agt gaa cga caa aat ggc gtc ctg aac agt tgg act gat cag gac agc     576
Ser Glu Arg Gln Asn Gly Val Leu Asn Ser Trp Thr Asp Gln Asp Ser
            160                 165                 170

aaa gac agc acc tac agc atg agc agc acc ctc acg ttg acc aag gac     624
Lys Asp Ser Thr Tyr Ser Met Ser Ser Thr Leu Thr Leu Thr Lys Asp
        175                 180                 185

gag tat gaa cga cat aac agc tat acc tgt gag gcc act cac aag aca     672
```

```
Glu Tyr Glu Arg His Asn Ser Tyr Thr Cys Glu Ala Thr His Lys Thr
    190                 195                 200

tca act tca ccc att gtc aag agc ttc aac agg aat gag tgt tag          717
Ser Thr Ser Pro Ile Val Lys Ser Phe Asn Arg Asn Glu Cys
205                 210                 215


<210>  185
<211>  238
<212>  PRT
<213>  Mus musculus

<400>  185

Met Glu Thr Asp Thr Leu Leu Leu Trp Val Leu Leu Leu Trp Val Pro
-20                 -15                 -10                 -5

Gly Ser Thr Gly Asp Ile Val Leu Thr Gln Ser Pro Ala Ser Leu Ala
            -1  1               5                   10

Val Ser Leu Gly Gln Ser Val Thr Ile Ser Cys Arg Ala Ser Glu Ser
        15                  20                  25

Val Glu Tyr Tyr Gly Thr Ser Leu Met Gln Trp Tyr Gln Gln Lys Pro
    30                  35                  40

Gly Gln Pro Pro Lys Leu Leu Ile Tyr Gly Ala Ser Asn Val Glu Ser
45                  50                  55                  60

Gly Val Pro Ala Arg Phe Ser Gly Ser Gly Ser Gly Thr Asp Phe Ser
                65                  70                  75

Leu Asn Ile His Pro Val Glu Glu Asp Asp Ile Ala Met Tyr Phe Cys
            80                  85                  90

Gln Gln Ser Arg Lys Val Pro Trp Thr Phe Gly Gly Gly Thr Lys Leu
        95                  100                 105

Glu Ile Lys Arg Ala Asp Ala Ala Pro Thr Val Ser Ile Phe Pro Pro
    110                 115                 120

Ser Ser Glu Gln Leu Thr Ser Gly Gly Ala Ser Val Val Cys Phe Leu
125                 130                 135                 140

Asn Asn Phe Tyr Pro Lys Asp Ile Asn Val Lys Trp Lys Ile Asp Gly
                145                 150                 155

Ser Glu Arg Gln Asn Gly Val Leu Asn Ser Trp Thr Asp Gln Asp Ser
```

```
            160                 165                 170

Lys Asp Ser Thr Tyr Ser Met Ser Ser Thr Leu Thr Leu Thr Lys Asp
        175                 180                 185

Glu Tyr Glu Arg His Asn Ser Tyr Thr Cys Glu Ala Thr His Lys Thr
    190                 195                 200

Ser Thr Ser Pro Ile Val Lys Ser Phe Asn Arg Asn Glu Cys
205                 210                 215


<210>  186
<211>  717
<212>  DNA
<213>  Artificial

<220>
<221>  source
<223>  /note= "Description of artificial sequence: An artificially
       synthesized nucleotide sequence"


<220>
<221>  CDS
<222>  (1)..(714)

<220>
<221>  sig_peptide
<222>  (1)..(60)

<220>
<221>  mat_peptide
<222>  (61)..(714)

<400>  186
atg gag aca gac aca ctc ctg cta tgg gtg ctg ctg ctc tgg gtt cca       48
Met Glu Thr Asp Thr Leu Leu Leu Trp Val Leu Leu Leu Trp Val Pro
-20                 -15                 -10                 -5

ggc tcc act ggt gac att gtg ctc acc caa tct cca gct tct ttg gct       96
Gly Ser Thr Gly Asp Ile Val Leu Thr Gln Ser Pro Ala Ser Leu Ala
            -1  1               5                   10

gtg tct cta ggg cag agt gtc acc atc tcc tgc aga gcc agt gaa agt      144
Val Ser Leu Gly Gln Ser Val Thr Ile Ser Cys Arg Ala Ser Glu Ser
        15                  20                  25

gtt gaa tat tat ggc act agt tta atg cag tgg tac caa cag aaa cca      192
Val Glu Tyr Tyr Gly Thr Ser Leu Met Gln Trp Tyr Gln Gln Lys Pro
    30                  35                  40

gga cag cca ccc aaa ctc ctc atc tat ggt gca tcc aac gta gaa tct      240
Gly Gln Pro Pro Lys Leu Leu Ile Tyr Gly Ala Ser Asn Val Glu Ser
45                  50                  55                  60

ggg gtc cct gcc agg ttt agt ggc agt ggg tct ggg aca gac ttc agc      288
```

```
Gly Val Pro Ala Arg Phe Ser Gly Ser Gly Ser Gly Thr Asp Phe Ser
                65                  70                  75

ctc aac atc cat cct gtg gag gag gat gat att gca atg tat ttc tgt      336
Leu Asn Ile His Pro Val Glu Glu Asp Asp Ile Ala Met Tyr Phe Cys
            80                  85                  90

cag caa agt agg aag gtt ccg tgg acg ttc ggt gga ggc acc aag ctg      384
Gln Gln Ser Arg Lys Val Pro Trp Thr Phe Gly Gly Gly Thr Lys Leu
        95                  100                 105

gaa atc aaa cgt acg gtg gct gca cca tct gtc ttc atc ttc ccg cca      432
Glu Ile Lys Arg Thr Val Ala Ala Pro Ser Val Phe Ile Phe Pro Pro
    110                 115                 120

tct gat gag cag ttg aaa tct gga act gcc tct gtt gtg tgc ctg ctg      480
Ser Asp Glu Gln Leu Lys Ser Gly Thr Ala Ser Val Val Cys Leu Leu
125                 130                 135                 140

aat aac ttc tat ccc aga gag gcc aaa gta cag tgg aag gtg gat aac      528
Asn Asn Phe Tyr Pro Arg Glu Ala Lys Val Gln Trp Lys Val Asp Asn
                145                 150                 155

gcc ctc caa tcg ggt aac tcc cag gag agt gtc aca gag cag gac agc      576
Ala Leu Gln Ser Gly Asn Ser Gln Glu Ser Val Thr Glu Gln Asp Ser
            160                 165                 170

aag gac agc acc tac agc ctc agc agc acc ctg acg ctg agc aaa gca      624
Lys Asp Ser Thr Tyr Ser Leu Ser Ser Thr Leu Thr Leu Ser Lys Ala
        175                 180                 185

gac tac gag aaa cac aaa gtc tac gcc tgc gaa gtc acc cat cag ggc      672
Asp Tyr Glu Lys His Lys Val Tyr Ala Cys Glu Val Thr His Gln Gly
    190                 195                 200

ctg agc tcg ccc gtc aca aag agc ttc aac agg gga gag tgt tga          717
Leu Ser Ser Pro Val Thr Lys Ser Phe Asn Arg Gly Glu Cys
205                 210                 215


<210>  187
<211>  238
<212>  PRT
<213>  Artificial

<220>
<221>  source
<223>  /note= "Description of artificial sequence: Synthetic
       Construct"

<400>  187

Met Glu Thr Asp Thr Leu Leu Leu Trp Val Leu Leu Leu Trp Val Pro
-20                 -15                 -10                 -5

Gly Ser Thr Gly Asp Ile Val Leu Thr Gln Ser Pro Ala Ser Leu Ala
            -1  1               5                   10
```

```
Val Ser Leu Gly Gln Ser Val Thr Ile Ser Cys Arg Ala Ser Glu Ser
        15                  20                  25

Val Glu Tyr Tyr Gly Thr Ser Leu Met Gln Trp Tyr Gln Gln Lys Pro
    30                  35                  40

Gly Gln Pro Pro Lys Leu Leu Ile Tyr Gly Ala Ser Asn Val Glu Ser
45                  50                  55                  60

Gly Val Pro Ala Arg Phe Ser Gly Ser Gly Ser Gly Thr Asp Phe Ser
                65                  70                  75

Leu Asn Ile His Pro Val Glu Glu Asp Asp Ile Ala Met Tyr Phe Cys
            80                  85                  90

Gln Gln Ser Arg Lys Val Pro Trp Thr Phe Gly Gly Gly Thr Lys Leu
        95                  100                 105

Glu Ile Lys Arg Thr Val Ala Ala Pro Ser Val Phe Ile Phe Pro Pro
    110                 115                 120

Ser Asp Glu Gln Leu Lys Ser Gly Thr Ala Ser Val Val Cys Leu Leu
125                 130                 135                 140

Asn Asn Phe Tyr Pro Arg Glu Ala Lys Val Gln Trp Lys Val Asp Asn
                145                 150                 155

Ala Leu Gln Ser Gly Asn Ser Gln Glu Ser Val Thr Glu Gln Asp Ser
            160                 165                 170

Lys Asp Ser Thr Tyr Ser Leu Ser Ser Thr Leu Thr Leu Ser Lys Ala
        175                 180                 185

Asp Tyr Glu Lys His Lys Val Tyr Ala Cys Glu Val Thr His Gln Gly
    190                 195                 200

Leu Ser Ser Pro Val Thr Lys Ser Phe Asn Arg Gly Glu Cys
205                 210                 215


<210>  188
<211>  25
<212>  DNA
<213>  Artificial

<220>
<221>  source
<223>  /note= "Description of artificial sequence: An artificially
```

```
        synthesized primer sequence"

<400>  188
atgtgtcact gcagccaggg accaa                                         25
```